# EUROPEAN PATENT APPLICATION

(11) **EP 1 477 490 A1**
(43) Date of publication of application: **17.11.2004**
(21) Application number: 03707009.1
(22) Date of filing: 24.02.2003
(51) Int. Cl.: C07D 487/14, C07D 487/04, C07D 491/22, A61K 31/551, A61P 3/00, A61P 3/04, A61P 3/10, A61P 9/10, A61P 9/12, A61P 15/00, A61P 17/14, A61P 25/00, A61P 25/14, A61P 25/16, A61P 25/18, A61P 25/24, A61P 25/28, A61P 29/00, A61P 35/00, A61P 37/02, A61P 43/00

(54) **PYRROLOPYRIMIDINE DERIVATIVE**

(30) Priority: 22.02.2002 JP 2002046129
(71) Applicant: TEIJIN LIMITED, Osaka-shi, Osaka 541-0054 (JP)
(72) Inventor: KATAOKA, Kenichiro, c/o Teijin Limited, Hino-shi, Tokyo 191-0065 (JP); KOSUGI, Tomomi, c/o Teijin Limited, Hino-shi, Tokyo 191-0065 (JP); ISHII, Toshihiro, c/o Teijin Limited, Hino-shi, Tokyo 191-0065 (JP); TAKEUCHI, Takahiro, c/o Teijin Limited, Hino-shi, Tokyo 191-0065 (JP); TSUTSUMI, Takaharu, c/o Teijin Limited, Hino-shi, Tokyo 191-0065 (JP); NAKANO, Akira, c/o Teijin Limited, Hino-shi, Tokyo 191-0065 (JP); YAMAMOTO, Yoji, c/o Teijin Limited, Hino-shi, Tokyo 191-0065 (JP); YOSHIOKA, Noboru, c/o Teijin Limited, Hino-shi, Tokyo 191-0065 (JP)
(74) Representative: Hallybone, Huw George
(86) International application number: PCT/JP2003/001978
(87) International publication number: WO 2003/070730

(57) **Abstract**

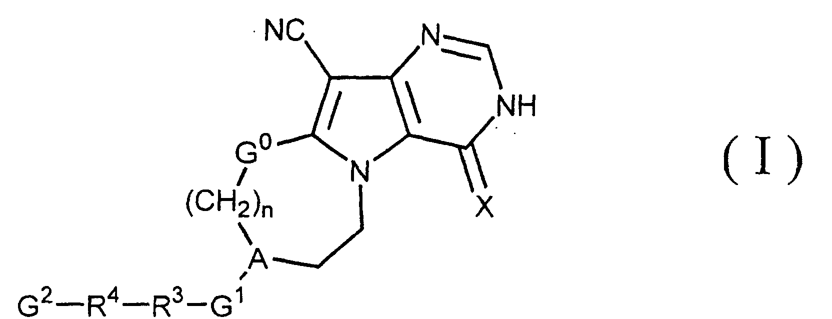

A pyrrolo[3,2-d]pyrimidine derivative represented by the formula (I) or a pharamaceutically acceptable salt of the derivative. The derivative or salt is useful as a GSK-3 inhibitor.

SEQUENCE LISTING

<110> TEIJIN LIMITED
      KATAOKA Kenichiro
      KOSUGI Tomomi
      ISHII Toshihiro
      TAKEUCHI Takahiro
      TSUTSUMI Takaharu
      NAKANO Akira
      YAMAMOTO Yoji
      YOSHIOKA Noboru

<120> Pyrrolopyrimidine derivatives

<130> M 0302

<150> JP P2002-046129
<151> 2002-2-22

<160> 1

<210> 1
<211> 26
<212> PRT
<213> Artificial Sequence

<220>
<223> Inventor: KATAOKA, Kenichiro; KOSUGI, Tomomi; ISHII,
Toshihiro; TAKEUCHI, Takahiro
      Inventor: TSUTSUMI, Takaharu; NAKANO, Akira; YAMAMOTO,
Yoji; YOSHIOKA, Noboru

<220>
<221> PHOSPHORYLATION
<222> 21
<223> O-LINKED

<220>
<223>  phospho-glycogen synthase peptide-2

<400> 1
Tyr Arg Arg Ala Ala Val Pro Pro Ser Pro Ser Leu Ser Arg His Ser
 1               5                  10                  15
Ser Pro His Gln Ser Glu Asp Glu Glu Glu
            20                  25

## Description

### TECHNICAL FIELD

The present invention relates to novel pyrrolopyrimidine derivatives for use as pharmaceutical agents having an activity of inhibiting glycogen synthase kinase-3 (GSK-3). More specifically, the present invention relates to novel pyrrolo[3,2-d]pyrimidine derivatives useful for use as pharmaceutical agents for treating and/or preventing diseases for which GSK-3 activity has been implicated as a causative agent, specifically impaired glucose tolerance, type 1 diabetes, type 2 diabetes, diabetic complications (retinopathy, nephropathy, neurotic disorders, macroangiopathy etc.), Alzheimer's disease, neurodegenerative diseases (AIDS encephalopathy, Huntington's disease, Parkinson's disease, amyotrophic lateral sclerosis, multiple sclerosis etc.), bipolar affective disorder (manic-depressive psychosis), traumatic encephalopathy and spinal injury, epilepsy, obesity, atherosclerosis, hypertension, polycystic ovary syndrome, Syndrome X, alopecia, inflammatory diseases (osteoarthritis, rheumatoid arthritis, atopic dermatitis, psoriasis, ulcerative colitis, Crohn's disease, sepsis, generalized inflammatory syndrome etc.), cancer, immune deficiency and the like.

### BACKGROUND ART

GSK-3 is a serine/threonine kinase, for which two types of isoforms (α type and β type, encoded by separate genes) have been identified (see Non-patent document 1). Either of GSK-3 isoforms assumes a monomer structure, and have been constantly activated in resting cells. originally GSK-3 was identified as a kinase that inhibits glycogen synthase kinase by directly phosphorylating the enzyme (see Non-patent document 2). Under insulin stimulation, it is believed, GSK-3 is inactivated which leads to the activation of glycogen synthase kinase and furthermore to the induction of insulin effect such as sugar transport. It is known that GSK-3 is also inactivated by other growth factors such as IGF-1 and FGF via signals from the receptor tyrosine kinase (see Non-patent document 3, Non-patent document 4, and Non-patent document 5).

GSK-3 inhibitors are useful for the treatment of various diseases for which GSK-3 activation is responsible. Furthermore, since the inhibition of GSK-3 simulates the activation of signaling pathway of growth factors, it is also useful for the treatment of diseases for which the inactivation of their signaling pathway is responsible. Various diseases for which GSK-3 inhibitors are thought to be useful are illustrated below.

Type 1 diabetes is caused by the autoimmune destruction of the insulin-producing cells, β-cells, in the pancreas leading to insulin deficiency. Therefore, in order to maintain life of patients with type 1 diabetes, the routine administration of insulin is imperative. The current insulin therapy, however, cannot reproduce the strict control of blood sugar levels which is attained by normal β-cells. Thus, type 1 diabetes tends to induce diabetic complications with retinopathy, nephropathy, neurotic disorders, macroangiopathy or the like.

Type 2 diabetes a multifactorial disease in which insulin resistance in the liver, skeletal muscles, and adipose tissues combined with deficient secretion of insulin from the pancreas causes high blood sugar. As a result, diabetic complications with retinopathy, nephropathy, neurotic disorders, macroangiopathy and the like are induced. Skeletal muscles are an important tissue in glucose incorporation by insulin stimulation, and the incorporated glucose is metabolized by either of the glycolysis/TCA cycle or glycogen accumulation. Glycogen accumulation in the skeletal muscles plays a very important role in glucose homeostasis, and in patients with type 2 diabetes the amount of glycogen accumulated in the skeletal muscles is decreased. GSK-3 is acting in the direction of increased blood glucose by phosphorylating glycogen synthase kinase thereby inhibiting the glycogen accumulation in the peripheral tissues and by lowering insulin reactivity.

Recently, it was reported that the expression of GSK-3 is enhanced in skeletal muscles of patients with type 2 diabetes and that an inverse correlation can be observed between GSK-3α activity in skeletal muscles and insulin effect (see Non-patent document 6). The excessive expression of GSK-3β and the activated GSK-3β mutants (S9A, S9E) in the HER-293 cells leads to the inhibition of glycogen synthase kinase activity (see Non-patent document 7). The excessive expression of GSK-3β in the CHO cells in which insulin receptors and insulin receptor substrate 1 (IRS-1) have been expressed leads to the decline of insulin effect (see Non-patent document 8). Recently, a study using C57BL/6J mice that show tendency of obese diabetic revealed a relationship between enhanced GSK-3 activity and the progress of insulin resistant/type 2 diabetes (see Non-patent document 9).

Conventionally, lithium salts have been used as pharmaceutical agents that inhibit GSK-3 activity (see Non-patent document 10). It has been reported that treatment with a lithium salt reduces blood sugar levels and ameliorates pathological conditions in either of type 1 diabetic and type 2 diabetic patients (see Non-patent document 11). However, it has been reported that lithium salts have a variety of effects on molecular targets other than GSK-3.

From the foregoing, it is thought that GSK-3 inhibitors can serve as effective pharmaceutical agents for ameliorating impaired glucose tolerance, type 1 diabetes, type 2 diabetes or complications thereof.

It has also been suggested that GSK-3 is involved in the progress of pathological conditions of Alzheimer's disease. Alzheimer's disease is characterized by the formation of senile plaques due to the deposition of amyloid β peptide (Aβ) in the brain and the ensuing formation of neurofibrillary changes. These changes lead to massive death of nerve cells leading to the appearance of dementia conditions. In this progress of pathological conditions, GSK-3 is believed to be involved in abnormal phosphorylation of tau protein which leads to neurofibrillary changes (see Non-patent document 12). There is also a report that GSK-3 inhibitors may prevent the death of nerve cells (see Non-patent document 13). Based on these findings, it is believed that the application of GSK-3 inhibitors to Alzheimer's disease can delay the progress of the pathological conditions. At present, as therapeutic agents for Alzheimer's disease, agents that perform symptomatic treatments are present (see Non-patent document 14) but no pharmaceutical agents are present that prevent the death of nerve cells and delay the progress of the pathological conditions. From the foregoing, GSK-3 inhibitors are considered to become pharmaceutical agents effective for ameliorating Alzheimer's dementia.

There is a report that GSK-3 inhibitors prevent the death of nerve cells, specifically the death of nerve cells due to hyperexcitation via glutamic acid (see Non-patent document 15 and Non-patent document 16). This suggests a possibility that GSK-3 inhibitors may be effective for the treatment of bipolar affective disorder (manic-depressive psychosis), epilepsy and many degenerative brain diseases and neurotic diseases. In addition to the above-mentioned Alzheimer's disease, neurodegenerative diseases include AIDS encephalopathy, Huntington's disease, Parkinson's disease, amyotrophic lateral sclerosis, multiple sclerosis, Pick's disease, progressive supranuclear palsy and the like. Also, the hyperexcitation via glutamic acid is considered to be a factor in brain disorders in stroke (cerebral infarction, cerebral hemorrhage, subarachnoid hemorrhage), traumatic encephalopathy and spinal injury, bacterial and virus infections and the like, and GSK-3 inhibitors are expected to be effective for these diseases. All of them are diseases accompanied by the death of nerve cells. At present, there are no pharmaceutical agents that effectively prevent the death of nerve cells, From the foregoing, it is thought that GSK-3 inhibitors may be pharmaceutical agents effective for the amelioration of neurodegenerative diseases, bipolar affective disorder (manic-depressive psychosis), epilepsy, stroke, traumatic encephalopathy and spinal injury, and the like.

Also, an in vitro study has been reported that Wint10B strongly inhibits the differentiation from pre-fatty cells to mature fatty cells (see Non-patent document 17). GSK-3 specific inhibitors simulate Wint10B signals in pre-fatty cells, i.e. stabilizes free αβ-catenin present in the cytoplasm, to inhibit the induction of c/EBPα and PPARγ, and by so doing inhibits fat formation (see Non-patent document 18). From the foregoing, GSK-3 inhibitors are expected to be pharmaceutical agents effective for the treatment of obesity.

Also, β-catenin is known to be a biological substrate for GSK-3. β-catenin is phosphorylated by GSK-3 and undergoes proteosome-dependent decomposition (see Non-patent document 19). On the other hand, the transient stabilization of β-catenin is thought to be responsible for hair growth (see Non-patent document 20). From the foregoing, GSK-3 inhibitors are expected to be pharmaceutical agents effective for the treatment of alopecia.

Furthermore, a study on fibroblasts derived from a GSK-3β-knock out mouse suggested that GSK-3β positively controls the activity of a transcription factor NFκB (see Non-patent document 21). NFκB is responsible for cellular response properties to a variety of inflammatory stimulations. From the foregoing, GSK-3 inhibitors are expected to be pharmaceutical agents effective for the treatment of inflammatory diseases such as osteoarthritis, rheumatoid arthritis, atopic dermatitis, psoriasis, ulcerative colitis, Crohn's disease, sepsis and generalized inflammatory syndrome by negatively controlling the NFκB activity.

A transcription factor NF-AT is dephosphorylated by calcineurin and potentiates immune reactions (see Non-patent document 22). Conversely GSK-3, by phosphorylating NF-AT and transporting it extranuclearly, acts in the direction of inhibiting the expression of early immune response genes. From the foregoing, GSK-3 inhibitors are expected to be pharmaceutical agents effective for immunopotentiation for cancer immunotherapy etc.

Substances that are conventionally known to have an activity of inhibiting GSK-3 include hymenialdisine derivatives (see Non-patent document 23 and Patent document 1), maleimide derivatives (see Non-patent document 24), Paullone derivatives (see Non-patent document 25 and Patent document 2), purine derivatives (see Patent document 3), pyrimidine and pyridine derivatives (see Patent document 4), hydroxyflavone derivatives (see Patent document 5), pyrimidone derivatives (see Patent document 6, Patent document 7, Patent document 8, Patent document 9, Patent document 19, Patent document 11, Patent document 12, and Patent document 13), pyrrole-2,5-dione derivatives (see Patent document 14 and Patent document 15), diamino-1,2,4-triazole-carboxylic acid derivatives (see Patent document 16), pyrazine derivatives (see Patent document 17), bicyclic inhibitors (see Patent document 18), indirubine derivatives (see Patent document 19), carboxamide derivatives (see Patent document 20), peptide inhibitors (see Patent document 21), 2,4-diaminothiazole derivatives (see Patent document 22), thiazolidine dione derivatives (see Patent document 23), aromatic amide derivatives (see Patent document 24), and the like.
Non-patent document 1: Trends in Biochem. Sci. 16: 177, 1991;
Non-patent document 2: Eur. J. Biochem. 107: 519, 1980;
Non-patent document 3: Biochem. J. (GB) 294: 625, 1993;
Non-patent document 4: Biochem. J. (GB) 303: 21, 1994;
Non-patent document 5: Biochem. J. (GB) 303: 27, 1994;
Non-patent document 6: Diabetes (USA) 49: 263, 2000;
Non-patent document 7: Proc. Natl. Acad. Sci. USA 93: 10228, 1996;
Non-patent document 8: Proc. Natl. Acad. Sci. USA 94: 9660, 1997;
Non-patent document 9: Diabetes (USA) 48: 1662, 1999;
Non-patent document 10: Proc. Natl. Acad. Sci. USA 93: 8455, 1996;
Non-patent document 11: Biol. Trace Elements Res. 60: 131, 1997;
Non-patent document 12: Acta Neuropathol. 103: 91, 2002;
Non-patent document 13: J. Neurochem. 77: 94, 2001;
Non-patent document 14: Expert Opin. Pharmacother. 1: 121, 1999;
Non-patent document 15: Proc. Natl. Acad. Sci. USA 95: 2642, 1998;
Non-patent document 16: J. Neurochem. 77: 94, 2001;
Non-patent document 17: Science 289: 950, 2000;
Non-patent document 18: J. Biol. Chem. 277: 30998, 2002;
Non-patent document 19: EMBO J. 17: 1371, 1998;
Non-patent document 20: Cell 95: 605, 1998;
Non-patent document 21: Nature 406: 86, 2000;
Non-patent document 22: Science 275: 1930, 1997;
Non-patent document 23: Chemistry & Biology 7: 51, 2000;
Non-patent document 24: Chemistry & Biology 7: 793, 2000;
Non-patent document 25: Eur. J. Biochem. 267: 5983, 2000;

Patent document 1: International Patent Publication WO 01/41768 brochure;
Patent document 2: International Patent Publication WO 01/60374 brochure;
Patent document 3: International Patent Publication WO 98/16528 brochure;
Patent document 4: International Patent Publication WO 99/65897 brochure;
Patent document 5: International Patent Publication WO 00/17184 brochure;
Patent document 6: International Patent Publication WO 00/18758 brochure;
Patent document 7: International Patent Publication WO 01/70683 brochure;
Patent document 8: International Patent Publication WO 01/70729 brochure;
Patent document 9: International Patent Publication WO 01/70728 brochure;
Patent document 10: International Patent Publication WO 01/70727 brochure;
Patent document 11: International Patent Publication WO 01/70727 brochure;
Patent document 12: International Patent Publication WO 01/70726 brochure;
Patent document 13: International Patent Publication WO 01/70725 brochure;
Patent document 14: International Patent Publication WO 00/21927 brochure;
Patent document 15: International Patent Publication WO 01/74771 brochure;
Patent document 16: International Patent Publication WO 01/09106 brochure;
Patent document 17: International Patent Publication WO 01/44206 brochure;
Patent document 18: International Patent Publication WO 01/44246 brochure;
Patent document 19: International Patent Publication WO 01/37819 brochure;
Patent document 20: International Patent Publication WO 01/42224 brochure;
Patent document 21: International Patent Publication WO 01/49709 brochure;
Patent document 22: International Patent Publication WO 01/56567 brochure;
Patent document 23: International Patent Publication WO 01/85685 brochure;
Patent document 24: International Patent Publication WO 01/81345 brochure.

It is an object of the present invention to provide clinically applicable novel compounds that have a potent and selective inhibitory activity against GSK-3.

### DISCLOSURE OF THE INVENTION

After intensive and extensive research to attain the above objective, the present inventors have found that novel pyrrolo[3,2-d]pyrimidine derivatives represented by the following formula (I) or pharmaceutically acceptable salts thereof exhibit excellent activity of inhibiting GSK-3, and thereby have completed the present invention.

Thus, the present invention is:
(1) A pyrrolo[3,2-d]pyrimidine derivative represented by Formula (I) or a pharmaceutically acceptable salt thereof

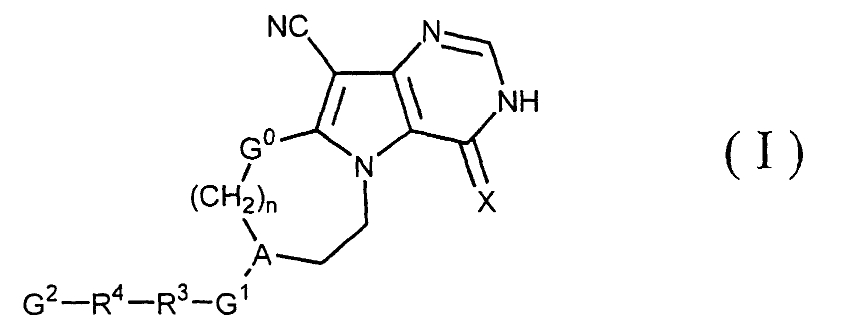

[In Formula (I), X represents an oxygen atom or a sulfur atom.

In Formula (I), n represents 0, 1, or 2.

In Formula (I), A represents a nitrogen atom or CH.

In Formula (I), G⁰ represents a divalent group of substituted or unsubstituted benzene, furan, thiophene, pyrrole, isoxazole, cyclopentane or cyclohexane, or a divalent group represented by -CR¹R²- (R¹ and R², which may be the same or different, represent a hydrogen atom, a substituted or unsubstituted aliphatic hydrocarbon group having one to four carbons, or NR¹⁰R²⁰ (R¹⁰ and R²⁰, which may be the same or different, represent a hydrogen atom, a substituted or unsubstituted aliphatic hydrocarbon group having one to four carbons), or an optionally substituted group in which R¹ and R² bind to each other and form a 3- to 7-membered ring together with a carbon atom (C in -CR¹R²-) to which R¹ and R² are bound, provided that R¹ and R² are not NR¹⁰R²⁰ at the same time).

In Formula (I), G¹ represents a single bond, or a group that binds A to which G¹ binds and R³ in the form of A-C(=O)-O-R³, A-C(=O)-R³, A-C(=O)-NR³⁰-R³, A-C(=S)-NR³¹-R³, A-C(=O)-NR³²-S(=O)₂-R³, or A-S(=O)₂-R³ (R³⁰ to R³² represent, independently from one another, a hydrogen atom or a substituted or unsubstituted aliphatic hydrocarbon group having one to four carbons).

In Formula (I), R³ represents a group selected from the following 1)-5).
1) a single bond,
2) a substituted or unsubstituted alicyclic hydrocarbon group having three to eight carbons (substituents are one or more substituents selected from the group consisting of a fluorine atom, a chlorine atom, a bromine atom, an iodine atom, a hydroxy group, an optionally substituted alkoxy group having one to seven carbons, an aryloxy group having six to ten carbons, an aralkoxy group having seven to nine carbons, an acyloxy group having two to seven carbons, an oxo group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, and an optionally substituted aliphatic hydrocarbon group having one to six carbons),
3) a substituted or unsubstituted aromatic hydrocarbon group having six to 14 carbons (substituents are one or more substituents selected from the group consisting of a fluorine atom, a chlorine atom, a bromine atom, an iodine atom, a hydroxy group, an optionally substituted alkoxy group having one to seven carbons, an aryloxy group having six to ten carbons, an aralkoxy group having seven to nine carbons, an acyloxy group having two to seven carbons, an oxo group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, and an optionally substituted aliphatic hydrocarbon group having one to six carbons),
4) a substituted or unsubstituted heterocyclic group containing, in the ring, one to four atoms selected from the group consisting of an oxygen atom, a nitrogen atom, and a sulfur atom (substituents are one or more substituents selected from the group consisting of a fluorine atom, a chlorine atom, a bromine atom, an iodine atom, a hydroxy group, an optionally substituted alkoxy group having one to seven carbons, an aryloxy group having six to ten carbons, an aralkoxy group having seven to nine carbons, an acyloxy group having two to seven carbons, an oxo group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, and an optionally substituted aliphatic hydrocarbon group having one to six carbons),
5) a substituted or unsubstituted aliphatic hydrocarbon group having one to ten carbons (substituents are one or more substituents selected from the group consisting of a fluorine atom, a chlorine atom, a bromine atom, an iodine atom, a hydroxy group, an optionally substituted alkoxy group having one to seven carbons, an optionally substituted phenylalkoxy group having seven to ten carbons, an alkoxy group having one to four carbons substituted with an optionally substituted heterocyclic group (containing, in the ring, one to four atoms selected from the group consisting of an oxygen atom, a nitrogen atom, and a sulfur atom), an optionally substituted aryloxy group having six to ten carbons, an acyloxy group having two to seven carbons, an oxo group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, an optionally substituted aromatic hydrocarbon group having six to 14 carbons, and an optionally substituted heterocyclic group (containing, in the ring, one to four atoms selected from the group consisting of an oxygen atom, a nitrogen atom, and a sulfur atom)).

In Formula (I), R⁴ represents a group selected from the following 1)-4).
1) a single bond,
2) a substituted or unsubstituted alicyclic hydrocarbon group having three to eight carbons (substituents are one or more substituents selected from the group consisting of a fluorine atom, a chlorine atom, a bromine atom, an iodine atom, a hydroxy group, an optionally substituted alkoxy group having one to seven carbons, an aryloxy group having six to ten carbons, an aralkoxy group having seven to nine carbons, an acyloxy group having two to seven carbons, an oxo group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, and an optionally substituted aliphatic hydrocarbon group having one to six carbons),
3) a substituted or unsubstituted aromatic hydrocarbon group having six to 14 carbons (substituents are one or more substituents selected from the group consisting of a fluorine atom, a chlorine atom, a bromine atom, an iodine atom, a hydroxy group, an optionally substituted alkoxy group having one to seven carbons, an aryloxy group having six to ten carbons, an aralkoxy group having seven to nine carbons, an acyloxy group having two to seven carbons, an oxo group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, and an optionally substituted aliphatic hydrocarbon group having one to six carbons),
4) a substituted or unsubstituted heterocyclic group containing, in the ring, one to four atoms selected from the group consisting of an oxygen atom, a nitrogen atom, and a sulfur atom (substituents are one or more substituents selected from the group consisting of a fluorine atom, a chlorine atom, a bromine atom, an iodine atom, a hydroxy group, an optionally substituted alkoxy group having one to seven carbons, an aryloxy group having six to ten carbons, an aralkoxy group having seven to nine carbons, an acyloxy group having two to seven carbons, an oxo group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, and an optionally substituted aliphatic hydrocarbon group having one to six carbons).

In Formula (I), G² represents a hydrogen atom, -C(=O)-OH, -C(=O)-NH-OH, -S(=O)₂-OH, or a 5-tetrazolyl group];
(2) A pyrrolo[3,2-d]pyrimidine derivative described in (1) or a pharmaceutically acceptable salt thereof, wherein A represents a nitrogen atom;
(3) A pyrrolo[3,2-d]pyrimidine derivative described in (2) or a pharmaceutically acceptable salt thereof, wherein G⁰ is a divalent group represented by -CR¹R²- (R¹ and R² are as defined above);
(4) A pyrrolo[3,2-d]pyrimidine derivative described in (2) or a pharmaceutically acceptable salt thereof, wherein G⁰ is a divalent group represented by -CR¹R²- wherein R¹ and R², which may be the same or different, are a hydrogen atom or an optionally substituted aliphatic hydrocarbon group having one to four carbons, or R¹ and R² bind to each other and form a cyclopropane ring together with a carbon atom to which R¹ and R² are bound;
(5) A pyrrolo[3,2-d]pyrimidine derivative described in (2) or a pharmaceutically acceptable salt thereof, wherein G⁰ is a divalent group represented by -CR¹R²- wherein R¹ and R², which may be the same or different, are a hydrogen atom or a methyl group, or R¹ and R² bind to each other and form a cyclopropane ring together with a carbon atom to which R¹ and R² are bound;
(6) A pyrrolo[3,2-d]pyrimidine derivative described in (2) or a pharmaceutically acceptable salt thereof, wherein G⁰ is a divalent group represented by -CR¹R²- wherein R¹ is an optionally substituted aliphatic hydrocarbon group having one to four carbons and R² is a hydrogen atom;
(7) A pyrrolo[3,2-d]pyrimidine derivative described in (2) or a pharmaceutically acceptable salt thereof, wherein G⁰ is a divalent group represented by -CR¹R²- wherein R¹ is a methyl group and R² is a hydrogen atom;
(8) A pyrrolo[3,2-d]pyrimidine derivative described in (2) or a pharmaceutically acceptable salt thereof, wherein G⁰ is a divalent group represented by -CR¹R²- wherein each of R¹ and R² is a methyl group, or R¹ and R² bind to each other and form a cyclopropane ring together with a carbon atom to which R¹ and R² are bound;
(9) A pyrrolo[3,2-d]pyrimidine derivative described in (2) or a pharmaceutically acceptable salt thereof, wherein G⁰ is a divalent group of an optionally substituted benzene, furan, thiophene, pyrrole, isoxazole, cyclopentane or cyclohexane, and G⁰, (CH₂)ₙ, A, -(CH₂)₂-, and a nitrogen atom and a carbon atom in the pyrrole ring of the pyrrolopyrimidine ring form a 10- to 12-membered bicyclic structure;
(10) A pyrrolo[3,2-d]pyrimidine derivative described in (2) or a pharmaceutically acceptable salt thereof, wherein G⁰ is a divalent group of an optionally substituted benzene, and G⁰, (CH₂)ₙ, A, -(CH₂)₂-, and a nitrogen atom and a carbon atom in the pyrrole ring of the pyrrolopyrimidine ring form a 10- to 12-membered bicyclic structure;
(11) A pyrrolo[3,2-d]pyrimidine derivative described in (2) or a pharmaceutically acceptable salt thereof, wherein G⁰ is a divalent group of benzene, furan, thiophene, pyrrole, isoxazole, cyclopentane or cyclohexane, and G⁰, (CH₂)ₙ, A, -(CH₂)₂-, and a nitrogen atom and a carbon atom in the pyrrole ring of the pyrrolopyrimidine ring form a 10- to 12-membered bicyclic structure, and said bicyclic structure has 3-5 substituents;
(12) A pyrrolo[3,2-d]pyrimidine derivative described in (2) or a pharmaceutically acceptable salt thereof, wherein G⁰ is a divalent group of an optionally substituted isoxazole, and G⁰, (CH₂)ₙ, A, -(CH₂)₂-, and a nitrogen atom and a carbon atom in the pyrrole ring of the pyrrolopyrimidine ring form a 10- to 12-membered bicyclic structure;
(13) A pyrrolo[3,2-d]pyrimidine derivative described in any of (2) to (12) or a pharmaceutically acceptable salt thereof, wherein R³ is a divalent group of an optionally substituted, saturated aliphatic hydrocarbon group having five to ten carbons, an optionally substituted alicyclic hydrocarbon group having five to eight carbons, an optionally substituted aromatic hydrocarbon group having six to ten carbons, or an optionally substituted heterocyclic group (containing one to four atoms selected from the group consisting of an oxygen atom, a nitrogen atom, and a sulfur atom);
(14) A pyrrolo[3,2-d]pyrimidine derivative described in any of (2) to (12) or a pharmaceutically acceptable salt thereof, wherein R³ is a divalent group of an optionally substituted heterocyclic group (containing, in the ring, one to four atoms selected from the group consisting of an oxygen atom, a nitrogen atom, and a sulfur atom);
(15) A pyrrolo[3,2-d]pyrimidine derivative described in any of (2) to (12) or a pharmaceutically acceptable salt thereof, wherein A-G¹-R³ represents a group that binds in the form of A-C(=O)-NH-R³, A-C(=S)-NH-R³, or A-C(=O)-NH-S(=O)₂-R³, and R³ is a divalent group of an optionally substituted aliphatic hydrocarbon group having one to ten carbons, an optionally substituted alicyclic hydrocarbon group having three to eight carbons, an optionally substituted aromatic hydrocarbon group having six to ten carbons, or an optionally substituted heterocyclic group (containing, in the ring, one to four atoms selected from the group consisting of an oxygen atom, a nitrogen atom, and a sulfur atom);
(16) A pyrrolo[3,2-d]pyrimidine derivative described in any of (2) to (12) or a pharmaceutically acceptable salt thereof, wherein A-G¹-R³ represents a group that binds in the form of A-C(=O)-NH-R³ or A-C(=S)-NH-R³, and R³ is a divalent group of an optionally substituted aliphatic hydrocarbon group having one to ten carbons, an optionally substituted alicyclic hydrocarbon group having three to eight carbons, an optionally substituted aromatic hydrocarbon group having six to ten carbons, or an optionally substituted heterocyclic group (containing, in the ring, one to four atoms selected from the group consisting of an oxygen atom, a nitrogen atom, and a sulfur atom);
(17) A pyrrolo[3,2-d]pyrimidine derivative described in any of (2) to (12) or a pharmaceutically acceptable salt thereof, wherein A-G¹-R³ represents a group that binds in the form of A-C(=O)-NH-R³, and R³ is a divalent group of an optionally substituted aliphatic hydrocarbon group having one to ten carbons, an optionally substituted alicyclic hydrocarbon group having three to eight carbons, an optionally substituted aromatic hydrocarbon group having six to ten carbons, or an optionally substituted heterocyclic group (containing, in the ring, one to four atoms selected from the group consisting of an oxygen atom, a nitrogen atom, and a sulfur atom);
(18) A pyrrolo[3,2-d]pyrimidine derivative described in any of (2) to (12) or a pharmaceutically acceptable salt thereof, wherein A-G¹-R³ represents a group that binds in the form of A-C(=O)-NH-R³, and R³ is a divalent group of an optionally substituted alkane having five to ten carbons, an optionally substituted alicyclic hydrocarbon group having five to eight carbons, an optionally substituted aromatic hydrocarbon group having six to ten carbons, or an optionally substituted heterocyclic group (containing, in the ring, one to four atoms selected from the group consisting of an oxygen atom, a nitrogen atom, and a sulfur atom);
(19) A pyrrolo[3,2-d]pyrimidine derivative described in any of (2) to (12) or a pharmaceutically acceptable salt thereof, wherein A-G¹-R³ represents a group that binds in the form of A-C(=O)-NH-R³, and R³ is a divalent group of an optionally substituted heterocyclic group (containing, in the ring, one to four atoms selected from the group consisting of an oxygen atom, a nitrogen atom, and a sulfur atom);
(20) A pyrrolo[3,2-d]pyrimidine derivative described in any of (2) to (19) or a pharmaceutically acceptable salt thereof, wherein A-G¹-R³ represents a group that binds in the form of A-C(=O)-R³, A-C(=O)-NH-R³, or A-C(=S)-NH-R³, and G² represents any of -C(=O)-OH, -C(=O)-NH-OH, -S(=O)₂-OH, and 5-tetrazolyl group;
(21) A pyrrolo[3,2-d]pyrimidine derivative described in any of (2) to (19) or a pharmaceutically acceptable salt thereof, wherein A-G¹-R³ represents a group that binds in the form of A-C(=O)-R³, A-C(=O)-NH-R³, or A-C(=S)-NH-R³, and G² represents -C(=O)-OH;
(22) A pyrrolo[3,2-d]pyrimidine derivative described in any of (2) to (19) or a pharmaceutically acceptable salt thereof, wherein A-G¹-R³ represents a group that binds in the form of A-C(=O)-NH-R³, and G² represents any of -C(=O)-OH, -C(=O)-NH-OH, -S(=O)₂-OH, and 5-tetrazolyl group;
(23) A pyrrolo[3,2-d]pyrimidine derivative described in any of (2) to (19) or a pharmaceutically acceptable salt thereof, wherein A-G¹-R³ represents a group that binds in the form of A-C(=O)-NH-R³, and G² represents -C(=O)-OH;
(24) A pyrrolo[3,2-d]pyrimidine derivative described in any of (2) to (12) or a pharmaceutically acceptable salt thereof, wherein -G¹- represents a single bond, and R³ is a divalent group of an alkane having two to six carbons substituted with an optionally substituted alkoxy group having one to four carbons, an optionally substituted phenylalkoxy group having seven to ten carbons, or an optionally substituted aryloxy group having six to ten carbons;
(25) A pyrrolo[3,2-d]pyrimidine derivative described in any of (2) to (12) or a pharmaceutically acceptable salt thereof, wherein -G¹- represents a single bond, and R³ is a divalent group of an alkane having two to four carbons substituted with an optionally substituted alkoxy group having one to four carbons;
(26) A pyrrolo[3,2-d]pyrimidine derivative described in any of (2) to (12) or a pharmaceutically acceptable salt thereof, wherein -G¹- represents a single bond, and R³ is a divalent group of an alkane having two to four carbons substituted with a phenylalkoxy group having seven to ten carbons;
(27) A pyrrolo[3,2-d]pyrimidine derivative described in any of (2) to (12) or a pharmaceutically acceptable salt thereof, wherein -G¹- represents a single bond, and R³ is a divalent group of an alkane having two to four carbons substituted with an alkoxy group having one to four carbons substituted with an optionally substituted heterocyclic group (containing, in the ring, one to four atoms selected from the group consisting of an oxygen atom, a nitrogen atom, and a sulfur atom);
(28) A pyrrolo[3,2-d]pyrimidine derivative described in any of (2) to (12) or a pharmaceutically acceptable salt thereof, wherein -G¹- represents a single bond, and R³ is a divalent group of an alkane having two to four carbons substituted with an optionally substituted phenoxy group;
(29) A pyrrolo[3,2-d]pyrimidine derivative described in any of (2) to (12) or a pharmaceutically acceptable salt thereof, wherein -G¹- represents a single bond, and R³ is a divalent group of an alkane having two to four carbons substituted with an optionally substituted benzyloxy group;
(30) A pyrrolo[3,2-d]pyrimidine derivative described in any of (2) to (12) or a pharmaceutically acceptable salt thereof, wherein -G¹- represents a single bond, and R³ represents -CH2-, and R⁴ is a divalent group of an aromatic hydrocarbon group having six to ten carbons said group having G² other than a hydrogen atom or a substituent at a carbon atom of R⁴ at a position adjacent to the carbon atom of R⁴ at which -R³- binds, or a heterocyclic group (containing, in the ring, one to four atoms selected from the group consisting of an oxygen atom, a nitrogen atom, and a sulfur atom) having G² other than a hydrogen atom or a substituent at an atom at a position adjacent to the carbon atom of R⁴ at which -R³- binds;
(31) A pyrrolo[3,2-d]pyrimidine derivative described in any of (2) to (30) or a pharmaceutically acceptable salt thereof, wherein X is an oxygen atom;
(32) A pyrrolo[3,2-d]pyrimidine derivative described in any of (2) to (30) or a pharmaceutically acceptable salt thereof, wherein X is a sulfur atom;
(33) A pyrrolo[3,2-d]pyrimidine derivative described in any of (2) to (30) or a pharmaceutically acceptable salt thereof, wherein G⁰ is a divalent group represented by -CR¹R²-, wherein R¹ and R², which may be the same or different, are a hydrogen atom or a methyl group, n represents 1, and X is a sulfur atom;
(34) A pyrrolo[3,2-d]pyrimidine derivative described in (1) or a pharmaceutically acceptable salt thereof, wherein A represents CH;
(35) A pyrrolo[3,2-d]pyrimidine derivative described in (34) or a pharmaceutically acceptable salt thereof, wherein G⁰ is a divalent group represented by -CR¹R²-, wherein R¹ and R², which may be the same or different, are a hydrogen atom or a substituted or unsubstituted aliphatic hydrocarbon group having one to four carbons, or R¹ and R² bind to each other and form a cyclopropane ring together with a carbon atom to which R¹ and R² are bound;
(36) A pyrrolo[3,2-d]pyrimidine derivative described in (34) or a pharmaceutically acceptable salt thereof, wherein G⁰ is a divalent group represented by -CR¹R²-, wherein R¹ and R², which may be the same or different, are a hydrogen atom or a methyl group, or R¹ and R² bind to each other and form a cyclopropane ring together with a carbon atom to which R¹ and R² are bound;
(37) A pyrrolo[3,2-d]pyrimidine derivative described in (34) or a pharmaceutically acceptable salt thereof, wherein G⁰ is a divalent group represented by -CR¹R²-, wherein R¹ is a substituted or unsubstituted aliphatic hydrocarbon group having one to four carbons and R² is a hydrogen atom;
(38) A pyrrolo[3,2-d]pyrimidine derivative described in (34) or a pharmaceutically acceptable salt thereof, wherein G⁰ is a divalent group represented by -CR¹R²-, wherein R¹ is a methyl group and R² is a hydrogen atom;
(39) A pyrrolo[3,2-d]pyrimidine derivative described in (34) or a pharmaceutically acceptable salt thereof, wherein G⁰ is a divalent group represented by -CR¹R²-, wherein both of R¹ and R² are a methyl group, or R¹ and R² bind to each other and form a cyclopropane ring together with a carbon atom to which R¹ and R² are bound;
(40) A pyrrolo[3,2-d]pyrimidine derivative described in (34) or a pharmaceutically acceptable salt thereof, wherein G⁰ represents a divalent group of an optionally substituted benzene, furan, thiophene, pyrrole, isoxazole, cyclopentane or cyclohexane, and G⁰, (CH₂)ₙ, A, -(CH₂)₂-, and a nitrogen atom and a carbon atom in the pyrrole ring of the pyrrolopyrimidine ring form a 10- to 12-membered bicyclic structure;
(41) A pyrrolo[3,2-d]pyrimidine derivative described in (34) or a pharmaceutically acceptable salt thereof, wherein G⁰ represents a divalent group of optionally substituted benzene, and G⁰, (CH₂)ₙ, A, -(CH₂)₂-, and a nitrogen atom and a carbon atom in the pyrrole ring of the pyrrolopyrimidine ring form a 10- to 12-membered bicyclic structure;
(42) A pyrrolo[3,2-d]pyrimidine derivative described in (34) or a pharmaceutically acceptable salt thereof, wherein G⁰ represents a divalent group of a substituted benzene, furan, thiophene, pyrrole, isoxazole, cyclopentane or cyclohexane, and G⁰, (CH₂)ₙ, A, -(CH₂)₂-, and a nitrogen atom and a carbon atom in the pyrrole ring of the pyrrolopyrimidine ring form a 10- to 12-membered bicyclic structure and said bicyclic structure has 3-5 substituents;
(43) A pyrrolo[3,2-d]pyrimidine derivative described in (34) or a pharmaceutically acceptable salt thereof, wherein G⁰ represents a divalent group of an optionally substituted isoxazole, and G⁰, (CH₂)ₙ, A, -(CH₂)₂-, and a nitrogen atom and a carbon atom in the pyrrole ring of the pyrrolopyrimidine ring form a 10- to 12-membered bicyclic structure;
(44) A GSK-3 inhibitor comprising a pyrrolo[3,2-d]pyrimidine derivative described in any of (1) to (43) or a pharmaceutically acceptable salt thereof;
(45) A pharmaceutical composition comprising a pyrrolo[3,2-d]pyrimidine derivative described in any of (1) to (43) or a pharmaceutically acceptable salt thereof and a pharmaceutically acceptable carrier;
(46) A therapeutic or preventive agent for a disease in which GSK-3 is involved, said agent comprising as an active ingredient a pyrrolo[3,2-d]pyrimidine derivative described in any of (1) to (43) or a pharmaceutically acceptable salt thereof;
(47) A therapeutic or preventive agent according to claim (46) wherein a disease in which GSK-3 is involved is one selected from the group consisting of diabetes, diabetic complications, Alzheimer's disease, neurodegenerative diseases, manic-depressive psychosis, traumatic encephalopathy, alopecia, inflammatory diseases, cancer, and immune deficiency;
(48) A pyrrolo[3,2-d]pyrimidine derivative represented by Formula (II) 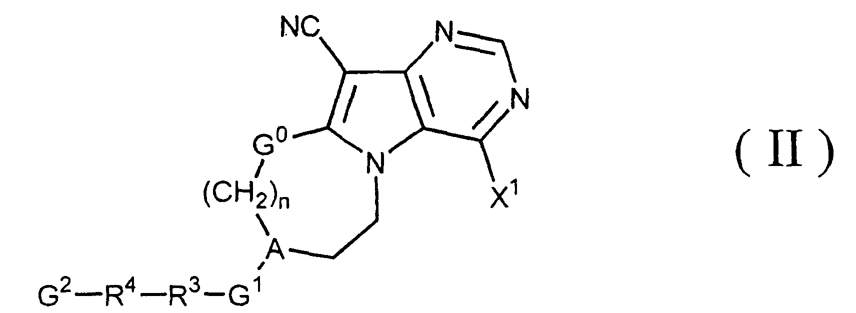 [In Formula (II), n, A, R³, R⁴, G⁰, G¹, and G² are as defined for Formula (I). X¹ represents a chlorine atom, a bromine atom, an iodine atom, or an alkyl or arylsulfonyl group having one to eight carbons that may be substituted with a fluorine atom, a chlorine atom, or a bromine atom.]
(49) A pyrrolo[3,2-d]pyrimidine derivative described in (48) wherein X¹ is a chlorine atom or a trifluoromethylsulfonyloxy group.

### BEST MODE FOR CARRYING OUT THE INVENTION

In the above Formula (I), G⁰ represents a divalent group of a substituted or unsubstituted benzene, furan, thiophene, pyrrole, isoxazole, cyclopentane, or cyclohexane, or a divalent group represented by -CR¹R²- (R¹ and R², which may be the same or different, represent a hydrogen atom, a substituted or unsubstituted aliphatic hydrocarbon group having one to four carbons, or NR¹⁰R²⁰ (R¹⁰ and R²⁰, which may be the same or different, represent a hydrogen atom or a substituted or unsubstituted aliphatic hydrocarbon group having one to four carbons), or an optionally substituted group in which R¹ and R² bind to each other and form a 3- to 7-membered ring together with a carbon atom (C in -CR¹R²-) to which R¹ and R² are bound, provided that R¹ and R² are not NR¹⁰R²⁰ at the same time).

When G⁰ is a divalent group of a substituted or unsubstituted benzene, furan, thiophene, pyrrole, isoxazole, cyclopentane, or cyclohexane, examples of a divalent group of benzene, furan, thiophene, pyrrole, isoxazole, cyclopentane, or cyclohexane include 1,2-phenylene, 1,3-phenylene, 2,3-furandiyl, 3,4-furandiyl, 2,4-furandiyl, 2,5-furandiyl, 2,3-thiophenediyl, 3,4-thiophenediyl, 2,4-thiophenediyl, 2,5-thiophenediyl, 1,2-pyrrolediyl, 1,3-pyrrolediyl, 2,3-pyrrolediyl, 3,4-pyrrolediyl, 2,4-pyrrolediyl, 2,5-pyrrolediyl, 3,4-isoxazolediyl, 3,5-isoxazolediyl, 4,5-isoxazolediyl, 1,2-cyclopentylene, 1,3-cyclopentylene, 1,2-cyclohexylene, and 1,3-cyclohexylene. G⁰, a divalent group of benzene, furan, thiophene, pyrrole, isoxazole, cyclopentane, or cyclohexane, may be substituted with one or more substituents selected from the group consisting of a fluorine atom, a chlorine atom, a bromine atom, an iodine atom, a hydroxy group, a methoxy group, an ethoxy group, an oxo group, a cyano group, a carboxyl group, a carbamoyl group, an amino group, a nitro group, and a sulpho group. G⁰, a divalent group of a substituted or unsubstituted benzene, furan, thiophene, pyrrole, isoxazole, cyclopentane, or cyclohexane is preferably 1,2-phenylene.

When G⁰ represents a divalent group represented by -CR¹R²- (R¹ and R², which may be the same or different, represent a hydrogen atom, a substituted or unsubstituted aliphatic hydrocarbon group having one to four carbons, or NR¹⁰R²⁰ (R¹⁰ and R²⁰, which may be the same or different, represent a hydrogen atom or a substituted or unsubstituted aliphatic hydrocarbon group having one to four carbons), or a group in which R¹ and R² bind to each other and form a 3- to 7-membered ring together with a carbon atom (C in -CR¹R²- ) to which R¹ and R² are bound, provided that R¹ and R² are not NR¹⁰R²⁰ at the same time), the above Formula (I) represents a pyrrolo[3,2-d]pyrimidine derivative represented by the following Formula (Ia):

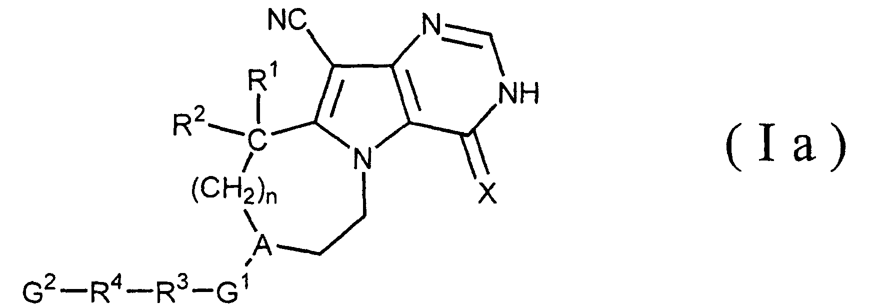

[In Formula (Ia), A, R¹, R², R³, R⁴, G¹, G², and X are as defined for Formula (I)].

When R¹ and R² represent a substituted or unsubstituted aliphatic hydrocarbon group having one to four carbons, examples of such an aliphatic hydrocarbon group having one to four carbons include methyl, ethyl, propyl, isopropyl, butyl, isobutyl, s-butyl, t-butyl, vinyl, 1-propenyl, 2-propenyl, 1-butenyl, 2-butenyl, 3-butenyl, ethinyl, 1-propynyl, 2-propynyl, 1-butynyl, 2-butynyl, and 3-butynyl. An aliphatic hydrocarbon group having one to four carbons may be substituted with one or more substituents selected from the group consisting of a fluorine atom, a chlorine atom, a bromine atom, an iodine atom, a hydroxy group, a methoxy group, an ethoxy group, an oxo group, a cyano group, a carboxyl group, a carbamoyl group, an amino group, a nitro group, a sulpho group, and a phenyl group. Preferred examples of such R¹ and R² comprising a substituted or unsubstituted aliphatic hydrocarbon group having one to four carbons include methyl, trifluoromethyl, ethyl, propyl, and isopropyl.

When R¹ and R² represent NR¹⁰R²⁰ (R¹⁰ and R²⁰, which may be the same or different, represent a hydrogen atom, a substituted or unsubstituted aliphatic hydrocarbon group having one to four carbons, or a substituted or unsubstituted alkylene group having two to five carbons that is formed by the binding of R¹⁰ and R²⁰), examples of R¹⁰ and R²⁰, an aliphatic hydrocarbon group having one to four carbons, include methyl, ethyl, propyl, isopropyl, butyl, isobutyl, s-butyl, t-butyl, 2-propenyl, 2-butenyl, 3-butenyl, 2-propynyl, 2-butynyl, and 3-butynyl.
Examples of an alkylene group having two to five carbons that is formed by the binding of R¹⁰ and R²⁰ include 1,2-ethylene, 1,3-propylene, 1,4-butylene, 1,5-pentylene. R¹⁰ and R²⁰, an aliphatic hydrocarbon group having one to four carbons, and an alkylene group having two to five carbons that are formed by the binding of R¹⁰ and R²⁰ may be substituted with one or more substituents selected from the group consisting of a fluorine atom, a chlorine atom, a bromine atom, an iodine atom, a hydroxy group, a methoxy group, an ethoxy group, a t-butoxy group, an oxo group, a cyano group, a carboxyl group, a carbamoyl group, an amino group, a sulpho group, and a phenyl group. Preferred examples of such R¹ and R², NR¹⁰R²⁰, include amino and dimethyl. However, R¹ and R² are not NR¹⁰R²⁰ at the same time.

When R¹ and R² bind to each other and form a 3- to 7-membered ring together with a carbon atom to which R¹ and R² are bound, examples of a group forming such a 3-to 7-membered ring include cyclopropane, cyclobutane, cyclopentane, cyclohexane, cycloheptane tetrahydrofuran, tetrahydropyran, pyrrolidine, and piperidine. A group forming such a 3- to 7-membered ring may be substituted with one or more substituents selected from the group consisting of a fluorine atom, a chlorine atom, a bromine atom, an iodine atom, a hydroxy group, a methoxy group, an ethoxy group, an oxo group, a cyano group, a carboxyl group, a carbamoyl group, an amino group, a sulpho group, and a phenyl group. Preferred examples of a group forming such a 3- to 7-membered ring include cyclopropane.

As preferred examples of R¹ and R², there can be mentioned a hydrogen atom, a methyl group, an ethyl group, and one in which R¹ and R² bind to each other and form cyclopropane with a carbon atom to which they are bound, with the methyl group being preferred.

When G⁰ in Formula (I) represents a divalent group of a substituted or unsubstituted benzene, furan, thiophene, pyrrole, isoxazole, cyclopentane, or cyclohexane, G⁰, (CH₂)ₙ, A, -(CH₂)₂-, and a nitrogen atom and a carbon atom in the pyrrole ring of the pyrrolopyrimidine ring may form a 10- to 12-membered bicyclic structure. At this time, G⁰ is preferably a substituted or unsubstituted benzene, furan, thiophene, pyrrole, or isoxazole.

As specific examples of said bicyclic structure, there can be mentioned 1H,2H,3H,4H,5H-benzo[f]1,4-diazaperhydroepine, 1H,2H,3H,4H,5H,6H, benzo[f]1,4-diazaperhydroocine, 1H,2H,3H,4H,5H-thiopheno[2,3-f]1,4-diazepine, 1H,2H,3H,4H,5H-furano[2,3-f]1,4-diazepine, 1H,2H,3H,4H,5H-pyrrolo[2,3-f]1,4-diazepine, 4H,5H,6H,7H,8H-isoxazolo[5,4-f]1,4-diazepine, 2,5-diazabicyclo[5,3,1]undeca-1(11),7,9-triene, 2,5-diaza-10-thiabicyclo[[5,2,1]deca-1(9),7-diene, 2,5-diaza-10-oxabicyclo[5,2,1]deca-1(9),7-diene, 2,5,10-triazabicyclo[5,2,1]deca-1(9),7-diene, 2,5-diazabicyclo[5,4,0]undecane, 2,5-diazabicyclo[5,3,0]decane, 1H,2H,3H,4H,5H,6H-benzo[f]azaperhydroocine, 1H,2H,3H,4H,5H-benzo[e]azaperhydroocine, 4H,5H,6H,7H,8H-thiopheno[3,2-e]azepine, 4H,5H,6H,7H,8H-furano[3,2-e]azepine, 3-aza-10-thiabicyclo[5,2,1]deca-2(9),7-diene, 3-aza-10-oxabicyclo[5,2,1]deca-1(9),7-diene, 3-azabicyclo[5,4,0]undecane, 3-azabicyclo[5,3,0]decane and the like.

In the above Formula (I), X represents a sulfur atom or an oxygen atom. Thus, a pyrrolo[3,2-d]pyrimidine derivative of the above Formula (I) represents a pyrrolo[3,2-d]pyrimidine derivative represented by the following Formula (Ib): 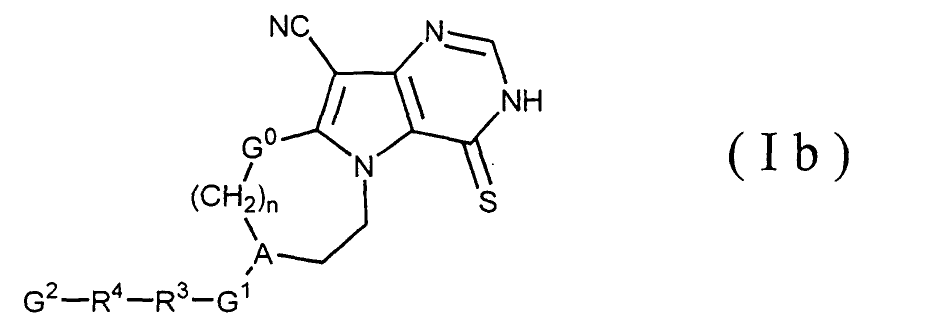 [in Formula (Ib), n, A, R³, R⁴, G⁰, G¹, and G² are as defined for Formula (I)],
and the following Formula (Ic): 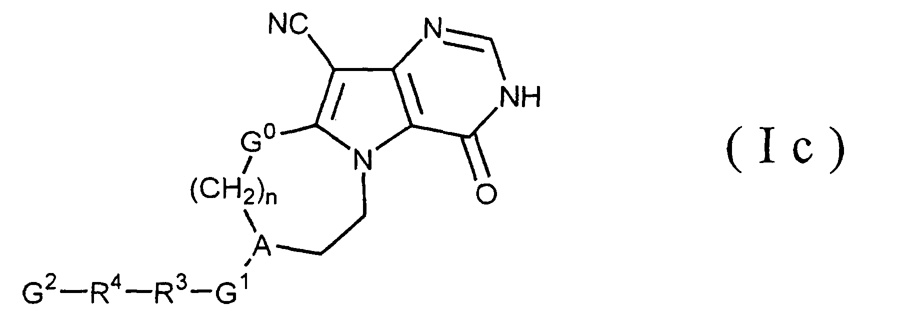 [in Formula (Ic), n, A, R³, R⁴, G⁰, G¹, and G² are as defined for Formula (I)]. A preferred X is a sulfur atom.

In the above Formula (I), n represents 0, 1 or 2. Thus, when n represents 0, the pyrrolo[3,2-d]pyrimidine derivative of the above Formula (I) represents a pyrrolo[3,2-d]pyrimidine derivative represented by the following Formula (Id): 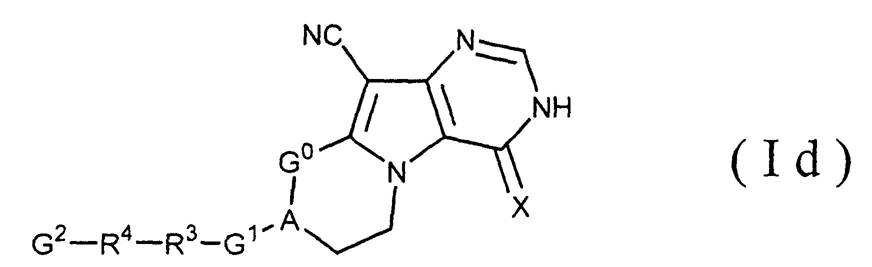 [in Formula (Id), A, R³, R⁴, G⁰, G¹, G², and X are as defined for Formula (I)], and when n represents 1, a pyrrolo[3,2-d]pyrimidine derivative represented by the following Formula (Ie): 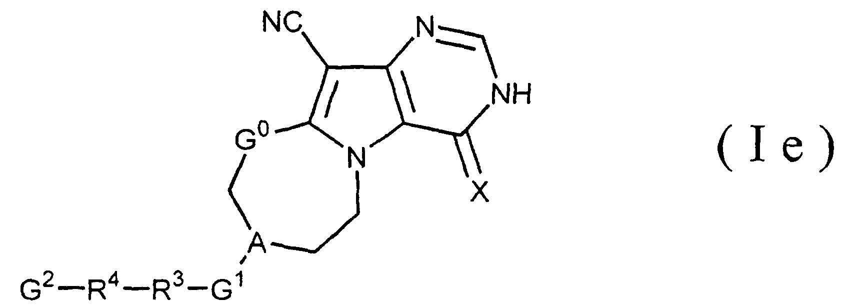 [in Formula (Ie), A, R³, R⁴, G⁰, G¹, G² and X are as defined for Formula (I)], and when n represents 2, a pyrrolo[3,2-d]pyrimidine derivative represented by the following Formula (If): 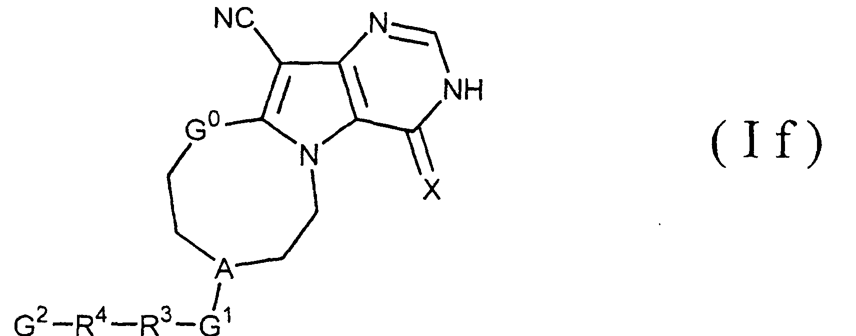 [in Formula (If), A, R³, R⁴, G⁰, G¹, G² and X are as defined for Formula (I)]. Preferred n is 1.

In the above Formula (I), A represents a nitrogen atom or CH. Thus, when A represents a nitrogen atom, it represents a pyrrolo[3,2-d]pyrimidine derivative represented by Formula (Ig): 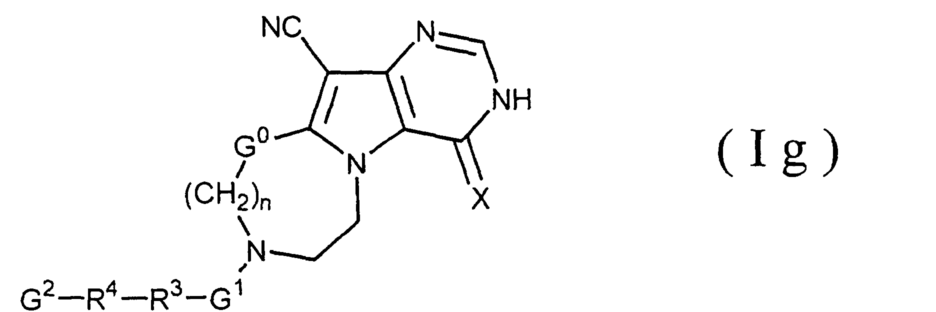 [in Formula (Ig), n, R³, R⁴, G⁰, G¹, G² and X are as defined for Formula (I)], and when A represents CH, it represents a pyrrolo[3,2-d]pyrimidine derivative represented by the following Formula (Ih): 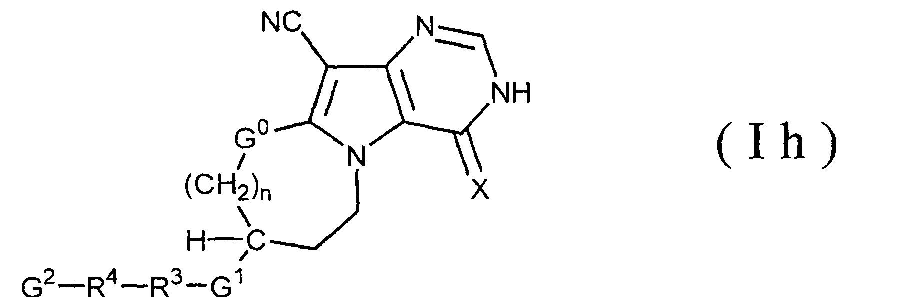 [in Formula (Ih), n, R³, R⁴, G⁰, G¹, G² and X are as defined for Formula (I)]. Preferred A is a nitrogen atom.

In the above Formula (I), G¹ represents a single bond, or a group that binds A bound to G¹ and R³ in the form of A-C(=O)-O-R³, A-C(=O)-R³, A-C(=O)-NR³⁰-R³, A-C(=S)-NR³¹-R³, A-C(=O)-NR³²-S(=O)₂-R³, or A-S(=O)₂-R³ (R³⁰ to R³² represent, independently from one another, a hydrogen atom or a substituted or unsubstituted aliphatic hydrocarbon group having one to four carbons).

When A and R³ to which G¹ binds are bound in the form of A-C(=O)-NR³⁰-R³ (R³⁰ represents a hydrogen atom or a substituted or unsubstituted aliphatic hydrocarbon group having one to four carbons), examples of an aliphatic hydrocarbon group having one to four carbons of R³⁰ include methyl, ethyl, propyl, isopropyl, butyl, isobutyl, s-butyl, t-butyl, 2-propenyl, 2-butenyl, 3-butenyl, 2-propynyl, 2-butynyl, and 3-butynyl. An aliphatic hydrocarbon group having one to four carbons of R³⁰ may be substituted with one or more substituents selected from the group consisting of a fluorine atom, a chlorine atom, a bromine atom, an iodine atom, a hydroxy group, a methoxy group, an ethoxy group, an oxo group, a cyano group, a carboxyl group, a carbamoyl group, an amino group, a sulpho group, and a phenyl group. As preferred examples of such R³⁰, there can be mentioned a hydrogen atom, a methyl, an ethyl, and a propyl group, with a hydrogen atom being most preferred.

When A and R³ to which G¹ binds are bound in the form of A-C(=S)-NR³¹-R³ (R³¹ represents a hydrogen atom or a substituted or unsubstituted aliphatic hydrocarbon group having one to four carbons), examples of an aliphatic hydrocarbon group having one to four carbons or R³¹ include the same ones as described for the above examples of R³⁰. As preferred examples of such R³¹, there can be mentioned a hydrogen atom, a methyl, an ethyl, and a propyl group, with a hydrogen atom being most preferred.

When A and R³ to which G¹ binds are bound in the form of A-C(=O)-NR³²-S(=O)₂-R³ (R³² represents a hydrogen atom or a substituted or unsubstituted aliphatic hydrocarbon group having one to four carbons), examples of an aliphatic hydrocarbon group having one to four carbons of R³² include the same ones as described for the above examples of R³⁰. As preferred examples of such R³², there can be mentioned a hydrogen atom, a methyl, an ethyl, and a propyl group, with a hydrogen atom being most preferred.

As preferred examples of such G¹, there can be mentioned a single bond, or a group that binds A and R³ to which G¹ binds in the form of A-C(=O)-R³, A-C(=O)-NH-R³, or A-C(=S)-NH-R³.

In above Formula (I), R³ represents a group selected from the following 1)-5).
1) a single bond,
2) a substituted or unsubstituted alicyclic hydrocarbon group having three to eight carbons (substituents are one or more substituents selected from the group consisting of a fluorine atom, a chlorine atom, a bromine atom, an iodine atom, a hydroxy group, an optionally substituted alkoxy group having one to seven carbons, an aryloxy group having six to ten carbons, an aralkoxy group having seven to nine carbons, an acyloxy group having two to seven carbons, an oxo group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, and an optionally substituted aliphatic hydrocarbon group having one to six carbons),
3) a substituted or unsubstituted aromatic hydrocarbon group having six to 14 carbons (substituents are one or more substituents selected from the group consisting of a fluorine atom, a chlorine atom, a bromine atom, an iodine atom, a hydroxy group, an optionally substituted alkoxy group having one to seven carbons, an aryloxy group having six to ten carbons, an aralkoxy group having seven to nine carbons, an acyloxy group having two to seven carbons, an oxo group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, and an optionally substituted aliphatic hydrocarbon group having one to six carbons),
4) a substituted or unsubstituted heterocyclic group containing, in the ring, one to four atoms selected from the group consisting of an oxygen atom, a nitrogen atom, and a sulfur atom (substituents are one or more substituents selected from the group consisting of a fluorine atom, a chlorine atom, a bromine atom, an iodine atom, a hydroxy group, an optionally substituted alkoxy group having one to seven carbons, an aryloxy group having six to ten carbons, an aralkoxy group having seven to nine carbons, an acyloxy group having two to seven carbons, an oxo group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, and an optionally substituted aliphatic hydrocarbon group having one to six carbons),
5) a substituted or unsubstituted aliphatic hydrocarbon group having one to ten carbons (substituents are one or more substituents selected from the group consisting of a fluorine atom, a chlorine atom, a bromine atom, an iodine atom, a hydroxy group, an optionally substituted alkoxy group having one to seven carbons, an optionally substituted phenylalkoxy group having seven to ten carbons, an alkoxy group having one to four carbons substituted with an optionally substituted heterocyclic group (containing, in the ring, one to four atoms selected from the group consisting of an oxygen atom, a nitrogen atom, and a sulfur atom), an optionally substituted aryloxy group having six to ten carbons, an acyloxy group having two to seven carbons, an oxo group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, an optionally substituted aromatic hydrocarbon group having six to 14 carbons, and an optionally substituted heterocyclic group (containing, in the ring, one to four atoms selected from the group consisting of an oxygen atom, a nitrogen atom, and a sulfur atom)).

In the above Formula (I), when R³ represents a substituted or unsubstituted alicyclic hydrocarbon group having three to eight carbons, examples of an alicyclic hydrocarbon group having three to eight carbons include cyclopropane, cyclobutane, cyclopentane, cyclopentene, cyclohexane, cyclohexene, cycloheptane, cycloheptene, cyclooctane, bicyclo[2.2.1]heptane, bicyclo[2.2.1]heptene, bicyclo[3.1.1]heptane, and bicycle[2.2.2]octane. As preferred examples of such an alicyclic hydrocarbon group having three to eight carbons, there can be mentioned an alicyclic hydrocarbon group having five to eight carbons such as cyclopentane, cyclopentene, cyclohexane, cyclohexene, cycloheptane, cycloheptene, and cyclooctane, with cyclopentane and cyclohexane being most preferred.

As a substituent comprising an alicyclic hydrocarbon group having three to eight carbons for substitution of R³, there can be mentioned a fluorine atom, a chlorine atom, a bromine atom, an iodine atom, a hydroxy group, an alkoxy group having one to seven carbons comprising a linear or branched alkyl group or a cycloalkyl group and an oxy group such as methoxy, ethoxy, propoxy, isopropoxy, butoxy, isobuboxy, s-butoxy, t-butoxy, pentyloxy, isopentyloxy, neopentyloxy, t-pentyloxy, hexyloxy, isohexyloxy, 2-methylpentyloxy, 1-ethylbutoxy, cyclopropyloxy, cyclobutyloxy, cyclopentyloxy, cyclohexyloxy, cycloproylmethyloxy, cycloproylethyloxy, cyclopentylmethyloxy, and cyclohexylmethyloxy; an aryloxy group having six to ten carbons such as phenoxy, 1-naphthoxy, and 2-naphthoxy; an aralkoxy group having seven to nine carbons such as benzyloxy, α-phenethyloxy, β-phenethyloxy, and 3-phenylpropyloxy; an acyloxy group having two to seven carbons such as acetoxy, propionyloxy, butylyloxy, isobutylyloxy, valeryloxy, isovaleryloxy, pivaloyloxy, and hexanoyloxy; an oxo group; an alkylsulfonyloxy group having one to six carbons comprising a linear or branched alkyl group and a sulfonyloxy group such as methylsulfonyloxy, ethylsulfonyloxy, propylsulfonyloxy, butylsulfonyloxy, and t-butylsulfonyloxy; an acyl group having two to seven carbons such as acetyl, propionyl, butylyl, isobutylyl, valeryl, isovaleryl, pivaloyl, and hexanoyl; a carboxyl group; an alkoxycarbonyl group comprising a linear or branched alkyl group and an oxycarbonyl group such as methoxycarbonyl, ethoxycarbonyl, propoxycarbonyl, isopropoxycarbonyl, butoxycarbonyl, isobutoxycarbonyl, s-butoxycarbonyl, and t-butoxycarbonyl; a carbamoyl group; an alkylcarbamoyl group having two to seven carbons comprising a linear or branched alkyl group or a cycloalkyl group and a carbamoyl group such as N-methylcarbamoyl, N-ethylcarbamoyl, N-propylcarbamoyl, N-isopropylcarbamoyl, N-butylcarbamoyl, N-isobutylcarbamoyl, N-s-butylcarbamoyl, N-t-butylcarbamoyl, N-pentylcarbamoyl, N-cyclopropylcarbamoyl, N-cyclobutylcarbamoyl, N-cyclopentylcarbamoyl, N-cyclohexylcarbamoyl, N-cycloheptylcarbamoyl, N-cyclopropylmethylcarbamoyl, N,N-dimecarbamoyl, N-ethyl-N-methylcarbamoyl, N,N-diethylcarbamoyl, and N,N-dipropylcarbamoyl; an amino group; an alkylamino group having one to six carbons comprising a linear or branched alkyl group or a cycloalkyl group and an amino group such as methylamino, ethylamino, propylamino, isopropylamino, butylamino, isobutylamino, s-butylamino, t-butylamino, pentylamino, hexylamino, cyclopropylamino, cyclobutylamino, cyclopentylamino, cyclohexylamino, cyclopropylmethylamino, dimethylamino, N-ethylmethylamino, diethylamino, N-methylpropylamino, N-methylisopropylamino, N-methylbutylamino, N-methyl-t-butylamino, N-ethylisopropylamino, dipropylamino, diisopropylamino, and ethylbutylamino; an acylamino group having two to seven carbons such as acetylamino, propionylamino, butylylamino, isobutylylamino, valerylamino, and hexanoylamino; an alkoxycarbonylamino group having two to eight carbons such as methoxycarbonylamino, ethoxycarbonylamino, and t-butoxycarbonylamino; an alkylsulfonylamino group having one to six carbons such as methylsulfonylamino, ethylsulfonylamino, butylsulfonylamino, and t-butylsulfonylamino; a cyano group; a nitro group; an alkylthio group having one to six carbons such as methylthio, ethylthio, propylthio, isopropylthio, butylthio, isobutylthio, s-butylthio, t-butylthio, pentylthio, and hexylthio; an alkylsulfinyl group having one to six carbons comprising a linear or branched alkyl group or a cycloalkyl group and a sulfinyl group such as methylsulfinyl, ethylsulfinyl, propylsulfinyl, isopropylsulfinyl, butylsulfinyl, isobutylsulfinyl, s-butylsulfinyl, t-butylsulfinyl, pentylsulfinyl, and cyclopentylsulfinyl; an alkylsulfonyl group having one to six carbons comprising a linear or branched alkyl group or a cycloalkyl group and a sulfonyl group such as methylsulfonyl, ethylsulfonyl, propylsulfonyl, isopropylsulfonyl, butylsulfonyl, isobutylsulfonyl, s-butylsulfonyl, t-butylsulfonyl, pentylsulfonyl, hexylsulfonyl, cyclopentylsulfonyl, and cyclohexylsulfonyl; a sulpho group; a sulfamoyl group; an aminosulfonyl group having one to six carbons comprising a linear or branched alkyl group or a cycloalkyl group and an aminosulfonyl group such as methylaminosulfonyl, ethylaminosulfonyl, propylaminosulfonyl, isopropylaminosulfonyl, butylaminosulfonyl, isobutylaminosulfonyl, s-butylaminosulfonyl, pentylaminosulfonyl, dimethylaminosulfonyl, N-ethyl-N-methylaminosulfonyl, diethylaminosulfonyl, dipropylaminosulfonyl, cyclopropylaminosulfonyl, cyclopentylaminosulfonyl, cyclohexylaminosulfonyl, and cyclopropylmethylaminosulfonyl; an alicyclic hydrocarbon group having three to six carbons such as cyclopropyl, cyclobutyl, cyclopentyl, and cyclohexyl; and a linear or branched aliphatic hydrocarbon group having one to six carbons that may contain an unsaturated bond such as methyl, ethyl, vinyl, ethynyl, propyl, 1-propenyl, 2-propenyl, isopropyl, isopropenyl, 1-propinyl, 2-propinyl, butyl, isobutyl, s-butyl, t-butyl, 1-butenyl, 2-butenyl, 3-butenyl, 1-methyl-1-propenyl, 2-methyl-1-propenyl, 1-methyl-2-propenyl, 2-methyl-2-propenyl, 1-butynyl, 2-butynyl, pentyl, isopentyl, neopentyl, t-pentyl, 1-pentenyl, 2-pentenyl, 3-pentenyl, 4-pentenyl, hexyl, 5-hexenyl, 4-methyl-3-pentenyl, isohexyl, 2-methylpentyl, and 1-ethylbutyl.

An alkyl group according to the present invention including the definition of substituents of an alicyclic hydrocarbon group having three to eight carbons for substitution of the above R³ represents, for example, a linear or branched saturated aliphatic hydrocarbon group such as methyl, ethyl, propyl, isopropyl, butyl, pentyl, hexyl, heptyl, octyl, isopropyl, isobutyl, s-butyl, t-butyl, isopentyl, neopentyl, t-pentyl, and isohexyl. A cycloalkyl group according to the present invention including the definition of substituents of an alicyclic hydrocarbon group having three to eight carbons for substitution of the above R³ represents, for example, a saturated alicyclic hydrocarbon group such as cyclopropyl, cyclobutyl, and cyclohexyl.

As a substituent of an alicyclic hydrocarbon group having three to eight carbons for substitution of said R³, an alkoxy group having one to seven carbons, an acyl group having two to seven carbons, an alkylcarbamoyl group having two to seven carbons, an alkylamino group having one to six carbons, an acylamino group having two to seven carbons, an alicyclic hydrocarbon group having three to six carbons, and an aliphatic hydrocarbon group having one to six carbons may further be substituted with (one or more substituents selected from the group consisting of a fluorine atom, a chlorine atom, a bromine atom, an iodine atom, a hydroxy group, an alkoxy group having one to six carbons such as methoxy, ethoxy, propoxy, isopropoxy, butoxy, isobutoxy, s-butoxy, t-butoxy, pentyloxy, and cyclopropyloxy, a methoxymethyloxy group, a 2-methoxyethoxy group, a formyl group, a trifluoroacetyl group, an acyl group having two to seven carbons such as acetyl, propionyl, butylyl, isobutylyl, valeryl, and isovaleryl, an oxo group, a carboxyl group, an alkoxycarbonyl group having two to seven carbons such as methoxycarbonyl, ethoxycarbonyl, propoxycarbonyl, isopropoxycarbonyl, butoxycarbonyl, isobutoxycarbonyl, and t-butoxycarbonyl, a carbamoyl group, an alkylcarbamoyl group having two to seven carbons such as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropylcarbamoyl, N-butylcarbamoyl, N-cyclopropylcarbamoyl, and N-cyclopropylmethylcarbamoyl, an amino group, an alkylamino group having one to six carbons such as methylamino, ethylamino, propylamino, isopropylamino, dimethylamino, N-ethylmethylamino, diethylamino, N-methylpropylamino, N-methylisopropylamino, cyclopropylamino, and cyclopropylmethylamino, a cyclic amino group having four to six carbons containing, in the ring, one to two atoms selected from the group consisting of an oxygen atom, a nitrogen atom, and a sulfur atom, such as 1-pyrrolidinyl, piperadinyl, 4-methylpiperadinyl, piperidino, and morpholino, a trifluoroacetylamino group, an acylamino group having one to seven carbons such as formylamino, acetylamino, propionylamino, butylylamino, isobutylylamino, and valerylamino, an alkylsulfonylamino group having one to six carbons such as methylsulfonylamino, ethylsulfonylamino, propylsulfonylamino, and butylylsulfonylamino, a nitro group, and a cyano group).

In the above Formula (I), when R³ represents a substituted or unsubstituted aromatic hydrocarbon group having six to 14 carbons, examples of an aromatic hydrocarbon group having six to 14 carbons include a divalent group containing, in the ring, at least one aromatic ring such as benzene, indene, indane, naphthalene, 1,2-dihydronaphthalene, 1,2,3,4-tetrahydronaphthalene, azulene, acenaphthylene, acenaphthene, fluorene, phenanthrene, and anthracene. Preferred examples of an aromatic hydrocarbon group having six to 14 carbons of R³ include an aromatic hydrocarbon group having six to ten carbons such as benzene, indene, indane, naphthalene, 1,2-dihydronaphthalene, and 1,2,3,4-tetrahydronaphthalene, and a further preferred example is a divalent group of benzene, with 1,3-phenylene and 1,4-phenylene being most preferred.

As a substituent of an aromatic hydrocarbon group having six to 14 carbons for substitution of R³, there can be mentioned a fluorine atom, a chlorine atom, a bromine atom, an iodine atom, a hydroxy group, an optionally substituted alkoxy group having one to seven carbons, an aryloxy group having six to ten carbons, an aralkoxy group having seven to nine carbons, an acyloxy group having two to seven carbons, an oxy group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, and an optionally substituted aliphatic hydrocarbon group having one to six carbons.

The definition of a substituent of an aromatic hydrocarbon group having six to 14 carbons for substitution of R³ is the same as for a substituent of an alicyclic hydrocarbon group having three to eight carbons for substitution of the above R³. Specific examples of a substituent of an aromatic hydrocarbon group having six to 14 carbons for substitution of said R³ include the same one as that described as specific examples of a substituent of an alicyclic hydrocarbon group having three to eight carbons for substitution of the above R³.

Preferred examples of a substituent of an aromatic hydrocarbon group having six to 14 carbons for substitution of R³ include a fluorine atom, a chlorine atom, a bromine atom; an alkoxy group having one to six carbons comprising a linear or branched alkyl group and an oxy group such as methoxy, ethoxy, propoxy, isopropoxy, butoxy, isobutoxy, s-butoxy, t-butoxy, pentyloxy, isopentyloxy, neopentyloxy, t-pentyloxy, and hexyloxy; a cyano group; a nitro group; a carboxyl group; a hydroxy group; an amino group; a mono- or di-alkylamino group comprising a linear or branched alkyl and an amino group such as methylamino, ethylamino, propylamino, isopropylamino, butylamino, isobutylamino, s-butylamino, t-butylamino, pentylamino, hexylamino, dimethylamino, N-ethylmethylamino, diethylamino, N-methylpropylamino, N-methylisopropylamino, N-methylbutylamino, N-methyl-t-butylamino, N-ethylisopropylamino, dipropylamino, diisopropylamino, and ethylbutylamino; a carbamoyl group; an aminosulfonyl group; an alicyclic hydrocarbon group having three to six carbons such as cyclopropyl, cyclobutyl, cyclopentyl, and cyclohexyl; an acyl group having two to seven carbons such as acetyl, propionyl, butylyl, isobutylyl, pivaloyl, and hexanoyl; an alkylthio group having one to six carbons such as methylthio, ethylthio, propylthio, isopropylthio, butylthio, isobutylthio, s-butylthio, t-butylthio, pentylthio, and hexylthio; an alkylsulfonyl group having one to six carbons such as methylsulfonyl, ethylsulfonyl, propylsulfonyl, isopropylsulfonyl, butylsulfonyl, isobutylsulfonyl, s-butylsulfonyl, t-butylsulfonyl, pentylsulfonyl, and hexylsulfonyl; an alkoxycarbonyl group having two to seven carbons such as acetoxy, propionyloxy, butylyloxy, isobutylyloxy, valeryloxy, isovaleryloxy, pivaloyloxy, and hexanoyloxy; an acylamino group having two to seven carbons such as acetylamino, propionylamino, butylylamino, isobutylylamino, valerylamino, and hexanoylamino; a trifluoromethyl group; and a trifluoromethoxy group; and a linear or branched aliphatic hydrocarbon group having one to six carbons that may contain an unsaturated bond such as methyl, ethyl, vinyl, ethynyl, propyl, 1-propenyl, 2-propenyl, isopropyl, isopropenyl, 1-propinyl, 2-propinyl, butyl, isobutyl, s-butyl, t-butyl, 1-butenyl, 2-butenyl, 3-butenyl, 1-methyl-1-propenyl, 2-methyl-1-propenyl, 1-methyl-2-propenyl, 2-methyl-2-propenyl, 1-butynyl, 2-butynyl, pentyl, isopentyl, neopentyl, t-pentyl, 1-pentenyl, 2-pentenyl, 3-pentenyl, 4-pentenyl, hexyl, 5-hexenyl, 4-methyl-3-pentenyl, isohexyl, 2-methylpentyl, and 1-ethylbutyl. Among them, more preferred examples of a substituent of an aromatic hydrocarbon group having six to 14 carbons for substitution of R³ include a fluorine atom, a chlorine atom, a bromine atom, an alkoxy group having one to six carbons, a cyano group, a nitro group, a carboxyl group, a hydroxy group, an amino group, a mono- or di-alkylamino group having one to six carbons, a carbamoyl group, an alicyclic hydrocarbon group having three to six carbons, an acyl group having two to seven carbons, an alkylsulfonyl group having one to six carbons, an alkoxycarboxyl group having two to seven carbons, an acylamino group having two to seven carbons, a trifluoromethyl group, a trifluoromethoxy group, and a saturated alkyl group having one to six carbons such as methyl, ethyl, propyl, isopropyl, butyl, isobutyl, s-butyl, t-butyl, pentyl, isopentyl, neopentyl, t-pentyl, hexyl, isohexyl, 2-methylpentyl, and 1-ethylbutyl.

An alkoxy group having one to seven carbons, an acyl group having two to seven carbons, an alkylcarbamoyl group having two to seven carbons, an alkylamino group having one to six carbons, an acylamino group having two to seven carbons, an alicyclic hydrocarbon group having three to six carbons, and an aliphatic hydrocarbon group having one to six carbons as a substituent of an aromatic hydrocarbon group having six to 14 carbons for substitution of said R³ may further be substituted with (one or more substituents selected from the group consisting of a fluorine atom, a chlorine atom, a bromine atom, an iodine atom, a hydroxy group, an alkoxy group having one to six carbons such as methoxy, ethoxy, propoxy, isopropoxy, butoxy, isobutoxy, s-butoxy, t-butoxy, pentyloxy, and cyclopropyloxy, a methoxymethyloxy group, a 2-methoxyethoxy group, a formyl group, a trifluoroacetyl group, an acyl group having two to seven carbons such as acetyl, propionyl, butylyl, isobutylyl, valeryl, and isovaleryl, an oxo group, a carboxyl group, an alkoxycarbonyl group having two to seven carbons such as methoxycarbonyl, ethoxycarbonyl, propoxycarbonyl, isopropoxycarbonyl, butoxycarbonyl, isobutoxycarbonyl, and t-butoxycarbonyl, a carbamoyl group, an alkylcarbamoyl group having two to seven carbons such as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropylcarbamoyl, N-butylcarbamoyl, N-cyclopropylcarbamoyl, and N-cyclopropylmethylcarbamoyl, an amino group, an alkylamino group having one to six carbons such as methylamino, ethylamino, propylamino, isopropylamino, dimethylamino, N-ethylmethylamino, diethylamino, N-methylpropylamino, N-methylisopropylamino, cyclopropylamino, and cyclopropylmethylamino, a cyclic amino group having four to six carbons containing, in the ring, one to two atoms selected from the group consisting of an oxygen atom, a nitrogen atom, and a sulfur atom, such as 1-pyrrolidinyl, piperadinyl, 4-methylpiperadinyl, piperidino, and morpholino, a trifluoroacetylamino group, an acylamino group having one to seven carbons such as formylamino, acetylamino, propionylamino, butylylamino, isobutylylamino, and valerylamino, an alkylsulfonylamino group having one to six carbons such as methylsulfonylamino, ethylsulfonylamino, propylsulfonylamino, and butylylsulfonylamino, a nitro group, and a cyano group).

In the above Formula (I), when R³ represents a substituted or unsubstituted heterocyclic group containing, in the ring, one to four atoms selected from the group consisting of an oxygen atom, a nitrogen atom, and a sulfur atom, examples of a heterocyclic group containing, in the ring, one to four atoms selected from the group consisting of an oxygen atom, a nitrogen atom, and a sulfur atom include a monocyclic, bicyclic or tricyclic divalent group such as furan, thiophene, pyrrole, pyrroline, pyrrolidine, oxazole, oxazolidine, isoxazole, isoxazolidine, thiazole, thiazolidine, isothiazole, isothiazolidine, furazane, imidazole, imidazoline, imidazolidine, pyrrazole, pyrrazoline, pyrrazolidine, triazole, thiadiazole, oxadiazole, tetrazole, pyran, tetrahydropyran, thiopyran, tetrahydrothiopyran, tetrahydrofuran, 1,3-dioxoran, 1,4-dioxane, pyridine, pirazine, pyrimidine, pyridadine, benzofuran, dibenzofuran, 1,4-dioxacycloheptane, benzothiophene, indole, 1,2-methylenedioxybenzene, benzimidazole, benzothiazole, benzoxazole, chroman, isochroman, quinoline, decahydroquinoline, isoquinoline, phthalazine, cinnoline, 1,8-naphthilidine, 1,2,3,4-tetrahydroisoquinoline, quinazoline, quinoxaline, purine, pteridine, azetidine, morpholine, thiomorpholine, piperidine, homopiperidine, piperadine, homopiperadine, indoline, isoindoline, phenoxadine, phenazine, phenothiazine, pyrrolopyrimidine, pyrazolopyrimidine, and quinuclidine.

As examples of a heterocyclic group of said R³ containing, in the ring, one to four atoms selected from the group consisting of an oxygen atom, a nitrogen atom, and a sulfur atom, there can be mentioned a monocyclic or bicyclic divalent group of an aromatic heterocycle having two to nine carbons containing, in the ring, one to three atoms selected from the group consisting of an oxygen atom, a nitrogen atom, and a sulfur atom, such as furan, pyrrole, thiophene, pyrrazole, oxazole, thiazole, isoxazole, isothiazole, pyrrazole, imidazole, pyridine, pyrimidine, pyradine, pyridadine, benzothiophene, benzofuran, 1,2-methylenedioxybenzene, benzimidazole, indole, quinoline, isoquinoline, quinazoline, purine, phthalazine, cinnoline, 1,8-naphthilidine, and pteridine.

As examples of a substituent of a heterocyclic group of R³ containing, in the ring, one to four atoms selected from the group consisting of an oxygen atom, a nitrogen atom, and a sulfur atom, there can be mentioned a fluorine atom, a chlorine atom, a bromine atom, an iodine atom, a hydroxy group, an optionally substituted alkoxy group having one to seven carbons, an aryloxy group having six to ten carbons, an aralkoxy group having seven to nine carbons, an acyloxy group having two to seven carbons, an oxo group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, and an optionally substituted aliphatic hydrocarbon group having one to six carbons.

The definition of a substituent of a heterocyclic group containing, in the ring for substitution of said R³, one to four atoms selected from the group consisting of an oxygen atom, a nitrogen atom, and a sulfur atom is the same as for a substituent of an alicyclic hydrocarbon group having three to eight carbons for substitution of the above R³. As specific examples of a substituent of a heterocyclic group containing, in the ring of said R³, one to four atoms selected from the group consisting of an oxygen atom, a nitrogen atom, and a sulfur atom, there can be mentioned the same one as that described as specific examples of a substituent of an alicyclic hydrocarbon group having three to eight carbons for substitution of the above R³.

As preferred examples of a substituent of a heterocyclic group of said R³ containing, in the ring, one to four atoms selected from the group consisting of an oxygen atom, a nitrogen atom, and a sulfur atom, there can be mentioned a fluorine atom, a chlorine atom, a bromine atom, an iodine atom, an alkoxy group having one to six carbons comprising a linear or branched alkyl group and an oxy group such as methoxy, ethoxy, propoxy, isopropoxy, butoxy, isobutoxy, s-butoxy, t-butoxy, pentyloxy, isopentyloxy, neopentyloxy, t-pentyloxy, and hexyloxy; a cyano group; a nitro group; a carboxyl group; a hydroxy group; an amino group; a mono- or di-alkylamino group comprising a linear or branched alkyl and an amino group such as methylamino, ethylamino, propylamino, isopropylamino, butylamino, isobutylamino, s-butylamino, t-butylamino, pentylamino, hexylamino, dimethylamino, N-ethylmethylamino, diethylamino, N-methylpropylamino, N-methylisopropylamino, N-methylbutylamino, N-methyl-t-butylamino, N-ethylisopropylamino, dipropylamino, diisopropylamino, and ethylbutylamino; a carbamoyl group; an aminosulfonyl group; an alicyclic hydrocarbon group having three to six carbons such as cyclopropyl, cyclobutyl, cyclopentyl, and cyclohexyl; an acyl group having two to seven carbons such as acetyl, propionyl, butylyl, isobutylyl, pivaloyl, and hexanoyl; an alkylthio group having one to six carbons such as methylthio, ethylthio, propylthio, isopropylthio, butylthio, isobutylthio, s-butylthio, t-butylthio, pentylthio, and hexylthio; an alkylsulfonyl group having one to six carbons such as methylsulfonyl, ethylsulfonyl, propylsulfonyl, isopropylsulfonyl, butylsulfonyl, isobutylsulfonyl, s-butylsulfonyl, t-butylsulfonyl, pentylsulfonyl, and hexylsulfonyl; an alkoxycarbonyl group having two to seven carbons such as acetoxy, propionyloxy, butylyloxy, isobutylyloxy, valeryloxy, isovaleryloxy, pivaloyloxy, and hexanoyloxy; an acylamino group having two to seven carbons s.uch as acetylamino, propionylamino, butylylamino, isobutylylamino, valerylamino, and hexanoylamino; a trifluoromethyl group; a trifluoromethoxy group; and a linear or branched aliphatic hydrocarbon group having one to six carbons that may contain an unsaturated bond such as methyl, ethyl, vinyl, ethynyl, propyl, 1-propenyl, 2-propenyl, isopropyl, isopropenyl, 1-propinyl, 2-propinyl, butyl, isobutyl, s-butyl, t-butyl, 1-butenyl, 2-butenyl, 3-butenyl, 1-methyl-1-propenyl, 2-methyl-1-propenyl, 1-methyl-2-propenyl, 2-methyl-2-propenyl, 1-butynyl, 2-butynyl, pentyl, isopentyl, neopentyl, t-pentyl, 1-pentenyl, 2-pentenyl, 3-pentenyl, 4-pentenyl, hexyl, 5-hexenyl, 4-methyl-3-pentenyl, isohexyl, 2-methylpentyl, and 1-ethylbutyl. Among them, as more preferred examples of a substituent of a heterocyclic group containing, in the ring of substitution, one to four atoms selected from the group consisting of an oxygen atom, a nitrogen atom, and a sulfur atom, there can be mentioned a fluorine atom, a chlorine atom, a bromine atom, an alkoxy group having one to six carbons, a cyano group, a nitro group, a carboxyl group, a hydroxy group, an amino group, a mono- or di-alkylamino group having one to six carbons, a carbamoyl group, an alicyclic hydrocarbon group having three to six carbons, an acyl group having two to seven carbons, an alkylsulfonyl group having one to six carbons, an alkoxycarboxyl group having two to seven carbons, a trifluoromethyl group, a trifluoromethoxy group, and a saturated alkyl group having one to six carbons such as methyl, ethyl, propyl, isopropyl, butyl, isobutyl, s-butyl, t-butyl, pentyl, isopentyl, neopentyl, t-pentyl, hexyl, isohexyl, 2-methylpentyl, and 1-ethylbutyl.

An alkoxy group having one to seven carbons, an acyl group having two to seven carbons, an alkylcarbamoyl group having two to seven carbons, an alkylamino group having one to six carbons, an acylamino group having two to seven carbons, an alicyclic hydrocarbon group having three to six carbons, and an aliphatic hydrocarbon group containing, in the ring of substitution of said R³, one to four atoms selected from the group consisting of an oxygen atom, a nitrogen atom, and a sulfur atom, may further be substituted with (one or more substituents selected from the group consisting of a fluorine atom, a chlorine atom, a bromine atom, an iodine atom, a hydroxy group, an alkoxy group having one to six carbons such as methoxy, ethoxy, propoxy, isopropoxy, butoxy, isobutoxy, s-butoxy, t-butoxy, pentyloxy, and cyclopropyloxy, a methoxymethyloxy group, a 2-methoxyethoxy group, a formyl group, a trifluoroacetyl group, an acyl group having two to seven carbons such as acetyl, propionyl, butylyl, isobutylyl, valeryl, and isovaleryl, an oxo group, a carboxyl group, an alkoxycarbonyl group having two to seven carbons such as methoxycarbonyl, ethoxycarbonyl, propoxycarbonyl, isopropoxycarbonyl, butoxycarbonyl, isobutoxycarbonyl, and t-butoxycarbonyl, a carbamoyl group, an alkylcarbamoyl group having two to seven carbons such as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropylcarbamoyl, N-butylcarbamoyl, N-cyclopropylcarbamoyl, and N-cyclopropylmethylcarbamoyl, an amino group, an alkylamino group having one to six carbons such as methylamino, ethylamino, propylamino, isopropylamino, dimethylamino, N-ethylmethylamino, diethylamino, N-methylpropylamino, N-methylisopropylamino, cyclopropylamino, and cyclopropylmethylamino, a cyclic amino group having four to six carbons containing, in the ring, one to two atoms selected from the group consisting of an oxygen atom, a nitrogen atom, and a sulfur atom, such as 1-pyrrolidinyl, piperadinyl, 4-methylpiperadinyl, piperidino, and morpholino, a trifluoroacetylamino group, an acylamino group having one to seven carbons such as formylamino, acetylamino, propionylamino, butylylamino, isobutylylamino, and valerylamino, an alkylsulfonylamino group having one to six carbons such as methylsulfonylamino, ethylsulfonylamino, propylsulfonylamino, and butylsulfonylamino, a nitro group, and a cyano group).

In the above Formula (I), when R³ represents a substituted or unsubstituted aliphatic hydrocarbon group having one to ten carbons, examples of an aliphatic hydrocarbon group having one to ten carbons of R³ include a divalent group of an alkane having one to four carbons such as methane, ethane, propane, isopropane, butane, isobutane, s-butane, and t-butane, an alkane having five to ten carbons such as pentane, isopentane, neopentane, t-pentane, 2-methylpentane, 4-methylpentane, 1-ethylbutane, hexane, heptane, 2-methylhexane, 5-methylhexane, 1,1-dimethylpentane, 6-methylheptane, octane, nonane, and decane; an alkene such as ethylene, propene, 2-methylpropene, 1-butene, 2-butene, 2-methylbutene, 1,3-butadiene, 1-pentene, 2-pentene, 4-methyl-1-pentene, 1-hexene, 2-hexene, 3-hexene, 1,5-hexadiene, 2-heptene, 2-octene, 2-nonene, and 2-decene; an alkyne such as acetylene, propyne, 1-butyne, 3-methyl-1-butyne, 3,3-dimethyl-1-butyne, 1-pentyne, 2-pentyne, 3-pentyne, 1-hexyne, 2-hexyne, 3-hexyne, 1-methyl-3-pentyne, 1-methyl-3-hexyne, 2-heptyne, 2-octyne, 2-nonyne, and 2-decyne. As preferred examples of an aliphatic hydrocarbon group having one to ten carbons of such R³, there can be mentioned a divalent group of an aliphatic hydrocarbon group having one to six carbons such as methane, ethane, propane, butane, pentane, hexane, ethylene, propene, 1-butene, acetylene, and propyne. Further preferred are methylene, 1,2-ethylene, and 1,3-propylene.

As a substituent of an aliphatic hydrocarbon group having one to ten carbons for substitution of R³, there can be mentioned a fluorine atom, a chlorine atom, a bromine atom, an iodine atom, a hydroxy group; an alkoxy group having one to seven carbons comprising a linear or branched alkyl group or a cycloalkyl group and an oxy group such as methoxy, ethoxy, propoxy, isopropoxy, butoxy, isobutoxy, s-butoxy, t-butoxy, pentyloxy, isopentyloxy, neopentyloxy, t-pentyloxy, hexyloxy, isohexyloxy, 2-methylpentyloxy, 1-ethylbutoxy, cyclopropyloxy, cyclobutyloxy, cyclopentyloxy, cyclohexyloxy, cyclopropylmethyloxy, cyclopropylethyloxy, cyclopentylmethyloxy, and cyclohexylmethyloxy; a phenylalkoxy group having seven to ten carbons such as benzyloxy, α-phenetyloxy, β-phenetyloxy, 3-phenylpropyloxy, 1-methyl-1-phenylethoxy, 1-methyl-2-phenyloxy, 2-methyl-2-phenylethoxy, 4-phenylbutoxy, 1-methyl-1-phenylpropyloxy, 2-methyl-1-phenylpropyloxy, 1-methyl-2-phenylpropyloxy, 1-methyl-3-phenylpropyloxy, and 2-methyl-3-phenylpropyloxy; an alkoxy group having one to four carbons substituted with a heterocyclic group (containing, in the ring, one to four atoms selected from the group consisting of an oxygen atom, a nitrogen atom, and a sulfur atom) such as 2-furylmethoxy, 2-(2-furyl)ethoxy, 3-(2-furyl)propoxy, 4-(2-furyl)butoxy, 3-furylmethoxy, 2-(3-furyl)ethoxy, 3-(3-furyl)propoxy, 4-(3-furyl)butoxy, 2-thienylmethoxy, 2-(2-thienyl)ethoxy, 3-(2-thienyl)propoxy, 4-(2-thienyl)butoxy, 3-thienylmethoxy, 2-(3-thienyl)ethoxy, 3-(3-thienyl)propoxy, 4-(3-thienyl)butoxy, 2-pyridylmethoxy, 2-(2-pyridyl)ethoxy, 3-pyridylmethoxy, 2-(3-pyridyl)ethoxy, 4-pyridylmethoxy, 2-(4-pyridyl)ethoxy, 2-indolylmethoxy, 3-indolylmethoxy, 2-benzofuranylmethoxy, 3-benzofuranylmethoxy, 2-thiazolylmethoxy, 4-thizolylmethoxy, 5-thizolylmethoxy, 2-oxazolylmethoxy, 4-oxazolylmethoxy, 5-oxazolylmethoxy, 3-isoxazolylmethoxy, 2-imidazolylmethoxy, 4-imidazolylmethoxy, and 5-tetrazolylmethoxy; an aryloxy group having six to ten carbons such as phenoxy, 1-naphthoxy, and 2-naphthoxy; an acyloxy group having two to seven carbons such as acetoxy, propionyloxy, butylyloxy, isobutylyloxy, valeryloxy, isovaleryloxy, pivaloyloxy, and hexanoyloxy; an oxy group; an alkylsulfonyloxy group having one to six carbons comprising a linear or branched alkyl group and a sulfonyloxy group such as methylsulfonyloxy, ethylsulfonyloxy, propylsulfonyloxy, butylsulfonyloxy, and t-butylsulfonyloxy; an acyl group having two to seven carbons such as acetyl, propionyl, butylyl, isobutylyl, valeryl, isovaleryl, pivaloyl, and hexanoyl; a carboxyl group; an alkoxycarbonyl group having two to seven carbons comprising a linear or branched alkyl group and an oxycarbonyl group such as methoxycarbonyl, ethoxycarbonyl, propoxycarbonyl, isopropoxycarbonyl, butoxycarbonyl, isobutoxycarbonyl, s-butoxycarbonyl, and t-butoxycarbonyl; a carbamoyl group; an alkylcarbamoyl group having two to seven carbons comprising a linear or branched alkyl group or a cycloalkyl group and a carbamoyl group such as N-methylcarbamoyl, N-ethylcarbamoyl, N-propylcarbamoyl, N-isopropylcarbamoyl, N-butylcarbamoyl, N-isobutylcarbamoyl, N-s-butylcarbamoyl, N-t-butylcarbamoyl, N-pentylcarbamoyl, N-cyclopropylcarbamoyl, N-cyclobutylcarbamoyl, N-cyclopentylcarbamoyl, N-cyclohexylcarbamoyl, N-cycloheptylcarbamoyl, N-cyclopropylmethylcarbamoyl, N,N-dimethylcarbamoyl, N-ethyl-N-methylcarbamoyl, N,N-diethylcarbamoyl, and N-dipropylcarbamoyl; an amino group; an alkylamino group having one to six carbons comprising an linear or branched alkyl group or a cycloalkyl group and an amino group such as methylamino, ethylamino, propylamino, isopropylamino, butylamino, isobutylamino, s-butylamino, t-butylamino, pentylamino, hexylamino, cyclopropylamino, cyclobutylamino, cyclopentylamino, cyclohexylamino, cyclopropylmethylamino, dimethylamino, N-ethylmethylamino, diethylamino, N-methylpropylamino, N-methylisopropylamino, N-methylbutylamino, N-methyl-t-butylamino, N-ethylisopropylamino, dipropylamino, diisopropylamino, and ethylbutylamino; an acylamino group having two to seven carbons such as acetylamino, propionylamino, butylylamino, isobutylylamino, valerylamino, and hexanoylamino; an alkoxycarbonylamino group having two to eight carbons such as methoxycarbonylamino, ethoxycarbonylamino, and t-butoxycarbonylamino; an alkylsulfonylamino group having one to six carbons such as methylsulfonylamino, ethylsulfonylamino, butylsulfonylamino, and t-butylsulfonylamino; a cyano group; a nitro group; an alkylthio group having one to six carbons such as methylthio, ethylthio, propylthio, isopropylthio, butylthio, isobutylthio, s-butylthio, t-butylthio, pentylthio, and hexylthio; an alkylsulfinyl group having one to six carbons comprising a linear or branched alkyl group or a cycloalkyl group and a sulfinyl group such as methylsulfinyl, ethylsulfinyl, propylsulfinyl, isopropylsulfinyl, butylsulfinyl, isobutylsulfinyl, s-butylsulfinyl, t-butylsulfinyl, pentylsulfinyl, and cyclopentylsulfinyl; an alkylsulfonyl group having one to six carbons comprising a linear or branched alkyl group or a cycloalkyl group and a sulfonyl group such as methylsulfonyl, ethylsulfonyl, propylsulfonyl, isopropylsulfonyl, butylsulfonyl, isobutylsulfonyl, s-butylsulfonyl, t-butylsulfonyl, pentylsulfonyl, hexylsulfonyl, cyclopentylsulfonyl, and cyclohexylsulfonyl; a sulpho group; a sulfamoyl group; an aminosulfonyl group having one to six carbons comprising a linear or branched alkyl group or a cycloalkyl group and an aminosulfonyl group such as methylaminosulfonyl, ethylaminosulfonyl, propylaminosulfonyl, isopropylaminosulfonyl, butylaminosulfonyl, isobutylaminosulfonyl, s-butylaminosulfonyl, pentylaminosulfonyl, dimethylaminosulfonyl, N-ethyl-N-methylaminosulfonyl, diethylaminosulfonyl, dipropylaminosulfonyl, cyclopropylaminosulfonyl, cyclopentylaminosulfonyl, cyclohexylaminosulfonyl, and cyclopropylmethylaminosulfonyl; an alicyclic hydrocarbon group having three to six carbons such as cyclopropyl, cyclobutyl, cyclopentyl, and cyclohexyl; an aromatic hydrocarbon group having six to 14 carbons which is a monovalent group of a monocyclic, bicyclic or tricyclic aromatic hydrocarbon such as benzene, naphthalene, indene, indane, 1,2,3,4-tetrahydronaphthalene, and fluorene; a heterocyclic group (containing, in the ring, one to four atoms selected from the group consisting of an oxygen atom, a nitrogen atom, and a sulfur atom) representing a monocyclic, bicyclic or tricyclic (containing, in the ring, one to four atoms selected from the group consisting of an oxygen atom, a nitrogen atom, and a sulfur atom) heterocyclic monovalent group such as furan, thiophene, pyrrole, pyrroline, pyrrolidine, oxazole, oxazolidine, isoxazole, isoxazolidine, thiazole, thiazolidine, isothiazole, isohiazolidine, imidazole, imidazoline, imidazolidine, pyrrazole, pyrrazoline, pyrrazolidine, triazole, thiadiazole, oxadiazole, tetrazole, pyran, tetrahydropyran, thiopyran, tetrahydrothiopyran, pyridine, pirazine, pyrimidine, pyridadine, benzofuran, dibenzofuran, benzothiophene, indole, benzimidazole, benzothiazole, benzoxazole, chroman, isochroman, quinoline, decahydroquinoline, isoquinoline, quinazoline, quinoxaline, purine, pteridine, azetidine, morpholine, thiomorpholine, piperidine, homopiperidine, piperadine, homopiperadine, indoline, isoindoline, phenoxadine, phenazine, phenothiazine, and quinuclidine.

As preferred examples of a substituent of an aliphatic hydrocarbon group having one to ten carbons for substitution as said R³, there can be mentioned a hydroxy group, an optionally substituted alkoxy group having one to seven carbons, an optionally substituted phenylalkoxy group having seven to ten carbons, an optionally substituted aryloxy group having six to ten carbons, an alkoxy having one to four carbons substituted with an optionally substituted heterocyclic group (containing, in the ring, one to four atoms selected from the group consisting of an oxygen atom, a nitrogen atom, and a sulfur atom), an oxo group, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkylsulfonylamino group having one to six carbons, a cyano group, an alkoxycarbonylamino group having two to eight carbons, an alkylthio group having one to six carbons, an alkylsulfonyl group having one to six carbons, a sulfamoyl group, an optionally substituted aromatic hydrocarbon group having six to 14 carbons, and an optionally substituted heterocyclic group (containing, in the ring, one to four atoms selected from the group consisting of an oxygen atom, a nitrogen atom, and a sulfur atom).

As preferred examples of a substituent of an aliphatic hydrocarbon group having one to ten carbons for substitution as said R³, there can be mentioned a hydroxy group, an optionally substituted alkoxy group having one to seven carbons, a carboxyl group, an amino group, an optionally substituted alkylamino group having one to six carbons, a cyano group, an alkoxycarbonylamino group having two to eight carbons, an acylamino group having two to seven carbons, an alkylthio group having one to six carbons, an optionally substituted aromatic hydrocarbon group having six to 14 carbons, and an optionally substituted heterocyclic group (containing, in the ring, one to four atoms selected from the group consisting of an oxygen atom, a nitrogen atom, and a sulfur atom).

A heterocyclic group (containing, in the ring, one to four atoms selected from the group consisting of an oxygen atom, a nitrogen atom, and a sulfur atom) as a substituent of an aliphatic hydrocarbon group having one to ten carbons for substitution as said R³ binds to an aliphatic hydrocarbon group having one to ten carbons as R³ on a carbon atom or a nitrogen atom.

As more preferred examples as R³ of a heterocyclic group (containing, in the ring, one to four atoms selected from the group consisting of an oxygen atom, a nitrogen atom, and a sulfur atom) that binds to an aliphatic hydrocarbon group having one to ten carbons on a carbon atom, there can be mentioned a monovalent group of a monocyclic or bicyclic aromatic hydrocarbon group having three to nine carbons containing, in the ring, one to two atoms selected from the group consisting of an oxygen atom, a nitrogen atom, and a sulfur atom, such as furan, pyrrole, thiophene, pyrrazole, oxazole, thiazole, isoxazole, isothiazole, pyrrazole, imidazole, pyridine, pyrimidine, pyradine, pyridadine, benzothiophene, benzofuran, 1,2-methylenedioxybenzene, benzimidazole, indole, quinoline, isoquinoline, and quinazoline.

As preferred examples as R³ of a heterocyclic group (containing, in the ring, one to four atoms selected from the group consisting of an oxygen atom, a nitrogen atom, and a sulfur atom) that binds to an aliphatic hydrocarbon group having one to ten carbons on a nitrogen atom, there can be mentioned a monovalent group of a monocyclic heterocyclic group having two to nine carbons containing, in the ring, one to two atoms selected from the group consisting of an oxygen atom, a nitrogen atom, and a sulfur atom, such as pyrrolidine, piperidine, morpholine, thiomorpholine, homopiperidine, homopiperadine, 1,2,3,6-tetrahydropyridine, or piperadine

An alkoxy having one to seven carbons, a phenylalkoxy group having seven to ten carbons, an aryloxy group having six to ten carbons, an alkoxy group having one to four carbons substituted with a heterocyclic group (containing, in the ring, one to four atoms selected from the group consisting of an oxygen atom, a nitrogen atom, and a sulfur atom), an acyl group having two to six carbons, an alkylcarbamoyl group having two to seven carbons, an alkylamino group having one to six carbons, an acylamino group having two to seven carbons, an alicyclic hydrocarbon group having three to six carbons, a aliphatic hydrocarbon group having one to six carbons, an aromatic hydrocarbon group having six to 14 carbons, and a heterocyclic group (containing, in the ring, one to four atoms selected from the group consisting of an oxygen atom, a nitrogen atom, and a sulfur atom) as a substituent of an aliphatic hydrocarbon group having one to ten carbons for substitution as said R, may further be substituted with (one or more substituents selected from the group consisting of a fluorine atom, a chlorine atom, a bromine atom, an iodine atom, a hydroxy group, an alkoxy group having one to six carbons such as methoxy, ethoxy, propoxy, isopropoxy, butoxy, isobutoxy, s-butoxy, t-butoxy, pentyloxy, and cyclopropyloxy, a methoxymethyloxy group, a 2-methoxyethoxy group, a formyl group, a trifluoroacetyl group, an acyl group having two to seven carbons such as acetyl, propionyl, butylyl, isobutylyl, valeryl, and isovaleryl, an oxo group, a carboxyl group, an alkoxycarbonyl group having two to seven carbons such as methoxycarbonyl, ethoxycarbonyl, propoxycarbonyl, isopropoxycarbonyl, butoxycarbonyl, isobutoxycarbonyl, and t-butoxycarbonyl, a carbamoyl group, an alkylcarbamoyl group having two to seven carbons such as N-methylcarbamoyl, N,N-dimethylcarbamoyl, N-ethylcarbamoyl, N-ethyl-N-methylcarbamoyl, N,N-diethylcarbamoyl, N-propylcarbamoyl, N-isopropylcarbamoyl, N-butylcarbamoyl, N-cyclopropylcarbamoyl, and N-cyclopropylmethylcarbamoyl, an amino group, an alkylamino group having one to six carbons such as methylamino, ethylamino, propylamino, isopropylamino, dimethylamino, N-ethylmethylamino, diethylamino, N-methylpropylamino, N-methylisopropylamino, cyclopropylamino, and cyclopropylmethylamino, a cyclic amino group having four to six carbons containing, in the ring, one to two atoms selected from the group consisting of an oxygen atom, a nitrogen atom, and a sulfur atom, such as 1-pyrrolidinyl, piperadinyl, 4-methylpiperadinyl, piperidino, and morpholino, a trifluoroacetylamino group, an acylamino group having one to seven carbons such as formylamino, acetylamino, propionylamino, butylylamino, isobutylylamino, and valerylamino, an alkylsulfonylamino group having one to six carbons such as methylsulfonylamino, ethylsulfonylamino, propylsulfonylamino, and butylylsulfonylamino, a nitro group, and a cyano group, an alkyl group having one to six carbons such as methyl, ethyl, propyl, isopropyl, butyl, isobutyl, s-butyl, and t-butyl, a trifluoromethyl group, and a trifluoromethoxy group).

When a substituent of an aliphatic hydrocarbon group having one to ten carbons for substitution as said R³ is an optionally substituted alkoxy group having one to seven carbons, an optionally substituted phenylalkoxy group having seven to ten carbons, an optionally substituted aryloxy group having six to ten carbons, and an alkoxy group having one to four carbons substituted with an optionally substituted heterocyclic group (containing, in the ring, one to four atoms selected from the group consisting of an oxygen atom, a nitrogen atom, and a sulfur atom), a preferred aliphatic hydrocarbon group having one to ten carbons of R³ is a divalent group of an alkane having two to six carbons such as ethane, propane, isopropane, butane, isobutane, s-butane, t-butane, pentane, isopentane, neopentane, t-pentane, 2-methylpentane, 4-methylpentane, 1-ethylbutane, and hexane. Furthermore, a divalent group of an alkane having two to four carbons such as ethane, propane, isopropane, butane, isobutane, s-butane, and t-butane are specifically preferred.

In the above Formula (I), R⁴ represents a group selected from the following 1)-4).
1) a single bond,
2) a substituted or unsubstituted alicyclic hydrocarbon group having three to eight carbons (substituents are one or more substituents selected from the group consisting of a fluorine atom, a chlorine atom, a bromine atom, an iodine atom, a hydroxy group, an optionally substituted alkoxy group having one to seven carbons, an aryloxy group having six to ten carbons, an aralkoxy group having seven to nine carbons, an acyloxy group having two to seven carbons, an oxo group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, and an optionally substituted aliphatic hydrocarbon group having one to six carbons),
3) a substituted or unsubstituted aromatic hydrocarbon group having six to 14 carbons (substituents are one or more substituents selected from the group consisting of a fluorine atom, a chlorine atom, a bromine atom, an iodine atom, a hydroxy group, an optionally substituted alkoxy group having one to seven carbons, an aryloxy group having six to ten carbons, an aralkoxy group having seven to nine carbons, an acyloxy group having two to seven carbons, an oxo group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, and an optionally substituted aliphatic hydrocarbon group having one to six carbons),
4) a substituted or unsubstituted heterocyclic group containing, in the ring, one to four atoms selected from the group consisting of an oxygen atom, a nitrogen atom, and a sulfur atom (substituents are one or more substituents selected from the group consisting of a fluorine atom, a chlorine atom, a bromine atom, an iodine atom, a hydroxy group, an optionally substituted alkoxy group having one to seven carbons, an aryloxy group having six to ten carbons, an aralkoxy group having seven to nine carbons, an acyloxy group having two to seven carbons, an oxo group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, and an optionally substituted aliphatic hydrocarbon group having one to six carbons).

In the above Formula (I), when R⁴ represents a substituted or unsubstituted alicyclic hydrocarbon group having three to eight carbons, as examples of such a substituted or unsubstituted alicyclic hydrocarbon group having three to eight carbons, there can be mentioned those that are the same as the one shown as an example of 2) a substituted or unsubstituted alicyclic hydrocarbon group having three to eight carbons of the above R³.

In the above Formula (I), when R⁴ represents a substituted or unsubstituted aromatic hydrocarbon group having six to 14 carbons, examples of such a substituted or unsubstituted aromatic hydrocarbon group having six to 14 carbons include those that are the same as the one shown as an example of 3) a substituted or unsubstituted aromatic hydrocarbon group having six to 14 carbons in the above R³. As examples of such an unsubstituted aromatic hydrocarbon group having six to 14 carbons, there can be mentioned a divalent group of benzene, with 1,2-phenylene being most preferred. As a substituent of an aromatic hydrocarbon group having six to 14 carbons for substitution, a fluorine atom, a hydroxy group, a methoxy group, a methylenedioxy group, a carboxyl group, a cyano group, and a nitro group are specifically preferred.

In the above Formula (I), when R⁴ represents a heterocyclic group containing, in the ring, one to four atoms selected from the group consisting of an oxygen atom, a nitrogen atom, and a sulfur atom, examples of such a substituted or unsubstituted heterocyclic group containing, in the ring, one to four atoms selected from the group consisting of an oxygen atom, a nitrogen atom, and a sulfur atom include those that are the same as the one shown as an example of 4) a substituted or unsubstituted heterocyclic group containing, in the ring, one to four atoms selected from the group consisting of an oxygen atom, a nitrogen atom, and a sulfur atom, of the above R³.

In the above Formula (I), G² represents any of a hydrogen atom, -C(=O)-OH, -C(=O)-NH-OH, -S(=O)₂-OH, and a 5-tetrazolyl group. As preferred ones of such G², there can be mentioned a hydrogen atom, -C(=O)-OH and -C(=O)-NH-OH, with -C(=O)-OH being most preferred.

In Formula (I) according to the present invention, preferred combinations of G²-R⁴-R³- are shown in Chemical formula 1 to Chemical formula 11. In the structures of Chemical formula 1 to Chemical formula 11, the symbol "-----" represents a binding site of G²-R⁴-R³- and G¹.

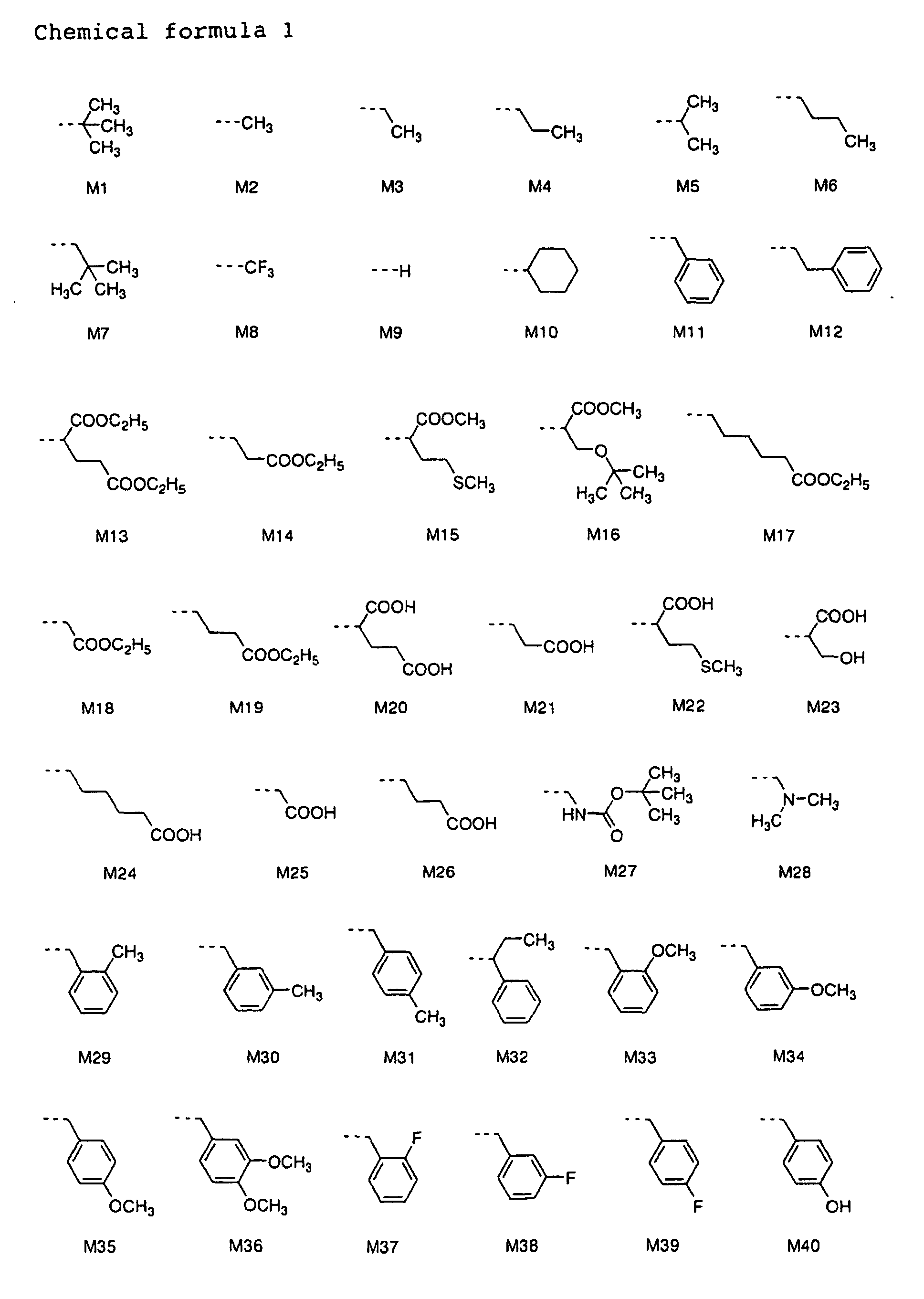

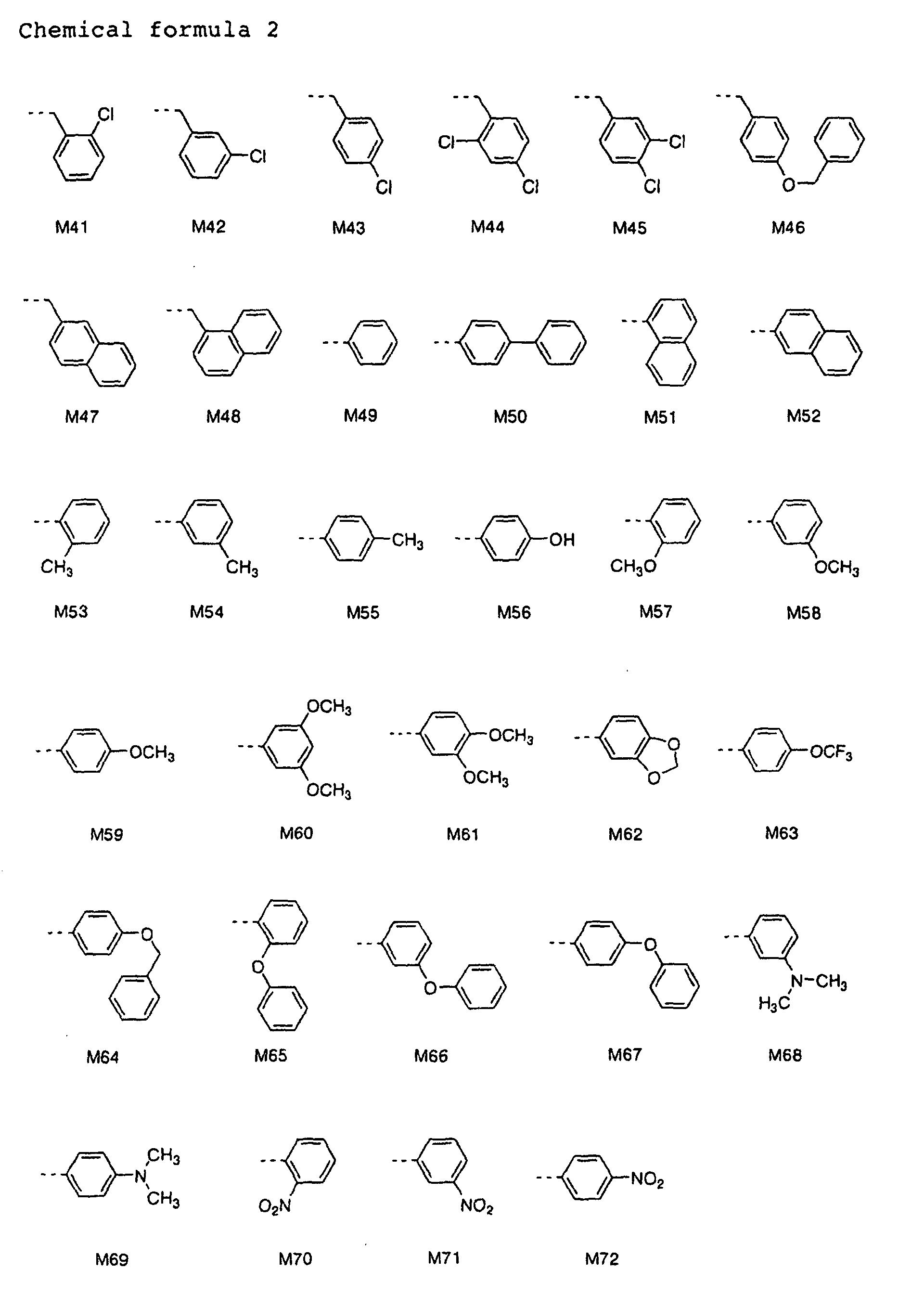

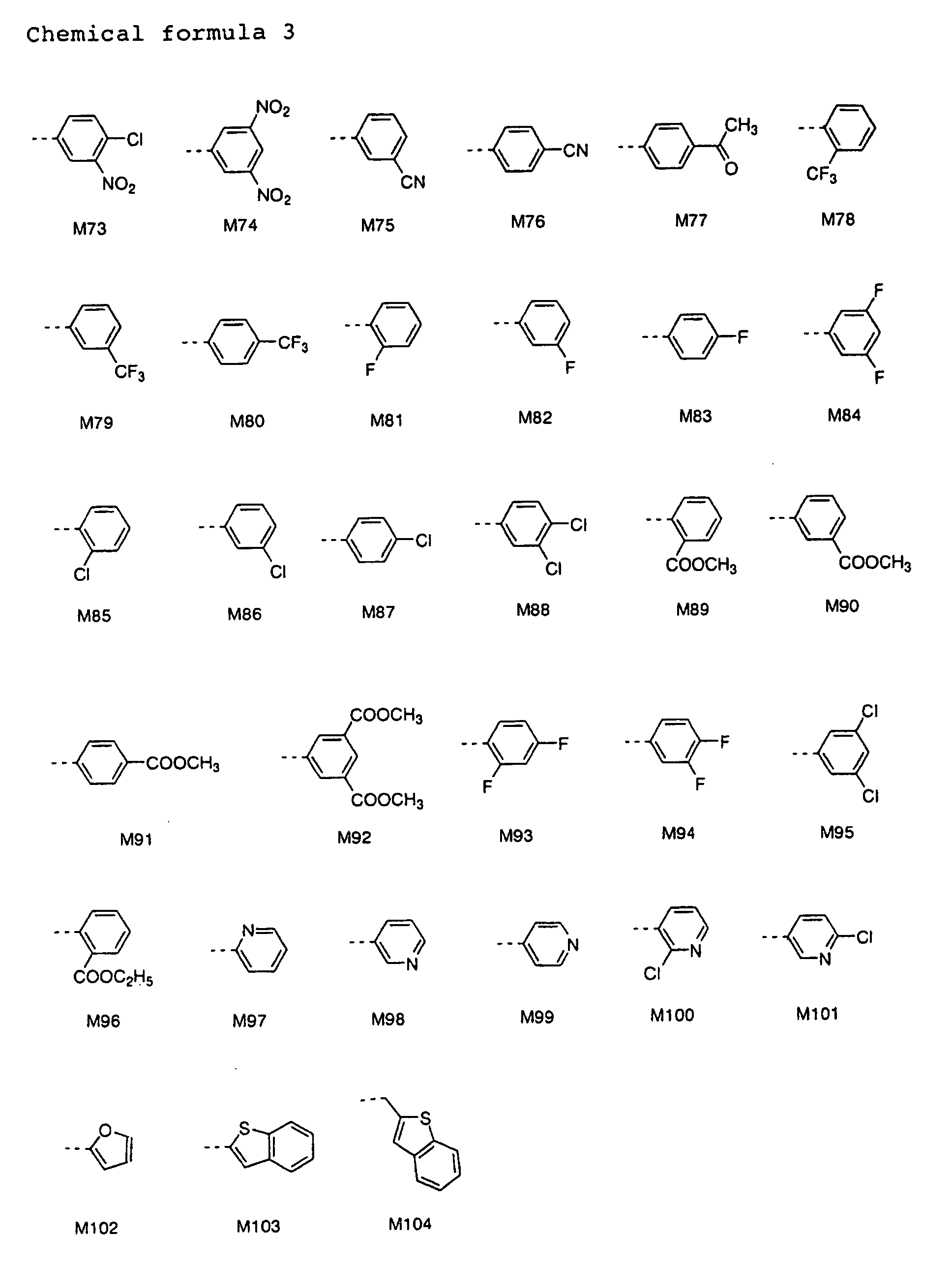

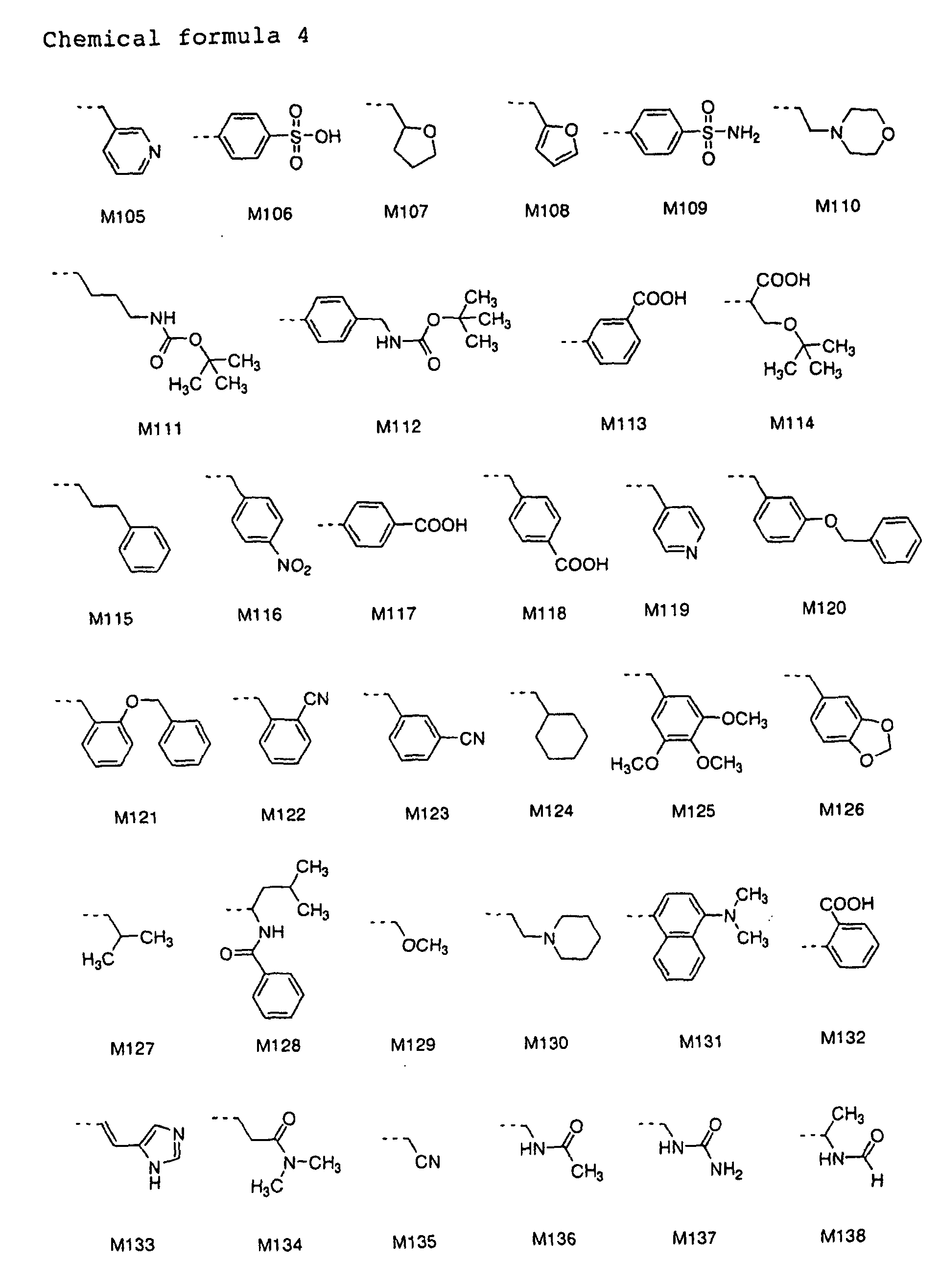

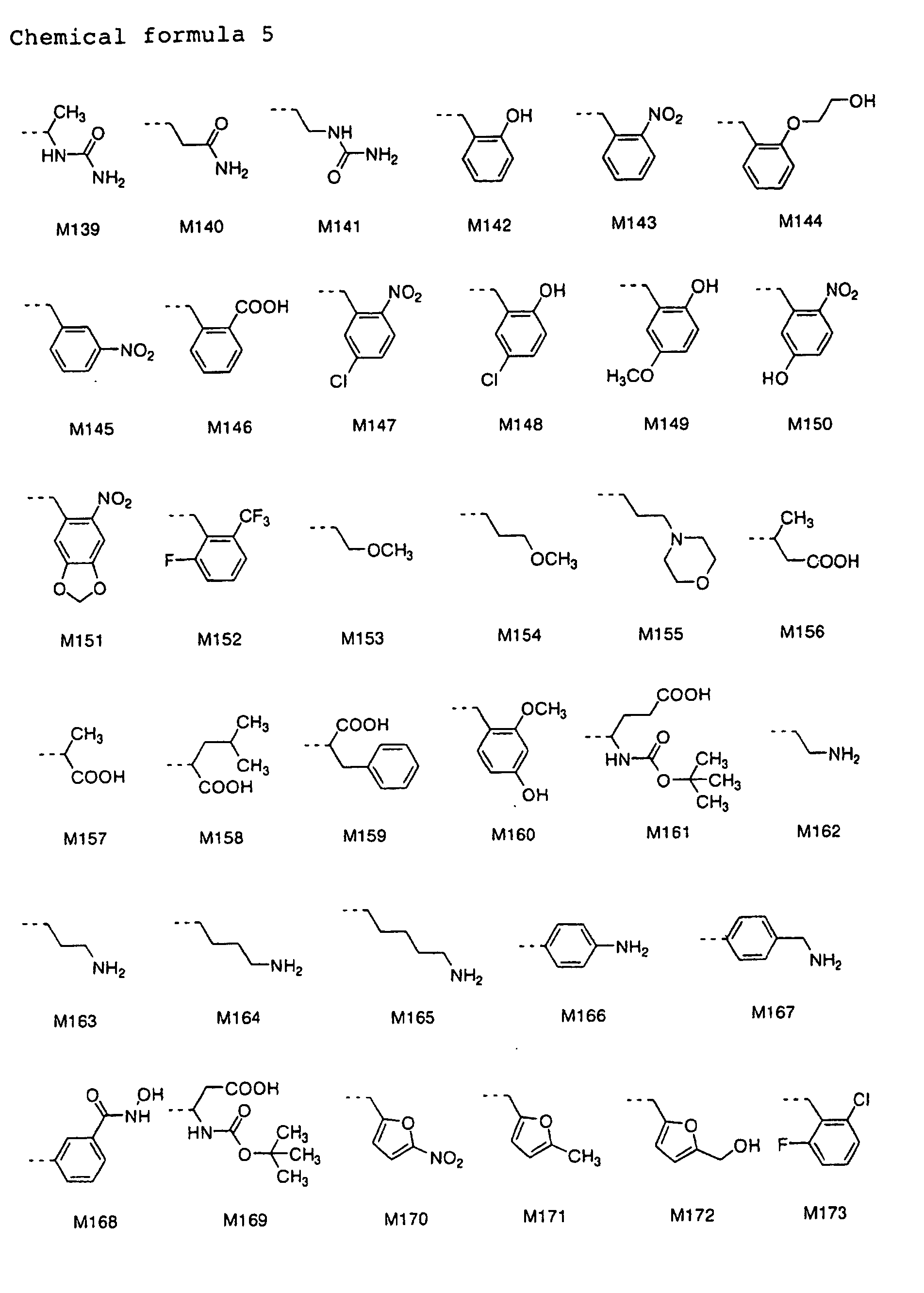

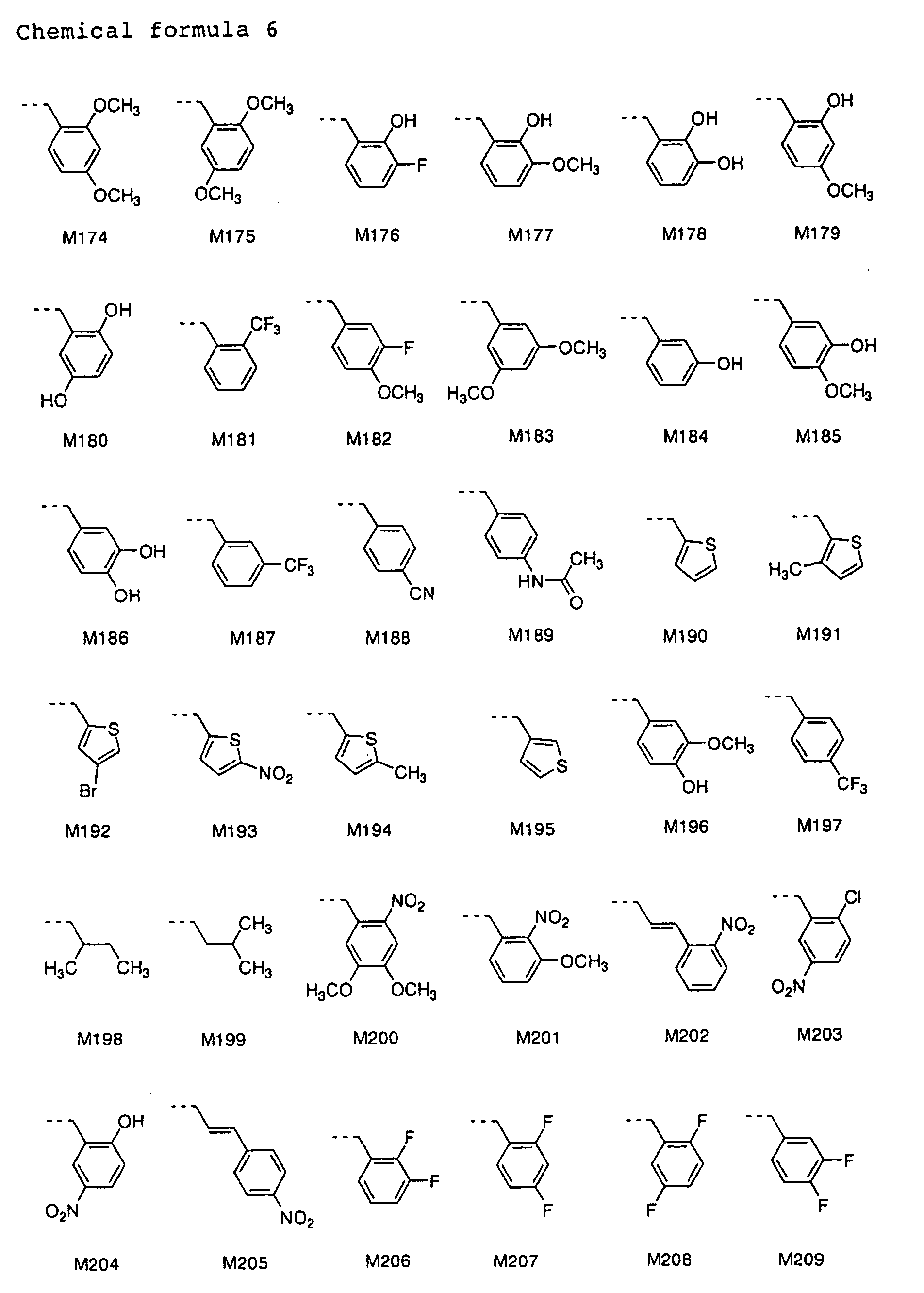

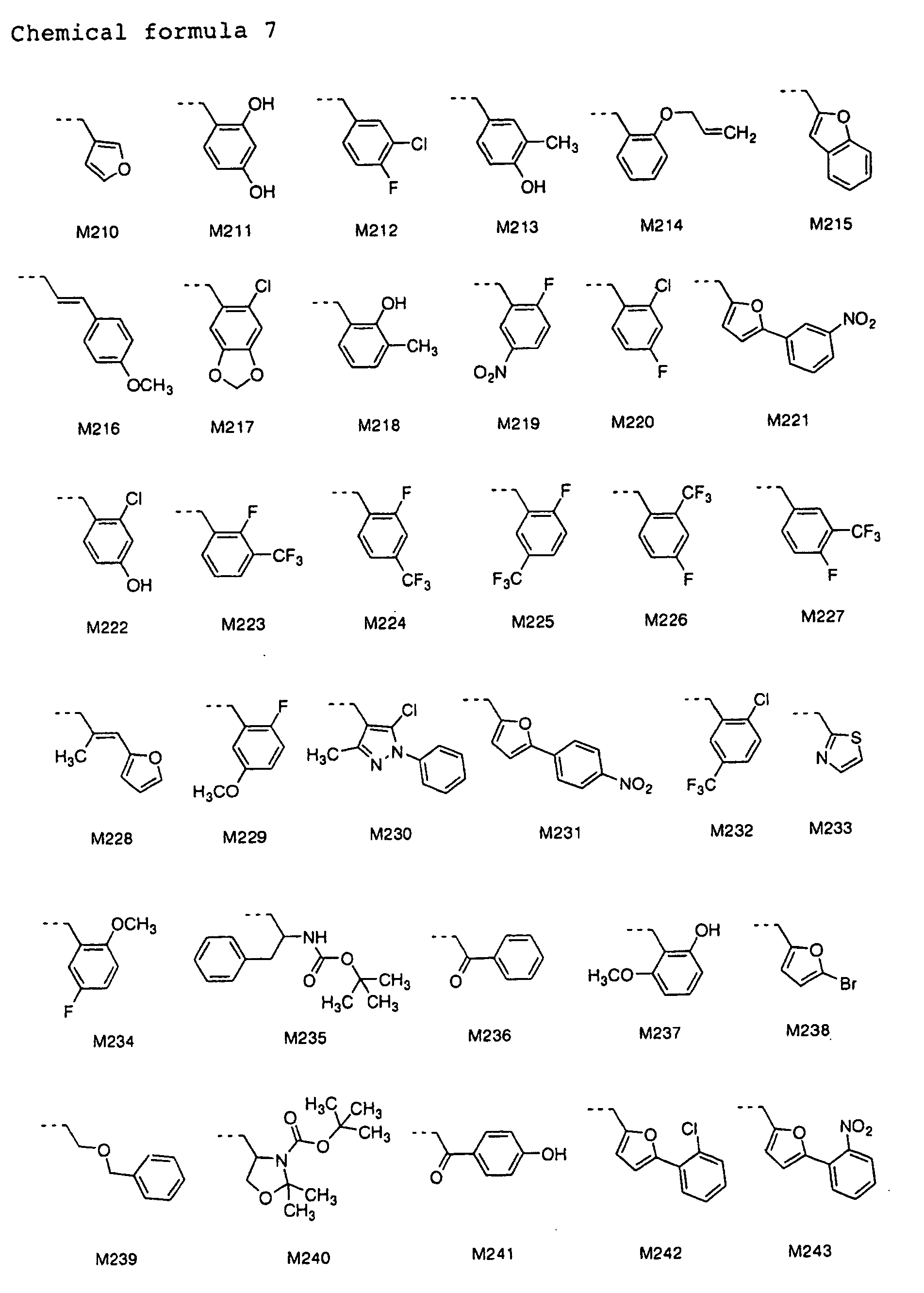

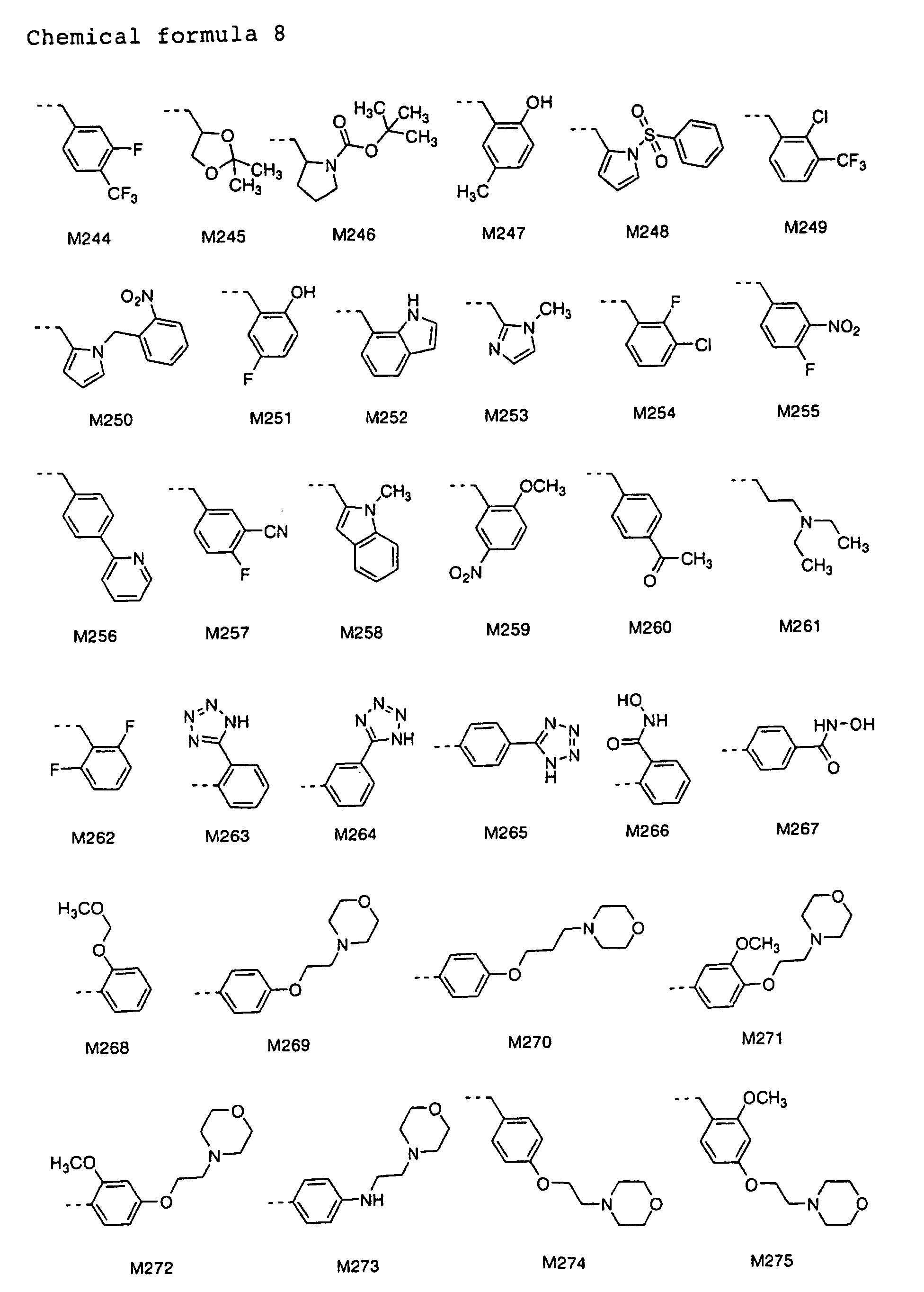

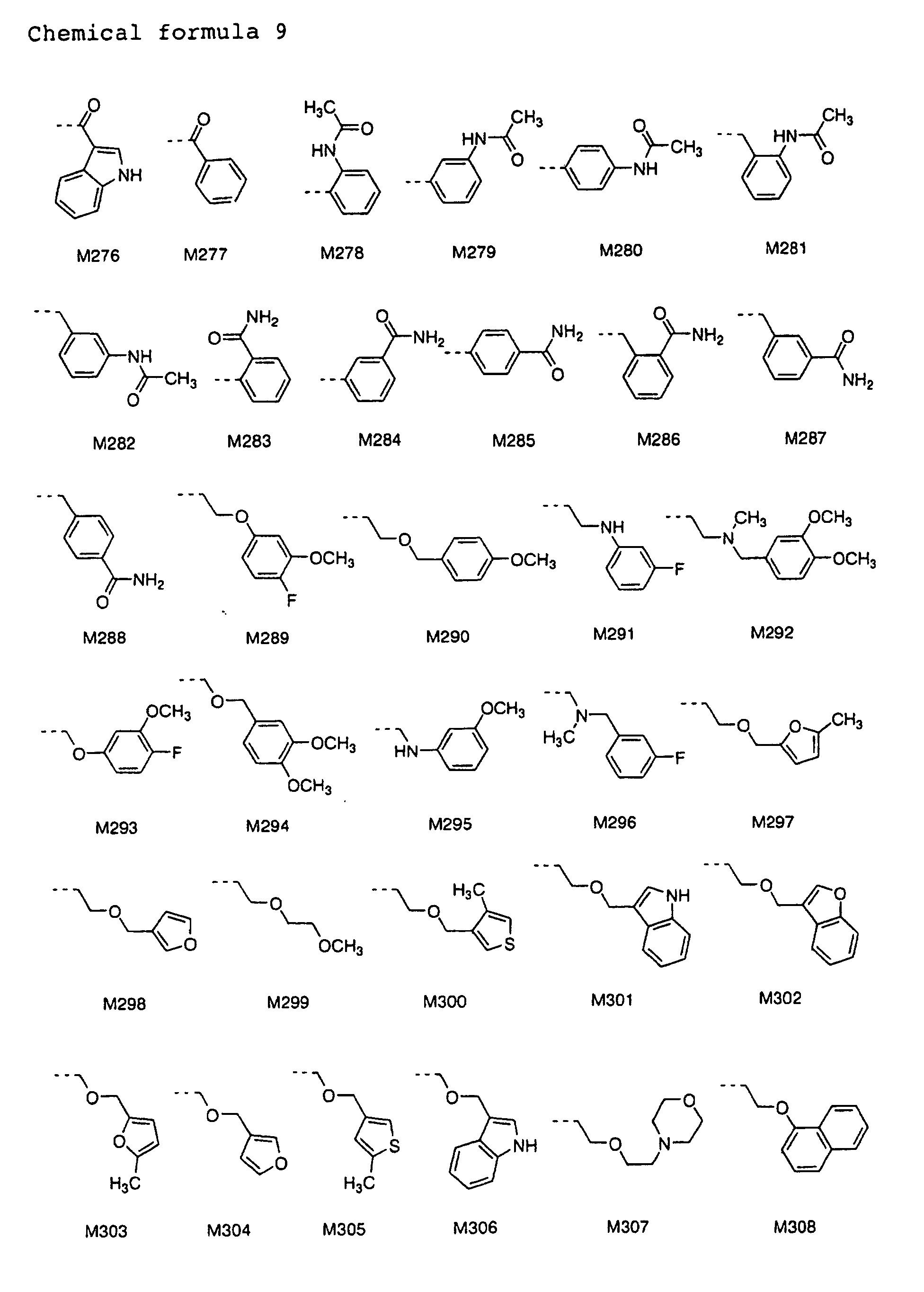

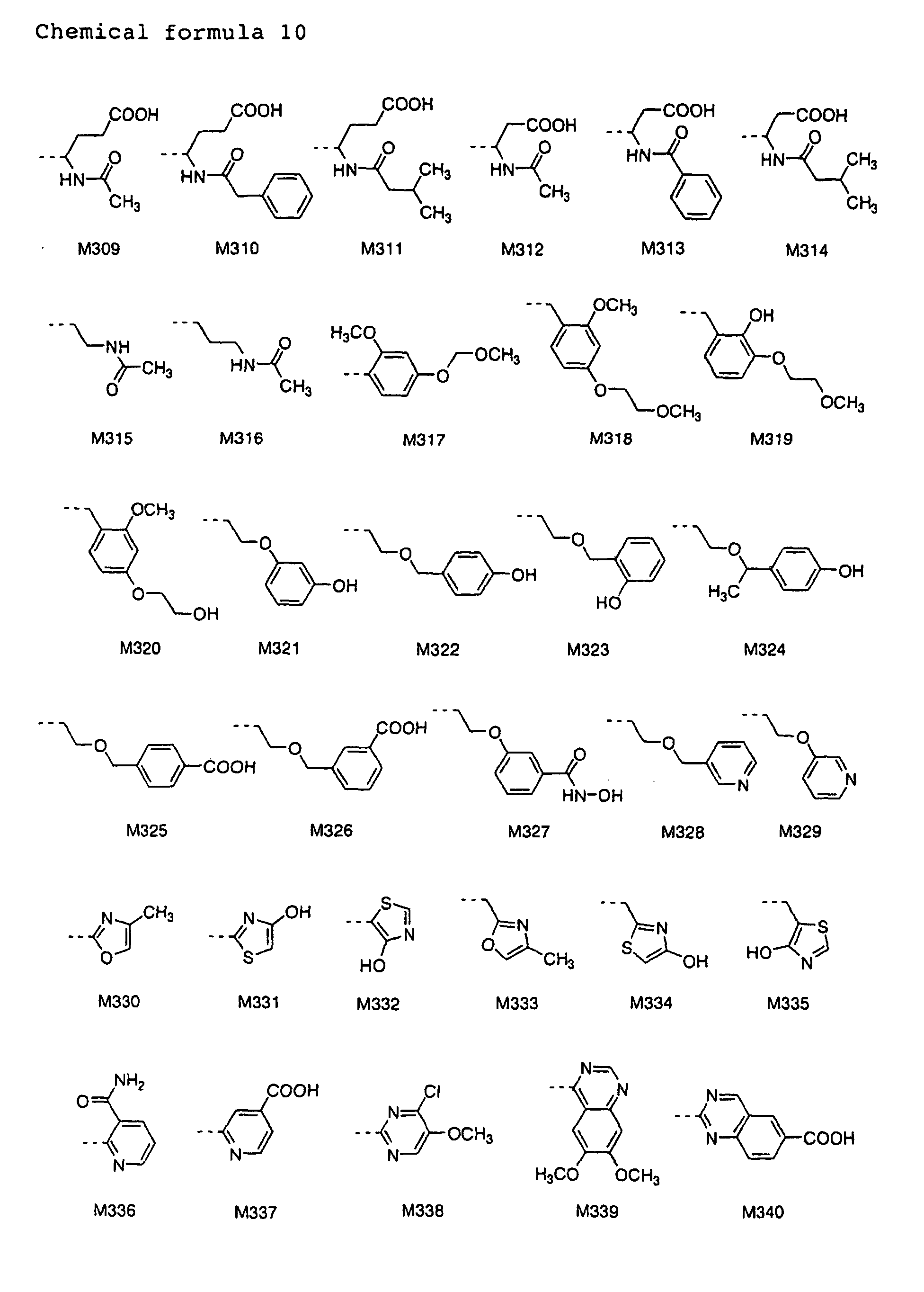

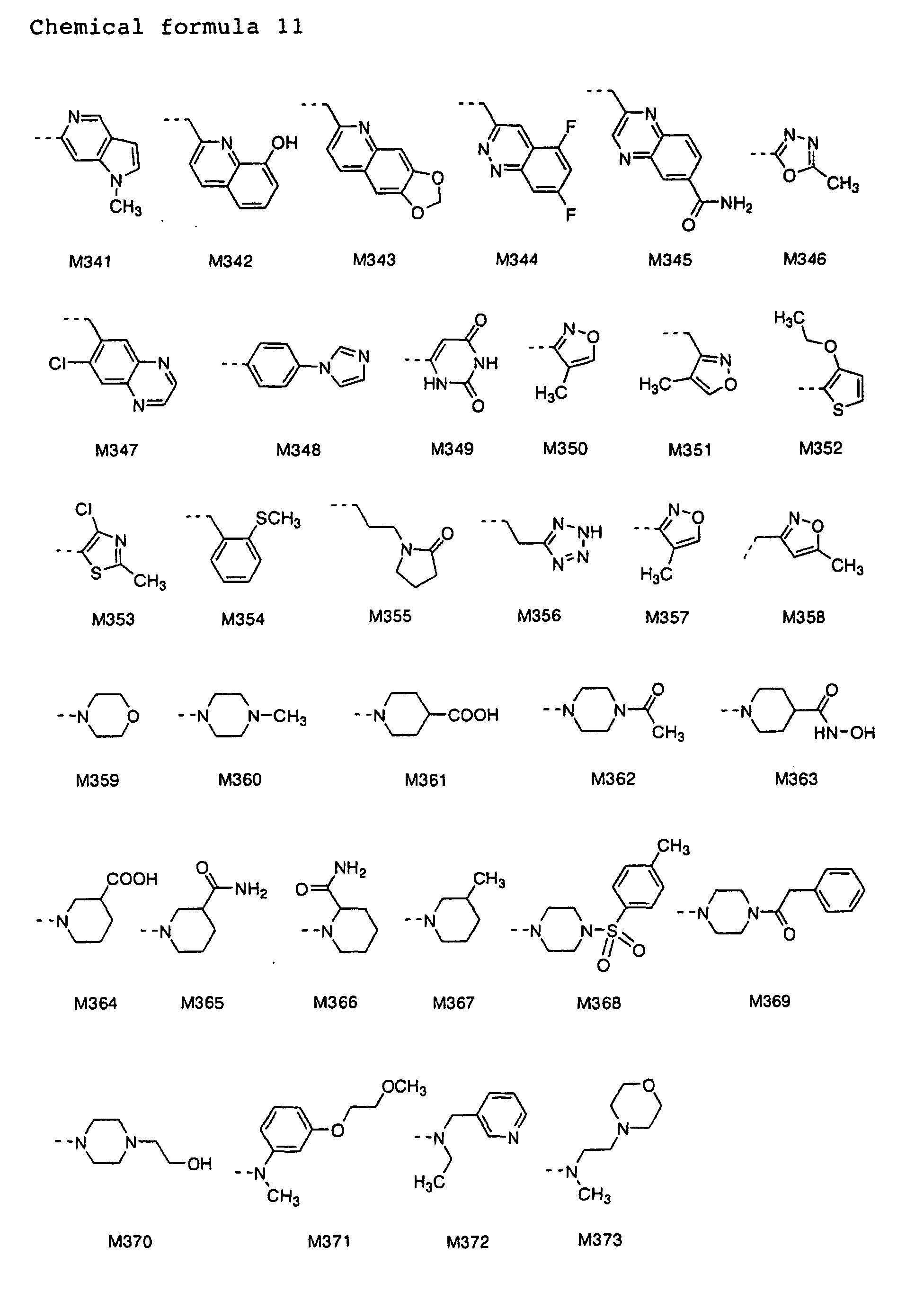

In Formula (I) according to the present invention, preferred combinations of G⁰ is in Chemical formula 12. In the structure of Chemical formula 12, the symbol "-----" represents a binding site of G⁰ and the pyrrole ring carbon to which G⁰ binds, and the symbol "-" represents a binding site of G⁰ and the carbon atom of (CH₂)ₙ to which G⁰ binds.

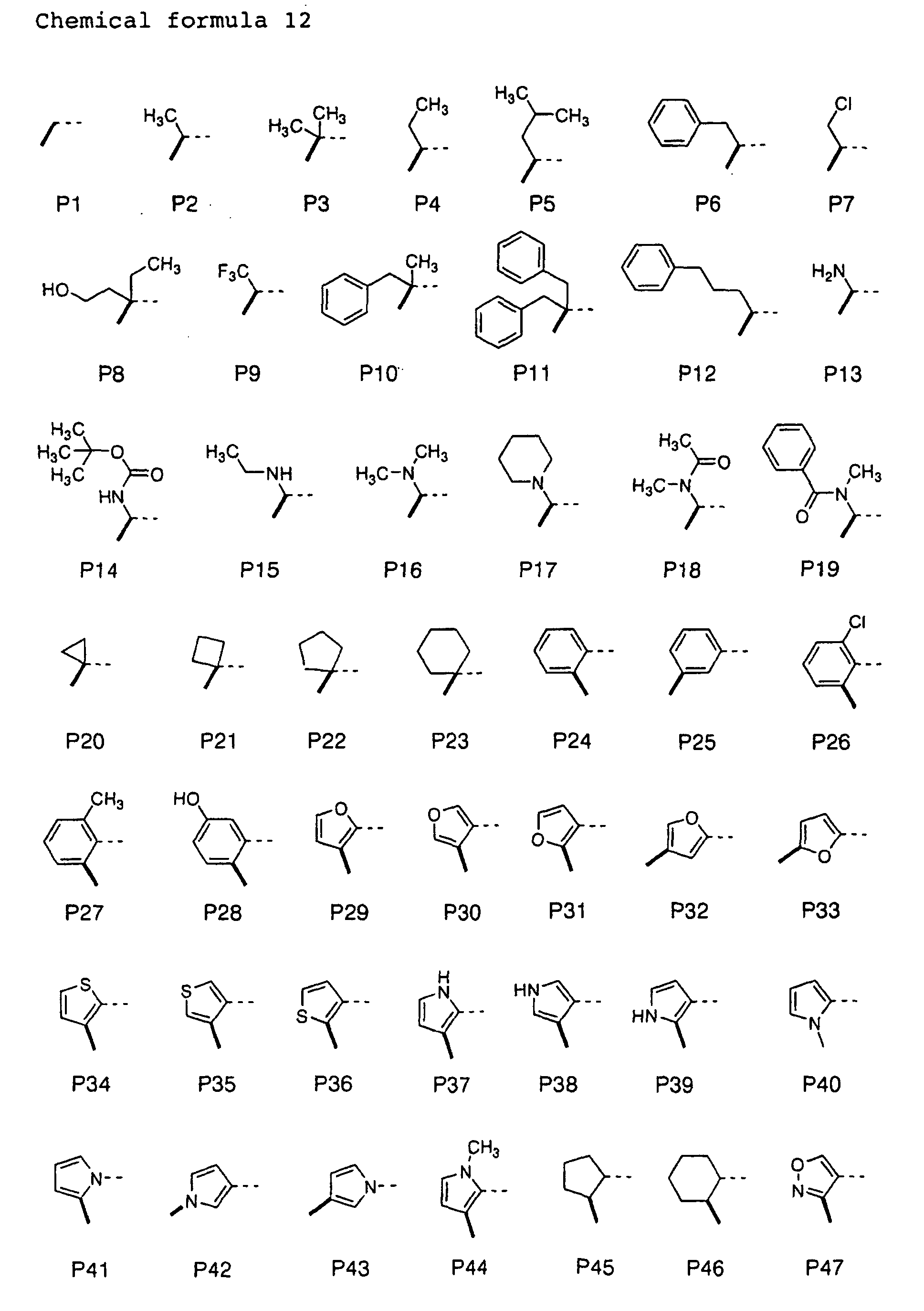

For the pyrrolo[3,2-d]pyrimidine derivative represented by the above Formula (I), there are tautomers represented by the following Formula (III): 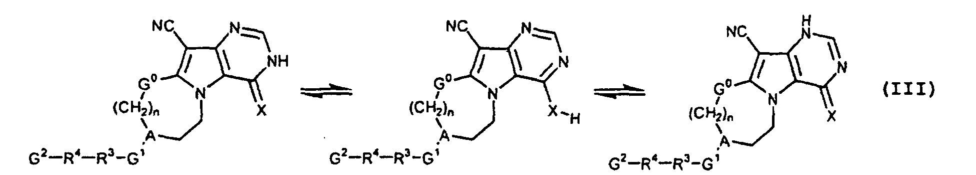 [wherein, n, A, R³, R⁴, G⁰, G¹, G², and X are as defined for n, A, R³, R⁴, G⁰, G¹, G², and X in the above Formula (I)]. However, all of these tautomers are considered to be within the scope of the present invention.

When an asymmetric structure is present on an atom constituting the molecule of the pyrrolo[3,2-d]pyrimidine derivative represented by the above Formula (I), optically active substances thereof and mixtures containing them at any ratio are considered to be within the scope of the present invention.

The pyrrolo[3,2-d]pyrimidine derivative represented by the above Formula (I) may have a basic group in the molecule, and, if this is the case, it can be converted to a medically acceptable acid additive salt as desired. Such an acid includes, for example, inorganic acids such as hydrochloric acid, hydrobromic acid, sulfuric acid, phosphoric acid, and carbonic acid; or organic acids such as acetic acid, citric acid, malic acid, oxalic acid, tartaric acid, lactic acid, maleic acid, fumaric acid, and methanesulfonic acid.

The pyrrolo[3,2-d]pyrimidine derivative represented by the above Formula (I) may have an acid group in the molecule, and if this is the case, it can be converted to a medically acceptable salt as desired. Such a salt includes, for example, a non-toxic cation salt, specifically an alkali metal ion such as Na⁺ and K⁺, an alkali earth metal ion such as Mg²⁺ and Ca²⁺, a metal ion such as Al³⁺ and Zn²⁺, an organic acid salt such as ammonia, triethylamine, ethylenediamine, propanediamine, pyrrolidine, piperidine, piperadine, pyridine, lysine, choline, ethanolamine, N,N-dimethylethanolamine, 4-hydroxypiperidine, glucosamine, N-methylglucamine or the like.

As preferred specific examples of the present invention, there can be mentioned compounds described in the following Table 1 to Table 59. In the following Table 1 to Table 59, each of M1-M37 and P1-P47 represents a substituent each defined in the above Chemical formula 1 to Chemical formula 12.

**Table 1**

| Compound No. | G⁰ | n | A | (A)-G¹-(R³) | -R³-R⁴-G² | X |
|---|---|---|---|---|---|---|
| 1 | P1 | 1 | N | -C(=O)-O- | M1 | O |
| 2 | P1 | 1 | N | -C(=O)-O- | M2 | O |
| 3 | P1 | 1 | N | -C(=O)-O- | M3 | O |
| 4 | P1 | 1 | N | -C(=O)-O- | M11 | O |
| 5 | P1 | 1 | N | -C(=O)- | M1 | O |
| 6 | P1 | 1 | N | -C(=O)- | M2 | O |
| 7 | P1 | 1 | N | -C(=O)- | M3 | O |
| 8 | P1 | 1 | N | -C(=O)- | M4 | O |
| 9 | P1 | 1 | N | -C(=O)- | M5 | O |
| 10 | P1 | 1 | N | -C(=O)- | M6 | O |
| 11 | P1 | 1 | N | -C(=O)- | M7 | O |
| 12 | P1 | 1 | N | -C(=O)- | M8 | O |
| 13 | P1 | 1 | N | -C(=O)- | M10 | O |
| 14 | P1 | 1 | N | -C(=O)- | M11 | O |
| 15 | P1 | 1 | N | -C(=O)- | M12 | O |
| 16 | P1 | 1 | N | -C(=O)- | M21 | O |
| 17 | P1 | 1 | N | -C(=O)- | M22 | O |
| 18 | P1 | 1 | N | -C(=O)- | M23 | O |
| 19 | P1 | 1 | N | -C(=O)- | M24 | O |
| 20 | P1 | 1 | N | -C(=O)- | M25 | O |
| 21 | P1 | 1 | N | -C(=O)- | M26 | O |
| 22 | P1 | 1 | N | -C(=O)- | M27 | O |
| 23 | P1 | 1 | N | -C(=O)- | M28 | O |
| 24 | P1 | 1 | N | -C(=O)- | M29 | O |
| 25 | P1 | 1 | N | -C(=O)- | M30 | O |
| 26 | P1 | 1 | N | -C(=O)- | M31 | O |
| 27 | P1 | 1 | N | -C(=O)- | M32 | O |
| 28 | P1 | 1 | N | -C(=O)- | M33 | O |
| 29 | P1 | 1 | N | -C(=O)- | M34 | O |
| 30 | P1 | 1 | N | -C(=O)- | M35 | O |
| 31 | P1 | 1 | N | -C(=O)- | M36 | O |
| 32 | P1 | 1 | N | -C(=O)- | M37 | O |
| 33 | P1 | 1 | N | -C(=O)- | M38 | O |
| 34 | P1 | 1 | N | -C(=O)- | M39 | O |
| 35 | P1 | 1 | N | -C(=O)- | M40 | O |
| 36 | P1 | 1 | N | -C(=O)- | M41 | O |
| 37 | P1 | 1 | N | -C(=O)- | M42 | O |
| 38 | P1 | 1 | N | -C(=O)- | M43 | O |
| 39 | P1 | 1 | N | -C(=O)- | M44 | O |

**Table 2**

| Compound No. | G⁰ | n | A | (A)-G¹-(R³) | -R³-R⁴-G² | X |
|---|---|---|---|---|---|---|
| 40 | P1 | 1 | N | -C(=O)- | M45 | O |
| 41 | P1 | 1 | N | -C(=O)- | M46 | O |
| 42 | P1 | 1 | N | -C(=O)- | M47 | O |
| 43 | P1 | 1 | N | -C(=O)- | M48 | O |
| 44 | P1 | 1 | N | -C(=O)- | M49 | O |
| 45 | P1 | 1 | N | -C(=O)- | M50 | O |
| 46 | P1 | 1 | N | -C(=O)- | M51 | O |
| 47 | P1 | 1 | N | -C(=O)- | M52 | O |
| 48 | P1 | 1 | N | -C(=O)- | M53 | O |
| 49 | P1 | 1 | N | -C(=O)- | M54 | O |
| 50 | P1 | 1 | N | -C(=O)- | M55 | O |
| 51 | P1 | 1 | N | -C(=O)- | M56 | O |
| 52 | P1 | 1 | N | -C(=O)- | M57 | O |
| 53 | P1 | 1 | N | -C(=O)- | M58 | O |
| 54 | P1 | 1 | N | -C(=O)- | M59 | O |
| 55 | P1 | 1 | N | -C(=O)- | M60 | O |
| 56 | P1 | 1 | N | -C(=O)- | M61 | O |
| 57 | P1 | 1 | N | -C(=O)- | M62 | O |
| 58 | P1 | 1 | N | -C(=O)- | M63 | O |
| 59 | P1 | 1 | N | -C(=O)- | M64 | O |
| 60 | P1 | 1 | N | -C(=O)- | M65 | O |
| 61 | P1 | 1 | N | -C(=O)- | M66 | O |
| 62 | P1 | 1 | N | -C(=O)- | M67 | O |
| 63 | P1 | 1 | N | -C(=O)- | M68 | O |
| 64 | P1 | 1 | N | -C(=O)- | M69 | O |
| 65 | P1 | 1 | N | -C(=O)- | M70 | O |
| 66 | P1 | 1 | N | -C(=O)- | M71 | O |
| 67 | P1 | 1 | N | -C(=O)- | M72 | O |
| 68 | P1 | 1 | N | -C(=O)- | M73 | O |
| 69 | P1 | 1 | N | -C(=O)- | M74 | O |
| 70 | P1 | 1 | N | -C(=O)- | M75 | O |
| 71 | P1 | 1 | N | -C(=O)- | M76 | O |
| 72 | P1 | 1 | N | -C(=O)- | M77 | O |
| 73 | P1 | 1 | N | -C(=O)- | M78 | O |
| 74 | P1 | 1 | N | -C(=O)- | M79 | O |
| 75 | P1 | 1 | N | -C(=O)- | M80 | O |
| 76 | P1 | 1 | N | -C(=O)- | M81 | O |
| 77 | P1 | 1 | N | -C(=O)- | M82 | O |
| 78 | P1 | | N | -C(=O)- | M83 | O |

**Table 3**

| Compound No. | G⁰ | n | A | (A)-G¹-(R³) | -R³-R⁴-G² | X |
|---|---|---|---|---|---|---|
| 79 | P1 | 1 | N | -C(=O)- | M84 | O |
| 80 | P1 | 1 | N | -C(=O)- | M85 | O |
| 81 | P1 | 1 | N | -C(=O)- | M86 | O |
| 82 | P1 | 1 | N | -C(=O)- | M87 | O |
| 83 | P1 | 1 | N | -C(=O)- | M88 | O |
| 84 | P1 | 1 | N | -C(=O)- | M89 | O |
| 85 | P1 | 1 | N | -C(=O)- | M90 | O |
| 86 | P1 | 1 | N | -C(=O)- | M81 | S |
| 87 | P1 | 1 | N | -C(=O)- | M82 | S |
| 88 | P1 | 1 | N | -C(=O)- | M83 | S |
| 89 | P1 | 1 | N | -C(=O)- | M84 | S |
| 90 | P1 | 2 | N | -C(=O)- | M85 | S |
| 91 | P1 | 2 | N | -C(=O)- | M86 | S |
| 92 | P1 | 2 | N | -C(=O)- | M87 | S |
| 93 | P1 | 1 | N | -C(=O)- | M88 | S |
| 94 | P1 | 1 | N | -C(=O)- | M89 | S |
| 95 | P1 | 1 | N | -C(=O)- | M90 | S |
| 96 | P1 | 1 | N | -C(=O)- | M91 | O |
| 97 | P1 | 1 | N | -C(=O)- | M92 | O |
| 98 | P1 | 1 | N | -C(=O)- | M93 | O |
| 99 | P1 | 1 | N | -C(=O)- | M94 | O |
| 100 | P1 | 1 | N | -C(=O)- | M95 | O |
| 101 | P1 | 1 | N | -C(=O)- | M96 | O |
| 102 | P1 | 1 | N | -C(=O)- | M97 | O |
| 103 | P1 | 1 | N | -C(=O)- | M98 | O |
| 104 | P1 | 1 | N | -C(=O)- | M99 | O |
| 105 | P1 | 1 | N | -C(=O)- | M100 | O |
| 106 | P1 | 1 | N | -C(=O)- | M101 | O |
| 107 | P1 | 1 | N | -C(=O)- | M102 | O |
| 108 | P1 | 1 | N | -C(=O)- | M103 | O |
| 109 | P1 | 1 | N | -C(=O)- | M104 | O |
| 110 | P1 | 1 | N | -C(=O)-NH- | M1 | O |
| 111 | P1 | 1 | N | -C(=O)-NH- | M2 | O |
| 112 | P1 | 1 | N | -C(=O)-NH- | M3 | O |
| 113 | P1 | 1 | N | -C(=O)-NH- | M4 | O |
| 114 | P1 | 1 | N | -C(=O)-NH- | M5 | O |
| 115 | P1 | 1 | N | -C(=O)-NH- | M6 | O |
| 116 | P1 | 1 | N | -C(=O)-NH- | M7 | O |
| 117 | P1 | 1 | N | -C(=O)-NH- | M9 | O |

**Table 4**

| Compound No. | G⁰ | n | A | (A)-G¹-(R³) | -R³-R⁴-G² | X |
|---|---|---|---|---|---|---|
| 118 | P1 | 1 | N | -C(=O)-NH- | M10 | O |
| 119 | P1 | 1 | N | -C(=O)-NH- | M11 | O |
| 120 | P1 | 1 | N | -C(=O)-NH- | M12 | O |
| 121 | P1 | 1 | N | -C(=O)-NH- | M13 | O |
| 122 | P1 | 1 | N | -C(=O)-NH- | M14 | O |
| 123 | P1 | 1 | N | -C(=O)-NH- | M15 | O |
| 124 | P1 | 1 | N | -C(=O)-NH- | M16 | O |
| 125 | P1 | 1 | N | -C(=O)-NH- | M17 | O |
| 126 | P1 | 1 | N | -C(=O)-NH- | M18 | O |
| 127 | P1 | 1 | N | -C(=O)-NH- | M19 | O |
| 128 | P1 | 1 | N | -C(=O)-NH- | M20 | O |
| 129 | P1 | 1 | N | -C(=O)-NH- | M21 | O |
| 130 | P1 | 1 | N | -C(=O)-NH- | M22 | O |
| 131 | P1 | 1 | N | -C(=O)-NH- | M23 | O |
| 132 | P1 | 1 | N | -C(=O)-NH- | M24 | 0 |
| 133 | P1 | 1 | N | -C(=O)-NH- | M25 | O |
| 134 | P1 | 1 | N | -C(=O)-NH- | M26 | O |
| 135 | P1 | 1 | N | -C(=O)-NH- | M29 | O |
| 136 | P1 | 1 | N | -C(=O)-NH- | M30 | O |
| 137 | P1 | 1 | N | -C(=O)-NH- | M31 | O |
| 138 | P1 | 1 | N | -C(=O)-NH- | M32 | O |
| 139 | P1 | 1 | N | -C(=O)-NH- | M33 | O |
| 140 | P1 | 1 | N | -C(=O)-NH- | M34 | O |
| 141 | P1 | 1 | N | -C(=O)-NH- | M35 | O |
| 142 | P1 | 1 | N | -C(=O)-NH- | M36 | O |
| 143 | P1 | 1 | N | -C(=O)-NH- | M37 | O |
| 144 | P1 | 1 | N | -C(=O)-NH- | M38 | O |
| 145 | P1 | 1 | N | -C(=O)-NH- | M39 | O |
| 146 | P1 | 1 | N | -C(=O)-NH- | M40 | O |
| 147 | P1 | 1 | N | -C(=O)-NH- | M41 | O |
| 148 | P1 | 1 | N | -C(=O)-NH- | M42 | O |
| 149 | P1 | 1 | N | -C(=O)-NH- | M43 | O |
| 150 | P1 | 1 | N | -C(=O)-NH- | M44 | O |
| 151 | P1 | 1 | N | -C(=O)-NH- | M45 | O |
| 152 | P1 | 1 | N | -C(=O)-NH- | M46 | O |
| 153 | P1 | 1 | N | -C(=O)-NH- | M47 | O |
| 154 | P1 | 1 | N | -C(=O)-NH- | M48 | O |
| 155 | P1 | 1 | N | -C(=O)-NH- | M49 | O |
| 156 | P1 | 1 | N | -C(=O)-NH- | M50 | O |

**Table 5**

| Compound No. | G⁰ | n | A | (A)-G¹-(R³) | -R³-R⁴-G² | X |
|---|---|---|---|---|---|---|
| 157 | P1 | 1 | N | -C(=O)-NH- | M51 | O |
| 158 | P1 | 1 | N | -C(=O)-NH- | M52 | O |
| 159 | P1 | 1 | N | -C(=O)-NH- | M53 | O |
| 160 | P1 | 1 | N | -C(=O)-NH- | M54 | O |
| 161 | P1 | 1 | N | -C(=O)-NH- | M55 | O |
| 162 | P1 | 1 | N | -C(=O)-NH- | M56 | O |
| 163 | P1 | 1 | N | -C(=O)-NH- | M57 | O |
| 164 | P1 | 1 | N | -C(=O)-NH- | M58 | O |
| 165 | P1 | 1 | N | -C(=O)-NH- | M59 | O |
| 166 | P1 | 1 | N | -C(=O)-NH- | M60 | O |
| 167 | P1 | 1 | N | -C(=O)-NH- | M61 | O |
| 168 | P1 | 1 | N | -C(=O)-NH- | M62 | O |
| 169 | P1 | 1 | N | -C(=O)-NH- | M63 | O |
| 170 | P1 | 1 | N | -C(=O)-NH- | M64 | O |
| 171 | P1 | 1 | N | -C(=O)-NH- | M65 | O |
| 172 | P1 | 1 | N | -C(=O)-NH- | M66 | O |
| 173 | P1 | 1 | N | -C(=O)-NH- | M67 | O |
| 174 | P1 | 1 | N | -C(=O)-NH- | M68 | O |
| 175 | P1 | 1 | N | -C(=O)-NH- | M69 | O |
| 176 | P1 | 1 | N | -C(=O)-NH- | M70 | O |
| 177 | P1 | 1 | N | -C(=O)-NH- | M71 | O |
| 178 | P1 | 1 | N | -C(=O)-NH- | M72 | O |
| 179 | P1 | 1 | N | -C(=O)-NH- | M73 | O |
| 180 | P1 | 1 | N | -C(=O)-NH- | M74 | O |
| 181 | P1 | 1 | N | -C(=O)-NH- | M75 | O |
| 182 | P1 | 1 | N | -C(=O)-NH- | M76 | O |
| 183 | P1 | 1 | N | -C(=O)-NH- | M77 | O |
| 184 | P1 | 1 | N | -C(=O)-NH- | M78 | O |
| 185 | P1 | 1 | N | -C(=O)-NH- | M79 | O |
| 186 | P1 | 1 | N | -C(=O)-NH- | M80 | O |
| 187 | P1 | 1 | N | -C(=O)-NH- | M81 | O |
| 188 | P1 | 1 | N | -C(=O)-NH- | M82 | O |
| 189 | P1 | 1 | N | -C(=O)-NH- | M83 | O |
| 190 | P1 | 1 | N | -C(=O)-NH- | M84 | O |
| 191 | P1 | 1 | N | -C(=O)-NH- | M85 | O |
| 192 | P1 | 1 | N | -C(=O)-NH- | M86 | O |
| 193 | P1 | 1 | N | -C(=O)-NH- | M87 | O |
| 194 | P1 | 1 | N | -C(=O)-NH- | M88 | O |
| 195 | P1 | 1 | N | -C(=O)-NH- | M89 | O |

**Table 6**

| Compound No. | G⁰ | n | A | (A)-G¹-(R³) | -R³-G⁴-G² | X |
|---|---|---|---|---|---|---|
| 196 | P1 | 1 | N | -C(=O)-NH- | M90 | O |
| 197 | P1 | 1 | N | -C(=O)-NH- | M91 | O |
| 198 | P1 | 1 | N | -C(=O)-NH- | M92 | O |
| 199 | P1 | 1 | N | -C(=O)-NH- | M93 | O |
| 200 | P1 | 1 | N | -C(=O)-NH- | M94 | O |
| 201 | P1 | 1 | N | -C(=O)-NH- | M95 | O |
| 202 | P1 | 1 | N | -C(=O)-NH- | M96 | O |
| 203 | P1 | 1 | N | -C(=S)-NH- | M1 | O |
| 204 | P1 | 1 | N | -C(=S)-NH- | M2 | O |
| 205 | P1 | 1 | N | -C(=S)-NH- | M3 | O |
| 206 | P1 | 1 | N | -C(=S)-NH- | M4 | O |
| 207 | P1 | 1 | N | -C(=S)-NH- | M5 | O |
| 208 | P1 | 1 | N | -C(=S)-NH- | M6 | O |
| 209 | P1 | 1 | N | -C(=S)-NH- | M7 | O |
| 210 | P1 | 1 | N | -C(=S)-NH- | M9 | O |
| 211 | P1 | 1 | N | -C(=S)-NH- | M10 | O |
| 212 | P1 | 1 | N | -C(=S)-NH- | M11 | O |
| 213 | P1 | 1 | N | -C(=S)-NH- | M12 | O |
| 214 | P1 | 1 | N | -C(=S)-NH- | M14 | O |
| 215 | P1 | 1 | N | -C(=S)-NH- | M18 | O |
| 216 | P1 | 1 | N | -C(=S)-NH- | M19 | O |
| 217 | P1 | 1 | N | -C(=S)-NH- | M29 | O |
| 218 | P1 | 1 | N | -C(=S)-NH- | M30 | O |
| 219 | P1 | 1 | N | -C(=S)-NH- | M31 | O |
| 220 | P1 | 1 | N | -C(=S)-NH- | M33 | O |
| 221 | P1 | 1 | N | -C(=S)-NH- | M34 | O |
| 222 | P1 | 1 | N | -C(=S)-NH- | M35 | O |
| 223 | P1 | 1 | N | -C(=S)-NH- | M41 | O |
| 224 | P1 | 1 | N | -C(=S)-NH- | M42 | O |
| 225 | P1 | 1 | N | -C(=S)-NH- | M43 | O |
| 226 | P1 | 1 | N | -C(=S)-NH- | M44 | O |
| 227 | P1 | 1 | N | -C(=S)-NH- | M47 | O |
| 228 | P1 | 1 | N | -C(=S)-NH- | M48 | O |
| 229 | P1 | 1 | N | -C(=S)-NH- | M49 | O |
| 230 | P1 | 1 | N | -C(=S)-NH- | M50 | O |
| 231 | P1 | 1 | N | -C(=S)-NH- | M51 | O |
| 232 | P1 | 1 | N | -C(=S)-NH- | M52 | O |
| 233 | P1 | 1 | N | -S(=O)₂- | M2 | O |

**Table 7**

| Compound No. | G⁰ | n | A | (A)-G¹-(R³) | -R³-R⁴-G² | X |
|---|---|---|---|---|---|---|
| 234 | P1 | 1 | N | -S(=O)₂- | M49 | O |
| 235 | P1 | 1 | N | -S(=O)₂- | M55 | O |
| 236 | P1 | 1 | N | -S(=O)₂- | M59 | O |
| 237 | P1 | 1 | N | -S(=O)₂- | M71 | O |
| 238 | P1 | 1 | N | -S(=O)₂- | M72 | O |
| 239 | P1 | 1 | N | -C(=O)-NH-S(=O)₂- | M49 | O |
| 240 | P1 | 1 | N | -C(=O)-NH-S(=O)₂- | M55 | O |
| 241 | P1 | 1 | N | -C(=O)-NH-S(=O)₂- | M59 | O |
| 242 | P1 | 1 | N | -C(=O)-NH-S(=O)₂- | M85 | O |
| 243 | P1 | 1 | N | -C(=O)-NH-S(=O)₂- | M87 | O |
| 244 | P1 | 1 | N | Single bond | M2 | O |
| 245 | P1 | 1 | N | Single bond | M3 | O |
| 246 | P1 | 1 | N | Single bond | M4 | O |
| 247 | P1 | 1 | N | Single bond | M5 | O |
| 248 | P1 | 1 | N | Single bond | M6 | O |
| 249 | P1 | 1 | N | Single bond | M9 | O |
| 250 | P1 | 1 | N | Single bond | M10 | O |
| 251 | P1 | 1 | N | Single bond | M11 | O |
| 252 | P1 | 1 | N | Single bond | M12 | O |
| 253 | P1 | 1 | N | Single bond | M14 | O |
| 254 | P1 | 1 | N | Single bond | M18 | O |
| 255 | P1 | 1 | N | Single bond | M21 | O |
| 256 | P1 | 1 | N | Single bond | M25 | O |
| 257 | P1 | 1 | N | Single bond | M29 | O |
| 258 | P1 | 1 | N | Single bond | M30 | O |
| 259 | P1 | 1 | N | Single bond | M31 | O |
| 260 | P1 | 1 | N | Single bond | M33 | O |
| 261 | P1 | 1 | N | Single bond | M34 | O |
| 262 | P1 | 1 | N | Single bond | M35 | O |
| 263 | P1 | 1 | N | Single bond | M36 | O |
| 264 | P1 | 1 | N | Single bond | M37 | O |
| 265 | P1 | 1 | N | Single bond | M38 | O |
| 266 | P1 | 1 | N | Single bond | M39 | O |
| 267 | P1 | 1 | N | Single bond | M40 | O |
| 268 | P1 | 1 | N | Single bond | M41 | O |
| 269 | P1 | 1 | N | Single bond | M42 | O |
| 270 | P1 | 1 | N | Single bond | M43 | O |

**Table 8**

| Compound No. | G⁰ | n | A | (A)-G¹-(R³) | -R³-R⁴-G² | X |
|---|---|---|---|---|---|---|
| 271 | P1 | 1 | N | Single bond | M44 | O |
| 272 | P1 | 1 | N | Single bond | M45 | O |
| 273 | P1 | 1 | N | Single bond | M46 | O |
| 274 | P1 | 1 | N | Single bond | M47 | O |
| 275 | P1 | 1 | N | Single bond | M48 | O |
| 276 | P1 | 1 | N | -C(=O)-O- | M1 | S |
| 277 | P1 | 1 | N | -C(=O)- | M2 | S |
| 278 | P1 | 1 | N | -C(=O)- | M3 | S |
| 279 | P1 | 1 | N | -C(=O)- | M5 | S |
| 280 | P1 | 1 | N | -C(=O)- | M8 | S |
| 281 | P1 | 1 | N | -C(=O)- | M10 | S |
| 282 | P1 | 1 | N | -C(=O)- | M11 | S |
| 283 | P1 | 1 | N | -C(=O)- | M12 | S |
| 284 | P1 | 1 | N | -C(=O)- | M27 | S |
| 285 | P1 | 1 | N | -C(=O)- | M28 | S |
| 286 | P1 | 1 | N | -C(=O)- | M33 | S |
| 287 | P1 | 1 | N | -C(=O)- | M34 | S |
| 288 | P1 | 1 | N | -C(=O)- | M35 | S |
| 289 | P1 | 1 | N | -C(=O)- | M36 | S |
| 290 | P1 | 1 | N | -C(=O)- | M41 | S |
| 291 | P1 | 1 | N | -C(=O)- | M42 | S |
| 292 | P1 | 1 | N | -C(=O)- | M43 | S |
| 293 | P1 | 1 | N | -C(=O)- | M44 | S |
| 294 | P1 | 1 | N | -C(=O)- | M45 | S |
| 295 | P1 | 1 | N | -C(=O)- | M47 | S |
| 296 | P1 | 1 | N | -C(=O)- | M48 | S |
| 297 | P1 | 1 | N | -C(=O)- | M49 | S |
| 298 | P1 | 1 | N | -C(=O)- | M51 | S |
| 299 | P1 | 1 | N | -C(=O)- | M52 | S |
| 300 | P1 | 1 | N | -C(=O)- | M53 | S |
| 301 | P1 | 1 | N | -C(=O)- | M54 | S |
| 302 | P1 | 1 | N | -C(=O)- | M55 | S |
| 303 | P1 | 1 | N | -C(=O)- | M57 | S |
| 304 | P1 | 1 | N | -C(=O)- | M58 | S |
| 305 | P1 | 1 | N | -C(=O)- | M59 | S |
| 306 | P1 | 1 | N | -C(=O)- | M61 | S |
| 307 | P1 | 1 | N | -C(=O)- | M62 | S |
| 308 | P1 | 1 | N | -C(=O)- | M63 | S |
| 309 | P1 | 1 | N | -C(=O)- | M64 | S |

**Table 9**

| Compound No. | G⁰ | n | A | (A)-G¹-(R³) | -R³-R⁴-G² | X |
|---|---|---|---|---|---|---|
| 310 | P1 | 1 | N | -C(=O)- | M69 | S |
| 311 | P1 | 1 | N | -C(=O)- | M72 | S |
| 312 | P1 | 1 | N | -C(=O)- | M76 | S |
| 313 | P1 | 1 | N | -C(=O)- | M77 | S |
| 314 | P1 | 1 | N | -C(=O)- | M80 | S |
| 315 | P1 | 1 | N | -C(=O)- | M85 | S |
| 316 | P1 | 1 | N | -C(=O)- | M86 | S |
| 317 | P1 | 1 | N | -C(=O)- | M87 | S |
| 318 | P1 | 1 | N | -C(=O)- | M91 | S |
| 319 | P1 | 1 | N | -C(=O)- | M95 | S |
| 320 | P1 | 1 | N | -C(=O)- | M97 | S |
| 321 | P1 | 1 | N | -C(=O)- | M98 | S |
| 322 | P1 | 1 | N | -C(=O)- | M99 | S |
| 323 | P1 | 1 | N | -C(=O)- | M102 | S |
| 324 | P1 | 1 | N | -C(=O)- | M103 | S |
| 325 | P1 | 1 | N | -C(=O)-NH- | M2 | S |
| 326 | P1 | 1 | N | -C(=O)-NH- | M3 | S |
| 327 | P1 | 1 | N | -C(=O)-NH- | M4 | S |
| 328 | P1 | 1 | N | -C(=O)-NH- | M5 | S |
| 329 | P1 | 1 | N | -C(=O)-NH- | M10 | S |
| 330 | P1 | 1 | N | -C(=O)-NH- | M11 | S |
| 331 | P1 | 1 | N | -C(=O)-NH- | M13 | S |
| 332 | P1 | 1 | N | -C(=O)-NH- | M14 | S |
| 333 | P1 | 1 | N | -C(=O)-NH- | M15 | S |
| 334 | P1 | 1 | N | -C(=O)-NH- | M16 | S |
| 335 | P1 | 1 | N | -C(=O)-NH- | M17 | S |
| 336 | P1 | 1 | N | -C(=O)-NH- | M18 | S |
| 337 | P1 | 1 | N | -C(=O)-NH- | M19 | S |
| 338 | P1 | 1 | N | -C(=O)-NH- | M33 | S |
| 339 | P1 | 1 | N | -C(=O)-NH- | M34 | S |
| 340 | P1 | 1 | N | -C(=O)-NH- | M35 | S |
| 341 | P1 | 1 | N | -C(=O)-NH- | M37 | S |
| 342 | P1 | 1 | N | -C(=O)-NH- | M38 | S |
| 343 | P1 | 1 | N | -C(=O)-NH- | M39 | S |
| 344 | P1 | 1 | N | -C(=O)-NH- | M41 | S |
| 345 | P1 | 1 | N | -C(=O)-NH- | M42 | S |
| 346 | P1 | 1 | N | -C(=O)-NH- | M43 | S |
| 347 | P1 | 1 | N | -C(=O)-NH- | M44 | S |
| 348 | P1 | 1 | N | -C(=O)-NH- | M45 | S |

**Table 10**

| Compound No. | G⁰ | n | A | (A)-G¹-(R³) | -R³-R⁴-G² | X |
|---|---|---|---|---|---|---|
| 349 | P1 | 1 | N | -C(=O)-NH- | M47 | S |
| 350 | P1 | 1 | N | -C(=O)-NH- | M48 | S |
| 351 | P1 | 1 | N | -C(=O)-NH- | M49 | S |
| 352 | P1 | 1 | N | -C(=O)-NH- | M51 | S |
| 353 | P1 | 1 | N | -C(=O)-NH- | M52 | S |
| 354 | P1 | 1 | N | -C(=O)-NH- | M53 | S |
| 355 | P1 | 1 | N | -C(=O)-NH- | M54 | S |
| 356 | P1 | 1 | N | -C(=O)-NH- | M55 | S |
| 357 | P1 | 1 | N | -C(=O)-NH- | M56 | S |
| 358 | P1 | 1 | N | -C(=O)-NH- | M57 | S |
| 359 | P1 | 1 | N | -C(=O)-NH- | M58 | S |
| 360 | P1 | 1 | N | -C(=O)-NH- | M59 | S |
| 361 | P1 | 1 | N | -C(=O)-NH- | M61 | S |
| 362 | P1 | 1 | N | -C(=O)-NH- | M62 | S |
| 363 | P1 | 1 | N | -C(=O)-NH- | M63 | S |
| 364 | P1 | 1 | N | -C(=O)-NH- | M64 | S |
| 365 | P1 | 1 | N | -C(=O)-NH- | M71 | S |
| 366 | P1 | 1 | N | -C(=O)-NH- | M85 | S |
| 367 | P1 | 1 | N | -C(=O)-NH- | M86 | S |
| 368 | P1 | 1 | N | -C(=O)-NH- | M87 | S |
| 369 | P1 | 1 | N | -C(=O)-NH- | M91 | S |
| 370 | P1 | 1 | N | -C(=S)-NH- | M2 | S |
| 371 | P1 | 1 | N | -C(=S)-NH- | M3 | S |
| 372 | P1 | 1 | N | -C(=S)-NH- | M5 | S |
| 373 | P1 | 1 | N | -C(=S)-NH- | M10 | S |
| 374 | P1 | 1 | N | -C(=S)-NH- | M11 | S |
| 375 | P1 | 1 | N | -C(=S)-NH- | M12 | S |
| 376 | P1 | 1 | N | -C(=S)-NH- | M29 | S |
| 377 | P1 | 1 | N | -C(=S)-NH- | M30 | S |
| 378 | P1 | 1 | N | -C(=S)-NH- | M31 | S |
| 379 | P1 | 1 | N | -C(=S)-NH- | M33 | S |
| 380 | P1 | 1 | N | -C(=S)-NH- | M34 | S |
| 381 | P1 | 1 | N | -C(=S)-NH- | M35 | S |
| 382 | P1 | 1 | N | -C(=S)-NH- | M36 | S |
| 383 | P1 | 1 | N | -C(=S)-NH- | M41 | S |
| 384 | P1 | 1 | N | -C(=S)-NH- | M42 | S |
| 385 | P1 | 1 | N | -C(=S)-NH- | M43 | S |
| 386 | P1 | 1 | N | -C(=S)-NH- | M44 | S |
| 387 | P1 | 1 | N | -C(=S)-NH- | M45 | S |

**Table 11**

| Compound No. | G⁰ | n | A | (A)-G¹-(R³) | -R³-R⁴-G² | X |
|---|---|---|---|---|---|---|
| 388 | P1 | 1 | N | -C(=S)-NH- | M46 | S |
| 389 | P1 | 1 | N | -C(=S)-NH- | M47 | S |
| 390 | P1 | 1 | N | -C(=S)-NH- | M48 | S |
| 391 | P1 | 1 | N | -C(=S)-NH- | M49 | S |
| 392 | P1 | 1 | N | -C(=S)-NH- | M51 | S |
| 393 | P1 | 1 | N | -C(=S)-NH- | M52 | S |
| 394 | P1 | 1 | N | -C(=S)-NH- | M53 | S |
| 395 | P1 | 1 | N | -C(=S)-NH- | M54 | S |
| 396 | P1 | 1 | N | -C(=S)-NH- | M55 | S |
| 397 | P1 | 1 | N | -C(=S)-NH- | M56 | S |
| 398 | P1 | 1 | N | -C(=S)-NH- | M57 | S |
| 399 | P1 | 1 | N | -C(=S)-NH- | M58 | S |
| 400 | P1 | 1 | N | -C(=S)-NH- | M59 | S |
| 401 | P1 | 1 | N | -C(=S)-NH- | M64 | S |
| 402 | P1 | 1 | N | -C(=S)-NH- | M85 | S |
| 403 | P1 | 1 | N | -C(=S)-NH- | M86 | S |
| 404 | P1 | 1 | N | -C(=S)-NH- | M87 | S |
| 405 | P1 | 1 | N | -C(=S)-NH- | M91 | S |
| 406 | P1 | 1 | N | -C(=S)-NH- | M95 | S |
| 407 | P1 | 1 | N | -C(=S)-NH- | M99 | S |
| 408 | P1 | 1 | N | -S(=O)₂- | M2 | S |
| 409 | P1 | 1 | N | -S(=O)₂- | M11 | S |
| 410 | P1 | 1 | N | -S(=O)₂- | M49 | S |
| 411 | P1 | 1 | N | Single bond | M2 | S |
| 412 | P1 | 1 | N | Single bond | M5 | S |
| 413 | P1 | 1 | N | Single bond | M9 | S |
| 414 | P1 | 1 | N | Single bond | M11 | S |
| 415 | P1 | 1 | N | Single bond | M12 | S |
| 416 | P1 | 1 | N | Single bond | M18 | S |
| 417 | P1 | 1 | N | Single bond | M25 | S |
| 418 | P1 | 1 | N | Single bond | M29 | S |
| 419 | P1 | 1 | N | Single bond | M30 | S |
| 420 | P1 | 1 | N | Single bond | M31 | S |
| 421 | P1 | 1 | N | Single bond | M33 | S |
| 422 | P1 | 1 | N | Single bond | M34 | S |
| 423 | P1 | 1 | N | Single bond | M35 | S |
| 424 | P1 | 1 | N | Single bond | M37 | S |
| 425 | P1 | 1 | N | Single bond | M38 | S |

**Table 12**

| Compound No. | G⁰ | n | A | (A)-G¹-(R³) | -R³-R⁴-G² | X |
|---|---|---|---|---|---|---|
| 426 | P1 | 1 | N | Single bond | M39 | S |
| 427 | P1 | 1 | N | Single bond | M40 | S |
| 428 | P1 | 1 | N | Single bond | M41 | S |
| 429 | P1 | 1 | N | Single bond | M42 | S |
| 430 | P1 | 1 | N | Single bond | M43 | S |
| 431 | P1 | 1 | N | Single bond | M44 | S |
| 432 | P1 | 1 | N | Single bond | M47 | S |
| 433 | P1 | 1 | N | Single bond | M48 | S |
| 434 | P1 | 0 | N | -C(=O)-O- | M1 | O |
| 435 | P1 | 0 | N | -C(=O)- | M2 | O |
| 436 | P1 | 0 | N | -C(=O)- | M3 | O |
| 437 | P1 | 0 | N | -C(=O)- | M4 | O |
| 438 | P1 | 0 | N | -C(=O)- | M5 | O |
| 439 | P1 | 0 | N | -C(=O)- | M8 | O |
| 440 | P1 | 0 | N | -C(=O)- | M10 | O |
| 441 | P1 | 0 | N | -C(=O)- | M11 | O |
| 442 | P1 | 0 | N | -C(=O)- | M12 | O |
| 443 | P1 | 0 | N | -C(=O)- | M14 | O |
| 444 | P1 | 0 | N | -C(=O)- | M18 | O |
| 445 | P1 | 0 | N | -C(=O)- | M21 | O |
| 446 | P1 | 0 | N | -C(=O)- | M25 | O |
| 447 | P1 | 0 | N | -C(=O)- | M27 | O |
| 448 | P1 | 0 | N | -C(=O)- | M28 | O |
| 449 | P1 | 0 | N | -C(=O)- | M49 | O |
| 450 | P1 | 0 | N | -C(=O)- | M51 | O |
| 451 | P1 | 0 | N | -C(=O)- | M52 | O |
| 452 | P1 | 0 | N | -C(=O)- | M59 | O |
| 453 | P1 | 0 | N | -C(=O)- | M85 | O |
| 454 | P1 | 0 | N | -C(=O)- | M86 | O |
| 455 | P1 | 0 | N | -C(=O)- | M87 | O |
| 456 | P1 | 0 | N | -C(=O)-NH- | M5 | O |
| 457 | P1 | 0 | N | -C(=O)-NH- | M10 | O |
| 458 | P1 | 0 | N | -C(=O)-NH- | M11 | O |
| 459 | P1 | 0 | N | -C(=O)-NH- | M12 | O |
| 460 | P1 | 0 | N | -C(=O)-NH- | M18 | O |
| 461 | P1 | 0 | N | -C(=O)-NH- | M25 | O |
| 462 | P1 | 0 | N | -C(=O)-NH- | M49 | O |
| 463 | P1 | 0 | N | -C(=O)-NH- | M51 | O |
| 464 | P1 | 0 | N | -C(=O)-NH- | M52 | O |

**Table 13**

| Compound No. | G⁰ | n | A | (A)-G¹-(R³) | -R³-R⁴-G² | X |
|---|---|---|---|---|---|---|
| 465 | P1 | 0 | N | -C(=O)-NH- | M53 | O |
| 466 | P1 | 0 | N | -C(=O)-NH- | M54 | O |
| 467 | P1 | 0 | N | -C(=O)-NH- | M55 | O |
| 468 | P1 | 0 | N | -C(=O)-NH- | M56 | O |
| 469 | P1 | 0 | N | -C(=O)-NH- | M57 | O |
| 470 | P1 | 0 | N | -C(=O)-NH- | M58 | O |
| 471 | P1 | 0 | N | -C(=O)-NH- | M59 | O |
| 472 | P1 | 0 | N | -C(=O)-NH- | M60 | O |
| 473 | P1 | 0 | N | -C(=O)-NH- | M61 | O |
| 474 | P1 | 0 | N | -C(=O)-NH- | M64 | O |
| 475 | P1 | 0 | N | -C(=O)-NH- | M85 | O |
| 476 | P1 | 0 | N | -C(=O)-NH- | M86 | O |
| 477 | P1 | 0 | N | -C(=O)-NH- | M87 | O |
| 478 | P1 | 2 | N | -C(=O)-NH- | M85 | O |
| 479 | P1 | 0 | N | -C(=O)-NH- | M90 | O |
| 480 | P1 | 0 | N | -C(=O)-NH- | M91 | O |
| 481 | P1 | 0 | N | -C(=S)-NH- | M2 | O |
| 482 | P1 | 0 | N | -C(=S)-NH- | M3 | O |
| 483 | P1 | 0 | N | -C(=S)-NH- | M4 | O |
| 484 | P1 | 0 | N | -C(=S)-NH- | M5 | O |
| 485 | P1 | 0 | N | -C(=S)-NH- | M8 | O |
| 486 | P1 | 0 | N | -C(=S)-NH- | M10 | O |
| 487 | P1 | 0 | N | -C(=S)-NH- | M11 | O |
| 488 | P1 | 0 | N | -C(=S)-NH- | M12 | O |
| 489 | P1 | 0 | N | -C(=S)-NH- | M14 | O |
| 490 | P1 | 0 | N | -C(=S)-NH- | M18 | O |
| 491 | P1 | 0 | N | -C(=S)-NH- | M21 | O |
| 492 | P1 | 0 | N | -C(=S)-NH- | M25 | O |
| 493 | P1 | 0 | N | -C(=S)-NH- | M27 | O |
| 494 | P1 | 0 | N | -C(=S)-NH- | M28 | O |
| 495 | P1 | 0 | N | -C(=S)-NH- | M49 | O |
| 496 | P1 | 0 | N | -C(=S)-NH- | M51 | O |
| 497 | P1 | 0 | N | -C(=S)-NH- | M52 | O |
| 498 | P1 | 0 | N | -C(=S)-NH- | M59 | O |
| 499 | P1 | 0 | N | -C(=S)-NH- | M85 | O |
| 500 | P1 | 0 | N | -C(=S)-NH- | M86 | O |
| 501 | P1 | 0 | N | -C(=S)-NH- | M87 | O |
| 502 | P1 | 0 | N | -C(=S)-NH- | M89 | O |
| 503 | P1 | 0 | N | -C(=S)-NH- | M90 | O |

**Table 14**

| Compound No. | G⁰ | n | A | (A)-G¹-(R³) | -R³-R⁴-G² | X |
|---|---|---|---|---|---|---|
| 504 | P1 | 0 | N | -C(=S)-NH- | M91 | O |
| 505 | P1 | 0 | N | -S(=O)₂- | M2 | O |
| 506 | P1 | 0 | N | -S(=O)₂- | M11 | O |
| 507 | P1 | 0 | N | -S(=O)₂- | M41 | O |
| 508 | P1 | 0 | N | Single bond | M2 | O |
| 509 | P1 | 0 | N | Single bond | M3 | O |
| 510 | P1 | 0 | N | Single bond | M11 | O |
| 511 | P1 | 0 | N | Single bond | M12 | O |
| 512 | P1 | 0 | N | Single bond | M18 | O |
| 513 | P1 | 0 | N | Single bond | M25 | O |
| 514 | P1 | 0 | N | Single bond | M35 | O |
| 515 | P1 | 0 | N | Single bond | M43 | O |
| 516 | P1 | 0 | N | -C(=O)-O- | M1 | S |
| 517 | P1 | 0 | N | -C(=O)- | M2 | S |
| 518 | P1 | 0 | N | -C(=O)- | M3 | S |
| 519 | P1 | 0 | N | -C(=O)- | M4 | S |
| 520 | P1 | 0 | N | -C(=O)- | M5 | S |
| 521 | P1 | 0 | N | -C(=O)- | M8 | S |
| 522 | P1 | 0 | N | -C(=O)- | M10 | S |
| 523 | P1 | 0 | N | -C(=O)- | M11 | S |
| 524 | P1 | 0 | N | -C(=O)- | M12 | S |
| 525 | P1 | 0 | N | -C(=O)- | M14 | S |
| 526 | P1 | 0 | N | -C(=O)- | M18 | S |
| 527 | P1 | 0 | N | -C(=O)- | M21 | S |
| 528 | P1 | 0 | N | -C(=O)- | M25 | S |
| 529 | P1 | 0 | N | -C(=O)- | M27 | S |
| 530 | P1 | 0 | N | -C(=O)- | M28 | S |
| 531 | P1 | 0 | N | -C(=O)- | M49 | S |
| 532 | P1 | 0 | N | -C(=O)- | M51 | S |
| 533 | P1 | 0 | N | -C(=O)- | M52 | S |
| 534 | P1 | 0 | N | -C(=O)- | M59 | S |
| 535 | P1 | 0 | N | -C(=O)- | M85 | S |
| 536 | P1 | 0 | N | -C(=O)- | M86 | S |
| 537 | P1 | 0 | N | -C(=O)- | M87 | S |
| 538 | P1 | 0 | N | -C(=O)-NH- | M5 | S |
| 539 | P1 | 0 | N | -C(=O)-NH- | M10 | S |
| 540 | P1 | 0 | N | -C(=O)-NH- | M11 | S |
| 541 | P1 | 0 | N | -C(=O)-NH- | M12 | S |

**Table 15**

| Compound No. | G⁰ | n | A | (A)-G¹-(R³) | -R³-R⁴-G² | X |
|---|---|---|---|---|---|---|
| 542 | P1 | 0 | N | -C(=O)-NH- | M18 | S |
| 543 | P1 | 0 | N | -C(=O)-NH- | M25 | S |
| 544 | P1 | 0 | N | -C(=O)-NH- | M49 | S |
| 545 | P1 | 0 | N | -C(=O)-NH- | M51 | S |
| 546 | P1 | 0 | N | -C(=O)-NH- | M52 | S |
| 547 | P1 | 0 | N | -C(=O)-NH- | M60 | S |
| 548 | P1 | 0 | N | -C(=O)-NH- | M61 | S |
| 549 | P1 | 0 | N | -C(=O)-NH- | M85 | S |
| 550 | P1 | 0 | N | -C(=O)-NH- | M86 | S |
| 551 | P1 | 0 | N | -C(=O)-NH- | M87 | S |
| 552 | P1 | 0 | N | -C(=S)-NH- | M5 | S |
| 553 | P1 | 0 | N | -C(=S)-NH- | M11 | S |
| 554 | P1 | 0 | N | -C(=S)-NH- | M41 | S |
| 555 | P1 | 0 | N | -C(=S)-NH- | M59 | S |
| 556 | P1 | 0 | N | -C(=S)-NH- | M87 | S |
| 557 | P1 | 0 | N | -S(=O)₂- | M2 | S |
| 558 | P1 | 0 | N | -S(=O)₂- | M11 | S |
| 559 | P1 | 0 | N | -S(=O)₂- | M41 | S |
| 560 | P1 | 0 | N | Single bond | M2 | S |
| 561 | P1 | 0 | N | Single bond | M3 | S |
| 562 | P1 | 0 | N | Single bond | M11 | S |
| 563 | P1 | 0 | N | Single bond | M12 | S |
| 564 | P1 | 0 | N | Single bond | M18 | S |
| 565 | P1 | 0 | N | Single bond | M25 | S |
| 566 | P1 | 0 | N | Single bond | M35 | S |
| 567 | P1 | 0 | N | Single bond | M43 | S |
| 568 | P1 | 0 | N | -C(=O)- | M13 | S |
| 569 | P1 | 0 | N | -C(=O)- | M15 | S |
| 570 | P1 | 0 | N | -C(=O)- | M16 | S |
| 571 | P1 | 0 | N | -C(=O)- | M17 | S |
| 572 | P1 | 0 | N | -C(=O)- | M19 | S |
| 573 | P1 | 0 | N | -C(=O)- | M20 | S |
| 574 | P1 | 0 | N | -C(=O)- | M22 | S |
| 575 | P1 | 0 | N | -C(=O)- | M23 | S |
| 576 | P1 | 0 | N | -C(=O)- | M24 | S |
| 577 | P1 | 0 | N | -C(=O)- | M33 | S |
| 578 | P1 | 0 | N | -C(=O)- | M34 | S |
| 579 | P1 | 0 | N | -C(=O)- | M35 | S |

**Table 16**

| Compound No. | G⁰ | n | A | (A)-G¹-(R³) | -R³-R⁴-G² | X |
|---|---|---|---|---|---|---|
| 580 | P1 | 0 | N | -C(=O)- | M40 | S |
| 581 | P1 | 0 | N | -C(=O)- | M57 | S |
| 582 | P1 | 0 | N | -C(=O)- | M60 | S |
| 583 | P1 | 0 | N | -C(=O)- | M62 | S |
| 584 | P1 | 0 | N | -C(=O)- | M70 | S |
| 585 | P1 | 0 | N | -C(=O)- | M77 | S |
| 586 | P1 | 0 | N | -C(=O)- | M83 | S |
| 587 | P1 | 0 | N | -C(=O)-NH- | M2 | S |
| 588 | P1 | 0 | N | -C(=O)-NH- | M3 | S |
| 589 | P1 | 0 | N | -C(=O)-NH- | M14 | S |
| 590 | P1 | 0 | N | -C(=O)-NH- | M19 | S |
| 591 | P1 | 0 | N | -C(=O)-NH- | M35 | S |
| 592 | P1 | 0 | N | -C(=O)-NH- | M56 | S |
| 593 | P1 | 0 | N | -C(=O)-NH- | M57 | S |
| 594 | P1 | 0 | N | -C(=O)-NH- | M58 | S |
| 595 | P1 | 0 | N | -C(=O)-NH- | M59 | S |
| 596 | P1 | 0 | N | -C(=O)-NH- | M62 | S |
| 597 | P1 | 0 | N | -C(=O)-NH- | M72 | S |
| 598 | P1 | 0 | N | -C(=O)-NH- | M77 | O |
| 599 | P1 | 0 | N | -C(=O)-NH- | M77 | S |
| 600 | P1 | 0 | N | -C(=O)-NH- | M90 | S |
| 601 | P1 | 0 | N | -C(=O)-NH- | M91 | S |
| 602 | P1 | 0 | N | -C(=O)-NH- | M113 | S |
| 603 | P1 | 0 | N | -C(=O)-NH- | M117 | S |
| 604 | P1 | 0 | N | -C(=O)-NH- | M118 | S |
| 605 | P1 | 0 | N | -C(=O)-NH- | M120 | S |
| 606 | P1 | 0 | N | -C(=O)-NH- | M126 | S |
| 607 | P1 | 0 | N | -C(=O)-NH- | M337 | S |
| 608 | P1 | 0 | N | -C(=O)-NH- | M339 | S |
| 609 | P1 | 0 | N | -C(=S)-NH- | M2 | S |
| 610 | P1 | 0 | N | -C(=S)-NH- | M14 | S |
| 611 | P1 | 0 | N | -C(=S)-NH- | M18 | S |
| 612 | P1 | 0 | N | -C(=S)-NH- | M21 | S |
| 613 | P1 | 0 | N | -C(=S)-NH- | M25 | S |
| 614 | P1 | 0 | N | -C(=S)-NH- | M26 | S |
| 615 | P1 | 0 | N | -C(=S)-NH- | M35 | S |
| 616 | P1 | 0 | N | -C(=S)-NH- | M49 | S |
| 617 | P1 | 0 | N | -C(=S)-NH- | M77 | O |
| 618 | P1 | 0 | N | -C(=S)-NH- | M77 | S |

**Table 17**

| Compound No. | G⁰ | n | A | (A)-G¹-(R³) | -R³-R⁴-G² | X |
|---|---|---|---|---|---|---|
| 619 | P1 | 0 | N | -C(=S)-NH- | M117 | S |
| 620 | P1 | 0 | N | Single bond | M19 | S |
| 621 | P1 | 0 | N | Single bond | M33 | O |
| 622 | P1 | 0 | N | Single bond | M33 | S |
| 623 | P1 | 0 | N | Single bond | M34 | S |
| 624 | P1 | 0 | N | Single bond | M36 | S |
| 625 | P1 | 0 | N | Single bond | M37 | O |
| 626 | P1 | 0 | N | Single bond | M37 | S |
| 627 | P1 | 0 | N | Single bond | M38 | S |
| 628 | P1 | 0 | N | Single bond | M39 | S |
| 629 | P1 | 0 | N | Single bond | M40 | S |
| 630 | P1 | 0 | N | Single bond | M41 | O |
| 631 | P1 | 0 | N | Single bond | M143 | S |
| 632 | P1 | 0 | N | Single bond | M174 | O |
| 633 | P1 | 0 | N | Single bond | M175 | S |
| 634 | P1 | 0 | N | Single bond | M190 | S |
| 635 | P1 | 0 | N | Single bond | M200 | S |
| 636 | P1 | 0 | N | Single bond | M201 | S |
| 637 | P1 | 0 | N | Single bond | M206 | S |
| 638 | P1 | 0 | N | Single bond | M207 | S |
| 639 | P1 | 0 | N | Single bond | M208 | S |
| 640 | P1 | 0 | N | Single bond | M209 | S |
| 641 | P1 | 0 | N | Single bond | M234 | S |
| 642 | P1 | 0 | N | Single bond | M239 | O |
| 643 | P1 | 0 | N | Single bond | M239 | S |
| 644 | P1 | 0 | N | Single bond | M275 | S |
| 645 | P1 | 0 | N | Single bond | M297 | S |
| 646 | P1 | 0 | N | Single bond | M298 | S |
| 647 | P1 | 0 | N | Single bond | M299 | S |
| 648 | P1 | 0 | N | Single bond | M300 | S |
| 649 | P1 | 0 | N | Single bond | M301 | S |
| 650 | P1 | 0 | N | Single bond | M302 | S |
| 651 | P1 | 0 | N | Single bond | M303 | S |
| 652 | P1 | 1 | N | -C(=O)- | M32 | S |
| 653 | P1 | 1 | N | -C(=O)- | M46 | S |
| 654 | P1 | 1 | N | -C(=O)- | M50 | S |
| 655 | P1 | 1 | N | -C(=O)- | M56 | S |
| 656 | P1 | 1 | N | -C(=O)- | M60 | S |
| 657 | P1 | 1 | N | -C(=O)- | M67 | S |

**Table 18**

| Compound No. | G⁰ | n | A | (A)-G¹-(R³) | -R³-R⁴-G² | X |
|---|---|---|---|---|---|---|
| 658 | P1 | 1 | N | -C(=O)- | M68 | S |
| 659 | P1 | 1 | N | -C(=O)- | M70 | S |
| 660 | P1 | 1 | N | -C(=O)- | M71 | S |
| 661 | P1 | 1 | N | -C(=O)- | M73 | S |
| 662 | P1 | 1 | N | -C(=O)- | M74 | S |
| 663 | P1 | 1 | N | -C(=O)- | M75 | S |
| 664 | P1 | 1 | N | -C(=O)- | M100 | S |
| 665 | P1 | 1 | N | -C(=O)- | M101 | S |
| 666 | P1 | 1 | N | -C(=O)- | M128 | S |
| 667 | P1 | 1 | N | -C(=O)- | M129 | O |
| 668 | P1 | 1 | N | -C(=O)- | M129 | S |
| 669 | P1 | 1 | N | -C(=O)- | M130 | S |
| 670 | P1 | 1 | N | -C(=O)- | M132 | S |
| 671 | P1 | 1 | N | -C(=O)- | M133 | O |
| 672 | P1 | 1 | N | -C(=O)- | M133 | S |
| 673 | P1 | 1 | N | -C(=O)- | M134 | O |
| 674 | P1 | 1 | N | -C(=O)- | M134 | S |
| 675 | P1 | 1 | N | -C(=O)- | M135 | O |
| 676 | P1 | 1 | N | -C(=O)- | M135 | S |
| 677 | P1 | 1 | N | -C(=O)- | M136 | O |
| 678 | P1 | 1 | N | -C(=O)- | M136 | S |
| 679 | P1 | 1 | N | -C(=O)- | M137 | O |
| 680 | P1 | 1 | N | -C(=O)- | M137 | S |
| 681 | P1 | 1 | N | -C(=O)- | M138 | O |
| 682 | P1 | 1 | N | -C(=O)- | M138 | S |
| 683 | P1 | 1 | N | -C(=O)- | M139 | O |
| 684 | P1 | 1 | N | -C(=O)- | M139 | S |
| 685 | P1 | 1 | N | -C(=O)- | M140 | O |
| 686 | P1 | 1 | N | -C(=O)- | M140 | S |
| 687 | P1 | 1 | N | -C(=O)- | M141 | O |
| 688 | P1 | 1 | N | -C(=O)- | M141 | S |
| 689 | P1 | 1 | N | -C(=O)- | M142 | S |
| 690 | P1 | 1 | N | -C(=O)- | M160 | S |
| 691 | P1 | 1 | N | -C(=O)- | M161 | O |
| 692 | P1 | 1 | N | -C(=O)- | M161 | S |
| 693 | P1 | 1 | N | -C(=O)- | M162 | S |
| 694 | P1 | 1 | N | -C(=O)- | M168 | S |
| 695 | P1 | 1 | N | -C(=O)- | M169 | O |
| 696 | P1 | 1 | N | -C(=O)- | M169 | S |

**Table 19**

| Compound No. | G⁰ | n | A | (A)-G¹-(R³) | -R³-R⁴-G² | X |
|---|---|---|---|---|---|---|
| 697 | P1 | 1 | N | -C(=O)- | M170 | S |
| 698 | P1 | 1 | N | -C(=O)- | M194 | S |
| 699 | P1 | 1 | N | -C(=O)- | M206 | S |
| 700 | P1 | 1 | N | -C(=O)- | M270 | S |
| 701 | P1 | 1 | N | -C(=O)- | M275 | S |
| 702 | P1 | 1 | N | -C(=O)- | M276 | O |
| 703 | P1 | 1 | N | -C(=O)- | M276 | S |
| 704 | P1 | 1 | N | -C(=O)- | M278 | S |
| 705 | P1 | 1 | N | -C(=O)- | M279 | S |
| 706 | P1 | 1 | N | -C(=O)- | M299 | S |
| 707 | P1 | 1 | N | -C(=O)- | M306 | S |
| 708 | P1 | 1 | N | -C(=O)- | M308 | S |
| 709 | P1 | 1 | N | -C(=O)- | M310 | S |
| 710 | P1 | 1 | N | -C(=O)- | M312 | S |
| 711 | P1 | 1 | N | -C(=O)- | M315 | S |
| 712 | P1 | 1 | N | -C(=O)- | M316 | S |
| 713 | P1 | 1 | N | -C(=O)- | M319 | S |
| 714 | P1 | 1 | N | -C(=O)- | M320 | S |
| 715 | P1 | 1 | N | -C(=O)- | M326 | S |
| 716 | P1 | 1 | N | -C(=O)- | M327 | S |
| 717 | P1 | 1 | N | -C(=O)- | M330 | S |
| 718 | P1 | 1 | N | -C(=O)- | M331 | S |
| 719 | P1 | 1 | N | -C(=O)- | M333 | S |
| 720 | P1 | 1 | N | -C(=O)- | M350 | S |
| 721 | P1 | 1 | N | -C(=O)- | M351 | S |
| 722 | P1 | 1 | N | -C(=O)- | M352 | S |
| 723 | P1 | 1 | N | -C(=O)- | M354 | S |
| 724 | P1 | 1 | N | -C(=O)-NH- | M1 | S |
| 725 | P1 | 1 | N | -C(=O)-NH- | M6 | S |
| 726 | P1 | 1 | N | -C(=O)-NH- | M7 | S |
| 727 | P1 | 1 | N | -C(=O)-NH- | M8 | S |
| 728 | P1 | 1 | N | -C(=O)-NH- | M9 | S |
| 729 | P1 | 1 | N | -C(=O)-NH- | M12 | S |
| 730 | P1 | 1 | N | -C(=O)-NH- | M20 | S |
| 731 | P1 | 1 | N | -C(=O)-NH- | M21 | S |
| 732 | P1 | 1 | N | -C(=O)-NH- | M22 | S |
| 733 | P1 | 1 | N | -C(=O)-NH- | M23 | S |
| 734 | P1 | 1 | N | -C(=O)-NH- | M24 | S |
| 735 | P1 | 1 | N | -C(=O)-NH- | M25 | S |

**Table 20**

| Compound No. | G⁰ | n | A | (A)-G¹-(R³) | -R³-R⁴-G² | X |
|---|---|---|---|---|---|---|
| 736 | P1 | 1 | N | -C(=O)-NH- | M26 | S |
| 737 | P1 | 1 | N | -C(=O)-NH- | M27 | S |
| 738 | P1 | 1 | N | -C(=O)-NH- | M28 | S |
| 739 | P1 | 1 | N | -C(=O)-NH- | M29 | S |
| 740 | P1 | 1 | N | -C(=O)-NH- | M30 | S |
| 741 | P1 | 1 | N | -C(=O)-NH- | M31 | S |
| 742 | P1 | 1 | N | -C(=O)-NH- | M32 | S |
| 743 | P1 | 1 | N | -C(=O)-NH- | M36 | S |
| 744 | P1 | 1 | N | -C(=O)-NH- | M40 | S |
| 745 | P1 | 1 | N | -C(=O)-NH- | M46 | S |
| 746 | P1 | 1 | N | -C(=O)-NH- | M50 | S |
| 747 | P1 | 1 | N | -C(=O)-NH- | M60 | S |
| 748 | P1 | 1 | N | -C(=O)-NH- | M65 | S |
| 749 | P1 | 1 | N | -C(=O)-NH- | M66 | S |
| 750 | P1 | 1 | N | -C(=O)-NH- | M67 | S |
| 751 | P1 | 1 | N | -C(=O)-NH- | M68 | S |
| 752 | P1 | 1 | N | -C(=O)-NH- | M69 | S |
| 753 | P1 | 1 | N | -C(=O)-NH- | M70 | S |
| 754 | P1 | 1 | N | -C(=O)-NH- | M72 | S |
| 755 | P1 | 1 | N | -C(=O)-NH- | M73 | S |
| 756 | P1 | 1 | N | -C(=O)-NH- | M74 | S |
| 757 | P1 | 1 | N | -C(=O)-NH- | M75 | S |
| 758 | P1 | 1 | N | -C(=O)-NH- | M76 | S |
| 759 | P1 | 1 | N | -C(=O)-NH- | M77 | S |
| 760 | P1 | 1 | N | -C(=O)-NH- | M78 | S |
| 761 | P1 | 1 | N | -C(=O)-NH- | M79 | S |
| 762 | P1 | 1 | N | -C(=O)-NH- | M80 | S |
| 763 | P1 | 1 | N | -C(=O)-NH- | M81 | S |
| 764 | P1 | 1 | N | -C(=O)-NH- | M82 | S |
| 765 | P1 | 1 | N | -C(=O)-NH- | M83 | S |
| 766 | P1 | 1 | N | -C(=O)-NH- | M84 | S |
| 767 | P1 | 1 | N | -C(=O)-NH- | M88 | S |
| 768 | P1 | 1 | N | -C(=O)-NH- | M89 | S |
| 769 | P1 | 1 | N | -C(=O)-NH- | M90 | S |
| 770 | P1 | 1 | N | -C(=O)-NH- | M92 | S |
| 771 | P1 | 1 | N | -C(=O)-NH- | M93 | S |
| 772 | P1 | 1 | N | -C(=O)-NH- | M94 | S |
| 773 | P1 | 1 | N | -C(=O)-NH- | M95 | S |
| 774 | P1 | 1 | N | -C(=O)-NH- | M96 | S |

**Table 21**

| Compound No. | G⁰ | n | A | (A)-G¹-(R³) | -R³-R⁴-G² | X |
|---|---|---|---|---|---|---|
| 775 | P1 | 1 | N | -C(=O)-NH- | M97 | S |
| 776 | P1 | 1 | N | -C(=O)-NH- | M98 | S |
| 777 | P1 | 1 | N | -C(=O)-NH- | M99 | S |
| 778 | P1 | 1 | N | -C(=O)-NH- | M100 | S |
| 779 | P1 | 1 | N | -C(=O)-NH- | M101 | S |
| 780 | P1 | 1 | N | -C(=O)-NH- | M102 | S |
| 781 | P1 | 1 | N | -C(=O)-NH- | M103 | S |
| 782 | P1 | 1 | N | -C(=O)-NH- | M104 | S |
| 783 | P1 | 1 | N | -C(=O)-NH- | M105 | S |
| 784 | P1 | 1 | N | -C(=O)-NH- | M106 | S |
| 785 | P1 | 1 | N | -C(=O)-NH- | M107 | S |
| 786 | P1 | 1 | N | -C(=O)-NH- | M108 | S |
| 787 | P1 | 1 | N | -C(=O)-NH- | M109 | S |
| 788 | P1 | 1 | N | -C(=O)-NH- | M110 | S |
| 789 | P1 | 1 | N | -C(=O)-NH- | M111 | S |
| 790 | P1 | 1 | N | -C(=O)-NH- | M112 | S |
| 791 | P1 | 1 | N | -C(=O)-NH- | M113 | O |
| 792 | P1 | 1 | N | -C(=O)-NH- | M113 | S |
| 793 | P1 | 1 | N | -C(=O)-NH- | M114 | S |
| 794 | P1 | 1 | N | -C(=O)-NH- | M115 | S |
| 795 | P1 | 1 | N | -C(=O)-NH- | M116 | S |
| 796 | P1 | 1 | N | -C(=O)-NH- | M117 | S |
| 797 | P1 | 1 | N | -C(=O)-NH- | M118 | S |
| 798 | P1 | 1 | N | -C(=O)-NH- | M119 | S |
| 799 | P1 | 1 | N | -C(=O)-NH- | M120 | S |
| 800 | P1 | 1 | N | -C(=O)-NH- | M121 | S |
| 801 | P1 | 1 | N | -C(=O)-NH- | M122 | S |
| 802 | P1 | 1 | N | -C(=O)-NH- | M123 | S |
| 803 | P1 | 1 | N | -C(=O)-NH- | M124 | S |
| 804 | P1 | 1 | N | -C(=O)-NH- | M125 | S |
| 805 | P1 | 1 | N | -C(=O)-NH- | M126 | S |
| 806 | P1 | 1 | N | -C(=O)-NH- | M127 | S |
| 807 | P1 | 1 | N | -C(=O)-NH- | M128 | S |
| 808 | P1 | 1 | N | -C(=O)-NH- | M129 | S |
| 809 | P1 | 1 | N | -C(=O)-NH- | M130 | S |
| 810 | P1 | 1 | N | -C(=O)-NH- | M131 | S |
| 811 | P1 | 1 | N | -C(=O)-NH- | M132 | S |
| 812 | P1 | 1 | N | -C(=O)-NH- | M133 | S |
| 813 | P1 | 1 | N | -C(=O)-NH- | M134 | S |

**Table 22**

| Compound No. | G⁰ | n | A | (A)-G¹-(R³) | -R³-R⁴-G² | X |
|---|---|---|---|---|---|---|
| 814 | P1 | 1 | N | -C(=O)-NH- | M135 | S |
| 815 | P1 | 1 | N | -C(=O)-NH- | M136 | S |
| 816 | P1 | 1 | N | -C(=O)-NH- | M137 | S |
| 817 | P1 | 1 | N | -C(=O)-NH- | M138 | S |
| 818 | P1 | 1 | N | -C(=O)-NH- | M139 | S |
| 819 | P1 | 1 | N | -C(=O)-NH- | M140 | S |
| 820 | P1 | 1 | N | -C(=O)-NH- | M141 | S |
| 821 | P1 | 1 | N | -C(=O)-NH- | M142 | S |
| 822 | P1 | 1 | N | -C(=O)-NH- | M143 | S |
| 823 | P1 | 1 | N | -C(=O)-NH- | M144 | S |
| 824 | P1 | 1 | N | -C(=O)-NH- | M145 | S |
| 825 | P1 | 1 | N | -C(=O)-NH- | M146 | S |
| 826 | P1 | 1 | N | -C(=O)-NH- | M147 | S |
| 827 | P1 | 1 | N | -C(=O)-NH- | M148 | S |
| 828 | P1 | 1 | N | -C(=O)-NH- | M149 | S |
| 829 | P1 | 1 | N | -C(=O)-NH- | M150 | S |
| 830 | P1 | 1 | N | -C(=O)-NH- | M151 | S |
| 831 | P1 | 1 | N | -C(=O)-NH- | M152 | S |
| 832 | P1 | 1 | N | -C(=O)-NH- | M153 | S |
| 833 | P1 | 1 | N | -C(=O)-NH- | M154 | S |
| 834 | P1 | 1 | N | -C(=O)-NH- | M155 | S |
| 835 | P1 | 1 | N | -C(=O)-NH- | M156 | S |
| 836 | P1 | 1 | N | -C(=O)-NH- | M157 | S |
| 837 | P1 | 1 | N | -C(=O)-NH- | M158 | S |
| 838 | P1 | 1 | N | -C(=O)-NH- | M159 | S |
| 839 | P1 | 1 | N | -C(=O)-NH- | M160 | S |
| 840 | P1 | 1 | N | -C(=O)-NH- | M161 | S |
| 841 | P1 | 1 | N | -C(=O)-NH- | M162 | S |
| 842 | P1 | 1 | N | -C(=O)-NH- | M163 | S |
| 843 | P1 | 1 | N | -C(=O)-NH- | M164 | S |
| 844 | P1 | 1 | N | -C(=O)-NH- | M165 | S |
| 845 | P1 | 1 | N | -C(=O)-NH- | M166 | S |
| 846 | P1 | 1 | N | -C(=O)-NH- | M167 | S |
| 847 | P1 | 1 | N | -C(=O)-NH- | M168 | S |
| 848 | P1 | 1 | N | -C(=O)-NH- | M169 | S |
| 849 | P1 | 1 | N | -C(=O)-NH- | M170 | S |
| 850 | P1 | 1 | N | -C(=O)-NH- | M171 | S |
| 851 | P1 | 1 | N | -C(=O)-NH- | M172 | S |
| 852 | P1 | 1 | N | -C(=O)-NH- | M173 | S |

**Table 23**

| Compound No. | G⁰ | n | A | (A)-G¹-(R³) | -R³-R⁴-G² | X |
|---|---|---|---|---|---|---|
| 853 | P1 | 1 | N | -C(=O)-NH- | M174 | S |
| 854 | P1 | 1 | N | -C(=O)-NH- | M175 | S |
| 855 | P1 | 1 | N | -C(=O)-NH- | M176 | S |
| 856 | P1 | 1 | N | -C(=O)-NH- | M177 | S |
| 857 | P1 | 1 | N | -C(=O)-NH- | M178 | S |
| 858 | P1 | 1 | N | -C(=O)-NH- | M179 | S |
| 859 | P1 | 1 | N | -C(=O)-NH- | M180 | S |
| 860 | P1 | 1 | N | -C(=O)-NH- | M181 | S |
| 861 | P1 | 1 | N | -C(=O)-NH- | M182 | S |
| 862 | P1 | 1 | N | -C(=O)-NH- | M183 | S |
| 863 | P1 | 1 | N | -C(=O)-NH- | M184 | S |
| 864 | P1 | 1 | N | -C(=O)-NH- | M185 | S |
| 865 | P1 | 1 | N | -C(=O)-NH- | M186 | S |
| 866 | P1 | 1 | N | -C(=O)-NH- | M187 | S |
| 867 | P1 | 1 | N | -C(=O)-NH- | M188 | S |
| 868 | P1 | 1 | N | -C(=O)-NH- | M189 | S |
| 869 | P1 | 1 | N | -C(=O)-NH- | M190 | S |
| 870 | P1 | 1 | N | -C(=O)-NH- | M191 | S |
| 871 | P1 | 1 | N | -C(=O)-NH- | M192 | S |
| 872 | P1 | 1 | N | -C(=O)-NH- | M193 | S |
| 873 | P1 | 1 | N | -C(=O)-NH- | M194 | S |
| 874 | P1 | 1 | N | -C(=O)-NH- | M195 | S |
| 875 | P1 | 1 | N | -C(=O)-NH- | M196 | S |
| 876 | P1 | 1 | N | -C(=O)-NH- | M197 | S |
| 877 | P1 | 1 | N | -C(=O)-NH- | M198 | S |
| 878 | P1 | 1 | N | -C(=O)-NH- | M199 | S |
| 879 | P1 | 1 | N | -C(=O)-NH- | M200 | S |
| 880 | P1 | 1 | N | -C(=O)-NH- | M201 | S |
| 881 | P1 | 1 | N | -C(=O)-NH- | M202 | S |
| 882 | P1 | 1 | N | -C(=O)-NH- | M203 | S |
| 883 | P1 | 1 | N | -C(=O)-NH- | M204 | S |
| 884 | P1 | 1 | N | -C(=O)-NH- | M205 | S |
| 885 | P1 | 1 | N | -C(=O)-NH- | M206 | S |
| 886 | P1 | 1 | N | -C(=O)-NH- | M207 | S |
| 887 | P1 | 1 | N | -C(=O)-NH- | M208 | S |
| 888 | P1 | 1 | N | -C(=O)-NH- | M209 | S |
| 889 | P1 | 1 | N | -C(=O)-NH- | M210 | S |
| 890 | P1 | 1 | N | -C(=O)-NH- | M211 | S |
| 891 | P1 | 1 | N | -C(=O)-NH- | M212 | S |

**Table 24**

| Compound No. | G⁰ | n | A | (A)-G¹-(R³) | -R³-R⁴-G² | X |
|---|---|---|---|---|---|---|
| 892 | P1 | 1 | N | -C(=O)-NH- | M213 | S |
| 893 | P1 | 1 | N | -C(=O)-NH- | M214 | S |
| 894 | P1 | 1 | N | -C(=O)-NH- | M215 | S |
| 895 | P1 | 1 | N | -C(=O)-NH- | M216 | S |
| 896 | P1 | 1 | N | -C(=O)-NH- | M217 | S |
| 897 | P1 | 1 | N | -C(=O)-NH- | M218 | S |
| 898 | P1 | 1 | N | -C(=O)-NH- | M219 | S |
| 899 | P1 | 1 | N | -C(=O)-NH- | M220 | S |
| 900 | P1 | 1 | N | -C(=O)-NH- | M221 | S |
| 901 | P1 | 1 | N | -C(=O)-NH- | M222 | S |
| 902 | P1 | 1 | N | -C(=O)-NH- | M223 | S |
| 903 | P1 | 1 | N | -C(=O)-NH- | M224 | S |
| 904 | P1 | 1 | N | -C(=O)-NH- | M225 | S |
| 905 | P1 | 1 | N | -C(=O)-NH- | M226 | S |
| 906 | P1 | 1 | N | -C(=O)-NH- | M227 | S |
| 907 | P1 | 1 | N | -C(=O)-NH- | M228 | S |
| 908 | P1 | 1 | N | -C(=O)-NH- | M229 | S |
| 909 | P1 | 1 | N | -C(=O)-NH- | M230 | S |
| 910 | P1 | 1 | N | -C(=O)-NH- | M231 | S |
| 911 | P1 | 1 | N | -C(=O)-NH- | M232 | S |
| 912 | P1 | 1 | N | -C(=O)-NH- | M233 | S |
| 913 | P1 | 1 | N | -C(=O)-NH- | M234 | S |
| 914 | P1 | 1 | N | -C(=O)-NH- | M235 | S |
| 915 | P1 | 1 | N | -C(=O)-NH- | M236 | S |
| 916 | P1 | 1 | N | -C(=O)-NH- | M237 | S |
| 917 | P1 | 1 | N | -C(=O)-NH- | M238 | S |
| 918 | P1 | 1 | N | -C(=O)-NH- | M239 | S |
| 919 | P1 | 1 | N | -C(=O)-NH- | M240 | S |
| 920 | P1 | 1 | N | -C(=O)-NH- | M241 | S |
| 921 | P1 | 1 | N | -C(=O)-NH- | M242 | S |
| 922 | P1 | 1 | N | -C(=O)-NH- | M243 | S |
| 923 | P1 | 1 | N | -C(=O)-NH- | M244 | S |
| 924 | P1 | 1 | N | -C(=O)-NH- | M245 | S |
| 925 | P1 | 1 | N | -C(=O)-NH- | M246 | S |
| 926 | P1 | 1 | N | -C(=O)-NH- | M247 | S |
| 927 | P1 | 1 | N | -C(=O)-NH- | M248 | S |
| 928 | P1 | 1 | N | -C(=O)-NH- | M249 | S |
| 929 | P1 | 1 | N | -C(=O)-NH- | M250 | S |
| 930 | P1 | 1 | N | -C(=O)-NH- | M251 | S |

**Table 25**

| Compound No. | G⁰ | n | A | (A)-G¹-(R³) | -R³-R⁴-G² | X |
|---|---|---|---|---|---|---|
| 931 | P1 | 1 | N | -C(=O)-NH- | M252 | S |
| 932 | P1 | 1 | N | -C(=O)-NH- | M253 | S |
| 933 | P1 | 1 | N | -C(=O)-NH- | M254 | S |
| 934 | P1 | 1 | N | -C(=O)-NH- | M255 | S |
| 935 | P1 | 1 | N | -C(=O)-NH- | M256 | S |
| 936 | P1 | 1 | N | -C(=O)-NH- | M257 | S |
| 937 | P1 | 1 | N | -C(=O)-NH- | M258 | S |
| 938 | P1 | 1 | N | -C(=O)-NH- | M259 | S |
| 939 | P1 | 1 | N | -C(=O)-NH- | M260 | S |
| 940 | P1 | 1 | N | -C(=O)-NH- | M261 | S |
| 941 | P1 | 1 | N | -C(=O)-NH- | M262 | S |
| 942 | P1 | 1 | N | -C(=O)-NH- | M263 | S |
| 943 | P1 | 1 | N | -C(=O)-NH- | M264 | S |
| 944 | P1 | 1 | N | -C(=O)-NH- | M265 | S |
| 945 | P1 | 1 | N | -C(=O)-NH- | M266 | S |
| 946 | P1 | 1 | N | -C(=O)-NH- | M267 | S |
| 947 | P1 | 1 | N | -C(=O)-NH- | M268 | S |
| 948 | P1 | 1 | N | -C(=O)-NH- | M269 | S |
| 949 | P1 | 1 | N | -C(=O)-NH- | M270 | S |
| 950 | P1 | 1 | N | -C(=O)-NH- | M271 | S |
| 951 | P1 | 1 | N | -C(=O)-NH- | M272 | S |
| 952 | P1 | 1 | N | -C(=O)-NH- | M273 | S |
| 953 | P1 | 1 | N | -C(=O)-NH- | M274 | S |
| 954 | P1 | 1 | N | -C(=O)-NH- | M275 | S |
| 955 | P1 | 1 | N | -C(=O)-NH- | M276 | S |
| 956 | P1 | 1 | N | -C(=O)-NH- | M277 | O |
| 957 | P1 | 1 | N | -C(=O)-NH- | M277 | S |
| 958 | P1 | 1 | N | -C(=O)-NH- | M278 | S |
| 959 | P1 | 1 | N | -C(=O)-NH- | M279 | S |
| 960 | P1 | 1 | N | -C(=O)-NH- | M280 | S |
| 961 | P1 | 1 | N | -C(=O)-NH- | M281 | S |
| 962 | P1 | 1 | N | -C(=O)-NH- | M282 | S |
| 963 | P1 | 1 | N | -C(=O)-NH- | M283 | S |
| 964 | P1 | 1 | N | -C(=O)-NH- | M284 | S |
| 965 | P1 | 1 | N | -C(=O)-NH- | M285 | S |
| 966 | P1 | 1 | N | -C(=O)-NH- | M286 | S |
| 967 | P1 | 1 | N | -C(=O)-NH- | M287 | S |
| 968 | P1 | 1 | N | -C(=O)-NH- | M288 | S |
| 969 | P1 | 1 | N | -C(=O)-NH- | M289 | S |

**Table 26**

| Compound No. | G⁰ | n | A | (A)-G¹-(R³) | -R³-R⁴-G² | X |
|---|---|---|---|---|---|---|
| 970 | P1 | 1 | N | -C(=O)-NH- | M290 | S |
| 971 | P1 | 1 | N | -C(=O)-NH- | M291 | S |
| 972 | P1 | 1 | N | -C(=O)-NH- | M292 | S |
| 973 | P1 | 1 | N | -C(=O)-NH- | M293 | S |
| 974 | P1 | 1 | N | -C(=O)-NH- | M294 | S |
| 975 | P1 | 1 | N | -C(=O)-NH- | M295 | S |
| 976 | P1 | 1 | N | -C(=O)-NH- | M296 | S |
| 977 | P1 | 1 | N | -C(=O)-NH- | M297 | S |
| 978 | P1 | 1 | N | -C(=O)-NH- | M298 | S |
| 979 | P1 | 1 | N | -C(=O)-NH- | M299 | S |
| 980 | P1 | 1 | N | -C(=O)-NH- | M300 | S |
| 981 | P1 | 1 | N | -C(=O)-NH- | M301 | S |
| 982 | P1 | 1 | N | -C(=O)-NH- | M302 | S |
| 983 | P1 | 1 | N | -C(=O)-NH- | M303 | S |
| 984 | P1 | 1 | N | -C(=O)-NH- | M304 | S |
| 985 | P1 | 1 | N | -C(=O)-NH- | M305 | S |
| 986 | P1 | 1 | N | -C(=O)-NH- | M306 | S |
| 987 | P1 | 1 | N | -C(=O)-NH- | M307 | S |
| 988 | P1 | 1 | N | -C(=O)-NH- | M308 | S |
| 989 | P1 | 1 | N | -C(=O)-NH- | M309 | S |
| 990 | P1 | 1 | N | -C(=O)-NH- | M310 | S |
| 991 | P1 | 1 | N | -C(=O)-NH- | M311 | S |
| 992 | P1 | 1 | N | -C(=O)-NH- | M312 | S |
| 993 | P1 | 1 | N | -C(=O)-NH- | M313 | S |
| 994 | P1 | 1 | N | -C(=O)-NH- | M314 | S |
| 995 | P1 | 1 | N | -C(=O)-NH- | M315 | S |
| 996 | P1 | 1 | N | -C(=O)-NH- | M316 | S |
| 997 | P1 | 1 | N | -C(=O)-NH- | M317 | S |
| 998 | P1 | 1 | N | -C(=O)-NH- | M318 | S |
| 999 | P1 | 1 | N | -C(=O)-NH- | M319 | S |
| 1000 | P1 | 1 | N | -C(=O)-NH- | M320 | S |
| 1001 | P1 | 1 | N | -C(=O)-NH- | M321 | S |
| 1002 | P1 | 1 | N | -C(=O)-NH- | M322 | S |
| 1003 | P1 | 1 | N | -C(=O)-NH- | M323 | S |
| 1004 | P1 | 1 | N | -C(=O)-NH- | M324 | S |
| 1005 | P1 | 1 | N | -C(=O)-NH- | M325 | S |
| 1006 | P1 | 1 | N | -C(=O)-NH- | M326 | S |
| 1007 | P1 | 1 | N | -C(=O)-NH- | M327 | S |
| 1008 | P1 | 1 | N | -C(=O)-NH- | M328 | S |

**Table 27**

| Compound No. | G⁰ | n | A | (A)-G¹-(R³) | -R³-R⁴-G² | X |
|---|---|---|---|---|---|---|
| 1009 | P1 | 1 | N | -C(=O)-NH- | M329 | S |
| 1010 | P1 | 1 | N | -C(=O)-NH- | M330 | S |
| 1011 | P1 | 1 | N | -C(=O)-NH- | M331 | S |
| 1012 | P1 | 1 | N | -C(=O)-NH- | M332 | S |
| 1013 | P1 | 1 | N | -C(=O)-NH- | M333 | S |
| 1014 | P1 | 1 | N | -C(=O)-NH- | M334 | S |
| 1015 | P1 | 1 | N | -C(=O)-NH- | M335 | S |
| 1016 | P1 | 1 | N | -C(=O)-NH- | M336 | S |
| 1017 | P1 | 1 | N | -C(=O)-NH- | M337 | S |
| 1018 | P1 | 1 | N | -C(=O)-NH- | M338 | S |
| 1019 | P1 | 1 | N | -C(=O)-NH- | M339 | S |
| 1020 | P1 | 1 | N | -C(=O)-NH- | M340 | S |
| 1021 | P1 | 1 | N | -C(=O)-NH- | M341 | S |
| 1022 | P1 | 1 | N | -C(=O)-NH- | M342 | S |
| 1023 | P1 | 1 | N | -C(=O)-NH- | M343 | S |
| 1024 | P1 | 1 | N | -C(=O)-NH- | M344 | S |
| 1025 | P1 | 1 | N | -C(=O)-NH- | M345 | S |
| 1026 | P1 | 1 | N | -C(=O)-NH- | M346 | S |
| 1027 | P1 | 1 | N | -C(=O)-NH- | M347 | S |
| 1028 | P1 | 1 | N | -C(=O)-NH- | M348 | S |
| 1029 | P1 | 1 | N | -C(=O)-NH- | M349 | S |
| 1030 | P1 | 1 | N | -C(=O)-NH- | M350 | S |
| 1031 | P1 | 1 | N | -C(=O)-NH- | M351 | S |
| 1032 | P1 | 1 | N | -C(=O)-NH- | M352 | S |
| 1033 | P1 | 1 | N | -C(=O)-NH- | M353 | S |
| 1034 | P1 | 1 | N | -C(=O)-NH- | M354 | S |
| 1035 | P1 | 1 | N | -C(=O)-NH- | M355 | S |
| 1036 | P1 | 1 | N | -C(=O)-NH- | M356 | S |
| 1037 | P1 | 1 | N | -C(=O)-NH- | M357 | S |
| 1038 | P1 | 1 | N | -C(=O)-NH- | M358 | S |
| 1039 | P1 | 1 | N | -C(=O)-NH-S(=O)₂- | M49 | S |
| 1040 | P1 | 1 | N | -C(=O)-NH-S(=O)₂- | M50 | S |
| 1041 | P1 | 1 | N | -C(=O)-NH-S(=O)₂- | M54 | S |
| 1042 | P1 | 1 | N | -C(=O)-NH-S(=O)₂- | M55 | S |
| 1043 | P1 | 1 | N | -C(=O)-NH-S(=O)₂- | M56 | S |
| 1044 | P1 | 1 | N | -C(=O)-NH-S(=O)₂- | M63 | S |
| 1045 | P1 | 1 | N | -C(=O)-NH-S(=O)₂- | M84 | S |

**Table 28**

| Compound No. | G⁰ | n | A | (A)-G¹-(R³) | -R³-R⁴-G² | X |
|---|---|---|---|---|---|---|
| 1046 | P1 | 1 | N | -C(=O)-NH-S(=O)₂- | M85 | S |
| 1047 | P1 | 1 | N | -C(=O)-NH-S(=O)₂- | M86 | S |
| 1048 | P1 | 1 | N | -C(=O)-NH-S(=O)₂- | M99 | S |
| 1049 | P1 | 1 | N | -C(=O)-NH-S(=O)₂- | M253 | S |
| 1050 | P1 | 1 | N | -C(=O)-NH-S(=O)₂- | M330 | S |
| 1051 | P1 | 1 | N | -C(=O)-NH-S(=O)₂- | M338 | S |
| 1052 | P1 | 1 | N | -C(=O)-NH-S(=O)₂- | M348 | S |
| 1053 | P1 | 1 | N | -C(=O)-O- | M2 | S |
| 1054 | P1 | 1 | N | -C(=O)-O- | M3 | S |
| 1055 | P1 | 1 | N | -C(=O)-O- | M4 | S |
| 1056 | P1 | 1 | N | -C(=O)-O- | M5 | O |
| 1057 | P1 | 1 | N | -C(=O)-O- | M5 | S |
| 1058 | P1 | 1 | N | -C(=O)-O- | M6 | S |
| 1059 | P1 | 1 | N | -C(=O)-O- | M7 | S |
| 1060 | P1 | 1 | N | -C(=O)-O- | M10 | S |
| 1061 | P1 | 1 | N | -C(=O)-O- | M11 | S |
| 1062 | P1 | 1 | N | -C(=O)-O- | M12 | S |
| 1063 | P1 | 1 | N | -C(=S)-NH- | M13 | S |
| 1064 | P1 | 1 | N | -C(=S)-NH- | M50 | S |
| 1065 | P1 | 1 | N | -C(=S)-NH- | M88 | S |
| 1066 | P1 | 1 | N | -C(=S)-NH- | M89 | O |
| 1067 | P1 | 1 | N | -C(=S)-NH- | M89 | S |
| 1068 | P1 | 1 | N | -C(=S)-NH- | M90 | O |
| 1069 | P1 | 1 | N | -C(=S)-NH- | M90 | S |
| 1070 | P1 | 1 | N | -C(=S)-NH- | M91 | O |
| 1071 | P1 | 1 | N | -C(=S)-NH- | M98 | O |
| 1072 | P1 | 1 | N | -C(=S)-NH- | M98 | S |
| 1073 | P1 | 1 | N | -C(=S)-NH- | M105 | O |
| 1074 | P1 | 1 | N | -C(=S)-NH- | M105 | S |
| 1075 | P1 | 1 | N | -C(=S)-NH- | M106 | O |
| 1076 | P1 | 1 | N | -C(=S)-NH- | M106 | S |
| 1077 | P1 | 1 | N | -C(=S)-NH- | M107 | O |
| 1078 | P1 | 1 | N | -C(=S)-NH- | M107 | S |
| 1079 | P1 | 1 | N | -C(=S)-NH- | M108 | O |
| 1080 | P1 | 1 | N | -C(=S)-NH- | M108 | S |
| 1081 | P1 | 1 | N | -C(=S)-NH- | M109 | O |
| 1082 | P1 | 1 | N | -C(=S)-NH- | M109 | S |

**Table 29**

| Compound No. | G⁰ | n | A | (A)-G¹-(R³) | -R³-R⁴-G² | X |
|---|---|---|---|---|---|---|
| 1083 | P1 | 1 | N | -C(=S)-NH- | M111 | O |
| 1084 | P1 | 1 | N | -C(=S)-NH- | M111 | S |
| 1085 | P1 | 1 | N | -C(=S)-NH- | M112 | O |
| 1086 | P1 | 1 | N | -C(=S)-NH- | M112 | S |
| 1087 | P1 | 1 | N | -C(=S)-NH- | M113 | O |
| 1088 | P1 | 1 | N | -C(=S)-NH- | M113 | S |
| 1089 | P1 | 1 | N | -C(=S)-NH- | M117 | O |
| 1090 | P1 | 1 | N | -C(=S)-NH- | M117 | S |
| 1091 | P1 | 1 | N | -C(=S)-NH- | M130 | O |
| 1092 | P1 | 1 | N | -C(=S)-NH- | M130 | S |
| 1093 | P1 | 1 | N | -C(=S)-NH- | M131 | O |
| 1094 | P1 | 1 | N | -C(=S)-NH- | M131 | S |
| 1095 | P1 | 1 | N | -C(=S)-NH- | M132 | O |
| 1096 | P1 | 1 | N | -C(=S)-NH- | M132 | S |
| 1097 | P1 | 1 | N | -C(=S)-NH- | M153 | O |
| 1098 | P1 | 1 | N | -C(=S)-NH- | M153 | S |
| 1099 | P1 | 1 | N | -C(=S)-NH- | M154 | O |
| 1100 | P1 | 1 | N | -C(=S)-NH- | M154 | S |
| 1101 | P1 | 1 | N | -C(=S)-NH- | M155 | O |
| 1102 | P1 | 1 | N | -C(=S)-NH- | M155 | S |
| 1103 | P1 | 1 | N | -C(=S)-NH- | M156 | O |
| 1104 | P1 | 1 | N | -C(=S)-NH- | M156 | S |
| 1105 | P1 | 1 | N | -C(=S)-NH- | M162 | O |
| 1106 | P1 | 1 | N | -C(=S)-NH- | M162 | S |
| 1107 | P1 | 1 | N | -C(=S)-NH- | M163 | O |
| 1108 | P1 | 1 | N | -C(=S)-NH- | M163 | S |
| 1109 | P1 | 1 | N | -C(=S)-NH- | M164 | O |
| 1110 | P1 | 1 | N | -C(=S)-NH- | M164 | S |
| 1111 | P1 | 1 | N | -C(=S)-NH- | M165 | O |
| 1112 | P1 | 1 | N | -C(=S)-NH- | M165 | S |
| 1113 | P1 | 1 | N | -C(=S)-NH- | M166 | O |
| 1114 | P1 | 1 | N | -C(=S)-NH- | M166 | S |
| 1115 | P1 | 1 | N | -C(=S)-NH- | M167 | O |
| 1116 | P1 | 1 | N | -C(=S)-NH- | M167 | S |
| 1117 | P1 | 1 | N | -C(=S)-NH- | M261 | O |
| 1118 | P1 | 1 | N | -C(=S)-NH- | M261 | S |
| 1119 | P1 | 1 | N | -C(=S)-NH- | M333 | S |
| 1120 | P1 | 1 | N | -C(=S)-NH- | M334 | S |
| 1121 | P1 | 1 | N | -C(=S)-NH- | M346 | S |

**Table 30**

| Compound No. | G⁰ | n | A | (A)-G¹-(R³) | -R³-R⁴-G² | X |
|---|---|---|---|---|---|---|
| 1122 | P1 | 1 | N | -C(=S)-NH- | M348 | S |
| 1123 | P1 | 1 | N | -C(=S)-NH- | M350 | S |
| 1124 | P1 | 1 | N | -C(=S)-NH- | M352 | S |
| 1125 | P1 | 1 | N | -C(=S)-NH- | M353 | S |
| 1126 | P1 | 1 | N | -S(=O)₂- | M5 | S |
| 1127 | P1 | 1 | N | -S(=O)₂- | M47 | S |
| 1128 | P1 | 1 | N | -S(=O)₂- | M50 | S |
| 1129 | P1 | 1 | N | -S(=O)₂- | M53 | S |
| 1130 | P1 | 1 | N | -S(=O)₂- | M55 | S |
| 1131 | P1 | 1 | N | -S(=O)₂- | M57 | S |
| 1132 | P1 | 1 | N | -S(=O)₂- | M59 | S |
| 1133 | P1 | 1 | N | -S(=O)₂- | M60 | S |
| 1134 | P1 | 1 | N | -S(=O)₂- | M72 | S |
| 1135 | P1 | 1 | N | -S(=O)₂- | M73 | S |
| 1136 | P1 | 1 | N | -S(=O)₂- | M96 | S |
| 1137 | P1 | 1 | N | -S(=O)₂- | M195 | S |
| 1138 | P1 | 1 | N | -S(=O)₂- | M220 | S |
| 1139 | P1 | 1 | N | Single bond | M3 | S |
| 1140 | P1 | 1 | N | Single bond | M4 | S |
| 1141 | P1 | 1 | N | Single bond | M6 | S |
| 1142 | P1 | 1 | N | Single bond | M7 | S |
| 1143 | P1 | 1 | N | Single bond | M8 | S |
| 1144 | P1 | 1 | N | Single bond | M10 | S |
| 1145 | P1 | 1 | N | Single bond | M13 | S |
| 1146 | P1 | 1 | N | Single bond | M14 | S |
| 1147 | P1 | 1 | N | Single bond | M15 | O |
| 1148 | P1 | 1 | N | Single bond | M15 | S |
| 1149 | P1 | 1 | N | Single bond | M16 | S |
| 1150 | P1 | 1 | N | Single bond | M17 | S |
| 1151 | P1 | 1 | N | Single bond | M19 | S |
| 1152 | P1 | 1 | N | Single bond | M20 | S |
| 1153 | P1 | 1 | N | Single bond | M21 | S |
| 1154 | P1 | 1 | N | Single bond | M22 | S |
| 1155 | P1 | 1 | N | Single bond | M23 | S |
| 1156 | P1 | 1 | N | Single bond | M24 | S |
| 1157 | P1 | 1 | N | Single bond | M26 | S |

**Table 31**

| Compound No. | G⁰ | n | A | (A)-G¹-(R³) | -R³-R⁴-G² | X |
|---|---|---|---|---|---|---|
| 1158 | P1 | 1 | N | Single bond | M27 | S |
| 1159 | P1 | 1 | N | Single bond | M28 | S |
| 1160 | P1 | 1 | N | Single bond | M32 | S |
| 1161 | P1 | 1 | N | Single bond | M36 | S |
| 1162 | P1 | 1 | N | Single bond | M45 | S |
| 1163 | P1 | 1 | N | Single bond | M46 | S |
| 1164 | P1 | 1 | N | Single bond | M82 | S |
| 1165 | P1 | 1 | N | Single bond | M104 | S |
| 1166 | P1 | 1 | N | Single bond | M105 | S |
| 1167 | P1 | 1 | N | Single bond | M107 | S |
| 1168 | P1 | 1 | N | Single bond | M108 | S |
| 1169 | P1 | 1 | N | Single bond | M110 | S |
| 1170 | P1 | 1 | N | Single bond | M111 | S |
| 1171 | P1 | 1 | N | Single bond | M114 | S |
| 1172 | P1 | 1 | N | Single bond | M115 | S |
| 1173 | P1 | 1 | N | Single bond | M116 | S |
| 1174 | P1 | 1 | N | Single bond | M118 | S |
| 1175 | P1 | 1 | N | Single bond | M119 | O |
| 1176 | P1 | 1 | N | Single bond | M119 | S |
| 1177 | P1 | 1 | N | Single bond | M120 | S |
| 1178 | P1 | 1 | N | Single bond | M121 | S |
| 1179 | P1 | 1 | N | Single bond | M122 | S |
| 1180 | P1 | 1 | N | Single bond | M123 | S |
| 1181 | P1 | 1 | N | Single bond | M124 | S |
| 1182 | P1 | 1 | N | Single bond | M125 | S |
| 1183 | P1 | 1 | N | Single bond | M126 | S |
| 1184 | P1 | 1 | N | Single bond | M127 | S |
| 1185 | P1 | 1 | N | Single bond | M128 | S |
| 1186 | P1 | 1 | N | Single bond | M129 | S |
| 1187 | P1 | 1 | N | Single bond | M130 | S |
| 1188 | P1 | 1 | N | Single bond | M133 | S |
| 1189 | P1 | 1 | N | Single bond | M134 | S |
| 1190 | P1 | 1 | N | Single bond | M140 | S |
| 1191 | P1 | 1 | N | Single bond | M141 | S |
| 1192 | P1 | 1 | N | Single bond | M142 | S |
| 1193 | P1 | 1 | N | Single bond | M143 | S |
| 1194 | P1 | 1 | N | Single bond | M144 | S |
| 1195 | P1 | 1 | N | Single bond | M145 | O |
| 1196 | P1 | 1 | N | Single bond | M145 | S |

**Table 32**

| Compound No. | G⁰ | n | A | (A)-G¹-(R³) | -R³-R⁴-G² | X |
|---|---|---|---|---|---|---|
| 1197 | P1 | 1 | N | Single bond | M146 | O |
| 1198 | P1 | 1 | N | Single bond | M146 | S |
| 1199 | P1 | 1 | N | Single bond | M147 | O |
| 1200 | P1 | 1 | N | Single bond | M147 | S |
| 1201 | P1 | 1 | N | Single bond | M148 | S |
| 1202 | P1 | 1 | N | Single bond | M149 | S |
| 1203 | P1 | 1 | N | Single bond | M150 | S |
| 1204 | P1 | 1 | N | Single bond | M151 | S |
| 1205 | P1 | 1 | N | Single bond | M152 | S |
| 1206 | P1 | 1 | N | Single bond | M153 | S |
| 1207 | P1 | 1 | N | Single bond | M154 | O |
| 1208 | P1 | 1 | N | Single bond | M154 | S |
| 1209 | P1 | 1 | N | Single bond | M155 | S |
| 1210 | P1 | 1 | N | Single bond | M156 | S |
| 1211 | P1 | 1 | N | Single bond | M157 | S |
| 1212 | P1 | 1 | N | Single bond | M158 | S |
| 1213 | P1 | 1 | N | Single bond | M159 | S |
| 1214 | P1 | 1 | N | Single bond | M160 | S |
| 1215 | P1 | 1 | N | Single bond | M161 | O |
| 1216 | P1 | 1 | N | Single bond | M161 | S |
| 1217 | P1 | 1 | N | Single bond | M162 | S |
| 1218 | P1 | 1 | N | Single bond | M163 | S |
| 1219 | P1 | 1 | N | Single bond | M164 | S |
| 1220 | P1 | 1 | N | Single bond | M165 | S |
| 1221 | P1 | 1 | N | Single bond | M169 | S |
| 1222 | P1 | 1 | N | Single bond | M170 | S |
| 1223 | P1 | 1 | N | Single bond | M171 | S |
| 1224 | P1 | 1 | N | Single bond | M172 | S |
| 1225 | P1 | 1 | N | Single bond | M173 | S |
| 1226 | P1 | 1 | N | Single bond | M174 | S |
| 1227 | P1 | 1 | N | Single bond | M175 | S |
| 1228 | P1 | 1 | N | Single bond | M176 | S |
| 1229 | P1 | 1 | N | Single bond | M177 | S |
| 1230 | P1 | 1 | N | Single bond | M178 | S |
| 1231 | P1 | 1 | N | Single bond | M179 | S |
| 1232 | P1 | 1 | N | Single bond | M180 | S |
| 1233 | P1 | 1 | N | Single bond | M181 | O |
| 1234 | P1 | 1 | N | Single bond | M181 | S |
| 1235 | P1 | 1 | N | Single bond | M182 | S |

**Table 33**

| Compound No. | G⁰ | n | A | (A)-G¹-(R³) | -R³-R⁴-G² | X |
|---|---|---|---|---|---|---|
| 1236 | P1 | 1 | N | Single bond | M183 | S |
| 1237 | P1 | 1 | N | Single bond | M184 | S |
| 1238 | P1 | 1 | N | Single bond | M185 | S |
| 1239 | P1 | 1 | N | Single bond | M186 | S |
| 1240 | P1 | 1 | N | Single bond | M187 | S |
| 1241 | P1 | 1 | N | Single bond | M188 | S |
| 1242 | P1 | 1 | N | Single bond | M189 | S |
| 1243 | P1 | 1 | N | Single bond | M190 | S |
| 1244 | P1 | 1 | N | Single bond | M191 | S |
| 1245 | P1 | 1 | N | Single bond | M192 | S |
| 1246 | P1 | 1 | N | Single bond | M193 | O |
| 1247 | P1 | 1 | N | Single bond | M193 | S |
| 1248 | P1 | 1 | N | Single bond | M194 | O |
| 1249 | P1 | 1 | N | Single bond | M194 | S |
| 1250 | P1 | 1 | N | Single bond | M195 | S |
| 1251 | P1 | 1 | N | Single bond | M196 | S |
| 1252 | P1 | 1 | N | Single bond | M197 | S |
| 1253 | P1 | 1 | N | Single bond | M198 | S |
| 1254 | P1 | 1 | N | Single bond | M199 | O |
| 1255 | P1 | 1 | N | Single bond | M199 | S |
| 1256 | P1 | 1 | N | Single bond | M200 | S |
| 1257 | P1 | 1 | N | Single bond | M201 | S |
| 1258 | P1 | 1 | N | Single bond | M202 | S |
| 1259 | P1 | 1 | N | Single bond | M203 | S |
| 1260 | P1 | 1 | N | Single bond | M204 | S |
| 1261 | P1 | 1 | N | Single bond | M205 | S |
| 1262 | P1 | 1 | N | Single bond | M206 | S |
| 1263 | P1 | 1 | N | Single bond | M207 | S |
| 1264 | P1 | 1 | N | Single bond | M208 | S |
| 1265 | P1 | 1 | N | Single bond | M209 | S |
| 1266 | P1 | 1 | N | Single bond | M210 | S |
| 1267 | P1 | 1 | N | Single bond | M211 | S |
| 1268 | P1 | 1 | N | Single bond | M212 | S |
| 1269 | P1 | 1 | N | Single bond | M213 | S |
| 1270 | P1 | 1 | N | Single bond | M214 | S |
| 1271 | P1 | 1 | N | Single bond | M215 | S |
| 1272 | P1 | 1 | N | Single bond | M216 | S |
| 1273 | P1 | 1 | N | Single bond | M217 | S |
| 1274 | P1 | 1 | N | Single bond | M218 | S |

**Table 34**

| Compound No. | G⁰ | n | A | (A)-G¹-(R³) | -R³-R⁴-G² | X |
|---|---|---|---|---|---|---|
| 1275 | P1 | 1 | N | Single bond | M219 | S |
| 1276 | P1 | 1 | N | Single bond | M220 | S |
| 1277 | P1 | 1 | N | Single bond | M221 | S |
| 1278 | P1 | 1 | N | Single bond | M222 | S |
| 1279 | P1 | 1 | N | Single bond | M223 | S |
| 1280 | P1 | 1 | N | Single bond | M224 | S |
| 1281 | P1 | 1 | N | Single bond | M225 | S |
| 1282 | P1 | 1 | N | Single bond | M226 | S |
| 1283 | P1 | 1 | N | Single bond | M227 | S |
| 1284 | P1 | 1 | N | Single bond | M228 | S |
| 1285 | P1 | 1 | N | Single bond | M229 | S |
| 1286 | P1 | 1 | N | Single bond | M230 | S |
| 1287 | P1 | 1 | N | Single bond | M231 | S |
| 1288 | P1 | 1 | N | Single bond | M232 | S |
| 1289 | P1 | 1 | N | Single bond | M233 | S |
| 1290 | P1 | 1 | N | Single bond | M234 | S |
| 1291 | P1 | 1 | N | Single bond | M235 | S |
| 1292 | P1 | 1 | N | Single bond | M236 | S |
| 1293 | P1 | 1 | N | Single bond | M237 | S |
| 1294 | P1 | 1 | N | Single bond | M238 | S |
| 1295 | P1 | 1 | N | Single bond | M239 | S |
| 1296 | P1 | 1 | N | Single bond | M240 | S |
| 1297 | P1 | 1 | N | Single bond | M241 | S |
| 1298 | P1 | 1 | N | Single bond | M242 | S |
| 1299 | P1 | 1 | N | Single bond | M243 | S |
| 1300 | P1 | 1 | N | Single bond | M244 | S |
| 1301 | P1 | 1 | N | Single bond | M245 | S |
| 1302 | P1 | 1 | N | Single bond | M246 | S |
| 1303 | P1 | 1 | N | Single bond | M247 | S |
| 1304 | P1 | 1 | N | Single bond | M248 | S |
| 1305 | P1 | 1 | N | Single bond | M249 | O |
| 1306 | P1 | 1 | N | Single bond | M249 | S |
| 1307 | P1 | 1 | N | Single bond | M250 | O |
| 1308 | P1 | 1 | N | Single bond | M250 | S |
| 1309 | P1 | 1 | N | Single bond | M251 | S |
| 1310 | P1 | 1 | N | Single bond | M252 | S |
| 1311 | P1 | 1 | N | Single bond | M253 | S |
| 1312 | P1 | 1 | N | Single bond | M254 | S |
| 1313 | P1 | 1 | N | Single bond | M255 | S |

**Table 35**

| Compound No. | G⁰ | n | A | (A)-G¹-(R³) | -R³-R⁴-G² | X |
|---|---|---|---|---|---|---|
| 1314 | P1 | 1 | N | Single bond | M256 | O |
| 1315 | P1 | 1 | N | Single bond | M256 | S |
| 1316 | P1 | 1 | N | Single bond | M257 | S |
| 1317 | P1 | 1 | N | Single bond | M258 | S |
| 1318 | P1 | 1 | N | Single bond | M259 | S |
| 1319 | P1 | 1 | N | Single bond | M260 | S |
| 1320 | P1 | 1 | N | Single bond | M261 | O |
| 1321 | P1 | 1 | N | Single bond | M261 | S |
| 1322 | P1 | 1 | N | Single bond | M262 | S |
| 1323 | P1 | 1 | N | Single bond | M274 | S |
| 1324 | P1 | 1 | N | Single bond | M275 | S |
| 1325 | P1 | 1 | N | Single bond | M276 | S |
| 1326 | P1 | 1 | N | Single bond | M277 | O |
| 1327 | P1 | 1 | N | Single bond | M277 | S |
| 1328 | P1 | 1 | N | Single bond | M281 | S |
| 1329 | P1 | 1 | N | Single bond | M282 | S |
| 1330 | P1 | 1 | N | Single bond | M286 | S |
| 1331 | P1 | 1 | N | Single bond | M287 | O |
| 1332 | P1 | 1 | N | Single bond | M287 | S |
| 1333 | P1 | 1 | N | Single bond | M288 | S |
| 1334 | P1 | 1 | N | Single bond | M289 | S |
| 1335 | P1 | 1 | N | Single bond | M290 | S |
| 1336 | P1 | 1 | N | Single bond | M291 | S |
| 1337 | P1 | 1 | N | Single bond | M292 | S |
| 1338 | P1 | 1 | N | Single bond | M297 | S |
| 1339 | P1 | 1 | N | Single bond | M298 | S |
| 1340 | P1 | 1 | N | Single bond | M299 | S |
| 1341 | P1 | 1 | N | Single bond | M300 | S |
| 1342 | P1 | 1 | N | Single bond | M301 | S |
| 1343 | P1 | 1 | N | Single bond | M302 | S |
| 1344 | P1 | 1 | N | Single bond | M307 | S |
| 1345 | P1 | 1 | N | Single bond | M308 | S |
| 1346 | P1 | 1 | N | Single bond | M315 | S |
| 1347 | P1 | 1 | N | Single bond | M316 | S |
| 1348 | P1 | 1 | N | Single bond | M318 | S |
| 1349 | P1 | 1 | N | Single bond | M319 | S |
| 1350 | P1 | 1 | N | Single bond | M320 | S |
| 1351 | P1 | 1 | N | Single bond | M321 | S |
| 1352 | P1 | 1 | N | Single bond | M322 | S |

**Table 36**

| Compound No. | G⁰ | n | A | (A)-G¹-(R³) | -R³-R⁴-G² | X |
|---|---|---|---|---|---|---|
| 1353 | P1 | 1 | N | Single bond | M323 | S |
| 1354 | P1 | 1 | N | Single bond | M324 | S |
| 1355 | P1 | 1 | N | Single bond | M325 | S |
| 1356 | P1 | 1 | N | Single bond | M326 | S |
| 1357 | P1 | 1 | N | Single bond | M327 | O |
| 1358 | P1 | 1 | N | Single bond | M327 | S |
| 1359 | P1 | 1 | N | Single bond | M328 | S |
| 1360 | P1 | 1 | N | Single bond | M329 | S |
| 1361 | P1 | 1 | N | Single bond | M333 | S |
| 1362 | P1 | 1 | N | Single bond | M334 | S |
| 1363 | P1 | 1 | N | Single bond | M335 | S |
| 1364 | P1 | 1 | N | Single bond | M342 | S |
| 1365 | P1 | 1 | N | Single bond | M343 | S |
| 1366 | P1 | 1 | N | Single bond | M344 | S |
| 1367 | P1 | 1 | N | Single bond | M345 | S |
| 1368 | P1 | 1 | N | Single bond | M347 | S |
| 1369 | P1 | 1 | N | Single bond | M351 | S |
| 1370 | P1 | 1 | N | Single bond | M354 | S |
| 1371 | P1 | 1 | N | Single bond | M355 | O |
| 1372 | P1 | 1 | N | Single bond | M355 | S |
| 1373 | P1 | 1 | N | Single bond | M356 | S |
| 1374 | P1 | 1 | N | Single bond | M358 | O |
| 1375 | P1 | 1 | N | Single bond | M358 | S |
| 1376 | P1 | 2 | N | -C(=O)- | M2 | O |
| 1377 | P1 1 | 2 | N | -C(=O)- | M2 | S |
| 1378 | P1 | 2 | N | -C(=O)- | M5 | S |
| 1379 | P1 | 2 | N | -C(=O)- | M8 | O |
| 1380 | P1 | 2 | N | -C(=O)- | M8 | S |
| 1381 | P1 | 2 | N | -C(=O)- | M10 | S |
| 1382 | P1 | 2 | N | -C(=O)- | M11 | O |
| 1383 | P1 | 2 | N | -C(=O)- | M11 | S |
| 1384 | P1 | 2 | N | -C(=O)- | M12 | S |
| 1385 | P1 | 2 | N | -C(=O)- | M13 | S |
| 1386 | P1 | 2 | N | -C(=O)- | M14 | S |
| 1387 | P1 | 2 | N | -C(=O)- | M15 | S |
| 1388 | P1 | 2 | N | -C(=O)- | M16 | S |
| 1389 | P1 | 2 | N | -C(=O)- | M17 | S |
| 1390 | P1 | 2 | N | -C(=O)- | M18 | S |
| 1391 | P1 | 2 | N | -C(=O)- | M19 | S |

**Table 37**

| Compound No. | G⁰ | n | A | (A)-G¹-(R³) | -R³-R⁴-G² | X |
|---|---|---|---|---|---|---|
| 1392 | P1 | 2 | N | -C(=O)- | M20 | S |
| 1393 | P1 | 2 | N | -C(=O)- | M21 | S |
| 1394 | P1 | 2 | N | -C(=O)- | M22 | S |
| 1395 | P1 | 2 | N | -C(=O)- | M23 | S |
| 1396 | P1 1 | 2 | N | -C(=O)- | M24 | S |
| 1397 | P1 | 2 | N | -C(=O)- | M25 | S |
| 1398 | P1 | 2 | N | -C(=O)- | M33 | S |
| 1399 | P1 | 2 | N | -C(=O)- | M34 | S |
| 1400 | P1 | 2 | N | -C(=O)- | M35 | S |
| 1401 | P1 | 2 | N | -C(=O)- | M40 | S |
| 1402 | P1 | 2 | N | -C(=O)- | M49 | O |
| 1403 | P1 | 2 | N | -C(=O)- | M49 | S |
| 1404 | P1 | 2 | N | -C(=O)- | M57 | S |
| 1405 | P1 | 2 | N | -C(=O)- | M60 | S |
| 1406 | P1 | 2 | N | -C(=O)- | M62 | S |
| 1407 | P1 | 2 | N | -C(=O)- | M70 | S |
| 1408 | P1 | 2 | N | -C(=O)- | M77 | S |
| 1409 | P1 | 2 | N | -C(=O)- | M83 | S |
| 1410 | P1 | 2 | N | -C(=O)-NH- | M2 | S |
| 1411 | P1 | 2 | N | -C(=O)-NH- | M3 | S |
| 1412 | P1 | 2 | N | -C(=O)-NH- | M5 | O |
| 1413 | P1 | 2 | N | -C(=O)-NH- | M5 | S |
| 1414 | P1 | 2 | N | -C(=O)-NH- | M10 | S |
| 1415 | P1 | 2 | N | -C(=O)-NH- | M11 | O |
| 1416 | P1 | 2 | N | -C(=O)-NH- | M11 | S |
| 1417 | P1 | 2 | N | -C(=O)-NH- | M14 | S |
| 1418 | P1 | 2 | N | -C(=O)-NH- | M18 | S |
| 1419 | P1 | 2 | N | -C(=O)-NH- | M19 | S |
| 1420 | P1 | 2 | N | -C(=O)-NH- | M25 | S |
| 1421 | P1 | 2 | N | -C(=O)-NH- | M35 | S |
| 1422 | P1 | 2 | N | -C(=O)-NH- | M49 | S |
| 1423 | P1 | 2 | N | -C(=O)-NH- | M56 | S |
| 1424 | P1 | 2 | N | -C(=O)-NH- | M57 | S |
| 1425 | P1 | 2 | N | -C(=O)-NH- | M58 | S |
| 1426 | P1 | 2 | N | -C(=O)-NH- | M59 | S |
| 1427 | P1 | 2 | N | -C(=O)-NH- | M60 | S |
| 1428 | P1 | 2 | N | -C(=O)-NH- | M62 | S |
| 1429 | P1 | 2 | N | -C(=O)-NH- | M72 | S |
| 1430 | P1 | 2 | N | -C(=O)-NH- | M77 | O |

**Table 38**

| Compound No. | G⁰ | n | A | (A)-G¹-(R³) | -R³-R⁴-G² | X |
|---|---|---|---|---|---|---|
| 1431 | P1 | 2 | N | -C(=O)-NH- | M77 | S |
| 1432 | P1 | 2 | N | -C(=O)-NH- | M90 | O |
| 1433 | P1 | 2 | N | -C(=O)-NH- | M90 | S |
| 1434 | P1 | 2 | N | -C(=O)-NH- | M91 | S |
| 1435 | P1 | 2 | N | -C(=O)-NH- | M113 | S |
| 1436 | P1 | 2 | N | -C(=O)-NH- | M117 | S |
| 1437 | P1 | 2 | N | -C(=O)-NH- | M118 | S |
| 1438 | P1 | 2 | N | -C(=O)-NH- | M120 | S |
| 1439 | P1 | 2 | N | -C(=O)-NH- | M126 | S |
| 1440 | P1 | 2 | N | -C(=O)-NH- | M337 | S |
| 1441 | P1 | 2 | N | -C(=O)-NH- | M339 | S |
| 1442 | P1 | 2 | N | -C(=S)-NH- | M2 | S |
| 1443 | P1 | 2 | N | -C(=S)-NH- | M5 | O |
| 1444 | P1 | 2 | N | -C(=S)-NH- | M5 | S |
| 1445 | P1 | 2 | N | -C(=S)-NH- | M11 | O |
| 1446 | P1 | 2 | N | -C(=S)-NH- | M14 | S |
| 1447 | P1 | 2 | N | -C(=S)-NH- | M18 | S |
| 1448 | P1 | 2 | N | -C(=S)-NH- | M21 | S |
| 1449 | P1 | 2 | N | -C(=S)-NH- | M25 | S |
| 1450 | P1 | 2 | N | -C(=S)-NH- | M26 | S |
| 1451 | P1 | 2 | N | -C(=S)-NH- | M35 | S |
| 1452 | P1 | 2 | N | -C(=S)-NH- | M49 | S |
| 1453 | P1 | 2 | N | -C(=S)-NH- | M77 | O |
| 1454 | P1 | 2 | N | -C(=S)-NH- | M77 | S |
| 1455 | P1 | 2 | N | -C(=S)-NH- | M90 | O |
| 1456 | P1 | 2 | N | -C(=S)-NH- | M117 | S |
| 1457 | P1 | 2 | N | Single bond | M2 | S |
| 1458 | P1 | 2 | N | Single bond | M11 | O |
| 1459 | P1 | 2 | N | Single bond | M11 | S |
| 1460 | P1 | 2 | N | Single bond | M19 | S |
| 1461 | P1 | 2 | N | Single bond | M33 | O |
| 1462 | P1 | 2 | N | Single bond | M33 | S |
| 1463 | P1 | 2 | N | Single bond | M34 | S |
| 1464 | P1 | 2 | N | Single bond | M35 | S |
| 1465 | P1 | 2 | N | Single bond | M36 | S |
| 1466 | P1 | 2 | N | Single bond | M37 | O |
| 1467 | P1 | 2 | N | Single bond | M37 | S |
| 1468 | P1 | 2 | N | Single bond | M38 | S |
| 1469 | P1 | 2 | N | Single bond | M39 | S |

**Table 39**

| Compound No. | G⁰ | n | A | (A)-G¹-(R³) | -R³-R⁴-G² | X |
|---|---|---|---|---|---|---|
| 1470 | P1 | 2 | N | Single bond | M40 | S |
| 1471 | P1 | 2 | N | Single bond | M41 | O |
| 1472 | P1 | 2 | N | Single bond | M143 | S |
| 1473 | P1 | 2 | N | Single bond | M174 | O |
| 1474 | P1 | 2 | N | Single bond | M175 | S |
| 1475 | P1 | 2 | N | Single bond | M190 | S |
| 1476 | P1 | 2 | N | Single bond | M200 | S |
| 1477 | P1 | 2 | N | Single bond | M201 | S |
| 1478 | P1 | 2 | N | Single bond | M206 | S |
| 1479 | P1 | 2 | N | Single bond | M207 | S |
| 1480 | P1 | 2 | N | Single bond | M208 | S |
| 1481 | P1 | 2 | N | Single bond | M209 | S |
| 1482 | P1 | 2 | N | Single bond | M234 | S |
| 1483 | P1 | 2 | N | Single bond | M239 | O |
| 1484 | P1 | 2 | N | Single bond | M239 | S |
| 1485 | P1 | 2 | N | Single bond | M275 | S |
| 1486 | P1 | 2 | N | Single bond | M297 | S |
| 1487 | P1 | 2 | N | Single bond | M298 | S |
| 1488 | P1 | 2 | N | Single bond | M299 | S |
| 1489 | P1 | 2 | N | Single bond | M300 | S |
| 1490 | P1 | 2 | N | Single bond | M301 | S |
| 1491 | P1 | 2 | N | Single bond | M302 | S |
| 1492 | P1 | 2 | N | Single bond | M303 | S |
| 1493 | P2 | 0 | N | -C(=O)-NH-S(=O)₂- | M6 | S |
| 1494 | P2 | 0 | N | -C(=O)-O- | M1 | S |
| 1495 | P2 | 0 | N | Single bond | M13 | S |
| 1496 | P2 | 1 | N | -C(=O)- | M2 | S |
| 1497 | P2 | 1 | N | -C(=O)- | M10 | O |
| 1498 | P2 | 1 | N | -C(=O)- | M14 | S |
| 1499 | P2 | 1 | N | -C(=O)-NH- | M4 | S |
| 1500 | P2 | 1 | N | -C(=O)-O- | M8 | S |
| 1501 | P2 | 1 | N | Single bond | M11 | S |
| 1502 | P2 | 1 | N | Single bond | M15 | S |
| 1503 | P2 | 2 | N | -C(=O)- | M17 | S |
| 1504 | P2 | 2 | N | -C(=O)-NH- | M12 | S |
| 1505 | P2 | 2 | N | -C(=S)-NH- | M5 | S |
| 1506 | P3 | 0 | N | -C(=S)-NH- | M33 | S |
| 1507 | P3 | 0 | N | Single bond | M18 | S |

**Table 40**

| Compound No. | G⁰ | n | A | (A)-G¹-(R³) | -R³-R⁴-G² | X |
|---|---|---|---|---|---|---|
| 1508 | P3 | 0 | N | Single bond | M28 | S |
| 1509 | P3 | 1 | N | -C(=O)- | M24 | S |
| 1510 | P3 | 1 | N | -C(=O)-NH- | M19 | S |
| 1511 | P3 | 1 | N | -C(=O)-NH- | M25 | S |
| 1512 | P3 | 1 | N | -C(=O)-NH-S(=O)₂- | M21 | O |
| 1513 | P3 | 1 | N | -C(=O)-O- | M29 | S |
| 1514 | P3 | 1 | N | -C(=S)-NH- | M20 | S |
| 1515 | P3 | 1 | N | Single bond | M31 | O |
| 1516 | P3 | 1 | N | Single bond | M26 | S |
| 1517 | P3 | 2 | N | -C(=O)- | M27 | S |
| 1518 | P3 | 2 | N | -C(=O)-NH- | M32 | S |
| 1519 | P3 | 2 | N | Single bond | M22 | S |
| 1520 | P4 | 0 | N | -C(=O)- | M43 | S |
| 1521 | P4 | 0 | N | -C(=O)-NH- | M38 | S |
| 1522 | P4 | 0 | N | Single bond | M48 | S |
| 1523 | P4 | 1 | N | -C(=O)- | M40 | S |
| 1524 | P4 | 1 | N | -C(=O)-NH- | M45 | S |
| 1525 | P4 | 1 | N | -C(=O)-NH-S(=O)₂- | M34 | S |
| 1526 | P4 | 1 | N | -C(=O)-O- | M36 | S |
| 1527 | P4 | 1 | N | -C(=O)-O- | M49 | S |
| 1528 | P4 | 1 | N | -C(=S)-NH- | M46 | S |
| 1529 | P4 | 1 | N | Single bond | M35 | S |
| 1530 | P4 | 1 | N | Single bond | M39 | S |
| 1531 | P4 | 1 | N | Single bond | M41 | O |
| 1532 | P4 | 2 | N | -C(=O)-NH-S(=O)₂- | M47 | S |
| 1533 | P4 | 2 | N | -C(=O)-O- | M42 | S |
| 1534 | P5 | 0 | N | -C(=O)- | M53 | S |
| 1535 | P5 | 0 | N | -C(=O)- | M63 | S |
| 1536 | P5 | 1 | N | -C(=O)- | M50 | S |
| 1537 | P5 | 1 | N | -C(=O)- | M56 | S |
| 1538 | P5 | 1 | N | -C(=O)-NH- | M64 | S |
| 1539 | P5 | 1 | N | -C(=O)-NH-S(=O)₂- | M60 | S |
| 1540 | P5 | 1 | N | -C(=O)-O- | M55 | S |
| 1541 | P5 | 1 | N | -C(=S)-NH- | M59 | S |
| 1542 | P5 | 1 | N | Single bond | M61 | O |
| 1543 | P5 | 1 | N | Single bond | M54 | S |
| 1544 | P5 | 2 | N | -C(=O)-O- | M62 | S |
| 1545 | P5 | 2 | N | Single bond | M52 | S |

**Table 41**

| Compound No. | G⁰ | n | A | (A)-G¹-(R³) | -R³-R⁴-G² | X |
|---|---|---|---|---|---|---|
| 1546 | P5 | 2 | N | Single bond | M57 | S |
| 1547 | P6 | 0 | N | -C(=O)-NH-S(=O)₂- | M73 | S |
| 1548 | P6 | 0 | N | -C(=O)-O- | M68 | S |
| 1549 | P6 | 0 | N | Single bond | M78 | S |
| 1550 | P6 | 1 | N | -C(=O)- | M66 | S |
| 1551 | P6 | 1 | N | -C(=O)- | M69 | S |
| 1552 | P6 | 1 | N | -C(=O)- | M76 | S |
| 1553 | P6 | 1 | N | -C(=O)-NH- | M71 | O |
| 1554 | P6 | 1 | N | -C(=O)-O- | M81 | O |
| 1555 | P6 | 1 | N | -C(=O)-O- | M75 | S |
| 1556 | P6 | 1 | N | Single bond | M70 | S |
| 1557 | P6 | 1 | N | Single bond | M74 | S |
| 1558 | P6 | 1 | N | Single bond | M80 | S |
| 1559 | P6 | 2 | N | -C(=O)-NH- | M77 | S |
| 1560 | P6 | 2 | N | Single bond | M67 | S |
| 1561 | P7 | 0 | N | -C(=O)-O- | M88 | S |
| 1562 | P7 | 0 | N | Single bond | M83 | S |
| 1563 | P7 | 1 | N | -C(=O)- | M89 | S |
| 1564 | P7 | 1 | N | -C(=O)- | M95 | S |
| 1565 | P7 | 1 | N | -C(=O)-NH- | M84 | S |
| 1566 | P7 | 1 | N | -C(=O)-NH- | M90 | S |
| 1567 | P7 | 1 | N | -C(=O)-O- | M94 | S |
| 1568 | P7 | 1 | N | -C(=S)-NH- | M85 | S |
| 1569 | P7 | 1 | N | Single bond | M91 | O |
| 1570 | P7 | 1 | N | Single bond | M96 | S |
| 1571 | P7 | 2 | N | -C(=O)- | M82 | S |
| 1572 | P7 | 2 | N | -C(=O)- | M92 | S |
| 1573 | P7 | 2 | N | -C(=O)-NH- | M97 | S |
| 1574 | P7 | 2 | N | Single bond | M87 | S |
| 1575 | P8 | 0 | N | -C(=O)- | M108 | S |
| 1576 | P8 | 0 | N | -C(=O)-NH- | M103 | S |
| 1577 | P8 | 0 | N | -C(=S)-NH- | M98 | S |
| 1578 | P8 | 0 | N | Single bond | M113 | S |
| 1579 | P8 | 1 | N | -C(=O)- | M105 | S |
| 1580 | P8 | 1 | N | -C(=O)-NH- | M110 | S |
| 1581 | P8 | 1 | N | -C(=O)-NH-S(=O)₂- | M99 | S |
| 1582 | P8 | 1 | N | -C(=O)-O- | M101 | O |
| 1583 | P8 | 1 | N | -C(=S)-NH- | M111 | O |

**Table 42**

| Compound No. | G⁰ | n | A | (A)-G¹-(R³) | -R³-R⁴-G² | X |
|---|---|---|---|---|---|---|
| 1584 | P8 | 1 | N | Single bond | M104 | S |
| 1585 | P8 | 1 | N | Single bond | M109 | S |
| 1586 | P8 | 1 | N | Single bond | M106 | S |
| 1587 | P8 | 2 | N | -C(=O)- | M102 | S |
| 1588 | P8 | 2 | N | -C(=O)-NH-S(=O)₂- | M112 | S |
| 1589 | P9 | 0 | N | -C(=O)- | M118 | S |
| 1590 | P9 | 0 | N | -C(=O)-NH- | M123 | S |
| 1591 | P9 | 1 | N | -C(=O)- | M115 | S |
| 1592 | P9 | 1 | N | -C(=O)-NH- | M116 | S |
| 1593 | P9 | 1 | N | -C(=O)-NH- | M129 | S |
| 1594 | P9 | 1 | N | -C(=O)-NH-S(=O)₂- | M125 | S |
| 1595 | P9 | 1 | N | -C(=O)-O- | M120 | S |
| 1596 | P9 | 1 | N | -C(=S)-NH- | M124 | S |
| 1597 | P9 | 1 | N | Single bond | M126 | S |
| 1598 | P9 | 1 | N | Single bond | M119 | S |
| 1599 | P9 | 2 | N | -C(=O)-O- | M127 | S |
| 1600 | P9 | 2 | N | Single bond | M117 | S |
| 1601 | P9 | 2 | N | Single bond | M122 | S |
| 1602 | P10 | 0 | N | -C(=O)-NH-S(=O)₂- | M138 | S |
| 1603 | P10 | 0 | N | -C(=O)-O- | M133 | S |
| 1604 | P10 | 0 | N | Single bond | M143 | S |
| 1605 | P10 | 1 | N | -C(=O)- | M131 | O |
| 1606 | P10 | 1 | N | -C(=O)- | M141 | O |
| 1607 | P10 | 1 | N | -C(=O)- | M134 | S |
| 1608 | P10 | 1 | N | -C(=O)- | M144 | S |
| 1609 | P10 | 1 | N | -C(=O)-NH- | M136 | S |
| 1610 | P10 | 1 | N | -C(=O)-O- | M140 | S |
| 1611 | P10 | 1 | N | Single bond | M130 | S |
| 1612 | P10 | 1 | N | Single bond | M139 | S |
| 1613 | P10 | 1 | N | Single bond | M145 | S |
| 1614 | P10 | 2 | N | -C(=S)-NH- | M137 | S |
| 1615 | P10 | 2 | N | Single bond | M132 | S |
| 1616 | P11 | 0 | N | -C(=O)-O- | M153 | S |
| 1617 | P11 | 0 | N | Single bond | M148 | S |
| 1618 | P11 | 0 | N | Single bond | M158 | S |
| 1619 | P11 | 1 | N | -C(=O)- | M154 | S |
| 1620 | P11 | 1 | N | -C(=O)- | M160 | S |
| 1621 | P11 | 1 | N | -C(=O)-NH- | M155 | S |

**Table 43**

| Compound No. | G⁰ | n | A | (A)-G¹-(R³) | -R³-R⁴-G² | X |
|---|---|---|---|---|---|---|
| 1622 | P11 | 1 | N | -C(=O)-NH-S(=O)₂- | M151 | O |
| 1623 | P11 | 1 | N | -C(=O)-O- | M146 | S |
| 1624 | P11 | 1 | N | -C(=O)-O- | M159 | S |
| 1625 | P11 | 1 | N | -C(=S)-NH- | M150 | S |
| 1626 | P11 | 1 | N | Single bond | M161 | O |
| 1627 | P11 | 2 | N | -C(=O)- | M147 | S |
| 1628 | P11 | 2 | N | -C(=O)- | M157 | S |
| 1629 | P11 | 2 | N | Single bond | M152 | S |
| 1630 | P12 | 0 | N | -C(=O)- | M173 | S |
| 1631 | P12 | 0 | N | -C(=O)-NH- | M168 | S |
| 1632 | P12 | 1 | N | -C(=O)-NH- | M175 | S |
| 1633 | P12 | 1 | N | -C(=O)-NH-S(=O)₂- | M164 | S |
| 1634 | P12 | 1 | N | -C(=O)-O- | M166 | S |
| 1635 | P12 | 1 | N | -C(=S)-NH- | M176 | S |
| 1636 | P12 | 1 | N | Single bond | M165 | S |
| 1637 | P12 | 1 | N | Single bond | M169 | S |
| 1638 | P12 | 1 | N | Single bond | M174 | S |
| 1639 | P12 | 1 | N | Single bond | M171 | O |
| 1640 | P12 | 2 | N | -C(=O)- | M167 | S |
| 1641 | P12 | 2 | N | -C(=O)-NH- | M162 | S |
| 1642 | P12 | 2 | N | -C(=O)-O- | M172 | S |
| 1643 | P13 | 0 | N | -C(=O)-O- | M178 | S |
| 1644 | P13 | 0 | N | -C(=S)-NH- | M188 | S |
| 1645 | P13 | 0 | N | Single bond | M183 | S |
| 1646 | P13 | 1 | N | -C(=O)- | M179 | S |
| 1647 | P13 | 1 | N | -C(=O)- | M185 | S |
| 1648 | P13 | 1 | N | -C(=O)-NH- | M180 | O |
| 1649 | P13 | 1 | N | -C(=O)-NH-S(=O)₂- | M189 | S |
| 1650 | P13 | 1 | N | Single bond | M190 | O |
| 1651 | P13 | 1 | N | Single bond | M181 | S |
| 1652 | P13 | 1 | N | Single bond | M186 | S |
| 1653 | P13 | 2 | N | -C(=O)- | M182 | S |
| 1654 | P13 | 2 | N | -C(=O)- | M192 | S |
| 1655 | P13 | 2 | N | -C(=O)-NH- | M187 | S |
| 1656 | P14 | 0 | N | -C(=O)- | M195 | S |
| 1657 | P14 | 0 | N | -C(=O)-NH- | M193 | S |
| 1658 | P14 | 0 | N | -C(=O)-NH- | M206 | S |
| 1659 | P14 | 0 | N | -C(=S)-NH- | M201 | S |

**Table 44**

| Compound No. | G⁰ | n | A | (A)-G¹-(R³) | -R³-R⁴-G² | X |
|---|---|---|---|---|---|---|
| 1660 | P14 | 1 | N | -C(=O)- | M208 | S |
| 1661 | P14 | 1 | N | -C(=O)-NH-S(=O)₂- | M202 | S |
| 1662 | P14 | 1 | N | -C(=O)-O- | M197 | S |
| 1663 | P14 | 1 | N | -C(=O)-O- | M204 | S |
| 1664 | P14 | 1 | N | Single bond | M194 | S |
| 1665 | P14 | 1 | N | Single bond | M203 | S |
| 1666 | P14 | 1 | N | Single bond | M207 | S |
| 1667 | P14 | 1 | N | Single bond | M196 | S |
| 1668 | P14 | 2 | N | -C(=O)-NH- | M200 | O |
| 1669 | P14 | 2 | N | Single bond | M199 | S |
| 1670 | P15 | 0 | N | -C(=O)- | M211 | S |
| 1671 | P15 | 0 | N | -C(=O)- | M221 | S |
| 1672 | P15 | 0 | N | Single bond | M216 | S |
| 1673 | P15 | 1 | N | -C(=O)- | M218 | S |
| 1674 | P15 | 1 | N | -C(=O)- | M224 | S |
| 1675 | P15 | 1 | N | -C(=O)-NH- | M213 | S |
| 1676 | P15 | 1 | N | -C(=O)-O- | M217 | S |
| 1677 | P15 | 1 | N | -C(=O)-O- | M223 | S |
| 1678 | P15 | 1 | N | -C(=S)-NH- | M214 | S |
| 1679 | P15 | 1 | N | Single bond | M209 | S |
| 1680 | P15 | 1 | N | Single bond | M222 | S |
| 1681 | P15 | 2 | N | -C(=O)-NH-S(=O)₂- | M215 | S |
| 1682 | P15 | 2 | N | -C(=O)-O- | M210 | O |
| 1683 | P15 | 2 | N | Single bond | M220 | O |
| 1684 | P16 | 0 | N | -C(=O)-O- | M230 | O |
| 1685 | P16 | 0 | N | Single bond | M235 | S |
| 1686 | P16 | 1 | N | -C(=O)- | M231 | S |
| 1687 | P16 | 1 | N | -C(=O)- | M237 | S |
| 1688 | P16 | 1 | N | -C(=O)-NH- | M232 | S |
| 1689 | P16 | 1 | N | -C(=O)-NH-S(=O)₂- | M228 | S |
| 1690 | P16 | 1 | N | -C(=O)-O- | M236 | S |
| 1691 | P16 | 1 | N | -C(=S)-NH- | M227 | S |
| 1692 | P16 | 1 | N | Single bond | M229 | S |
| 1693 | P16 | 1 | N | Single bond | M238 | S |
| 1694 | P16 | 2 | N | -C(=O)- | M234 | S |
| 1695 | P16 | 2 | N | -C(=O)-NH- | M239 | S |
| 1696 | P16 | 2 | N | Single bond | M225 | S |
| 1697 | P17 | 0 | N | -C(=O)- | M250 | O |

**Table 45**

| Compound No. | G⁰ | n | A | (A)-G¹-(R³) | -R³-R⁴-G² | X |
|---|---|---|---|---|---|---|
| 1698 | P17 | 0 | N | -C(=O)-NH- | M245 | S |
| 1699 | P17 | 0 | N | Single bond | M255 | S |
| 1700 | P17 | 1 | N | -C(=O)-NH- | M252 | S |
| 1701 | P17 | 1 | N | -C(=O)-NH-S(=O)₂- | M241 | S |
| 1702 | P17 | 1 | N | -C(=O)-O- | M243 | S |
| 1703 | P17 | 1 | N | -C(=O)-O- | M256 | S |
| 1704 | P17 | 1 | N | -C(=S)-NH- | M253 | S |
| 1705 | P17 | 1 | N | Single bond | M242 | S |
| 1706 | P17 | 1 | N | Single bond | M246 | S |
| 1707 | P17 | 1 | N | Single bond | M251 | S |
| 1708 | P17 | 1 | N | Single bond | M248 | S |
| 1709 | P17 | 2 | N | -C(=O)- | M244 | S |
| 1710 | P17 | 2 | N | -C(=O)-O- | M249 | S |
| 1711 | P18 | 0 | N | -C(=O)- | M260 | O |
| 1712 | P18 | 0 | N | -C(=O)-NH- | M265 | S |
| 1713 | P18 | 1 | N | -C(=O)- | M270 | O |
| 1714 | P18 | 1 | N | -C(=O)- | M257 | S |
| 1715 | P18 | 1 | N | -C(=O)- | M263 | S |
| 1716 | P18 | 1 | N | -C(=O)-NH- | M258 | S |
| 1717 | P18 | 1 | N | -C(=O)-NH- | M271 | S |
| 1718 | P18 | 1 | N | -C(=O)-NH-S(=O)₂- | M267 | S |
| 1719 | P18 | 1 | N | -C(=O)-O- | M262 | S |
| 1720 | P18 | 1 | N | -C(=S)-NH- | M266 | S |
| 1721 | P18 | 2 | N | -C(=O)-O- | M269 | S |
| 1722 | P18 | 2 | N | Single bond | M259 | S |
| 1723 | P18 | 2 | N | Single bond | M264 | S |
| 1724 | P18 | 2 | N | Single bond | M272 | S |
| 1725 | P19 | 0 | N | -C(=O)- | M273 | S |
| 1726 | P19 | 0 | N | -C(=O)- | M283 | S |
| 1727 | P19 | 0 | N | -C(=O)-NH- | M278 | S |
| 1728 | P19 | 1 | N | -C(=O)- | M276 | S |
| 1729 | P19 | 1 | N | -C(=O)- | M286 | S |
| 1730 | P19 | 1 | N | -C(=O)-NH- | M284 | S |
| 1731 | P19 | 1 | N | -C(=O)-NH-S(=O)₂- | M280 | S |
| 1732 | P19 | 1 | N | -C(=S)-NH- | M279 | O |
| 1733 | P19 | 1 | N | Single bond | M281 | S |
| 1734 | P19 | 1 | N | Single bond | M285 | S |
| 1735 | P19 | 1 | N | Single bond | M274 | S |

**Table 46**

| Compound No. | G⁰ | n | A | (A)-G¹-(R³) | -R³-R⁴-G² | X |
|---|---|---|---|---|---|---|
| 1736 | P19 | 2 | N | Single bond | M277 | S |
| 1737 | P19 | 2 | N | Single bond | M287 | S |
| 1738 | P20 | 0 | N | -C(=O)-NH-S(=O)₂- | M293 | S |
| 1739 | P20 | 0 | N | -C(=O)-O- | M288 | S |
| 1740 | P20 | 0 | N | Single bond | M298 | S |
| 1741 | P20 | 1 | N | -C(=O)- | M299 | O |
| 1742 | P20 | 1 | N | -C(=O)-NH- | M291 | S |
| 1743 | P20 | 1 | N | -C(=O)-O- | M295 | S |
| 1744 | P20 | 1 | N | -C(=O)-O- | M301 | S |
| 1745 | P20 | 1 | N | Single bond | M290 | S |
| 1746 | P20 | 1 | N | Single bond | M294 | S |
| 1747 | P20 | 1 | N | Single bond | M300 | S |
| 1748 | P20 | 2 | N | -C(=O)- | M302 | S |
| 1749 | P20 | 2 | N | -C(=O)-NH- | M297 | S |
| 1750 | P20 | 2 | N | -C(=S)-NH- | M292 | S |
| 1751 | P21 | 0 | N | -C(=O)- | M316 | S |
| 1752 | P21 | 0 | N | -C(=O)-NH- | M308 | S |
| 1753 | P21 | 0 | N | -C(=S)-NH- | M309 | O |
| 1754 | P21 | 0 | N | Single bond | M315 | S |
| 1755 | P21 | 1 | N | -C(=O)- | M319 | O |
| 1756 | P21 | 1 | N | -C(=O)- | M304 | S |
| 1757 | P21 | 1 | N | -C(=O)- | M305 | S |
| 1758 | P21 | 1 | N | -C(=O)- | M306 | S |
| 1759 | P21 | 1 | N | -C(=O)-O- | M312 | S |
| 1760 | P21 | 1 | N | -C(=O)-O- | M318 | S |
| 1761 | P21 | 1 | N | Single bond | M311 | S |
| 1762 | P21 | 2 | N | -C(=O)- | M313 | S |
| 1763 | P21 | 2 | N | -C(=O)-NH- | M314 | S |
| 1764 | P21 | 2 | N | Single bond | M307 | S |
| 1765 | P22 | 0 | N | -C(=O)- | M329 | O |
| 1766 | P22 | 0 | N | -C(=O)-NH-S(=O)₂- | M323 | S |
| 1767 | P22 | 0 | N | -C(=S)-NH- | M322 | S |
| 1768 | P22 | 0 | N | Single bond | M330 | S |
| 1769 | P22 | 1 | N | -C(=O)- | M326 | S |
| 1770 | P22 | 1 | N | -C(=O)- | M332 | S |
| 1771 | P22 | 1 | N | -C(=O)-O- | M325 | S |
| 1772 | P22 | 1 | N | Single bond | M333 | S |
| 1773 | P22 | 2 | N | -C(=O)-NH- | M321 | S |

**Table 47**

| Compound No. | G⁰ | n | A | (A)-G¹-(R³) | -R³-R⁴-G² | X |
|---|---|---|---|---|---|---|
| 1774 | P22 | 2 | N | -C(=O)-NH- | M327 | S |
| 1775 | P22 | 2 | N | -C(=O)-NH- | M334 | S |
| 1776 | P22 | 2 | N | -C(=S)-NH- | M335 | S |
| 1777 | P22 | 2 | N | Single bond | M320 | S |
| 1778 | P22 | 2 | N | Single bond | M328 | S |
| 1779 | P23 | 0 | N | -C(=O)-NH-S(=O)₂- | M336 | S |
| 1780 | P23 | 0 | N | -C(=O)-O- | M344 | S |
| 1781 | P23 | 0 | N | Single bond | M337 | S |
| 1782 | P23 | 0 | N | Single bond | M343 | S |
| 1783 | P23 | 1 | N | -C(=O)- | M339 | O |
| 1784 | P23 | 1 | N | -C(=O)-NH- | M340 | S |
| 1785 | P23 | 1 | N | -C(=O)-NH- | M347 | S |
| 1786 | P23 | 1 | N | -C(=O)-O- | M351 | S |
| 1787 | P23 | 1 | N | Single bond | M346 | S |
| 1788 | P23 | 1 | N | Single bond | M350 | S |
| 1789 | P23 | 2 | N | -C(=O)- | M342 | S |
| 1790 | P23 | 2 | N | -C(=O)-NH-S(=O)₂- | M349 | O |
| 1791 | P23 | 2 | N | -C(=S)-NH- | M348 | S |
| 1792 | P23 | 2 | N | Single bond | M341 | S |
| 1793 | P24 | 0 | N | -C(=O)- | M352 | S |
| 1794 | P24 | 0 | N | -C(=O)- | M355 | S |
| 1795 | P24 | 0 | N | -C(=O)- | M358 | S |
| 1796 | P24 | 0 | N | -C(=O)- | M360 | S |
| 1797 | P24 | 0 | N | -C(=O)- | M361 | S |
| 1798 | P24 | 0 | N | Single bond | M11 | O |
| 1799 | P24 | 0 | N | Single bond | M11 | S |
| 1800 | P24 | 1 | N | -C(=O)- | M359 | S |
| 1801 | P24 | 1 | N | -C(=O)- | M362 | S |
| 1802 | P24 | 1 | N | -C(=O)- | M365 | S |
| 1803 | P24 | 1 | N | -C(=O)-NH- | M353 | S |
| 1804 | P24 | 1 | N | Single bond | M356 | S |
| 1805 | P24 | 2 | N | -C(=O)- | M363 | S |
| 1806 | P24 | 2 | N | -C(=O)-O- | M357 | O |
| 1807 | P24 | 2 | N | Single bond | M354 | S |
| 1808 | P25 | 0 | N | -C(=O)- | M367 | O |
| 1809 | P25 | 0 | N | -C(=O)- | M369 | S |
| 1810 | P25 | 0 | N | -C(=O)- | M370 | S |
| 1811 | P25 | 0 | N | -C(=O)- | M373 | S |

**Table 48**

| Compound No. | G⁰ | n | A | (A)-G¹-(R³) | -R³-R⁴-G² | X |
|---|---|---|---|---|---|---|
| 1812 | P25 | 0 | N | -C(=S)-NH- | M3 | S |
| 1813 | P25 | 0 | N | Single bond | M5 | S |
| 1814 | P25 | 1 | N | -C(=O)- | M368 | S |
| 1815 | P25 | 1 | N | -C(=O)- | M371 | S |
| 1816 | P25 | 1 | N | -C(=O)- | M7 | S |
| 1817 | P25 | 1 | N | -C(=O)-NH-S(=O)₂- | M4 | O |
| 1818 | P25 | 1 | N | Single bond | M1 | S |
| 1819 | P25 | 2 | N | -C(=O)- | M366 | S |
| 1820 | P25 | 2 | N | -C(=O)- | M372 | S |
| 1821 | P25 | 2 | N | -C(=O)-NH- | M2 | S |
| 1822 | P25 | 2 | N | -C(=O)-NH- | M8 | S |
| 1823 | P26 | 0 | N | -C(=O)- | M25 | S |
| 1824 | P26 | 0 | N | -C(=O)-NH- | M17 | S |
| 1825 | P26 | 0 | N | -C(=O)-O- | M14 | S |
| 1826 | P26 | 0 | N | Single bond | M16 | O |
| 1827 | P26 | 0 | N | Single bond | M10 | S |
| 1828 | P26 | 0 | N | Single bond | M20 | S |
| 1829 | P26 | 0 | N | Single bond | M26 | O |
| 1830 | P26 | 1 | N | -C(=O)- | M12 | S |
| 1831 | P26 | 1 | N | -C(=O)- | M15 | S |
| 1832 | P26 | 1 | N | -C(=O)-O- | M21 | S |
| 1833 | P26 | 1 | N | -C(=S)-NH- | M18 | S |
| 1834 | P26 | 1 | N | Single bond | M24 | S |
| 1835 | P26 | 2 | N | -C(=O)- | M22 | S |
| 1836 | P26 | 2 | N | -C(=O)-NH-S(=O)₂- | M19 | S |
| 1837 | P26 | 2 | N | Single bond | M13 | S |
| 1838 | P27 | 0 | N | -C(=O)- | M35 | S |
| 1839 | P27 | 0 | N | -C(=O)- | M41 | S |
| 1840 | P27 | 0 | N | -C(=O)-NH-S(=O)₂- | M32 | S |
| 1841 | P27 | 0 | N | -C(=O)-O- | M34 | S |
| 1842 | P27 | 0 | N | Single bond | M29 | S |
| 1843 | P27 | 1 | N | -C(=O)-NH- | M36 | O |
| 1844 | P27 | 1 | N | -C(=O)-NH- | M30 | S |
| 1845 | P27 | 1 | N | -C(=O)-O- | M27 | S |
| 1846 | P27 | 1 | N | Single bond | M33 | S |
| 1847 | P27 | 1 | N | Single bond | M42 | S |
| 1848 | P27 | 1 | N | Single bond | M39 | S |
| 1849 | P27 | 2 | N | -C(=O)- | M28 | S |

**Table 49**

| Compound No. | G⁰ | n | A | (A)-G¹-(R³) | -R³-R⁴-G² | X |
|---|---|---|---|---|---|---|
| 1850 | P27 | 2 | N | -C(=O)-O- | M40 | S |
| 1851 | P27 | 2 | N | -C(=S)-NH- | M31 | S |
| 1852 | P27 | 2 | N | Single bond | M37 | S |
| 1853 | P28 | 0 | N | -C(=O)-NH- | M56 | O |
| 1854 | P28 | 0 | N | -C(=O)-NH- | M43 | S |
| 1855 | P28 | 0 | N | -C(=O)-O- | M47 | S |
| 1856 | P28 | 0 | N | -C(=S)-NH- | M44 | S |
| 1857 | P28 | 0 | N | Single bond | M50 | S |
| 1858 | P28 | 0 | N | Single bond | M52 | S |
| 1859 | P28 | 1 | N | -C(=O)- | M48 | S |
| 1860 | P28 | 1 | N | -C(=O)- | M51 | S |
| 1861 | P28 | 1 | N | -C(=O)- | M54 | S |
| 1862 | P28 | 1 | N | -C(=O)-NH-S(=O)₂- | M45 | S |
| 1863 | P28 | 1 | N | -C(=S)-NH- | M57 | S |
| 1864 | P28 | 2 | N | -C(=O)-NH- | M49 | S |
| 1865 | P28 | 2 | N | -C(=O)-NH-S(=O)₂- | M58 | S |
| 1866 | P28 | 2 | N | Single bond | M46 | O |
| 1867 | P28 | 2 | N | Single bond | M55 | S |
| 1868 | P29 | 0 | N | -C(=O)- | M61 | S |
| 1869 | P29 | 0 | N | -C(=O)- | M74 | S |
| 1870 | P29 | 0 | N | -C(=O)-NH- | M62 | S |
| 1871 | P29 | 0 | N | -C(=O)-NH-S(=O)₂- | M71 | S |
| 1872 | P29 | 0 | N | -C(=S)-NH- | M70 | S |
| 1873 | P29 | 0 | N | Single bond | M59 | S |
| 1874 | P29 | 0 | N | Single bond | M65 | S |
| 1875 | P29 | 1 | N | -C(=O)-NH- | M69 | S |
| 1876 | P29 | 1 | N | -C(=O)-O- | M66 | O |
| 1877 | P29 | 1 | N | -C(=O)-O- | M60 | S |
| 1878 | P29 | 1 | N | Single bond | M63 | S |
| 1879 | P29 | 1 | N | Single bond | M72 | S |
| 1880 | P29 | 2 | N | -C(=O)- | M64 | S |
| 1881 | P29 | 2 | N | -C(=O)- | M67 | S |
| 1882 | P29 | 2 | N | -C(=O)-O- | M73 | S |
| 1883 | P30 | 0 | N | -C(=O)- | M77 | S |
| 1884 | P30 | 0 | N | -C(=O)- | M80 | S |
| 1885 | P30 | 0 | N | -C(=O)-NH- | M88 | S |
| 1886 | P30 | 0 | N | -C(=O)-O- | M86 | O |
| 1887 | P30 | 0 | N | -C(=O)-O- | M79 | S |

**Table 50**

| Compound No. | G⁰ | n | A | (A)-G¹-(R³) | -R³-R⁴-G² | X |
|---|---|---|---|---|---|---|
| 1888 | P30 | 0 | N | Single bond | M89 | S |
| 1889 | P30 | 1 | N | -C(=O)- | M87 | S |
| 1890 | P30 | 1 | N | -C(=O)- | M90 | S |
| 1891 | P30 | 1 | N | -C(=O)-NH- | M75 | S |
| 1892 | P30 | 1 | N | -C(=O)-NH-S(=O)₂- | M84 | S |
| 1893 | P30 | 1 | N | Single bond | M81 | S |
| 1894 | P30 | 1 | N | Single bond | M78 | S |
| 1895 | P30 | 2 | N | -C(=O)-NH- | M82 | S |
| 1896 | P30 | 2 | N | Single bond | M76 | O |
| 1897 | P30 | 2 | N | Single bond | M85 | S |
| 1898 | P31 | 0 | N | -C(=O)- | M106 | O |
| 1899 | P31 | 0 | N | -C(=O)-NH- | M95 | S |
| 1900 | P31 | 0 | N | -C(=O)-NH- | M101 | S |
| 1901 | P31 | 0 | N | -C(=O)-NH-S(=O)₂- | M97 | S |
| 1902 | P31 | 0 | N | -C(=O)-O- | M92 | S |
| 1903 | P31 | 0 | N | Single bond | M104 | S |
| 1904 | P31 | 1 | N | -C(=O)- | M93 | S |
| 1905 | P31 | 1 | N | -C(=O)-O- | M99 | S |
| 1906 | P31 | 1 | N | -C(=O)-O- | M105 | S |
| 1907 | P31 | 1 | N | -C(=S)-NH- | M96 | O |
| 1908 | P31 | 1 | N | Single bond | M102 | S |
| 1909 | P31 | 2 | N | -C(=O)- | M100 | S |
| 1910 | P31 | 2 | N | -C(=O)- | M103 | S |
| 1911 | P31 | 2 | N | Single bond | M94 | S |
| 1912 | P31 | 2 | N | Single bond | M91 | S |
| 1913 | P32 | 0 | N | -C(=O)- | M116 | O |
| 1914 | P32 | 0 | N | -C(=O)- | M119 | S |
| 1915 | P32 | 0 | N | -C(=O)-NH-S(=O)₂- | M110 | S |
| 1916 | P32 | 0 | N | -C(=S)-NH- | M122 | S |
| 1917 | P32 | 0 | N | Single bond | M107 | S |
| 1918 | P32 | 0 | N | Single bond | M115 | S |
| 1919 | P32 | 1 | N | -C(=O)-NH- | M108 | S |
| 1920 | P32 | 1 | N | -C(=O)-NH- | M114 | S |
| 1921 | P32 | 1 | N | Single bond | M111 | S |
| 1922 | P32 | 1 | N | Single bond | M120 | S |
| 1923 | P32 | 1 | N | Single bond | M117 | S |
| 1924 | P32 | 2 | N | -C(=O)-NH- | M121 | S |
| 1925 | P32 | 2 | N | -C(=O)-O- | M112 | S |

**Table 51**

| Compound No. | G⁰ | n | A | (A)-G¹-(R³) | -R³-R⁴-G² | X |
|---|---|---|---|---|---|---|
| 1926 | P32 | 2 | N | -C(=O)-O- | M118 | S |
| 1927 | P32 | 2 | N | -C(=S)-NH- | M109 | S |
| 1928 | P33 | 0 | N | -C(=O)-NH- | M134 | S |
| 1929 | P33 | 0 | N | -C(=O)-O- | M125 | S |
| 1930 | P33 | 0 | N | -C(=O)-O- | M131 | S |
| 1931 | P33 | 0 | N | Single bond | M124 | S |
| 1932 | P33 | 0 | N | Single bond | M133 | S |
| 1933 | P33 | 0 | N | Single bond | M137 | S |
| 1934 | P33 | 1 | N | -C(=O)- | M126 | O |
| 1935 | P33 | 1 | N | -C(=O)- | M129 | S |
| 1936 | P33 | 1 | N | -C(=O)- | M132 | S |
| 1937 | P33 | 1 | N | -C(=O)-NH-S(=O)₂- | M123 | S |
| 1938 | P33 | 1 | N | -C(=O)-O- | M138 | S |
| 1939 | P33 | 1 | N | -C(=S)-NH- | M135 | S |
| 1940 | P33 | 2 | N | -C(=O)-NH- | M127 | S |
| 1941 | P33 | 2 | N | -C(=O)-NH-S(=O)₂- | M136 | O |
| 1942 | P33 | 2 | N | Single bond | M130 | S |
| 1943 | P34 | 0 | N | -C(=O)- | M142 | S |
| 1944 | P34 | 0 | N | -C(=O)- | M152 | S |
| 1945 | P34 | 0 | N | -C(=O)-NH- | M140 | S |
| 1946 | P34 | 0 | N | -C(=O)-NH-S(=O)₂- | M149 | S |
| 1947 | P34 | 0 | N | -C(=O)-O- | M151 | S |
| 1948 | P34 | 0 | N | Single bond | M146 | O |
| 1949 | P34 | 1 | N | -C(=O)-NH- | M147 | S |
| 1950 | P34 | 1 | N | -C(=O)-NH- | M153 | S |
| 1951 | P34 | 1 | N | -C(=O)-O- | M144 | S |
| 1952 | P34 | 1 | N | Single bond | M141 | S |
| 1953 | P34 | 1 | N | Single bond | M150 | S |
| 1954 | P34 | 2 | N | -C(=O)- | M139 | S |
| 1955 | P34 | 2 | N | -C(=O)- | M145 | S |
| 1956 | P34 | 2 | N | -C(=S)-NH- | M148 | S |
| 1957 | P34 | 2 | N | Single bond | M154 | S |
| 1958 | P35 | 0 | N | -C(=O)- | M155 | S |
| 1959 | P35 | 0 | N | -C(=O)-NH- | M160 | S |
| 1960 | P35 | 0 | N | -C(=O)-O- | M164 | S |
| 1961 | P35 | 0 | N | -C(=O)-O- | M170 | S |
| 1962 | P35 | 0 | N | -C(=S)-NH- | M161 | S |
| 1963 | P35 | 0 | N | Single bond | M167 | S |

**Table 52**

| Compound No. | G⁰ | n | A | (A)-G¹-(R³) | -R³-R⁴-G² | X |
|---|---|---|---|---|---|---|
| 1964 | P35 | 0 | N | Single bond | M169 | S |
| 1965 | P35 | 1 | N | -C(=O)- | M165 | S |
| 1966 | P35 | 1 | N | -C(=O)- | M168 | S |
| 1967 | P35 | 1 | N | -C(=O)-NH-S(=O)₂- | M162 | S |
| 1968 | P35 | 1 | N | Single bond | M159 | S |
| 1969 | P35 | 1 | N | Single bond | M156 | O |
| 1970 | P35 | 2 | N | -C(=O)-NH- | M166 | O |
| 1971 | P35 | 2 | N | -C(=O)-O- | M157 | S |
| 1972 | P35 | 2 | N | Single bond | M163 | S |
| 1973 | P36 | 0 | N | -C(=O)- | M178 | S |
| 1974 | P36 | 0 | N | -C(=O)-NH- | M179 | S |
| 1975 | P36 | 0 | N | Single bond | M176 | O |
| 1976 | P36 | 0 | N | Single bond | M185 | S |
| 1977 | P36 | 0 | N | Single bond | M182 | S |
| 1978 | P36 | 1 | N | -C(=O)- | M171 | S |
| 1979 | P36 | 1 | N | -C(=O)-NH- | M186 | O |
| 1980 | P36 | 1 | N | -C(=O)-O- | M177 | S |
| 1981 | P36 | 1 | N | -C(=O)-O- | M183 | S |
| 1982 | P36 | 1 | N | -C(=S)-NH- | M174 | S |
| 1983 | P36 | 1 | N | Single bond | M180 | S |
| 1984 | P36 | 2 | N | -C(=O)- | M181 | S |
| 1985 | P36 | 2 | N | -C(=O)- | M184 | S |
| 1986 | P36 | 2 | N | -C(=O)-NH-S(=O)₂- | M175 | S |
| 1987 | P36 | 2 | N | Single bond | M172 | S |
| 1988 | P37 | 0 | N | -C(=O)- | M191 | S |
| 1989 | P37 | 0 | N | -C(=O)- | M194 | S |
| 1990 | P37 | 0 | N | -C(=O)- | M197 | S |
| 1991 | P37 | 0 | N | -C(=O)-O- | M196 | O |
| 1992 | P37 | 0 | N | -C(=S)-NH- | M187 | S |
| 1993 | P37 | 0 | N | -C(=S)-NH- | M200 | S |
| 1994 | P37 | 1 | N | -C(=O)-NH- | M192 | S |
| 1995 | P37 | 1 | N | -C(=O)-NH-S(=O)₂- | M201 | S |
| 1996 | P37 | 1 | N | Single bond | M189 | S |
| 1997 | P37 | 1 | N | Single bond | M198 | S |
| 1998 | P37 | 1 | N | Single bond | M195 | S |
| 1999 | P37 | 2 | N | -C(=O)-NH- | M199 | S |
| 2000 | P37 | 2 | N | -C(=O)-O- | M190 | S |
| 2001 | P37 | 2 | N | Single bond | M193 | S |

**Table 53**

| Compound No. | G⁰ | n | A | (A)-G¹-(R³) | -R³-R⁴-G² | X |
|---|---|---|---|---|---|---|
| 2002 | P37 | 2 | N | Single bond | M202 | S |
| 2003 | P38 | 0 | N | -C(=O)-NH- | M205 | S |
| 2004 | P38 | 0 | N | -C(=O)-NH- | M212 | S |
| 2005 | P38 | 0 | N | -C(=O)-NH-S(=O)₂- | M214 | S |
| 2006 | P38 | 0 | N | -C(=O)-O- | M209 | S |
| 2007 | P38 | 0 | N | Single bond | M206 | O |
| 2008 | P38 | 0 | N | Single bond | M215 | S |
| 2009 | P38 | 1 | N | -C(=O)- | M204 | S |
| 2010 | P38 | 1 | N | -C(=O)- | M207 | S |
| 2011 | P38 | 1 | N | -C(=O)- | M210 | S |
| 2012 | P38 | 1 | N | -C(=O)-O- | M216 | O |
| 2013 | P38 | 1 | N | -C(=S)-NH- | M213 | S |
| 2014 | P38 | 2 | N | -C(=O)- | M217 | S |
| 2015 | P38 | 2 | N | Single bond | M211 | S |
| 2016 | P38 | 2 | N | Single bond | M208 | S |
| 2017 | P39 | 0 | N | -C(=O)- | M223 | S |
| 2018 | P39 | 0 | N | -C(=O)- | M230 | S |
| 2019 | P39 | 0 | N | -C(=O)-NH-S(=O)₂- | M227 | S |
| 2020 | P39 | 0 | N | Single bond | M224 | S |
| 2021 | P39 | 0 | N | Single bond | M232 | S |
| 2022 | P39 | 0 | N | Single bond | M221 | S |
| 2023 | P39 | 1 | N | -C(=O)-NH- | M225 | S |
| 2024 | P39 | 1 | N | -C(=O)-NH- | M231 | S |
| 2025 | P39 | 1 | N | -C(=O)-O- | M222 | S |
| 2026 | P39 | 1 | N | Single bond | M219 | S |
| 2027 | P39 | 1 | N | Single bond | M228 | S |
| 2028 | P39 | 1 | N | Single bond | M234 | S |
| 2029 | P39 | 2 | N | -C(=O)- | M220 | S |
| 2030 | P39 | 2 | N | -C(=O)-O- | M229 | S |
| 2031 | P39 | 2 | N | -C(=O)-O- | M235 | S |
| 2032 | P39 | 2 | N | -C(=S)-NH- | M226 | O |
| 2033 | P40 | 0 | N | -C(=O)- | M236 | O |
| 2034 | P40 | 0 | N | -C(=O)-NH- | M251 | S |
| 2035 | P40 | 0 | N | -C(=O)-O- | M242 | S |
| 2036 | P40 | 0 | N | -C(=S)-NH- | M239 | S |
| 2037 | P40 | 0 | N | Single bond | M241 | S |
| 2038 | P40 | 0 | N | Single bond | M245 | S |
| 2039 | P40 | 0 | N | Single bond | M250 | S |

**Table 54**

| Compound No. | G⁰ | n | A | (A)-G¹-(R³) | -R³-R⁴-G² | X |
|---|---|---|---|---|---|---|
| 2040 | P40 | 1 | N | -C(=O)- | M246 | O |
| 2041 | P40 | 1 | N | -C(=O)- | M243 | S |
| 2042 | P40 | 1 | N | -C(=O)- | M249 | S |
| 2043 | P40 | 1 | N | -C(=O)-NH-S(=O)₂- | M240 | S |
| 2044 | P40 | 1 | N | -C(=S)-NH- | M252 | S |
| 2045 | P40 | 1 | N | Single bond | M237 | S |
| 2046 | P40 | 2 | N | -C(=O)-NH- | M238 | S |
| 2047 | P40 | 2 | N | -C(=O)-NH- | M244 | S |
| 2048 | P40 | 2 | N | Single bond | M247 | S |
| 2049 | P41 | 0 | N | -C(=O)- | M259 | S |
| 2050 | P41 | 0 | N | -C(=O)- | M269 | S |
| 2051 | P41 | 0 | N | -C(=O)-NH- | M257 | S |
| 2052 | P41 | 0 | N | -C(=O)-NH-S(=O)₂- | M266 | O |
| 2053 | P41 | 0 | N | -C(=O)-O- | M268 | S |
| 2054 | P41 | 0 | N | Single bond | M254 | S |
| 2055 | P41 | 0 | N | Single bond | M260 | S |
| 2056 | P41 | 1 | N | -C(=O)-NH- | M264 | S |
| 2057 | P41 | 1 | N | -C(=O)-O- | M255 | S |
| 2058 | P41 | 1 | N | -C(=O)-O- | M261 | S |
| 2059 | P41 | 1 | N | Single bond | M258 | S |
| 2060 | P41 | 1 | N | Single bond | M267 | S |
| 2061 | P41 | 2 | N | -C(=O)- | M256 | O |
| 2062 | P41 | 2 | N | -C(=O)- | M262 | S |
| 2063 | P41 | 2 | N | -C(=O)-NH-S(=O)₂- | M253 | S |
| 2064 | P41 | 2 | N | -C(=S)-NH- | M265 | S |
| 2065 | P42 | 0 | N | -C(=O)- | M272 | S |
| 2066 | P42 | 0 | N | -C(=O)- | M275 | S |
| 2067 | P42 | 0 | N | -C(=O)-NH- | M277 | S |
| 2068 | P42 | 0 | N | -C(=O)-O- | M281 | S |
| 2069 | P42 | 0 | N | Single bond | M284 | S |
| 2070 | P42 | 0 | N | Single bond | M286 | O |
| 2071 | P42 | 1 | N | -C(=O)- | M282 | S |
| 2072 | P42 | 1 | N | -C(=O)- | M285 | S |
| 2073 | P42 | 1 | N | -C(=O)-NH- | M270 | S |
| 2074 | P42 | 1 | N | -C(=O)-NH-S(=O)₂- | M279 | S |
| 2075 | P42 | 1 | N | Single bond | M276 | O |
| 2076 | P42 | 1 | N | Single bond | M273 | S |
| 2077 | P42 | 2 | N | -C(=O)-NH- | M283 | S |

**Table 55**

| Compound No. | G⁰ | n | A | (A)-G¹-(R³) | -R³-R⁴-G² | X |
|---|---|---|---|---|---|---|
| 2078 | P42 | 2 | N | -C(=O)-O- | M274 | S |
| 2079 | P42 | 2 | N | Single bond | M271 | S |
| 2080 | P42 | 2 | N | Single bond | M280 | S |
| 2081 | P43 | 0 | N | -C(=O)- | M295 | S |
| 2082 | P43 | 0 | N | -C(=O)-NH- | M296 | O |
| 2083 | P43 | 0 | N | -C(=O)-NH- | M290 | S |
| 2084 | P43 | 0 | N | -C(=O)-O- | M287 | S |
| 2085 | P43 | 0 | N | Single bond | M302 | S |
| 2086 | P43 | 0 | N | Single bond | M299 | S |
| 2087 | P43 | 1 | N | -C(=O)- | M288 | S |
| 2088 | P43 | 1 | N | -C(=O)-NH- | M303 | S |
| 2089 | P43 | 1 | N | -C(=O)-O- | M294 | S |
| 2090 | P43 | 1 | N | -C(=O)-O- | M300 | S |
| 2091 | P43 | 1 | N | -C(=S)-NH- | M291 | S |
| 2092 | P43 | 1 | N | Single bond | M297 | S |
| 2093 | P43 | 2 | N | -C(=O)- | M298 | S |
| 2094 | P43 | 2 | N | -C(=O)- | M301 | S |
| 2095 | P43 | 2 | N | -C(=O)-NH-S(=O)₂- | M292 | S |
| 2096 | P43 | 2 | N | Single bond | M289 | S |
| 2097 | P44 | 0 | N | -C(=O)- | M311 | S |
| 2098 | P44 | 0 | N | -C(=O)- | M314 | S |
| 2099 | P44 | 0 | N | -C(=O)-NH-S(=O)₂- | M305 | S |
| 2100 | P44 | 0 | N | -C(=O)-O- | M313 | S |
| 2101 | P44 | 0 | N | -C(=O)-O- | M320 | S |
| 2102 | P44 | 0 | N | -C(=S)-NH- | M304 | S |
| 2103 | P44 | 0 | N | -C(=S)-NH- | M317 | S |
| 2104 | P44 | 1 | N | -C(=O)-NH- | M309 | S |
| 2105 | P44 | 1 | N | -C(=O)-NH-S(=O)₂- | M318 | S |
| 2106 | P44 | 1 | N | Single bond | M306 | O |
| 2107 | P44 | 1 | N | Single bond | M315 | S |
| 2108 | P44 | 1 | N | Single bond | M312 | S |
| 2109 | P44 | 2 | N | -C(=O)-NH- | M316 | O |
| 2110 | P44 | 2 | N | -C(=O)-O- | M307 | S |
| 2111 | P44 | 2 | N | Single bond | M310 | S |
| 2112 | P44 | 2 | N | Single bond | M319 | S |
| 2113 | P45 | 0 | N | -C(=O)-NH- | M322 | S |
| 2114 | P45 | 0 | N | -C(=O)-NH- | M329 | S |
| 2115 | P45 | 0 | N | -C(=O)-NH- | M335 | S |

**Table 56**

| Compound No. | G⁰ | n | A | (A)-G¹-(R³) | -R³-R⁴-G² | X |
|---|---|---|---|---|---|---|
| 2116 | P45 | 0 | N | -C(=O)-NH-S(=O)₂- | M331 | S |
| 2117 | P45 | 0 | N | -C(=O)-O- | M326 | O |
| 2118 | P45 | 0 | N | Single bond | M332 | S |
| 2119 | P45 | 1 | N | -C(=O)- | M321 | S |
| 2120 | P45 | 1 | N | -C(=O)- | M324 | S |
| 2121 | P45 | 1 | N | -C(=O)- | M327 | S |
| 2122 | P45 | 1 | N | -C(=O)-O- | M333 | S |
| 2123 | P45 | 1 | N | -C(=S)-NH- | M330 | S |
| 2124 | P45 | 1 | N | Single bond | M336 | O |
| 2125 | P45 | 2 | N | -C(=O)- | M334 | S |
| 2126 | P45 | 2 | N | -C(=O)- | M337 | S |
| 2127 | P45 | 2 | N | Single bond | M328 | S |
| 2128 | P45 | 2 | N | Single bond | M325 | S |
| 2129 | P46 | 0 | N | -C(=O)- | M340 | S |
| 2130 | P46 | 0 | N | -C(=O)- | M347 | S |
| 2131 | P46 | 0 | N | -C(=O)- | M350 | S |
| 2132 | P46 | 0 | N | -C(=O)-NH-S(=O)₂- | M344 | S |
| 2133 | P46 | 0 | N | -C(=S)-NH- | M356 | O |
| 2134 | P46 | 0 | N | Single bond | M341 | S |
| 2135 | P46 | 0 | N | Single bond | M349 | S |
| 2136 | P46 | 1 | N | -C(=O)-NH- | M342 | S |
| 2137 | P46 | 1 | N | -C(=O)-NH- | M348 | S |
| 2138 | P46 | 1 | N | -C(=O)-O- | M339 | S |
| 2139 | P46 | 1 | N | Single bond | M345 | S |
| 2140 | P46 | 1 | N | Single bond | M354 | S |
| 2141 | P46 | 1 | N | Single bond | M351 | S |
| 2142 | P46 | 2 | N | -C(=O)-NH- | M355 | S |
| 2143 | P46 | 2 | N | -C(=O)-O- | M346 | O |
| 2144 | P46 | 2 | N | -C(=O)-O- | M352 | S |
| 2145 | P46 | 2 | N | -C(=S)-NH- | M343 | S |
| 2146 | P47 | 0 | N | -C(=O)-O- | M359 | S |
| 2147 | P47 | 0 | N | -C(=O)-O- | M365 | S |
| 2148 | P47 | 0 | N | Single bond | M358 | S |
| 2149 | P47 | 0 | N | Single bond | M362 | S |
| 2150 | P47 | 0 | N | Single bond | M367 | S |
| 2151 | P47 | 0 | N | Single bond | M371 | S |
| 2152 | P47 | 1 | N | -C(=O)- | M366 | O |
| 2153 | P47 | 1 | N | -C(=O)- | M360 | S |

**Table 57**

| Compound No. | G⁰ | n | A | (A)-G¹-(R³) | -R³-R⁴-G² | X |
|---|---|---|---|---|---|---|
| 2154 | P47 | 1 | N | -C(=O)- | M363 | S |
| 2155 | P47 | 1 | N | -C(=O)-NH-S(=O)₂- | M357 | S |
| 2156 | P47 | 1 | N | -C(=O)-O- | M372 | S |
| 2157 | P47 | 1 | N | -C(=S)-NH- | M369 | S |
| 2158 | P47 | 2 | N | -C(=O)- | M373 | S |
| 2159 | P47 | 2 | N | -C(=O)-NH- | M361 | S |
| 2160 | P47 | 2 | N | -C(=O)-NH-S(=O)₂- | M370 | S |
| 2161 | P47 | 2 | N | Single bond | M364 | S |
| 2162 | P2 | 1 | CH | -C(=O)-O- | M16 | S |
| 2163 | P2 | 1 | CH | Single bond | M7 | S |
| 2164 | P2 | 1 | CH | Single bond | M9 | S |
| 2165 | P3 | 0 | CH | -C(=O)-O- | M23 | S |
| 2166 | P3 | 1 | CH | -C(=O)- | M30 | S |
| 2167 | P4 | 1 | CH | Single bond | M44 | S |
| 2168 | P4 | 2 | CH | -C(=O)- | M37 | S |
| 2169 | P5 | 0 | CH | -C(=O)-NH- | M58 | S |
| 2170 | P5 | 1 | CH | -C(=O)-NH- | M51 | O |
| 2171 | P5 | 1 | CH | Single bond | M65 | S |
| 2172 | P6 | 1 | CH | -C(=O)- | M79 | S |
| 2173 | P6 | 2 | CH | -C(=S)-NH- | M72 | S |
| 2174 | P7 | 0 | CH | Single bond | M93 | S |
| 2175 | P7 | 1 | CH | -C(=O)-NH-S(=O)₂- | M86 | S |
| 2176 | P8 | 1 | CH | Single bond | M100 | S |
| 2177 | P8 | 2 | CH | -C(=O)-O- | M107 | S |
| 2178 | P9 | 0 | CH | -C(=O)- | M128 | S |
| 2179 | P9 | 1 | CH | -C(=O)- | M121 | O |
| 2180 | P9 | 1 | CH | -C(=O)-O- | M114 | S |
| 2181 | P10 | 1 | CH | Single bond | M135 | S |
| 2182 | P10 | 2 | CH | -C(=O)-NH- | M142 | S |
| 2183 | P11 | 1 | CH | -C(=O)-NH- | M149 | S |
| 2184 | P11 | 1 | CH | Single bond | M156 | S |
| 2185 | P12 | 0 | CH | -C(=S)-NH- | M163 | S |
| 2186 | P12 | 1 | CH | -C(=O)- | M170 | S |
| 2187 | P12 | 2 | CH | -C(=O)-NH-S(=O)₂- | M177 | S |
| 2188 | P13 | 1 | CH | -C(=O)-O- | M184 | S |
| 2189 | P13 | 1 | CH | -C(=O)-O- | M191 | S |
| 2190 | P13 | 1 | CH | Single bond | M9 | O |
| 2191 | P14 | 1 | CH | -C(=O)- | M198 | S |

**Table 58**

| Compound No. | G⁰ | n | A | (A)-G¹-(R³) | -R³-R⁴-G² | X |
|---|---|---|---|---|---|---|
| 2192 | P14 | 2 | CH | -C(=O)- | M205 | S |
| 2193 | P15 | 1 | CH | -C(=O)-NH- | M219 | S |
| 2194 | P15 | 1 | CH | Single bond | M212 | S |
| 2195 | P16 | 0 | CH | -C(=O)-NH- | M226 | S |
| 2196 | P16 | 0 | CH | -C(=S)-NH- | M240 | O |
| 2197 | P16 | 1 | CH | Single bond | M233 | S |
| 2198 | P17 | 1 | CH | -C(=O)- | M247 | S |
| 2199 | P17 | 2 | CH | -C(=O)-NH-S(=O)₂- | M254 | S |
| 2200 | P18 | 1 | CH | Single bond | M268 | S |
| 2201 | P18 | 1 | CH | Single bond | M261 | S |
| 2202 | P19 | 1 | CH | -C(=O)-O- | M275 | S |
| 2203 | P19 | 1 | CH | Single bond | M9 | O |
| 2204 | P19 | 2 | CH | -C(=O)-O- | M282 | S |
| 2205 | P20 | 0 | CH | -C(=O)- | M303 | S |
| 2206 | P20 | 1 | CH | -C(=O)- | M289 | S |
| 2207 | P20 | 1 | CH | -C(=O)- | M296 | S |
| 2208 | P21 | 1 | CH | -C(=O)-NH-S(=O)₂- | M310 | S |
| 2209 | P21 | 1 | CH | Single bond | M317 | S |
| 2210 | P22 | 1 | CH | -C(=O)-O- | M331 | S |
| 2211 | P22 | 1 | CH | Single bond | M324 | S |
| 2212 | P23 | 1 | CH | -C(=O)- | M345 | S |
| 2213 | P23 | 1 | CH | -C(=O)-O- | M338 | S |
| 2214 | P24 | 0 | CH | -C(=O)- | M364 | S |
| 2215 | P25 | 0 | CH | -C(=O)-O- | M6 | S |
| 2216 | P26 | 0 | CH | -C(=O)-NH- | M23 | S |
| 2217 | P27 | 0 | CH | -C(=O)- | M38 | S |
| 2218 | P28 | 0 | CH | -C(=O)-O- | M53 | S |
| 2219 | P29 | 0 | CH | Single bond | M68 | S |
| 2220 | P30 | 0 | CH | -C(=S)-NH- | M83 | S |
| 2221 | P31 | 0 | CH | Single bond | M98 | S |
| 2222 | P32 | 0 | CH | -C(=O)- | M113 | S |
| 2223 | P33 | 0 | CH | Single bond | M128 | S |
| 2224 | P34 | 0 | CH | Single bond | M143 | S |
| 2225 | P35 | 0 | CH | -C(=O)- | M158 | S |
| 2226 | P36 | 0 | CH | -C(=O)-NH- | M173 | S |
| 2227 | P37 | 0 | CH | -C(=O)-NH-S(=O)₂- | M188 | S |
| 2228 | P38 | 0 | CH | -C(=O)-NH- | M218 | S |
| 2229 | P38 | 0 | CH | -C(=O)-O- | M203 | S |

**Table 59**

| Compound No. | G⁰ | n | A | (A)-G¹-(R³) | -R³-R⁴-G² | X |
|---|---|---|---|---|---|---|
| 2230 | P39 | 0 | CH | -C(=O)- | M233 | S |
| 2231 | P40 | 0 | CH | -C(=O)-O- | M248 | S |
| 2232 | P41 | 0 | CH | Single bond | M263 | S |
| 2233 | P42 | 0 | CH | -C(=S)-NH- | M278 | S |
| 2234 | P43 | 0 | CH | Single bond | M293 | S |
| 2235 | P44 | 0 | CH | -C(=O)- | M308 | S |
| 2236 | P45 | 0 | CH | Single bond | M323 | S |
| 2237 | P46 | 0 | CH | -C(=O)- | M353 | S |
| 2238 | P46 | 0 | CH | Single bond | M338 | S |
| 2239 | P47 | 0 | CH | -C(=O)-NH- | M368 | S |
| 2240 | P1 | 1 | N | -C(=S)-NH- | M110 | S |
| 2241 | P1 | 1 | N | -C(=S)-NH- | M27 | S |
| 2242 | P1 | 1 | N | Single bond | M72 | S |
| 2243 | P1 | 0 | N | -C(=O)- | M127 | O |
| 2244 | P1 | 0 | N | -C(=O)- | M128 | O |

In the above Formula (II), n, A, R³, R⁴, G⁰, G¹ and G² are as defined for n, A, R³, R⁴, G⁰, G¹ and G², respectively, in the above Formula (I), and referred to as the same one illustrated in each of them.

In the above Formula (II), X¹ represents a chlorine atom, a bromine atom, an iodine atom, or an alkyl or arylsulfonyloxy group having one to eight carbons optionally substituted with a fluorine atom, a chlorine atom, or a bromine atom. When X¹ represents a chlorine atom, a bromine atom, an iodine atom, or an alkyl or arylsulfonyloxy group having one to eight carbons optionally substituted with a fluorine atom, a chlorine atom, or a bromine atom, examples of said a chlorine atom, a bromine atom, an iodine atom, or an alkyl or arylsulfonyloxy group having one to eight carbons optionally substituted with a fluorine atom, a chlorine atom, or a bromine atom include methylsulfonyloxy, trifluoromethylsulfonyloxy, ethylsulfonyloxy, propylsulfonyloxy, butylsulfonyloxy, nonafluorobutylsulfonyloxy, t-butylsulfonyloxy, phenylsulfonyloxy, p-bromophenylsulfonyloxy, p-toluylsulfonyloxy, benzylsulfonyloxy, α-phenetylsulfonyloxy, and β-phenetylsulfonyloxy. Preferred examples of said X¹ include a chlorine atom, a bromine atom, an iodine atom, and a trifluoromethylsulfonyloxy group, with a chlorine atom and a trifluoromethylsulfonyloxy group being most preferred.

A pyrrolo[3,2-d]pyrimidine derivative represented by the above Formula (Ib) can be synthesized from a pyrrolo[3,2-d]pyrimidine derivative represented by the above Formula (II) according to the following synthetic method (A). 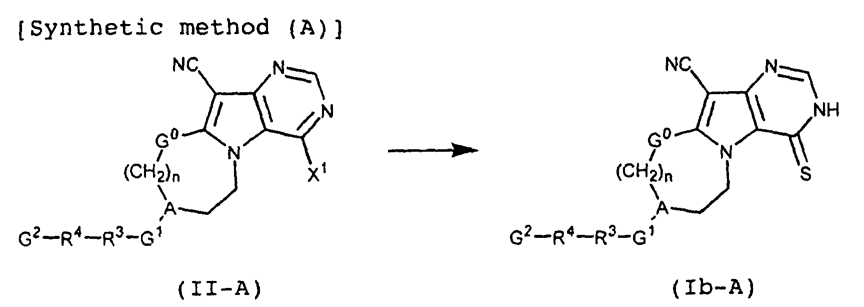 [wherein, n, A, R³, R⁴, G⁰, G¹ and G² are as defined for n, A, R³, R⁴, G⁰, G¹ and G², respectively, in the above Formula (I). X¹ is as defined in the above Formula (II)].

Thus, by reacting a pyrrolo[3,2-d]pyrimidine derivative (II-A) of the present invention to thiourea, a pyrrolo[3,2-d]pyrimidine derivative (Ib-A) of the present invention can be synthesized. In the thioxo reaction with thiourea, a reaction may be effected using a solvent such as dioxane, ethanol, and 2-propanol at a reaction temperature of 0°C to 150°C.

A pyrrolo[3,2-d]pyrimidine derivative represented by the above Formula (II) can be synthesized from a pyrrolo[3,2-d]pyrimidine derivative represented by the above Formula (Ic) according to the following synthetic method (B). 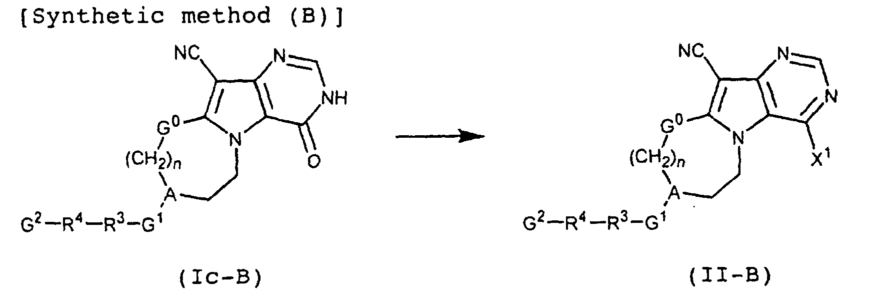 [wherein, n, A, R³, R⁴, G⁰, G¹ and G² are as defined for n, A, R³, R⁴, G⁰, G¹ and G², respectively, in the above Formula (I). X¹ is as defined in the above Formula (II)].

Thus, when, for example, X¹ is a chlorine atom, a pyrrolo[3,2-d]pyrimidine derivative (Ic-B) of the present invention can be reacted to phosphorus oxychloride to synthesize a pyrrolo[3,2-d]pyrimidine derivative (II-B) of the present invention. In the chlorination reaction with phosphorus oxychloride, a standard condition for the chlorination reaction is followed, and for example in the presence or absence of triethylamine, 4-dimethylaminopyridine or dimethylaniline, and in the presence or absence of a solvent such as acetonitrile, reaction may be carried out at a temperature range of 0°C to 150°C.

Also, when, for example, X¹ is a trifluoromethylsulfonyloxy group, a pyrrolo[3,2-d]pyrimidine derivative (Ic-B) of the present invention can be reacted to trifluoromethane sulfonic acid anhydride to synthesize a pyrrolo[3,2-d]pyrimidine derivative (II-B) of the present invention. In a trifluoromethyl sulfonyloxy reaction with trifluoromethane sulfonic acid anhydride, reaction may be carried out together with an amine such as pyridine and triethylamine in the presence or absence of a solvent such as dichloromethane at a temperature range of 0°C to 100°C.

Among the pyrrolo[3,2-d]pyrimidine derivatives represented by the above Formula (Ib), a pyrrolo[3,2-d]pyrimidine derivative represented by the above Formula (Ib-C) can be synthesized from a pyrrolo[3,2-d]pyrimidine derivative represented by the above Formula (Ic-C) according to the following synthetic method (C). 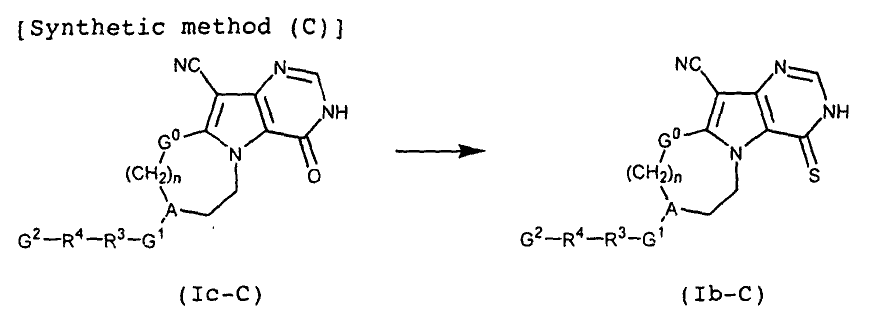 [wherein, n, A, R³, R⁴, G⁰, G¹ and G² are as defined for n, A, R³, R⁴, G⁰, G¹ and G², respectively, in the above Formula (I)].

Thus, a pyrrolo[3,2-d]pyrimidine derivative (Ic-C) of the present invention may be reacted to a Lawesson reagent described below to synthesize a pyrrolo[3,2-d]pyrimidine derivative (Ib-C) of the present invention.

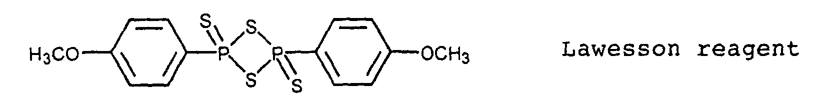

Reaction with a Lawesson reagent etc. may be carried out in an inert solvent such as benzene, toluene, and xylene at a temperature range of 10°C to 120°C for 1-24 hours to prepare a pyrrolo[3,2-d]pyrimidine derivative (Ib-C) of the present invention. Preferably, reaction is carried out in toluene at a temperature range of 60°C to 120°C for 2-12 hours.

Among the pyrrolo[3,2-d]pyrimidine derivatives represented by the above Formula (Ic), a pyrrolo[3,2-d]pyrimidine derivative represented by the above Formula (Ic-D2) can be synthesized from a pyrrolo[3,2-d]pyrimidine derivative represented by the above Formula (Ic-D1) according to the following synthetic method (D). 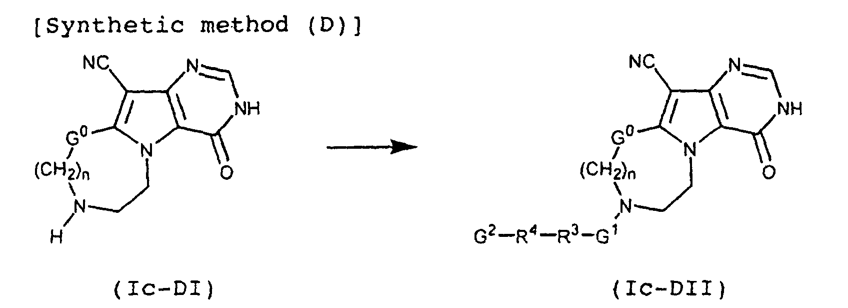 [wherein, n, A, R³, R⁴, G⁰, G¹ and G² are as defined for n, A, R³, R⁴, G⁰, G¹ and G², respectively, in the above Formula (I)].

Thus, a pyrrolo[3,2-d]pyrimidine derivative (Ic-D1) of the present invention may be reacted to a variety of electrophilic reagents to prepare a pyrrolo[3,2-d]pyrimidine derivative (Ic-DII) of the present invention.

When a carboxylic acid anhydride, a carboxylic acid chloride, a sulfonic acid chloride, an isocyanate, or an isothiocyanate is used as an electrophilic reagent, a pyrrolo[3,2-d]pyrimidine derivative (Ic-DI) of the present invention may be reacted in a solvent such as dichloromethane, chloroform, tetrahydrofuran, and dimethylformamide in the presence of pyridine, triethylamine, diisopropylethylamine etc. at a temperature range of 0°C to 60°C for 1-24 hours to prepare a pyrrolo[3,2-d]pyrimidine derivative (Ic-DII) of the present invention. Preferably, dichloromethane, tetrahydrofuran etc. as a solvent and triethylamine as a base are used, and reacted at a temperature range of 20°C to 60°C for 2-12 hours.

Also, when an aldehyde is used as an electrophilic reagent, and a reductive alkylation reaction is carried out to introduce a group represented by G²-R⁴-R³-G¹-, a pyrrolo[3,2-d]pyrimidine derivative (Ic-DII) of the present invention may be reacted in a suitable solvent or a solvent mixture such as water, methanol, ethanol, 2-propanol, acetic acid, methyl orthoformate, dichloromethane, and chloroform, using sodium triacetoxy borohydride, sodium cyanoborohydride, sodium tetrahydroborate as a reducing agent at a temperature range of 0°C to 60°C for 1-24 hours to prepare a pyrrolo[3,2-d]pyrimidine derivative (Ic-DII) of the present invention. Preferably, methanol, methyl orthoformate, acetic acid, dichloromethane, or a solvent mixture thereof is used, and reacted at a temperature range of 20°C to 60°C for 2-12 hours.

Also, when an alkylhalide is used as an electrophilic reagent, a pyrrolo[3,2-d]pyrimidine derivative (Ic-DI) of the present invention may be mixed with a variety of an alkyl chloride, an alkyl bromide, or an alkyl iodide in the presence of an organic or inorganic base, in a solvent such as dichloromethane, chloroform, acetone, and acetonitrile, and reacted at a temperature range of 0°C to 80°C for 1-24 hours to prepare a pyrrolo[3,2-d]pyrimidine derivative (Ic-DII) of the present invention. Preferably, triethylamine or potassium carbonate is used as a base and reacted in a solvent such as acetonitrile or acetone at a temperature range of 40°C to 80°C for 2-12 hours.

When a pyrrolo[3,2-d]pyrimidine derivative (Ic-DI) of the present invention and a carboxylic acid are reacted to prepare an amide compound, condensing agents known to those skilled in the art such as dicyclohexyl carbodiimide, disopropyl carbodiimide, carbonyl diimidazole, hydrochloric acid 1-ethyl-3-(3-dimethylaminopropyl)carbodiimide hydrochloride and the like may be used, and by reacting in a solvent such as dichloromethane, chloroform, tetrahydrofuran, dioxane, and dimethylformamide at a temperature range of 0°C to 60°C for 1-24 hours, a pyrrolo[3,2-d]pyrimidine derivative (Ic-DII) of the present invention can be prepared. Preferably, 1-ethyl-3-(3-dimethylaminopropyl)carbodiimide hydrochloride is used as a condensing agent, and reacted in dichloromethane or dimethylformamide at a temperature range of 20°C to 40°C for 2-12 hours. The resulting pyrrolo[3,2-d]pyrimidine derivative (1c-DII) is purified by a method known to those skilled in the art such as silica gel chromatography, recrystalization, or the like.

Among the pyrrolo[3,2-d]pyrimidine derivatives represented by the above Formula (Ib), a pyrrolo[3,2-d]pyrimidine derivative represented by the above Formula (Ib-EII) can be synthesized from a pyrrolo[3,2-d]pyrimidine derivative represented by the above Formula (Ib-EI) according to the following synthetic method (E). 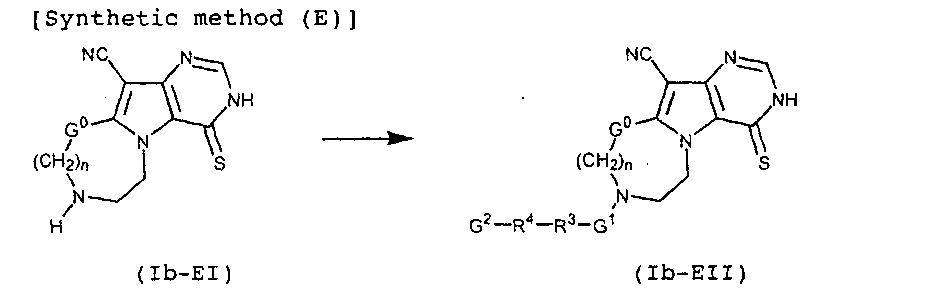 [wherein, n, R³, R⁴, G⁰, G¹ and G² are as defined for n, R³, R⁴, G⁰, G¹ and G², respectively, in the above Formula (I)].

Thus, a pyrrolo[3,2-d]pyrimidine derivative (Ia-EI) of the present invention may be reacted to a variety of electrophilic reagents to synthesize a pyrrolo[3,2-d]pyrimidine derivative (Ib-EII) of the present invention. Such a synthetic method is similar to that described in the above synthetic method (D) except that the alkylation reaction using an alkylhalide is omitted.

Among the pyrrolo[3,2-d]pyrimidine derivatives represented by the above Formula (Ic), a pyrrolo[3,2-d]pyrimidine derivative represented by the above Formula (Ic-F) can be synthesized from a pyrrolo[3,2-d]pyrimidine derivative represented by the above Formula (IV-F) according to the following synthetic method (F). 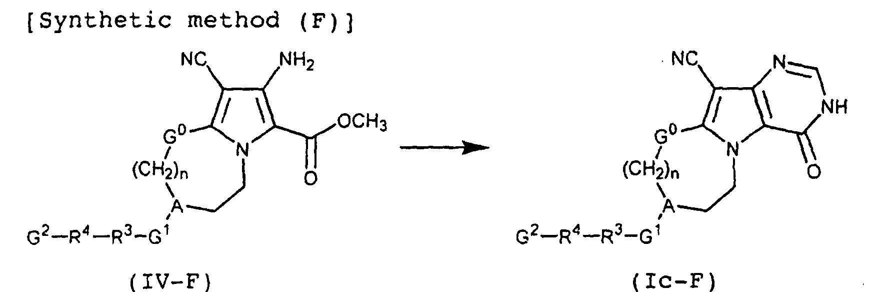 [wherein, n, A, R³, R⁴, G⁰, G¹ and G² are as defined for n, A, R³, R⁴, G⁰, G¹ and G², respectively, in the above Formula (I)].

Thus, by subjecting a pyrrole derivative represented by (IV-F) to a cyclization reaction using a formamidine or formamide, a pyrrolo[3,2-d]pyrimidine derivative (Ic-F) of the present invention can be synthesized.

For the cyclization reaction of the pyrrole derivative (IV-F) using formamidine, formamidine acetate, for example, is reacted in a solvent such as 2-propanol at a temperature range of 0°C to 150°C. In a cyclization reaction using formamide, reaction can be attained by reacting formamide in the presence of, for example, an alkoxydic base such as sodium methoxide, sodium ethoxide, and potassium t-butoxide. As an organic solvent used in the reaction, there can be mentioned polar solvents such as formamide, methanol, ethanol, acetonitrile, dimethylformamide, and dimethoxyethane. Preferably formamide and methanol are used. This reaction may be carried out at a temperature range of 20°C to 100°C for 1-24 hours. Preferably reaction is carried out at a temperature range of 50°C to 80°C for 1-12 hours.

When the pyrrolo[3,2-d]pyrimidine derivatives of the present invention synthesized by the above synthetic methods (A), (B), (C), (D), (E), and (F) have an easily convertible substituent such as an alkoxycarbonyl group, an acyloxy group, and an aromatic nitro group, they can be converted to pyrrolo[3,2-d]pyrimidine derivatives of the present invention having a carboxyl group, a hydroxy group, or an amino group, respectively, by subjecting them to a reaction known to those skilled in the art.

When the pyrrolo[3,2-d]pyrimidine derivatives of the present invention synthesized by the above synthetic methods (A), (B), (C), (D), (E), and (F) have a carboxyl group, they can be converted to pyrrolo[3,2-d]pyrimidine derivatives of the present invention having an alkoxycarbonyl group, a carbamoyl group, a N-alkylcarbamoyl group, and N-alkoxycarbamoyl group or the like, by subjecting them to a condensation reaction known to those skilled in the art.

When the pyrrolo[3,2-d]pyrimidine derivatives of the present invention synthesized by the above synthetic methods (A), (B), (C), (D), (E), and (F) have an amino group, they can be converted to pyrrolo[3,2-d]pyrimidine derivatives of the present invention having an acylamino group, an alkylsulfonylamino group or the like, by subjecting them to a condensation reaction known to those skilled in the art.

When they have an amino group, reductive alkylation known to those skilled in the art may be effected to convert to pyrrolo[3,2-d]pyrimidine derivatives of the present invention having a monoalkylamino group or a dialkylamino group.

When the pyrrolo[3,2-d]pyrimidine derivatives of the present invention synthesized by the above synthetic methods (A), (B), (C), (D), (E), and (F) have a hydroxy group, they can be converted to pyrrolo[3,2-d]pyrimidine derivatives of the present invention having an acyloxy group by subjecting them to a condensation reaction known to those skilled in the art.

When the pyrrolo[3,2-d]pyrimidine derivatives of the present invention synthesized by the above synthetic methods (A), (B), (C), (D), (E), and (F) have a formyl group, they can be converted to pyrrolo[3,2-d]pyrimidine derivatives of the present invention having an alkylmethyl group by subjecting them to reductive alkylation known to those skilled in the art.

In the synthetic method of the pyrrolo[3,2-d]pyrimidine derivatives represented by the above Formula (I), a pyrrole derivative for use as a starting material represented by the above Formula (IV-F) can be synthesized from a lactam derivative represented by the following Formula (VII-G) according to a synthetic method (G). 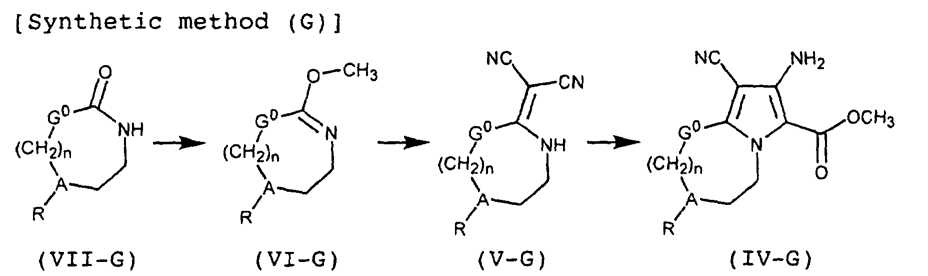 [wherein, n, A, and G⁰ are as defined for n, A, and G⁰, respectively, in the above Formula (I). R represents a group that is convertible to G²-R⁴-R³-G¹].

Thus, by methylating a lactam derivative (VII-G), an intermediate (VI-G) can be obtained, which is then reacted to malononitrile to obtain a malonomethyl derivative (V-G). This is reacted to methyl bromoacetate in the presence of a base, and further cyclized to synthesize a pyrrole derivative (IV-G).

As an example of a methylation reaction of a lactam derivative (VII-G) in the synthetic method (G), there can be mentioned, but not limited to, a method in which a methylation agent such as dimethyl sulfate and trimethyl tetrafluoroborate oxonium is used in a suitable organic solvent or an organic solvent mixture to methylate the oxygen atom of the carbonyl group. Preferably, using trimethyl tetrafluoroborate oxonium in a solvent such as dichloromethane, chloroform and dichloroethane, the reaction is stirred at a temperature range of -20°C to 80°C for 1-24 hours, followed by treatment with a suitable aqueous base to obtain an intermediate (VI-G). The aqueous base used herein is an aqueous solution of sodium carbonate, an aqueous solution of phosphate carbonate, an aqueous solution of sodium bicarbonate, an aqueous solution of potassium bicarbonate, or the like.

Then, malononitrile is reacted to the intermediate (VI-G) to obtain a malonomethylene derivative (V-G). The reaction proceeds by dissolving the intermediate (VI-G) and malononitrile in a suitable organic solvent such as methanol, ethanol, 2-propanol, benzene, toluene, and xylene, and stirring at a temperature range of 0°C to 130°C for 1-24 hours. A preferred example of a reaction condition include a system in which ethanol, toluene or a mixture thereof is used and stirred at a temperature range of 25°C to 80°C for 1-24 hours. The malonomethylene derivative (V-G) formed in this reaction is preferably purified by a method known to those skilled in the art such as silica gel chromatography or recrystalization.

The malonomethylene derivative (V-G) is then is reacted to methyl bromoacetate in a suitable polar organic solvent and in the presence of a suitable base to convert it to a pyrrole derivative (IV-G). As a suitable organic solvent, there can be mentioned acetone, acetonitrile, methylethylketone, tetrahydrofuran, or dimethylformamide, with acetone or acetonitrile being preferred. As a base, there can be mentioned an organic base such as pyridine, triethylamine, or diisopropylethylamine, and inorganic bases such as sodium carbonate, potassium carbonate, cesium carbonate, sodium bicarbonate, potassium bicarbonate or the like, with potassium carbonate or cesium carbonate being preferably used. The reaction proceeds at a temperature range of 20°C to 100°C for 1-24 hours. Preferably, reaction is carried out at a temperature range of 50°C to 80°C for 3-12 hours.

The thus obtained pyrrolo[3,2-d]pyrimidine derivative represented by the above Formula (I) has an effect of inhibiting GSK-3 activity, and thus can be used as a GSK-3 activity inhibitor as a clinically applicable preventive and/or therapeutic agent. As diseases that can be treated by GSK-3 activity inhibitors, there can be mentioned diabetes mellitus, diabetic complications, atherosclerosis, hypertension, obesity, Syndrome X, Alzheimer's disease, neurodegenerative diseases (AIDS encephalopathy, Huntington's disease, Parkinson's disease, cerebral ischemia), manic-depressive psychosis, traumatic encephalopathy, alopecia, inflammatory diseases, cancer, and immune deficiency.

Also, a pyrrolo[3,2-d]pyrimidine derivative represented by Formula (I) and a pharmaceutically acceptable salt thereof may be rendered a pharmaceutical composition together with a pharmaceutically acceptable carrier and/or diluent. The pharmaceutical composition may be formulated into various dosage forms, and administered orally or parenterally. As parenteral administration, there can be mentioned intravenous, subcutaneous, intramuscular, transdermal, and rectal administration.

Dosage forms for oral administration include, for example, tablets, pills, granules, powders, liquids, suspensions, syrups, and capsules.

As used herein tablets may be formed according to a standard method using a pharmaceutically acceptable carrier such as an excipient, a binder, a disintegrant, and the like. Pills, granules, and powders can also be formed according to a standard method using an excipient as for tablets. Methods of forming liquids, suspensions, and syrups are standard methods that use a glycerin ester, an alcohol, water, a vegetative oil, and the like. Capsules may be formed by filling granules, powders, or liquids into a capsule of gelatin etc. and by shaping it.

In the case of intravenous, subcutaneous, and intramuscular administration, agents for parenteral administration may be administered as injections. For injections, there are cases in which they are dissolved in a water-soluble liquid such as physiological saline, or cases in which they are dissolved in a non-aqueous liquid comprising an organic ester such as propylene glycol, polyethylene glycol, and a vegetative oil.

In the case of transdermal administration, dosage forms such as ointments and creams may be used. Ointments may be mixed with lipids or vaselin, and creams may be mixed with emulsifying agents, and then formed.

To these various pharmaceutical formulations, pharmaceutically acceptable carriers such as an isotonizing agent, a preservative, a disinfectant, a wetting agent, a buffering agent, an emulsifying agent, a dispersant, and a stabilizer can be added as desired.

Also, these pharmaceutical formulations may be sterilized, as desired, by filtration with a bacteria-retaining filter and by the blending of a bacteriocidal agent.

The dosage of pyrrolo[3,2-d]pyrimidine derivatives represented by the above Formula (I) and pharmaceutically acceptable salt thereof may vary with the type of diseases, the administration route, conditions, age, sex, body weight etc. of the patient, but generally it is about 1-500 mg/day/person for oral administration. For parenteral administration such as intravenous, subcutaneous, and transdermal administration, it is about 0.1-100mg/day/person.

### Examples

The present invention will now be illustrated with reference to the following examples, but it should be noted that the present invention is not limited to them in any way. For the data on the compounds synthesized in the following Examples, "HPLC retention time" indicates the retention time (unit: minutes) of the compound under the following analytical condition of HPLC analysis. High performance liquid chromatography (HPLC) analytical condition:

| | |
|---|---|
| System | Hewlett-Packard 1100 HPLC |
| Column | Cadenza CD-C18 (manufactured by imtakt) 100 |
| | x 4.6 mm |
| Solvent | A: H₂O/acetonitrile = 95/5 |
| | (0.05% trifluoroacetic acid) |
| | B: H₂O/acetonitrile = 5/95 |
| | (0.05% trifluoroacetic acid) |
| Flow rate | 1.0 mL/min |
| Gradient | |

0-1 min Solvent B 10% Solvent A 90%
1-14 min Solvent B 10% → 100% Solvent A 90% → 0%
14-16 min Solvent B 100% Solvent A 0%

### [Reference Example 1]

### Synthesis of tert-butyl 4-(hydroxyimino)piperidine carboxylate

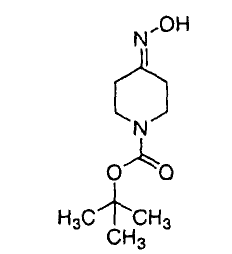

To a solution of tert-butyl 4-piperidinone carboxylate in ethanol (400 mL), hydroxylamine hydrochloride (29.34 g) and sodium acetate (34.64 g) were added, and stirred at 100°C for seven hours. The reaction mixture was cooled to room temperature, and the filtrate obtained by filtering off the solid was concentrated under reduced pressure. Water was added to the residue, and extracted twice with ethyl acetate. The combined organic layer was washed in a saturated aqueous solution of sodium bicarbonate and saturated saline, and then dried on anhydrous magnesium sulfate, and filtered. The solvent was evaporated under reduced pressure, dried under vacuum to obtain a title compound (quantitative yield) as a white solid compound.
¹H-NMR (400 MHz, DMSO-d₆) δ(ppm): 1.46 (s, 9H), 2.27 (m, 2H), 2.49 (m, 2H), 3.36-3.52 (m, 4H), 10.50 (s, 1H).

### [Reference Example 2]

### Synthesis of tert-butyl 5-oxo-1,4-diazaperhydroepin carboxylate

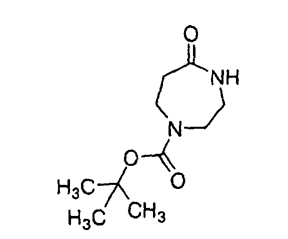

To a solution of tert-butyl 4-(hydroxyimino)piperidinone carboxylate (32.70 g) in acetonitrile (250 mL), 2-chloro-1,3-dimethylimidazolynium chloride (30.96 g) was added, to which triethylamine (51 mL) was added dropwise at room temperature over 20 minutes. After dropwise addition, it was further stirred at room temperature for 30 minutes, and then water (50 mL) was added and stirred overnight. After the reaction mixture was diluted with ethyl acetate, the organic layer was separated. The organic layer was washed in 0.1 mol/L hydrochloric acid, a saturated aqueous solution of sodium bicarbonate and saturated saline in this order, and then dried on anhydrous magnesium sulfate, and filtered. The solvent was evaporated under reduced pressure, dried under vacuum to obtain a crude title compound as a brown semi-solid compound. The product was used in the subsequent reaction without further purification.

### [Reference Example 3]

### Synthesis of tert-butyl 3-oxopiperadine carboxylate

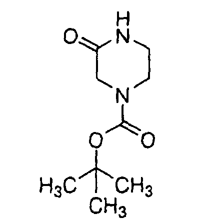

To a solution of piperadine-2-one (2.01 g) in dioxane (20 mL) and water (10 mL), an aqueous solution of two normal sodium hydroxide (10 mL) was added at room temperature and stirred. Then, a solution of tert-butyl dicarbonate (5.45 g) in dioxane (5 mL) was slowly added dropwise, and stirred as it is at room temperature for eight hours. Water was poured to the reaction mixture, and extracted twice with 50 mL of ethyl acetate. After the organic layers were combined and washed in saturated saline, it was dried on anhydrous magnesium sulfate, and filtered. The solvent was evaporated under reduced pressure, dried under vacuum to obtain a title compound (3.41 g, yield 68%) as a white solid compound. The product thus obtained was used in the subsequent reaction without further purification.
ESI/MS m/e: 201.2 (M⁺+H, C₉H₁₆N₂O₃)

### [Reference Example 4]

### Synthesis of 1-benzyl-2H,3H,4H-benzo[f]1,4-diazepine-5-one

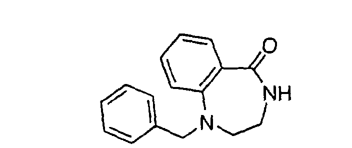

Under a nitrogen atmosphere, to methyl 2-[(2-aminoethyl)benzylamino]benzoic acid dihydrochloride (250 mg) in a solvent mixture of toluene-tetrahydrofuran (1:1, 10 mL), a solution of trimethylaluminum in hexane (15%, 0.8 mL) was added dropwise at 0°C. After stirring for 30 minutes, the reaction mixture was heated to 60°C and stirred for four hours. To the reaction mixture was added 20 mL of water and 20 mL of ethyl acetate, the organic layer was separated, and the aqueous layer was reextracted with 20 mL of ethyl acetate. After the organic layer was combined and dried, it was concentrated under reduced pressure, and the residue thus obtained was purified on silica gel chromatography (solvent: hexane/ethyl acetate = 1:1 → 1:1) to obtain a title compound (220 mg, yield 100%).
ESI/MS m/e: 253.4(M⁺+H, C₁₆H₁₆N₂O)

### [Reference Example 5]

### Synthesis of tert-butyl 5-methoxy-2H,3H,6H,7H-1,4-diazepine carboxylate

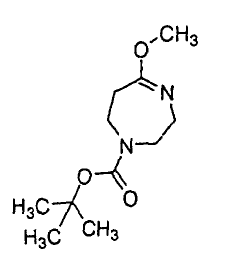

To a solution of tert-butyl 5-oxo-1,4-diazaperhydroepin carboxylate in dichloromethane (400 mL), trimethyl tetrafluoroborate oxonium (33.86 g) was added and stirred overnight at room temperature. To the reaction mixture were added an aqueous solution of saturated sodium bicarbonate (200 mL) and water (100 mL), and then after stirring for 20 minutes, the aqueous layer was removed and the organic layer was dried on anhydrous magnesium sulfate, and filtered. The solvent was evaporated under reduced pressure, and the residue dried under vacuum to obtain a crude title compound as a oily compound. The product thus obtained was used in the subsequent reaction without further purification.

### [Reference Example 6]

### Synthesis of tert-butyl 5-(dicyanomethylene)-1,4-diazepine carboxylate

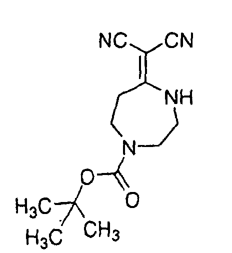

The crude product of tert-butyl 5-methoxy-2H,3H,6H,7H-1,4-diazepine carboxylate was dissolved in ethanol (200 mL) and toluene (100 mL), to which malononitrile (30.25 g) was added and stirred at 90°C for four hours. The reaction mixture was cooled to room temperature, and after the solvent was evaporated under reduced pressure, the residue was diluted with ethyl acetate and water, and the organic layer was separated. The aqueous layer was extracted twice with ethyl acetate. The combined organic layer was washed with saturate saline, and dried on anhydrous magnesium sulfate and filtered. The solvent was evaporated under reduced pressure, and the residue was purified on silica gel chromatography (hexane/ethyl acetate = 5/2) to obtain a brown solid. The solid was washed in a small amount of diethylether to obtain a title compound (7.32 g, yield 19% of three steps from Reference Example 2) as a white solid compound.
¹H-NMR (400 MHz, DMSO-d₆) δ (ppm): 1.40 (s, 9H), 2.83 (br, 2H), 3.43-3.52 (m, 6H), 9.06 (br, 1H).
13C-NMR (400 MHz, DMSO-d6): δ 27.93, 32.70, 41.84, 45.37, 48.38, 79.49, 115.35, 116.90, 154.00, 175.10.
ESI/MS m/e: 263.4(M⁺+H, C₁₃H₁₈N₄O₂)

### [Reference Example 7]

### Synthesis of tert-butyl 3-(dicyanomethylene)-piperadine carboxylate

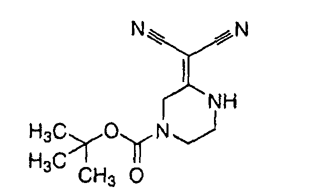

To a solution of crude tert-butyl 3-oxopperadine carboxylate (400 mg) in dichloromethane (4 mL), trimethyl tetrafluoroborate oxonium (470 mg) was added and stirred overnight at room temperature. To the reaction mixture were added an aqueous solution of saturated sodium bicarbonate (5 mL) and water (5 mL), and then after stirring for two hours, the aqueous layer was removed, and the organic layer was dried on anhydrous magnesium sulfate, and filtered. The solvent was evaporated under reduced pressure, and the residue was dried under vacuum to obtain crude tert-butyl 3-methoxy-1,2,5,6-tetrahydropyradine carboxylate (230 mg) as a pale yellow oily compound. Malononitrile (100 mg) was added to a solution of this crude product in ethanol (5 mL), which was then stirred overnight at room temperature, and then the solvent was evaporated, and the residue was extracted twice with ethyl acetate and water. The combined organic layer was washed with saturated saline, dried on anhydrous sodium sulfate and filtered. The solvent was evaporated under reduced pressure to obtain a title compound (395 mg) as a brown solid.
ESI/MS m/e: 249.2 (M⁺+H, C₁₂H₁₆N₄O₂)

### [Reference Example 8]

### Synthesis of [1-benzyl-2H,3H,4H-benzo[f]1,4-diazepine-5-ylidene]methane-1,1-dicarbonitrile

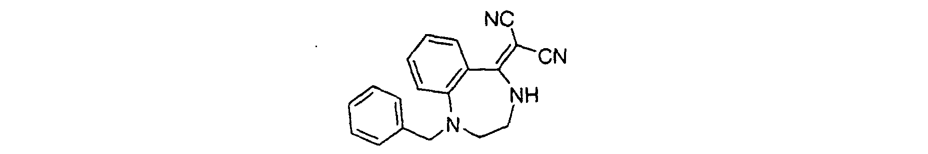

As in Reference Example 7, 1-benzyl-2H,3H,4H-benzo[f]1,4-diazepine-5-one (100 mg) was used to synthesize a title compound (74 mg, yield 62%).
ESI/MS m/e: 301.1 (M⁺+H, C₁₉H₁₆N₄)

### [Reference Example 9]

### Synthesis of methyl 8-amino 3-[(tert-butyl)oxocarbonyl]-9-cyano-1H,2H,4H,5H-pyrrolo[1,5-f]1,4-diazaperhydroepin-7-carboxylate

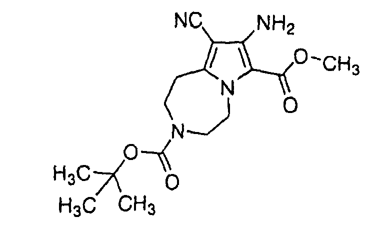

To a solution of tert-butyl 5-(dicyanomethylene)-1,4-diazaperhydroepin carboxylate (5.74 g) in acetonitrile (120 mL), cesium carbonate (14.25 g) and methyl bromoacetate (6.69 g) were added at room temperature, and then stirred at 90°C for five hours. After the reaction mixture was cooled to room temperature, the solid component was filtered off. After the solvent of the filtrate was evaporated under reduced pressure, water was added to the residue for dilution, and then extracted twice with ethyl acetate. The combined organic layer was washed with saturated saline, dried on anhydrous magnesium sulfate, and filtered. The solvent was evaporated under reduced pressure, and the residue was dried under vacuum. The resulting solid was washed in methanol to obtain a title compound (4.53 g, yield of 62%) as a pale yellow solid compound.
¹H-NMR (400 MHz, DMSO-d₆) δ (ppm): 1.39 (s, 9H), 2.90 (br, 2H), 3.52 (m, 4H), 3.72 (s, 3H), 4.55 (m, 2H), 5.80 (s, 1H).
ESI/MS m/e: 335.4 (M⁺+H, C₁₆H₂₂N₄O₄)

### [Reference Example 10]

### Synthesis of methyl 7-amino-2-[(tert-butyl)oxycarbonyl]-8-cyanopyrrolo[1,5-a]piperadine-6-carboxylate

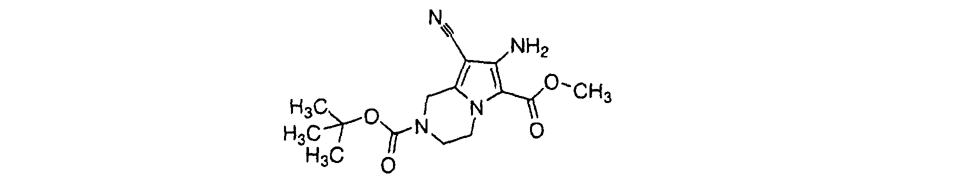

To a solution of tert-butyl 3-(dicyanomethylene)-piperadine carboxylate (395 mg) in acetonitrile (5 mL), potassium carbonate (350 mg) and methyl bromoacetate (325 mg) were added at room temperature, and then stirred at 90°C for five hours. After the reaction mixture was cooled to room temperature, 10 mL of water was added to the reaction mixture, and extracted twice with 20 mL of ethyl acetate. The combined organic layer was washed with saturated saline, dried on anhydrous magnesium sulfate, and filtered. The solvent was evaporated under reduced pressure, and the residue was dried under vacuum. The resulting solid was washed in methanol to obtain a crude title compound (390 mg) as a pale yellow solid compound. The product was used in the subsequent reaction without further purification. ESI/MS m/e: 321.0 (M⁺+H, C₁₅H₂₀N₄O₄)

### [Reference Example 11]

### Synthesis of methyl 10-amino-11-cyano-5-benzyl-6H,7H-benzo[f]pyrrolo[1,5-d]1,4-diazepine-9-carboxylate

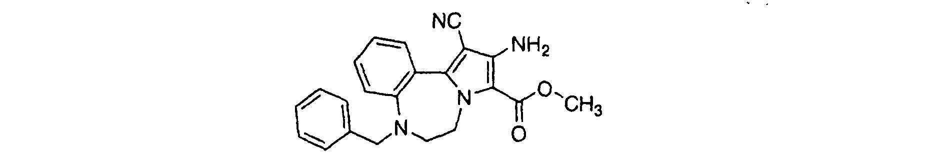

As in Reference Example 10, [1-benzyl-2H,3H,4H-benzo[f] 1,4-diazepine-5-ylidene]methane-1,1-dicarbonitrile (60 mg) was used to synthesize a crude title compound (83 mg)
ESI/MS m/e: 373.2 (M⁺+H, C₂₂H₂₀N₄O₂)

### [Working Example 1]

### Synthesis of tert-butyl 11-cyano-4-oxo-3-hydro-6H,7H,9H,10H-1,4-diazaperhydroepino[4',5'-1,5]pyrrolo[3,2-d]pyrimidine-8-carboxylate (Compound No. 1)

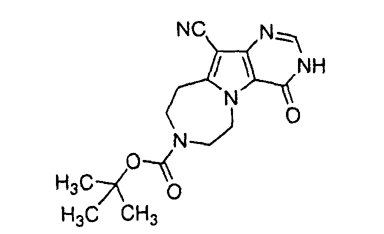

To methyl 8-amino 3-[(tert-butyl)oxocarbonyl]-9-cyano-1H,2H,4H,5H-pyrrolo[1,5-f]1,4-diazaperhydroepin-7-carboxylate (8.06 g), formamide (70 mL) and a solution of 28% sodium methoxide in methanol (45 mL) were added, and stirred at 90°C for three hours. After the reaction mixture was cooled to room temperature, acetic acid was slowly added dropwise for neutralization. The resulting solid was filtered, and sequentially washed in water and methanol to obtain a title compound (5.80 g, yield 73%) as a white solid compound.
HPLC retention time = 7.7 (min)
¹H-NMR (400 MHz, DMSO-d₆) δ (ppm): 1.41 (m, 9H), 3.12 (m, 2H), 3.60 (m, 2H), 3.65 (m, 2H), 4.84 (m, 2H), 7.96 (s, 1H), 12.41 (br, 1H).
ESI/MS m/e: 330.2 (M⁺+H, C₁₆H₁₉N₅O₃)

### [Working Example 2]

### Synthesis of tert-butyl 10-cyano-4-oxo-3-hydropyrimidino[4',5'-4,5]pyrrolo[2,1-c]piperadine-8-carboxylate (Compound No. 434)

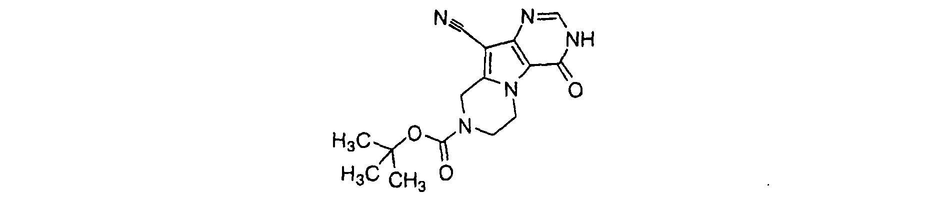

To crude methyl 7-amino 2-[(tert-butyl)oxocarbonyl]-8-cyanopyrrolo[1,5-a]piperadine-6-carboxylate (314 mg), formamide (5 mL) and a solution of 28% sodium methoxide in methanol (5 mL) were added, and stirred at 90°C for 12 hours. After the reaction mixture was cooled to room temperature and 25 mL of water was added, it was extracted twice with 20 mL of ethyl acetate. The organic layers were combined and washed with saturated saline, and then it was dried on anhydrous sodium sulfate and filtered. The solvent was evaporated under reduced pressure, and the residue was purified on silica gel chromatography (ethyl acetate) to obtain a title compound (80.6 mg) as a white solid compound.
¹H-NMR (400 MHz, DMSO-d₆) δ (ppm): 1.45 (m, 9H), 3.84 (t, J=5.1, 2H), 4.48 (t, J=5.1, 2H), 4.83 (s, 2H), 7.91 (s, 1H), 11.8 (brs, 1H)
ESI/MS m/e: 316.1 (M⁺+H, C₁₅H₁₇N₅O₃)

### [Working Example 3]

### Synthesis of 9-oxo-5-benzyl-10-hydro-6H,7H-benzo[f]pyrimidino[5',4'-2,3]pyrrolo[1,5-d]1,4-diazepine-13-carbonitrile (Compound No. 1798)

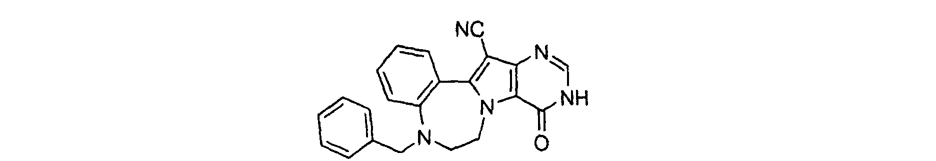

As in Working Example 2, methyl 10-amino-11-cyano-5-benzyl-6H,7H-benzo[f]pyrrolo[1,5-d]1,4-diazepine-9-carboxylate was used to obtain a title compound.
HPLC retention time = 9.7 (min)
ESI/MS m/e = 368.2 (M⁺+H: C₂₂H₁₇N₅O)

### [Working Example 4]

### Synthesis of 4-oxo-3-hydro-6H,7H,8H,9H,10H-1,4-diazaperhydroepino[1',7'-1,5]pyrrolo[3,2-d]pyrimidine-11-carboxynitrile (Compound No. 249) hydrochloride

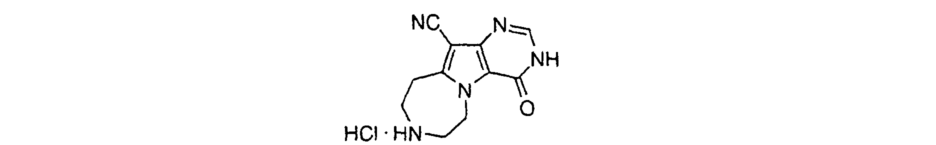

After tert-butyl 11-cyano-4-oxo-3-hydro-6H,7H,9H,10H-1,4-diazaperhydroepino[4',5'-1,5]pyrrolo[3,2-d]pyrimidine-8-carboxylate (5.80 g) was dissolved in 1,4-dioxane (100 mL) and methanol (40 mL), 4 mol/L hydrochloric acid / 1,4-dioxane solution (20 mL) was added thereto, which was stirred at room temperature for two hours and then at 60°C for four hours. The reaction mixture was cooled to room temperature, and the solvent was evaporated under reduced pressure. The residue was dried under vacuum to obtain a title compound (quantitative yield) as a white solid compound.
¹H-NMR (400 MHz, DMSO-d₆) δ (ppm): 3.32-3.55 (m, 6H), 5.09 (br, 2H), 7.99 (s, 1H), 9.92 (br, 2H), 12.55 (br, 1H).
ESI/MS m/e: 230.3 (M⁺+H, C₁₁H₁₁N₅O)

### [Working Example 5]

### Synthesis of 4-oxo-8-(2,2,2-trifluoroacetyl)-3-hydro-6H,7H,9H,10H-1,4-diazaperhydroepino[1',7'-1,5]pyrrolo[3,2-d]pyrimidine-11-carbonitrile (Compound No. 12)

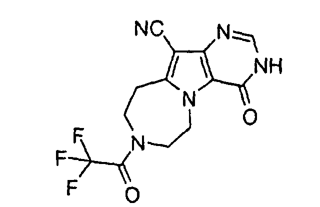

To a solution of 4-oxo-3-hydro-6H,7H,8H,9H,10H-1,4-diazaperhydroepino[1',7'-1,5]pyrrolo[3,2-d]pyrimidine-11-carbonitrile hydrochloride (2.05 g) in tetrahydrofuran (40 mL), triethylamine (32 mL) was added and stirred. Then, after slowly adding dropwise trifluoroacetic acid anhydride (16.2 g), it was stirred at room temperature for three hours. Methanol was added to the reaction mixture to stop the reaction, and then the solvent was evaporated under reduced pressure. Ethyl acetate was added to the residue to separate the organic layer. After the organic layer was washed with water and saturated saline, it was dried on anhydrous magnesium sulfate, and filtered. The solvent was evaporated under reduced pressure, and the resulting solid was washed with a small amount of methanol and diethylether, and recovered. The solvent of the filtrate was evaporated under reduced pressure, and the resulting solid was washed sequentially with a small amount of methanol and diethylether, and recovered. The solvent of the filtrate was evaporated under reduced pressure, and the resulting solid was washed as described above, and was combined with the solid recovered earlier to obtain a title compound (1.75 g, yield 70%) as a white solid.
¹H-NMR (400 MHz, DMSO-d₆) δ (ppm): 3.24-3.32 (m, 2H), 3.84 (m, 2H), 3.93 (m, 2H), 5.00 (m, 2H), 7.98 (s, 1H), 12.48 (s, 1H).
ESI/MS m/e: 326.1 (M⁺+H, C₁₃H₁₀F₃N₅O₂)

### [Working Example 6]

### Synthesis of 8-[(4-methylphenyl)carbonyl]4-oxo-3-hydro-6H,7H,9H,10H-1,4-diazaperhydroepino[1',7'-5,1]pyrrolo[3,2-d]pyrimidine-11-carbonitrile (Compound No. 50)

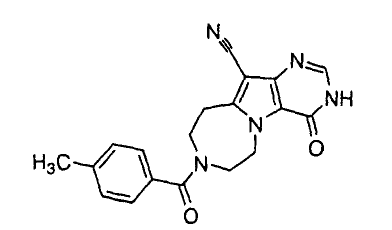

To a solution of 4-oxo-3-hydro-6H,7H,8H,9H,10H-1,4-diazaperhydroepino[1',7'-1,5]pyrrolo[3,2-d]pyrimidine-11-carbonitrile hydrochloride (25 mg) in N,N-dimethylformamide/dichloromethane/triethylamine = 10/10/1 (2 mL), 4-methylbenzoyl chloride (15 mg) was added, and stirred at room temperature for five hours. Methanol was added to the reaction mixture to stop the reaction, and then the solvent was evaporated under reduced pressure. The residue was purified on a preparative HPLC to obtain a title compound as a white solid compound.
HPLC retention time = 7.0 (min)
ESI/MS m/e: 384.1 (M⁺+H, C₁₉H₁₇N₅O₂)

### [Working Example 7]

### Synthesis of (11-cyano-4-oxo(3-hydro-6H,7H,9H,10H-1,4-diazaperhydroepino[4',5'-1,5]pyrrolo[3,2-d]pyrimidine-8-yl))-N-(4-methylphenyl)carboxamide (Compound No. 161)

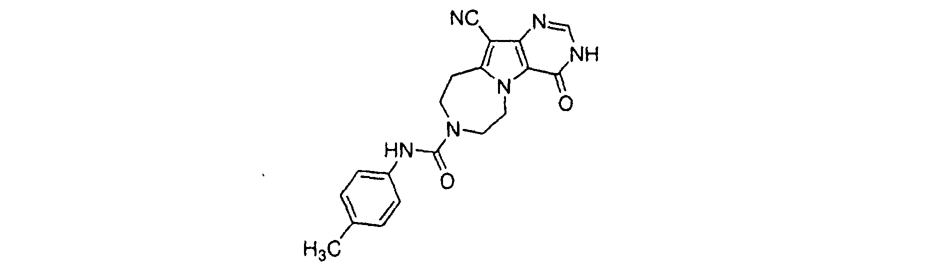

To a solution of 4-oxo-3-hydro-6H,7H,8H,9H,10H-1,4-diazaperhydroepino[1',7'-1,5]pyrrolo[3,2-d]pyrimidine-11-carbonitrile hydrochloride (25 mg) in N,N-dimethylformamide/dichloromethane/triethylamine = 10/10/1 (2 mL), 4-methylphenylisocyanate (13 mg) was added, and stirred at room temperature for five hours. Methanol was added to the reaction mixture to stop the reaction, and then the solvent was evaporated under reduced pressure. The residue was purified on a preparative HPLC to obtain a title compound as a white solid compound.
HPLC retention time = 7.3 (min)
¹H-NMR (400 MHz, DMSO-d₆) δ (ppm): 2.23 (s, 3H), 3.15 (m, 2H), 3.74 (m, 2H), 3.81 (m, 2H), 4.91 (m, 2H), 7.05 (d, 8.6 Hz, 2H), 7.35 (d, 8.5 Hz, 2H), 7.97 (s, 1H), 8.52 (s, 1H), 12.38 (br, 1H).
ESI/MS m/e: 363.3 (M⁺+H, C₁₉H₁₈N₆O₂)

### [Working Example 8]

### Synthesis of 8-[(4-methoxyphenyl)sulfonyl]-4-oxo-3-hydro-6H,7H,9H,10H-1,4-diazaperhydroepino[1',7'-1,5]pyrrolo[3,2-d]pyrimidine-11-carbonitrile (Compound No. 236)

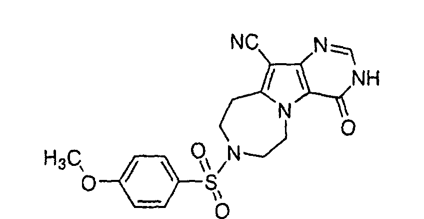

To a solution of 4-oxo-3-hydro-6H,7H,8H,9H,10H-1,4-diazaperhydroepino[1',7'-1,5]pyrrolo[3,2-d]pyrimidine-11-carbonitrile hydrochloride (25 mg) in N,N-dimethylformamide/dichloromethane/triethylamine = 10/10/1 (2 mL), 4-methoxybenzenesulfonyl chloride (19 mg) was added, and stirred at room temperature for five hours. Methanol was added to the reaction mixture to stop the reaction, and then the solvent was evaporated under reduced pressure. The residue was purified on a preparative HPLC to obtain a title compound as a white solid compound.
HPLC retention time = 7.6 (min)
¹H-NMR (400 MHz, DMSO-d₆) δ (ppm): 3.20 (m, 2H), 3.25-3.43 (m, 4H), 3.81 (m, 3H), 4.90 (m, 2H), 7.10 (d, 9.0 Hz, 2H), 7.70 (d, 9.0 Hz, 2H), 7.93 (d, 3.7 Hz, 1H), 12.38 (br, 1H). ESI/MS m/e: 400.2 (M⁺+H, C₁₈H₁₇N₅O₄S)

### [Working Example 9]

### Synthesis of 4-oxo-8-benzyl-3-hydro-6H,7H,9H,10H-1,4-diazaperhydroepino[1',7'-1,5]pyrrolo[3,2-d]pyrimidine-11-carbonitrile (Compound No. 251)

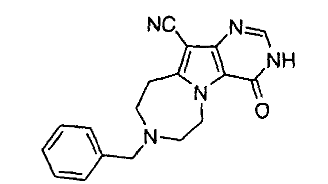

After tert-butyl 11-cyano-4-oxo-3-hydro-6H,7H,9H,10H-1,4-diazaperhydroepino[4',5'-1,5]pyrrolo[3,2-d]pyrimidine-8-carboxylate (355 mg) was dissolved in 1,4-dioxane (10 mL) and methanol (10 mL), 4 mol/L hydrochloric acid / 1,4-dioxane solution (1 mL) was added thereto, which was stirred at room temperature for three hours. 4 mol/L hydrochloric acid / 1,4-dioxane solution (0.5 mL) was further added and stirred for five hours. The solvent of the reaction mixture was evaporated under reduced pressure, and the residue was dried under vacuum. To a solution of the white solid compound obtained in methanol, Dowex (-OH form) was added till the liquid becomes neutral, and stirred at room temperature for desalting. From the reaction mixture, the solid component was filtered off, and the solvent was evaporated under reduced pressure, and the residue was dried under vacuum. The white solid compound obtained (309 mg) was dissolved in N,N-dimethylformamide (5 mL) and tetrahydrofuran (10 mL), to which trimethyl orthoformate (1 mL) and benzaldehyde (286 mg) were added and stirred at room temperature. To this reaction mixture was added triacetoxy sodium borohydride (2.29 g) and stirred at room temperature for two hours. Furthermore, benzaldehyde (286 mg) was added and stirred overnight at room temperature. Methanol was added to the reaction mixture to stop the reaction, and then the solvent was evaporated under reduced pressure. The residue was diluted with ethyl acetate and saturated sodium bicarbonate, and after separating the organic layer it was extracted twice with ethyl acetate. The combined organic layer was washed with saturated saline, and dried on anhydrous magnesium sulfate, and filtered. The solvent was evaporated under reduced pressure and the residue was purified on silica gel chromatography (ethyl acetate) to obtain a title compound (193 mg, yield 45%) as a white solid compound.
HPLC retention time = 4.0 (min)
ESI/MS m/e: 320.2 (M⁺+H, C₁₈H₁₇N₅O)

### [Working Example 10]

### Synthesis of 4-chloro-8-(2,2,2-trifluoroacetyl)-6H,7H,9H,10H-1,4-diazaperhydroepino[1',7'-1,5]pyrrolo[3,2-d]pyrimidine-11-carbonitrile

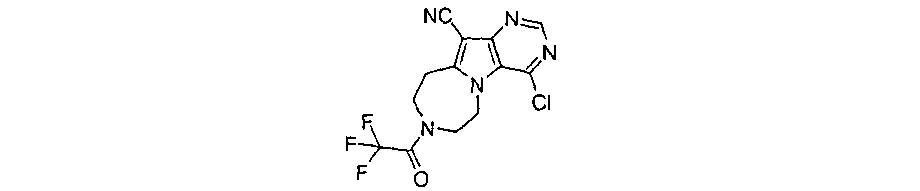

To a solution of 4-oxo-8-(2,2,2-trifluoroacetyl)-3-hydro-6H,7H,9H,10H-1,4-diazaperhydroepino[1',7'-1,5]pyrrolo[3,2-d]pyrimidine-11-carbonitrile (2.02 g) in acetonitrile (50 mL) were added N,N-dimethylaniline (0.752 g) and phosphorus oxychloride (19.03 g), and stirred at 100°C for four hours. The reaction mixture was cooled to room temperature, and the solvent was evaporated under reduced pressure. The residue was dried under vacuum to obtain a crude title compound as a green solid compound. The product was used in the subsequent reaction without further purification.
ESI/MS m/e: 344.1 (M⁺+H, C₁₃H₉C₁F₃N₅O)

### [Working Example 11]

### Synthesis of 4-thioxo-8-(2,2,2-trifluoroacetyl)-3-hydro-6H,7H,9H,10H-1,4-diazaperhydroepino[1',7'-1,5]pyrrolo[3,2-d]pyrimidine-11-carbonitrile (Compound No. 280)

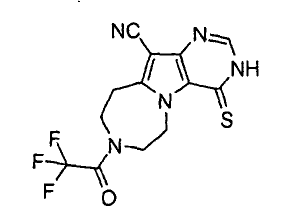

After crude 4-chloro-8-(2,2,2-trifluoroacetyl)-6H,7H,9H,10H-1,4-diazaperhydroepino[1',7'-1,5]pyrrolo[3,2-d]pyrimidine-11-carbonitrile was dissolved in 1,4-dioxane (40 mL) and isopropanol (20 mL), thiourea (40 mL) was added thereto, which was stirred at 80°C for three hours. The reaction mixture was cooled to room temperature, and the solvent was evaporated under reduced pressure. The resulting solid was washed with water, and further with ethanol and diethylether to recover the solid. These filtrates were combined and extracted with ethyl acetate. The organic layer was washed with saturated saline, dried on anhydrous magnesium sulfate, and filtered. The solvent was evaporated under reduced pressure, and the resulting solid was washed with methanol to obtain a title compound (2.19 g, quantitative yield) as a white solid compound. HPLC retention time = 8.0 (min)
¹H-NMR (400 MHz, DMSO-d₆) δ (ppm): 3.32 (m, 2H), 3.89 (m, 4H), 5.70 (m, 2H), 8.17 (s, 1H), 13.78 (s, 1H).
ESI/MS m/e: 342.1 (M⁺+H, C₁₃H₁₀F₃N₅OS)

### [Working Example 12]

### Synthesis of 5-benzyl-9-thioxo-10-hydro-6H,7H-benzo[f]pyrimidino[5',4'-2,3]pyrrolo[1,5-d]1,4-diazepine-13-carbonitrile (Compound No. 1799)

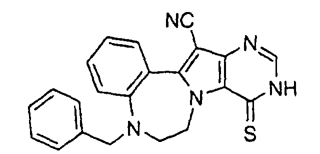

As in Working Example 11 and Working Example 12, 9-oxo-5-benzyl-10-hydro-6H,7H-benzo[f]pyrimidino[5',4'-2,3]pyrrolo[1,5-d]1,4-diazepine-13-carbonitrile was used to obtain a title compound.
HPLC retention time = 11.4 (min)
ESI/MS m/e = 384.2 (M⁺+H: C₂₂H₁₇N₅S)

### [Working Example 13]

### Synthesis of 4-thioxo-3-hydro-6H,7H,9H,10H-1,4-diazaperhydroepino[1',7'-1,5]pyrrolo[3,2-d]pyrimidine-11-carbonitrile (Compound No. 413)

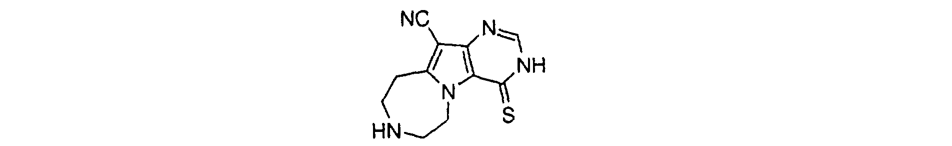

To 4-thioxo-8-(2,2,2-trifluoroacetyl)-3-hydro-6H,7H,9H,10H-1,4-diazaperhydroepino[1',7'-1,5]pyrrolo[3,2-d]pyrimidine-11-carbonitrile (2.26 g) in methanol (50 mL), 2 mol/L ammonia/methanol solution (30 mL) was added and stirred at room temperature for four hours, and the solvent was evaporated under reduced pressure. The residue was dried under vacuum to obtain a title compound (quantitative yield) as a white solid compound.
HPLC retention time = 1.502 (min)
¹H-NMR (400 MHz, DMSO-d₆) δ (ppm): 2.91 (br, 4H), 3.14 (m, 2H), 5.48 (br, 2H), 7.04 (br, 1H), 8.12 (s, 1H).
ESI/MS m/e: 246.3 (M⁺+H, C₁₁H₁₁N₅OS)

### [Working Example 14]

### Synthesis of 8-(phenylcarbonyl)-4-thioxo-3-hydro-6H,7H,9H,10H-1,4-diazaperhydroepino[1',7'-5,1]pyrrolo[3,2-d]pyrimidine-11-carbonitrile (Compound No. 297)

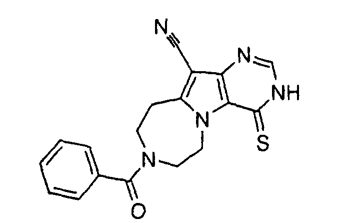

To a solution (2 mL) of 4-oxo-3-hydro-6H,7H,8H,9H,10H-1,4-diazaperhydroepino[1',7'-1,5]pyrrolo[3,2-d]pyrimidine-11-carbonitrile (25 mg) in N,N-dimethylformamide/dichloromethane/triethylamine = 10/10/1, benzoyl chloride (13 mg) was added, and stirred at room temperature for five hours. Methanol was added to the reaction mixture to stop the reaction, and then the solvent was evaporated under reduced pressure. The residue was purified on a preparative HPLC to obtain a title compound as a white solid compound.
HPLC retention time = 7.4 (min)
ESI/MS m/e: 350.0 (M⁺+H, C₁₈H₁₅N₅OS)

### [Working Example 15]

### Synthesis of 8-(2-cyanoacetyl)-4-thioxo-3-hydro-6H,7H,9H,10H-1,4-diazaperhydroepino[1',7'-1,5]pyrrolo[3,2-d]pyrimidine-11-carbonitrile (Compound No. 676)

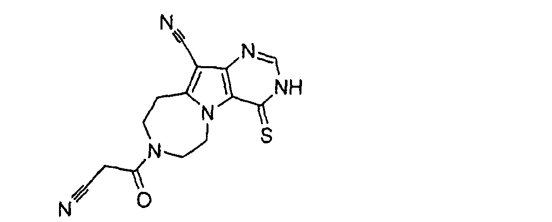

To a solution (1.5 mL) of 4-thioxo-3-hydro-6H,7H,8H,9H,10H-1,4-diazaperhydroepino[1',7'-1,5]pyrrolo[3,2-d]pyrimidine-11-carbonitrile (30 mg) in N,N-dimethylformamide/triethylamine = 2/1, cyano acetate(21 mg) was added, and furthermore a solution of 1-ethyl-3-(3'-dimethylaminopropyl)carbodiimide hydrochloride (70 mg) and N-hydroxybenzotriazole (25 mg) in dichloromethane (1 mL) was added thereto, and stirred overnight at room temperature. Methanol was added to the reaction mixture to stop the reaction, and then the solvent was evaporated under reduced pressure. The residue was purified on a preparative HPLC to obtain a title compound as a white solid compound.
HPLC retention time = 5.0 (min)
ESI/MS m/e: 313.1 (M⁺+H, C₁₄H₁₂N₆OS)

### [Working Example 16]

### Synthesis of (11-cyano-4-thioxo(3-hydro-6H,7H,9H,10H-1,4-diazaperhydroepino[4',5'-1,5]pyrrolo[3,2-d]pyrimidine-8-yl))-N-(4-methylphenyl)carboxamide (Compound No. 356)

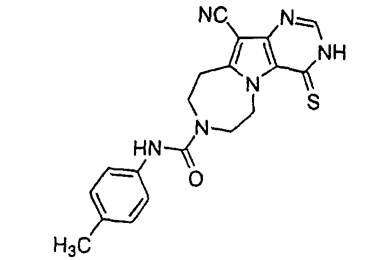

To a solution (2 mL) of 4-thioxo-3-hydro-6H,7H,8H,9H,10H-1,4-diazaperhydroepino[1',7'-1,5]pyrrolo[3,2-d]pyrimidine-11-carbonitrile (25 mg) in N,N-dimethylformamide/dichloromethane/triethylamine = 10/10/1, 4-methylphenyl isocyanate (13 mg) was added, and stirred at room temperature for five hours. Methanol was added to the reaction mixture to stop the reaction, and then the solvent was evaporated under reduced pressure. The residue was purified on a preparative HPLC to obtain a title compound as a white solid compound.
HPLC retention time = 8.6 (min)
¹H-NMR (400 MHz, DMSO-d₆) δ (ppm): 2.22 (s, 3H), 3.23 (m, 2H), 3.79 (m, 4H), 5.59 (m, 2H), 7.05 (d, 8.6 Hz, 2H), 7.33 (d, 8.6 Hz, 2H), 8.14 (d, 3.6 Hz, 1H), 8.56 (s, 1H), 13.73 (br, 1H).
ESI/MS m/e: 379.1 (M⁺+H, C₁₉H₁₈N₆OS)

### [Working Example 17]

### Synthesis of 8-(phenylsulfonyl)-4-thioxo-3-hydro-6H,7H,9H,10H-1,4-diazaperhydroepino[1',7'-5,1]pyrrolo[3,2-d]pyrimidine-11-carbonitrile (Compound No. 410)

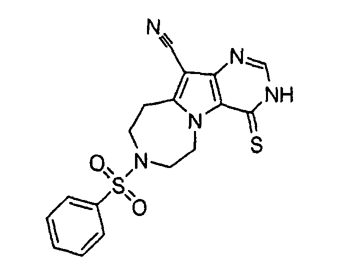

To a solution (2 mL) of 4-thioxo-3-hydro-6H,7H,8H,9H,10H-1,4-diazaperhydroepino[1',7'-1,5]pyrrolo[3,2-d]pyrimidine-11-carbonitrile (25 mg) in N,N-dimethylformamide/dichloromethane/triethylamine = 10/10/1, benzenesulfonyl chloride (16 mg) was added, and stirred at room temperature for five hours. Methanol was added to the reaction mixture to stop the reaction, and then the solvent was evaporated under reduced pressure. The residue was purified on a preparative HPLC to obtain a title compound as a white solid compound.
HPLC retention time = 8.7 (min)
ESI/MS m/e: 386.1 (M⁺+H, C₁₇H₁₅N₅O₂S₂)

### [Working Example 18]

### Synthesis of 3-[(11-cyano-4-thioxo-3-hydro-6H,7H,9H,10H-1,4-diazaperhydroepino[4',5'-1,5]pyrrolo[3,2-d]pyrimidine-8-yl)carbonylamino]benzoic acid (Compound No. 792)

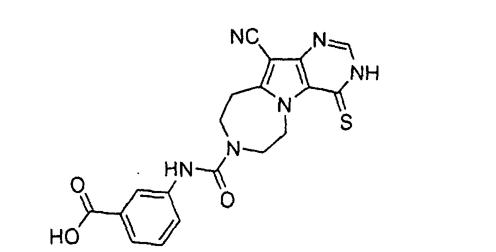

To ethyl 3-isocyanate benzoate (585 mg), a solution (20 mL) of 4-thioxo-3-hydro-6H,7H,8H,9H,10H-1,4-diazaperhydroepino[1',7'-1,5]pyrrolo[3,2-d]pyrimidine-11-carbonitrile (500 mg) in N,N-dimethylformamide/dichloromethane/triethylamine = 10/10/1 was added, and stirred at room temperature for five hours. Methanol (5 mL) was added dropwise to stop the reaction, and then the solvent was evaporated under reduced pressure. The reaction mixture was dried under vacuum, and washed with methanol to obtain a crude reaction product. This was dissolved in 1,4-dioxane (15 mL), and 1 mol/L aqueous solution of sodium hydroxide (10 mL) was added thereto, and stirred overnight at room temperature. Acetic acid was added to stop the reaction, and the solvent was evaporated under reduced pressure. Ethyl acetate and water were added to the residue to separate the organic layer. The aqueous layer was extracted with ethyl acetate, and the combined organic layer was washed with saturated saline, and dried on anhydrous magnesium sulfate, and filtered. The solvent was evaporated under reduced pressure to obtain a title compound (633 mg, yield 76%) as a white solid compound. HPLC retention time = 6.7 (min)
¹H-NMR (400 MHz, DMSO-d₆) δ (ppm): 3.27 (m, 2H), 3.81 (br, 4H), 5.62 (m, 2H), 7.36 (t, 7.8 Hz, 1H), 7.53 (m, 1H), 7.78 (m, 1H), 8.09 (s, 1H), 8.15 (s, 1H), 8.87 (s, 1H), 12.89 (br, 1H), 13.74 (br, 1H).
ESI/MS m/e: 409.2 (M⁺+H, C₁₉H₁₆N₆O₃S)

### [Working Example 19]

### Synthesis of N-(3-(N-hydroxycarbamoyl)phenyl)(11-cyano-4-thioxo-(3-hydro-6H,7H,9H,10H-1,4-diazaperhydroepino[4',5'-1,5]pyrrolo[3,2-d]pyrimidine-8-yl))carboxamide (Compound No. 847)

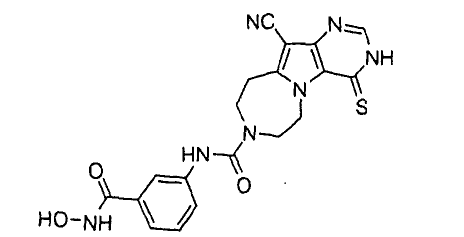

O-(tetrahydro-2H-pyrane-2-yl)hydroxylamine (86 mg), 1-ethyl-3-(3'-dimethylaminopropyl)carbodiimide hydrochloride (140 mg), N-hydroxybenzotriazole (50 mg), and 3-[(11-cyano-4-thioxo-3-hydro-6H,7H,9H,10H-1,4-diazaperhydroepino[4',5'-1,5]pyrrolo[3,2-d]pyrimidine-8-yl)carbonylamino]benzoic acid (100 mg) were dissolved in N,N-dimethylformamide (2 mL), to which triethylamine (0.3 mL) was added, and stirred at room temperature for six hours. Water and an excess of ethyl acetate were added to the reaction mixture to separate the organic layer, and further extracted with ethyl acetate. From the combined organic layer, the solvent was evaporated under reduced pressure. The residue was purified on a preparative HPLC to obtain a crude reaction product. 1,4-dioxane (3 mL) and 0.1 mol/L hydrochloric acid (0.6 mL) were added thereto, and stirred overnight at room temperature. The reaction mixture was neutralized with a saturated sodium bicarbonate solution, and the solvent was evaporated under reduced pressure. The residue was purified on a preparative HPLC to obtain a title compound (23 mg, yield 22%) as a white solid compound.
HPLC retention time = 5.2 (min)
¹H-NMR (400 MHz, DMSO-d₆) δ (ppm): 3.26 (br, 2H), 3.81 (br, 4H), 5.62 (br, 2H), 7.30 (m, 2H), 7.68 (m, 1H), 7.88 (s, 1H), 8.15 (m, 1H), 8.85 (s, 1H), 11.14 (s, 1H), 13.76 (s, 1H).
ESI/MS m/e: 424.3 (M⁺+H, C₁₉H₁₇N₇O₃S)

### [Working Example 20]

### Synthesis of 8-benzyl-4-thioxo-3-hydro-6H,7H,9H,10H-1,4-diazaperhydroepino[1',7'-1,5]pyrrolo[3,2-d]pyrimidine-11-carbonitrile (Compound No. 414)

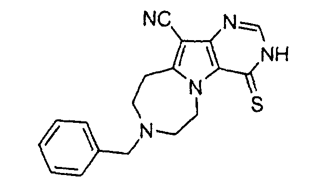

To a solution (34 mL) of 4-thioxo-3-hydro-6H,7H,8H,9H,10H-1,4-diazaperhydroepino[1',7'-1,5]pyrrolo[3,2-d]pyrimidine-11-carbonitrile (1.00 g) in N,N-dimethylformamide/dichloromethane/trimethyl orthoformate = 10/5/2, benzaldehyde was added, and stirred at room temperature for one hour. To this reaction mixture was added triacetoxy sodium borohydride (1.74 g) and stirred at room temperature for five hours. Methanol was added to the reaction mixture to stop the reaction, and then the solvent was evaporated under reduced pressure. The residue was purified on a cation exchange column to obtain a title compound (1.27 g, yield 92%) as a pale yellow solid compound.
HPLC retention time = 5.0 (min)
ESI/MS m/e: 336.4 (M⁺+H, C₁₈H₁₇N₅S)

### [Working Example 21]

### Synthesis of 4-[(3,3-dimethyl-3-silabutoxy)methylthio]-8-benzyl-6H,7H,9H,10H-1,4-diazaperhydroepino[1',7'-1,5]pyrrolo[3,2-d]pyrimidine-11-carbonitrile

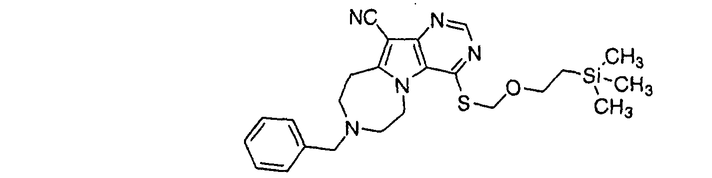

A solution (26 mL) of 8-benzyl-4-thioxo-3-hydro-6H,7H,9H,10H-1,4-diazaperhydroepino[1',7'-1,5]pyrrolo[3,2-d]pyrimidine-11-carbonitrile (534 mg) in N,N-dimethylformamide/tetrahydrofuran/triethylamine = 3/20/3 was cooled to 0°C under a nitrogen atmosphere, to which 2-(chloromethoxy)ethyltrimethylsilane (423 µl) was added dropwise. The reaction mixture was allowed to return to room temperature and stirred at room temperature for two hours, to which methanol was added to stop the reaction. The solvent was evaporated under reduced pressure, and the residue was purified on a silica gel chromatography (hexane/ethyl acetate = 3/1) to obtain a title compound (637 mg, yield 86%) as a pale yellow oily compound.
ESI/MS m/e: 466.3 (M⁺+H, C₂₄H₃₁N₅OSSi)

### [Working Example 22]

### Synthesis of 10-methyl-8-benzyl-4-thioxo-3-hydro-6H,7H,9H,10H-1,4-diazaperhydroepino[1',7'-1,5]pyrrolo[3,2-d]pyrimidine-11-carbonitrile (Compound No. 1501)

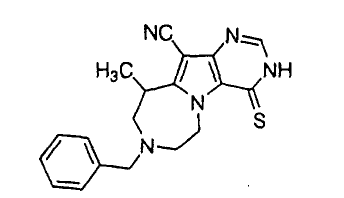

A solution (2 mL) of 4-[(3,3-dimethyl-3-silabutoxy)methylthio]-8-benzyl-6H,7H,9H,10H-1,4-diazaperhydroepino[1',7'-1,5]pyrrolo[3,2-d]pyrimidine-11-carbonitrile (85 mg) in tetrahydrofuran was cooled to -78°C under a nitrogen atmosphere, and lithium bis(trimethylsilyl)amide (365 µl, 1.0 mol/L tetrahydrofuran solution) was added dropwise. After stirring at -78°C for 30 minutes, methyl iodide (23 µl) was added dropwise, and was further stirred at -78°C for two hours. To the reaction mixture, acetic acid was added to neutralize, and after returning to room temperature ethyl acetate and water were added for dilution, and the organic layer was separated. The aqueous layer was extracted with ethyl acetate, and the solvent of the combined organic layer was evaporated under reduced pressure. A solution (3 mL) of trifluoroacetic acid/dichloromethane = 1/10 was added to the residue, and stirred for two hours. The solvent was evaporated under reduced pressure, and the residue was purified on a preparative HPLC to obtain a title compound (30 mg, yield 46%) as a pale yellow oily compound.
HPLC retention time = 5.6 (min)
¹H-NMR (400 MHz, DMSO-d₆) δ (ppm): 1.57 (br, 3H), 3.07-4.33 (m, 9H), 7.45 (m, 5H), 8.19 (s, 1H), 13.85 (br, 1H). ESI/MS m/e: 350.2 (M⁺+H, C₁₉H₁₉N₅S)

### [Working Examples 23-361]

Each of the following compounds of the present invention was synthesized using respective starting material and reactants according to either of the methods described in Working Examples 1-22. ESI/MS data of HPLC/mass spectrometry, the retention time and purity of each compound in HPLC analysis under the following analytical condition, and the synthetic method and the corresponding Working Example number are summarized in Table 60 - Table 69. In the tables, Compound Nos. indicate those in Table 1 - Table 5 that were listed as preferred examples described above.

**Table 60**

| Work. Ex. No. | Compound No. | Composition | ESI MS m/e | HPLC (min) | Purity (%) | Synthetic meth. |
|---|---|---|---|---|---|---|
| 23 | 6 | C13 H13 N5 O2 | 272.1 | 2.8 | 99 | Work. Ex. 6 |
| 24 | 14 | C19 H17 N5 O2 | 348.4 | 6.4 | 89 | Work. Ex. 6 |
| 25 | 27 | C21 H21 N5 O2 | 376.5 | 7.9 | 100 | Work. Ex. 6 |
| 26 | 29 | C20 H19 N5 O3 | 378.3 | 6.4 | 100 | Work. Ex. 6 |
| 27 | 30 | C20 H19 N5 O3 | 378.5 | 6.3 | 100 | Work. Ex. 6 |
| 28 | 31 | C21 H21 N5 O4 | 408.5 | 5.5 | 100 | Work. Ex. 6 |
| 29 | 38 | C19 H16 Cl N5 O2 | 382.4 | 7.4 | 100 | Work. Ex. 6 |
| 30 | 41 | C26 H23 N5 O3 | 454.6 | 9.0 | 98 | Work. Ex. 6 |
| 31 | 44 | C18 H15 N5 O2 | 334.3 | 6.1 | 99 | Work. Ex. 6 |
| 32 | 46 | C22 H17 N5 O2 | 384.3 | 7.5 | 100 | Work. Ex. 6 |
| 33 | 47 | C22 H17 N5 O2 | 384.4 | 7.9 | 100 | Work. Ex. 6 |
| 34 | 48 | C19 H17 N5 O2 | 348.6 | 6.6 | 100 | Work. Ex. 6 |
| 35 | 52 | C19 H17 N5 O3 | 364.5 | 6.2 | 95 | Work. Ex. 6 |
| 36 | 53 | C19 H17 N5 O3 | 364.5 | 6.5 | 100 | Work. Ex. 6 |
| 37 | 54 | C19 H17 N5 O3 | 364.3 | 6.4 | 95 | Work. Ex. 6 |
| 38 | 63 | C20 H20 N6 O2 | 377.3 | 4.5 | 80 | Work. Ex. 6 |
| 39 | 65 | C18 H14 N6 O4 | 379.4 | 6.1 | 100 | Work. Ex. 6 |
| 40 | 66 | C18 H14 N6 O4 | 379.4 | 6.5 | 100 | Work. Ex. 6 |
| 41 | 67 | C18 H14 N6 O4 | 379.1 | 6.6 | 98 | Work. Ex. 6 |
| 42 | 70 | C19 H14 N6 O2 | 359.4 | 5.9 | 100 | Work. Ex. 6 |
| 43 | 71 | C19 H14 N6 O2 | 359.4 | 6.0 | 97 | Work. Ex. 6 |
| 44 | 96 | C20 H17 N5 O4 | 392.4 | 6.5 | 97 | Work. Ex. 6 |
| 45 | 102 | C17 H14 N6 O2 | 335.4 | 4.5 | 100 | Work. Ex. 6 |
| 46 | 103 | C17 H14 N6 O2 | 335.4 | 2.1 | 98 | Work. Ex. 6 |
| 47 | 104 | C17 H14 N6 O2 | 335.4 | 1.8 | 98 | Work. Ex. 6 |
| 48 | 105 | C17 H13 Cl N6 O2 | 369.2 | 5.1 | 97 | Work. Ex. 6 |
| 49 | 106 | C17 H13 Cl N6 O2 | 369.4 | 5.8 | 100 | Work. Ex. 6 |
| 50 | 114 | C15 H18 N6 O2 | 315.3 | 5.2 | 99 | Work. Ex. 7 |
| 51 | 119 | C19 H18 N6 O2 | 363.5 | 6.5 | 100 | Work. Ex. 7 |
| 52 | 135 | C20 H20 N6 O2 | 377.5 | 7.2 | 100 | Work. Ex. 7 |
| 53 | 136 | C20 H20 N6 O2 | 377.5 | 7.3 | 100 | Work. Ex. 7 |
| 54 | 137 | C20 H20 N6 O2 | 377.5 | 7.3 | 100 | Work. Ex. 7 |
| 55 | 141 | C20 H20 N6 O3 | 393.5 | 6.5 | 100 | Work. Ex. 7 |
| 56 | 143 | C19 H17 F N6 O2 | 381.6 | 6.7 | 100 | Work. Ex. 7 |
| 57 | 144 | C19 H17 F N6 O2 | 381.6 | 6.8 | 100 | Work. Ex. 7 |
| 58 | 145 | C19 H17 F N6 O2 | 381.5 | 6.8 | 100 | Work. Ex. 7 |
| 59 | 147 | C19 H17 Cl N6 O2 | 397.5 | 7.3 | 100 | Work. Ex. 7 |

**Table 61**

| Work. Ex. No. | Compound No. | Composition | ESI MS m/e | HPLC (min) | Purity (%) | Synthetic meth. |
|---|---|---|---|---|---|---|
| 60 | 150 | C19 H16 C12 N6 O2 | 431.5 | 8.4 | 100 | Work. Ex. 7 |
| 61 | 151 | C19 H16 C12 N6 O2 | 431.5 | 8.4 | 100 | Work. Ex. 7 |
| 62 | 155 | C18 H16 N6 O2 | 349.2 | 6.5 | 93 | Work. Ex. 7 |
| 63 | 156 | C24 H20 N6 O2 | 425.5 | 9.2 | 100 | Work. Ex. 7 |
| 64 | 157 | C22 H18 N6 O2 | 399.5 | 7.4 | 94 | Work. Ex. 7 |
| 65 | 158 | C22 H18 N6 O2 | 399.5 | 8.3 | 90 | Work. Ex. 7 |
| 66 | 159 | C19 H18 N6 O2 | 363.4 | 6.5 | 100 | Work. Ex. 7 |
| 67 | 160 | C19 H18 N6 O2 | 363.5 | 7.3 | 100 | Work. Ex. 7 |
| 68 | 163 | C19 H18 N6 O3 | 379.4 | 6.8 | 100 | Work. Ex. 7 |
| 69 | 164 | C19 H18 N6 O3 | 379.5 | 6.8 | 100 | Work. Ex. 7 |
| 70 | 165 | C19 H18 N6 O3 | 379.2 | 6.4 | 97 | Work. Ex. 7 |
| 71 | 166 | C20 H20 N6 O4 | 409.5 | 7.0 | 100 | Work. Ex. 7 |
| 72 | 167 | C20 H20 N6 O4 | 409.6 | 5.9 | 85 | Work. Ex. 7 |
| 73 | 168 | C19 H16 N6 O4 | 393.6 | 6.3 | 96 | Work. Ex. 7 |
| 74 | 169 | C19 H15 F3 N6 O3 | 433.5 | 8.8 | 100 | Work. Ex. 7 |
| 75 | 170 | C25 H22 N6 O3 | 455.6 | 9.0 | 89 | Work. Ex. 7 |
| 76 | 171 | C24 H20 N6 O3 | 441.6 | 8.5 | 100 | Work. Ex. 7 |
| 77 | 172 | C24 H20 N6 O3 | 441.6 | 9.3 | 84 | Work. Ex. 7 |
| 78 | 173 | C24 H20 N6 O3 | 441.6 | 9.1 | 78 | Work. Ex. 7 |
| 79 | 175 | C20 H21 N7 O2 | 392.6 | 3.5 | 100 | Work. Ex. 7 |
| 80 | 176 | C18 H15 N7 O4 | 394.5 | 7.3 | 100 | Work. Ex. 7 |
| 81 | 177 | C18 H15 N7 O4 | 394.5 | 7.3 | 87 | Work. Ex. 7 |
| 82 | 178 | C18 H15 N7 O4 | 394.1 | 7.3 | 93 | 'Work. Ex. 7 |
| 83 | 179 | C18 H14 C1 N7 O4 | 428.4 | 8.4 | 100 | Work. Ex. 7 |
| 84 | 180 | C18 H14 N8 O6 | 439.5 | 8.3 | 97 | Work. Ex. 7 |
| 85 | 181 | C19 H15 N7 O2 | 374.5 | 6.7 | 100 | Work. Ex. 7 |
| 86 | 182 | C19 H15 N7 O2 | 374.5 | 6.6 | 100 | Work. Ex. 7 |
| 87 | 183 | C20 H18 N6 O3 | 391.5 | 6.2 | 100 | Work. Ex. 7 |
| 88 | 184 | C19 H15 F3 N6 O2 | 417.4 | 7.2 | 100 | Work. Ex. 7 |
| 89 | 186 | C19 H15 F3 N6 O2 | 417.5 | 8.7 | 100 | Work. Ex. 7 |
| 90 | 187 | C18 H15 F N6 O2 | 367.3 | 6.3 | 100 | Work. Ex. 7 |
| 91 | 188 | C18 H15 F N6 O2 | 367.4 | 7.2 | 100 | Work. Ex. 7 |
| 92 | 189 | C18 H15 F N6 O2 | 367.4 | 6.9 | 100 | Work. Ex. 7 |
| 93 | 190 | C18 H14 F2 N6 O2 | 385.6 | 7.9 | 100 | Work. Ex. 7 |
| 94 | 191 | C18 H15 Cl N6 O2 | 383.4 | 6.9 | 100 | Work. Ex. 7 |
| 95 | 192 | C18 H15 Cl N6 O2 | 383.4 | 7.9 | 100 | Work. Ex. 7 |
| 96 | 193 | C18 H15 Cl N6 O2 | 383.4 | 7.9 | 100 | Work. Ex. 7 |

**Table 62**

| Work. Ex. No. | Compound No. | Composition | ESI MS m/e | HPLC (min) | Purity (%) | Synthetic meth. |
|---|---|---|---|---|---|---|
| 97 | 194 | C18 H14 Cl2 N6 O2 | 417.4 | 8.9 | 100 | Work. Ex. 7 |
| 98 | 195 | C20 H18 N6 O4 | 407.5 | 8.2 | 100 | Work. Ex. 7 |
| 99 | 196 | C20 H18 N6 O4 | 407.5 | 7.0 | 100 | Work. Ex. 7 |
| 100 | 197 | C20 H18 N6 O4 | 407.6 | 6.9 | 100 | Work. Ex. 7 |
| 101 | 198 | C22 H20 N6 O6 | 465.6 | 7.6 | 98 | Work. Ex. 7 |
| 102 | 199 | C18 H14 F2 N6 O2 | 385.3 | 6.6 | 100 | Work. Ex. 7 |
| 103 | 200 | C18 H14 F2 N6 O2 | 385.4 | 7.5 | 100 | Work. Ex. 7 |
| 104 | 201 | C18 H14 Cl2 N6 O2 | 417.4 | 9.3 | 100 | Work. Ex. 7 |
| 105 | 202 | C21 H20 N6 O4 | 421.6 | 9.1 | 100 | Work. Ex. 7 |
| 106 | 235 | C18 H17 N5 O3 S | 384.2 | 8.0 | 92 | Work. Ex. 8 |
| 107 | 238 | C17 H14 N6 O5 S | 415.2 | 7.9 | 96 | Work. Ex. 8 |
| 108 | 239 | C18 H16 N6 O4 S | 413.5 | 6.0 | 100 | Work. Ex. 7 |
| 109 | 240 | C19 H18 N6 O4 S | 427.5 | 6.8 | 89 | Work. Ex. 7 |
| 110 | 242 | C18 H15 Cl N6 O4 S | 447.4 | 6.3 | 92 | Work. Ex. 7 |
| 111 | 282 | C19 H17 N5 O S | 364.3 | 7.5 | 97 | Work. Ex. 14 |
| 112 | 283 | C20 H19 N5 O S | 378.1 | 8.6 | 95 | Work. Ex. 14 |
| 113 | 330 | C19 H18 N6 O S | 379.1 | 7.7 | 94 | Work. Ex. 16 |
| 114 | 331 | C21 H26 N6 O5 S | 475.2 | 8.0 | 98 | Work. Ex. 16 |
| 115 | 332 | C17 H20 N6 O3 S | 389.2 | 6.4 | 97 | Work. Ex. 16 |
| 116 | 333 | C18 H22 N6 O3 S2 | 435.2 | 7.3 | 98 | Work. Ex. 16 |
| 117 | 334 | C20 H26 N6 O4 S | 447.3 | 8.2 | 98 | Work. Ex. 16 |
| 118 | 335 | C20 H26 N6 O3 S | 431.1 | 8.0 | 97 | Work. Ex. 16 |
| 119 | 336 | C16 H18 N6 O3 S | 375.2 | 5.9 | 97 | Work. Ex. 16 |
| 120 | 337 | C18 H22 N6 O3 S | 403.2 | 6.8 | 98 | Work. Ex. 16 |
| 121 | 340 | C20 H20 N6 O2 S | 409.4 | 7.7 | 100 | Work. Ex. 16 |
| 122 | 341 | C19 H17 F N6 O S | 397.4 | 7.9 | 100 | Work. Ex. 16 |
| 123 | 342 | C19 H17 F N6 O S | 397.4 | 8.0 | 96 | Work. Ex. 16 |
| 124 | 344 | C19 H17 Cl N6 O S | 413.4 | 8.5 | 100 | Work. Ex. 16 |
| 125 | 347 | C19 H16 Cl2 N6 O S | 447.3 | | 97 | Work. Ex. 16 |
| 126 | 351 | C18 H16 N6 O S | 365.2 | 7.8 | 82 | Work. Ex. 16 |
| 127 | 353 | C22 H18 N6 O S | 415.4 | 9.5 | 100 | Work. Ex. 16 |
| 128 | 354 | C19 H18 N6 O S | 379.4 | 7.8 | 95 | Work. Ex. 16 |
| 129 | 355 | C19 H18 N6 O S | 379.0 | 8.6 | 92 | Work. Ex. 16 |
| 130 | 359 | C19 H18 N6 O2 S | 395.4 | 8.0 | 93 | Work. Ex. 16 |
| 131 | 360 | C19 H18 N6 O2 S | 395.2 | 7.6 | 87 | Work. Ex. 16 |
| 132 | 361 | C20 H20 N6 O3 S | 425.4 | 7.0 | 100 | Work. Ex. 16 |
| 133 | 363 | C19 H15 F3 N6 O2 S | 449.4 | 10.0 | 100 | Work. Ex. 16 |

**Table 63**

| Work. Ex. No. | Compound No. | Composition | ESI MS m/e | HPLC (min) | Purity (%) | Synthetic meth. |
|---|---|---|---|---|---|---|
| 134 | 365 | C18 H15 N7 O3 S | 410.3 | 8.5 | 93 | Work. Ex. 16 |
| 135 | 366 | C18 H15 Cl N6 O S | 399.3 | 8.3 | 100 | Work. Ex. 16 |
| 136 | 368 | C18 H15 Cl N6 O S | 399.3 | 9.1 | 100 | Work. Ex. 16 |
| 137 | 374 | C19 H18 N6 S2 | 395.3 | 9.2 | 98 | Work. Ex. 16 |
| 138 | 375 | C20 H20 N6 S2 | 409.3 | 9.9 | 98 | Work. Ex. 16 |
| 139 | 391 | C18 H16 N6 S2 | 381.3 | 8.5 | 97 | Work. Ex. 16 |
| 140 | 405 | C20 H18 N6 02 S2 | 439.2 | 8.7 | 95 | Work. Ex. 16 |
| 141 | 415 | C19 H19 N5 S | 350.0 | 5.9 | 97 | Work. Ex. 20 |
| 142 | 420 | C19 H19 N5 S | 350.1 | 5.9 | 95 | Work. Ex. 20 |
| 143 | 421 | C19 H19 N5 O S | 366.4 | 5.6 | 97 | Work. Ex. 20 |
| 144 | 422 | C19 H19 N5 O S | 366.0 | 5.5 | 95 | Work. Ex. 20 |
| 145 | 423 | C19 H19 N5 O S | 366.2 | 5.4 | 89 | Work. Ex. 20 |
| 146 | 424 | C18 H16 F N5 S | 354.2 | 5.1 | 100 | Work. Ex. 20 |
| 147 | 425 | C18 H16 F N5 S | 354.0 | 5.5 | 100 | Work. Ex. 20 |
| 148 | 426 | C18 H16 F N5 S | 354.1 | 5.4 | 94 | Work. Ex. 20 |
| 149 | 427 | C18 H17 N5 O S | 352.3 | 3.2 | 96 | Work. Ex. 20 |
| 150 | 428 | C18 H16 Cl N5 S | 370.0 | 6.0 | 100 | Work. Ex. 20 |
| 151 | 432 | C22 H19 N5 S | 386.2 | 6.8 | 95 | Work. Ex. 20 |
| 152 | 433 | C22 H19 N5 S | 386.2 | 6.8 | 95 | Work. Ex. 20 |
| 153 | 435 | C12 H11 N5 O2 | 258.3 | 2.2 | 98 | Work. Ex. 6 |
| 154 | 439 | C12 H8 F3 N5 O2 | 312.2 | 5.9 | 100 | Work. Ex. 5 |
| 155 | 449 | C17 H13 N5 O2 | 320.1 | 5.8 | 100 | Work. Ex. 6 |
| 156 | 456 | C14 H16 N6 O2 | 301.2 | 5.1 | 91 | Work. Ex. 7 |
| 157 | 462 | C17 H14 N6 O2 | 335.3 | 6.5 | 98 | Work. Ex. 7 |
| 158 | 521 | C12 H8 F3 N5 O S | 328.1 | 7.8 | 99 | Work. Ex. 11 |
| 159 | 668 | C14 H15 N5 O2 S | 318.3 | 4.9 | 98 | Work. Ex. 14 |
| 160 | 672 | C17 H15 N7 O S | 364.0 | 3.8 | 95 | Work. Ex. 18 |
| 161 | 674 | C17 H20 N6 O2 S | 371.3 | 5.1 | 95 | Work. Ex. 18 |
| 162 | 678 | C15 H16 N6 O2 S | 343.4 | 3.6 | 97 | Work. Ex. 15 |
| 163 | 680 | C14 H15 N7 O2 S | 344.3 | 2.6 | 95 | Work. Ex. 15 |
| 164 | 682 | C15 H16 N6 O2 S | 343.3 | 4.3 | 93 | Work. Ex. 15 |
| 165 | 684 | C15 H17 N7 O2 S | 358.2 | 4.0 | 89 | Work. Ex. 15 |
| 166 | 686 | C15 H16 N6 O2 S | 343.2 | 3.5 | 95 | Work. Ex. 15 |
| 167 | 688 | C15 H17 N7 O2 S | 358.2 | 3.4 | 96 | Work. Ex. 15 |
| 168 | 692(±) | C21 H26 N6 O5 S | 475.3 | 7.1 | 99 | Work. Ex. 15 |
| 169 | 692(R) | C21 H26 N6 O5 S | 475.3 | 7.1 | 95 | Work. Ex. 15 |
| 170 | 696(S) | C20 H24 N6 O5 S | 461.3 | 7.0 | 92 | Work. Ex. 15 |

**Table 64**

| Work. Ex. No. | Compound No. | Composition | ESI MS m/e | HPLC (min) | Purity (%) | Synthetic meth. |
|---|---|---|---|---|---|---|
| 171 | 696(R) | C20 H24 N6 O5 S | 461.4 | 7.0 | 93 | Work. Ex. 15 |
| 172 | 702 | C21 H16 N6 O3 | 401.4 | 6.7 | 100 | Work. Ex. 6 |
| 173 | 731 | C15 H16 N6 O3 S | 361.3 | 4.6 | 99 | Work. Ex. 18 |
| 174 | 732 | C17 H20 N6 O3 S2 | 421.5 | 6.3 | 96 | Work. Ex. 18 |
| 175 | 734 | C18 H22 N6 O3 S | 403.2 | 5.8 | 99 | Work. Ex. 18 |
| 176 | 735 | C14 H14 N6 O3 S | 347.2 | 4.1 | 97 | Work. Ex. 18 |
| 177 | 736 | C16 H18 N6 O3 S | 375.1 | 4.9 | 96 | Work. Ex. 18 |
| 178 | 739 | C20 H20 N6 O S | 393.4 | 8.3 | 100 | Work. Ex. 16 |
| 179 | 740 | C20 H20 N6 O S | 393.4 | 8.5 | 95 | Work. Ex. 16 |
| 180 | 741 | C20 H20 N6 O S | 393.4 | 8.5 | 72 | Work. Ex. 16 |
| 181 | 747 | C20 H20 N6 O3 S | 425.4 | 8.2 | 93 | Work. Ex. 16 |
| 182 | 749 | C24 H20 N6 O2 S | 457.4 | 10.5 | 100 | Work. Ex. 16 |
| 183 | 750 | C24 H20 N6 O2 S | 457.4 | 10.3 | 100 | Work. Ex. 16 |
| 184 | 752 | C20 H21 N7 O S | 408.4 | 4.7 | 100 | Work. Ex. 16 |
| 185 | 753 | C18 H15 N7 O3 S | 410.4 | 8.6 | 100 | Work. Ex. 16 |
| 186 | 755 | C18 H14 Cl N7 O3 S | 444.4 | 9.5 | 93 | Work. Ex. 16 |
| 187 | 756 | C18 H14 N8 O5 S | 455.4 | 9.4 | 90 | Work. Ex. 16 |
| 188 | 758 | C19 H15 N7 O S | 390.4 | 7.8 | 99 | Work. Ex. 16 |
| 189 | 759 | C20 H18 N6 O2 S | 407.4 | 7.3 | 100 | Work. Ex. 16 |
| 190 | 760 | C19 H15 F3 N6 O S | 433.4 | 8.4 | 95 | Work. Ex. 16 |
| 191 | 763 | C18 H15 F N6 O S | 383.3 | 7.7 | 83 | Work. Ex. 16 |
| 192 | 764 | C18 H15 F N6 O S | 383.3 | 8.5 | 94 | Work. Ex. 16 |
| 193 | 765 | C18 H15 F N6 O S | 383.3 | 8.1 | 98 | Work. Ex. 16 |
| 194 | 769 | C20 H18 N6 O3 S | 423.4 | 8.2 | 100 | Work. Ex. 16 |
| 195 | 770 | C22 H20 N6 O5 S | 481.4 | 8.7 | 89 | Work. Ex. 16 |
| 196 | 771 | C18 H14 F2 N6 O S | 401.3 | 7.9 | 100 | Work. Ex. 16 |
| 197 | 772 | C18 H14 F2 N6 O S | 401.3 | 8.8 | 99 | Work. Ex. 16 |
| 198 | 773 | C18 H14 C12 N6 O S | 433.3 | 10.7 | 100 | Work. Ex. 16 |
| 199 | 791 | C19 H16 N6 O4 | 393.5 | 5.6 | 100 | Work. Ex. 7 |
| 200 | 793(S) | C19 H24 N6 O4 S | 433.5 | 7.0 | 99 | Work. Ex. 18 |
| 201 | 793(R) | C19 H24 N6 O4 S | 433.4 | 7.0 | 96 | Work. Ex. 18 |
| 202 | 796 | C19 H16 N6 O3 S | 409.3 | 6.4 | 99 | Work. Ex. 18 |
| 203 | 811 | C19 H16 N6 O3 S | 409.3 | 8.0 | 98 | Work. Ex. 18 |
| 204 | 836 | C15 H16 N6 O3 S | 361.2 | 4.9 | 93 | Work. Ex. 18 |
| 205 | 837 | C18 H22 N6 O3 S | 403.4 | 7.1 | 99 | Work. Ex. 18 |
| 206 | 838 | C21 H20 N6 O3 S | 437.4 | | 97 | Work. Ex. 18 |
| 207 | 956 | C19 H16 N6 O3 | 377.5 | 5.7 | 100 | Work. Ex. 7 |

**Table 65**

| Work. Ex. No. | Compound No. | Composition | ESI MS m/e | HPLC (min) | Purity (%) | Synthetic meth. |
|---|---|---|---|---|---|---|
| 208 | 1067 | C20 H18 N6 O2 S2 | 439.1 | 9.7 | 91 | Work. Ex. 16 |
| 209 | 1069 | C20 H18 N6 O2 S2 | 439.3 | 8.6 | 89 | Work. Ex. 16 |
| 210 | 1072 | C17 H15 N7 S2 | 382.2 | 4.8 | 99 | Work. Ex. 16 |
| 211 | 1074 | C18 H17 N7 S2 | 396.2 | 4.9 | 96 | Work. Ex. 16 |
| 212 | 1076 | C18 H16 N6 03 S3 | 461.1 | 4.4 | 87 | Work. Ex. 16 |
| 213 | 1078 | C17 H20 N6 O S2 | 389.2 | 7.6 | 97 | Work. Ex. 16 |
| 214 | 1080 | C17 H16 N6 O S2 | 385.0 | 8.5 | 98 | Work. Ex. 16 |
| 215 | 1082 | C18 H17 N7 O2 S3 | 460.3 | 6.6 | 81 | Work. Ex. 16 |
| 216 | 1084 | C21 H29 N7 O2 S2 | 476.4 | 9.3 | 84 | Work. Ex. 16 |
| 217 | 1087 | C19 H16 N6 O3 S | 409.4 | 6.1 | 99 | Work. Ex. 7 |
| 218 | 1088 | C19 H16 N6 O2 S2 | 425.1 | 7.1 | 91 | Work. Ex. 18 |
| 219 | 1090 | C19 H16 N6 O2 S2 | 425.1 | 7.0 | 97 | Work. Ex. 18 |
| 220 | 1092 | C19 H25 N7 S2 | 416.2 | 5.5 | 86 | Work. Ex. 16 |
| 221 | 1094 | C24 H23 N7 S2 | 474.2 | 6.2 | 84 | Work. Ex. 16 |
| 222 | 1096 | C19 H16 N6 O2 S2 | 425.3 | 8.5 | 91 | Work. Ex. 18 |
| 223 | 1098 | C15 H18 N6 O S2 | 363.3 | 6.9 | 98 | Work. Ex. 16 |
| 224 | 1100 | C16 H20 N6 O S2 | 377.3 | 7.3 | 98 | Work. Ex. 16 |
| 225 | 1102 | C19 H25 N7 O S2 | 432.4 | 4.8 | 98 | Work. Ex. 16 |
| 226 | 1104 | C16 H18 N6 O2 S2 | 391.1 | 6.4 | 98 | Work. Ex. 18 |
| 227 | 1106 | C14 H17 N7 S2 | 348.1 | 4.2 | 99 | Work. Ex. 16 |
| 228 | 1108 | C15 H19 N7 S2 | 362.1 | 4.3 | 99 | Work. Ex. 16 |
| 229 | 1110 | C16 H21 N7 S2 | 376.4 | 4.6 | 96 | Work. Ex. 16 |
| 230 | 1112 | C17 H23 N7 S2 | 390.3 | 5.0 | 92 | Work. Ex. 16 |
| 231 | 1114 | C18 H17 N7 S2 | 396.5 | 4.7 | 95 | Work. Ex. 16 |
| 232 | 1116 | C19 H19 N7 S2 | 410.4 | 4.9 | 92 | Work. Ex. 16 |
| 233 | 1118 | C19 H27 N7 S2 | 418.3 | 5.4 | 83 | Work. Ex. 16 |
| 234 | 1141 | C15 H19 N5 S | 302.1 | 4.1 | 69 | Work. Ex. 20 |
| 235 | 1161 | C20 H21 N5 O2 S | 396.0 | 5.0 | 95 | Work. Ex. 20 |
| 236 | 1163 | C25 H23 N5 O S | 442.4 | 7.9 | 100 | Work. Ex. 20 |
| 237 | 1166 | C17 H16 N6 S | 337.4 | 2.3 | 99 | Work. Ex. 20 |
| 238 | 1172 | C20 H21 N5 S | 364.2 | 6.5 | 94 | Work. Ex. 20 |
| 239 | 1174 | C19 H17 N5 O2 S | 380.3 | 3.7 | 87 | Work. Ex. 20 |
| 240 | 1176 | C17 H16 N6 S | 337.1 | 3.2 | 95 | Work. Ex. 20 |
| 241 | 1177 | C25 H23 N5 O S | 442.5 | 7.9 | 99 | Work. Ex. 20 |
| 242 | 1178 | C25 H23 N5 O S | 442.5 | 7.6 | 100 | Work. Ex. 20 |
| 243 | 1179 | C19 H16 N6 S | 361.2 | 6.2 | 90 | Work. Ex. 20 |
| 244 | 1180 | C19 H16 N6 S | 361.3 | 5.1 | 87 | Work. Ex. 20 |

**Table 66**

| Work. Ex. No. | Compound No. | Composition | ESI MS m/e | HPLC (min) | Purity (%) | Synthetic meth. |
|---|---|---|---|---|---|---|
| 245 | 1181 | C18 H23 N5 S | 342.4 | 6.0 | 99 | Work. Ex. 20 |
| 246 | 1182 | C21 H23 N5 O3 S | 427.2 | 5.5 | 93 | Work. Ex. 20 |
| 247 | 1183 | C19 H17 N5 O2 S | 380.1 | 5.2 | 93 | Work. Ex. 20 |
| 248 | 1184 | C15 H19 N5 S | 302.1 | 3.5 | 95 | Work. Ex. 20 |
| 249 | 1192 | C18 H17 N5 O S | 352.2 | 4.7 | 96 | Work. Ex. 20 |
| 250 | 1193 | C18 H16 N6 O2 S | 381.2 | 6.2 | 99 | Work. Ex. 20 |
| 251 | 1194 | C20 H21 N5 O2 S | 396.3 | 4.9 | 95 | Work. Ex. 20 |
| 252 | 1196 | C18 H16 N6 O2 S | 381.0 | 5.8 | 98 | Work. Ex. 20 |
| 253 | 1198 | C19 H17 N5 O2 S | 380.1 | 4.5 | 99 | Work. Ex. 20 |
| 254 | 1200 | C18 H15 C1 N6 O2 S | 415.4 | 9.2 | 78 | Work. Ex. 20 |
| 255 | 1201 | C18 H16 C1 N5 O S | 386.3 | 5.9 | 92 | Work. Ex. 20 |
| 256 | 1202 | C19 H19 N5 O2 S | 382.3 | 4.8 | 94 | Work. Ex. 20 |
| 257 | 1203 | C18 H16 N6 O3 S | 397.3 | 5.0 | 87 | Work. Ex. 20 |
| 258 | 1204 | C19 H16 N6 O4 S | 425.4 | 6.1 | 83 | Work. Ex. 20 |
| 259 | 1205 | C19 H15 F4 N5 S | 422.3 | 8.8 | 97 | Work. Ex. 20 |
| 260 | 1214 | C19 H19 N5 O2 S | 382.1 | 4.5 | 98 | Work. Ex. 20 |
| 261 | 1222 | C16 H14 N6 O3 S | 371.0 | 6.0 | 74 | Work. Ex. 20 |
| 262 | 1223 | C17 H17 N5 O S | 340.0 | 5.0 | 96 | Work. Ex. 20 |
| 263 | 1224 | C17 H17 N5 O2 S | 356.0 | 2.2 | 100 | Work. Ex. 20 |
| 264 | 1225 | C18 H15 C1 F N5 S | 387.9 | 6.4 | 96 | Work. Ex. 20 |
| 265 | 1226 | C20 H21 N5 O2 S | 396.0 | 6.0 | 95 | Work. Ex. 20 |
| 266 | 1227 | C20 H21 N5 O2 S | 396.0 | 5.9 | 96 | Work. Ex. 20 |
| 267 | 1228 | C18 H16 F N5 O S | 370.0 | 4.7 | 78 | Work. Ex. 20 |
| 268 | 1229 | C19 H19 N5 O2 S | 382.0 | 5.0 | 98 | Work. Ex. 20 |
| 269 | 1230 | C18 H17 N5 O2 S | 368.1 | 3.6 | 100 | Work. Ex. 20 |
| 270 | 1231 | C19 H19 N5 O2 S | 382.0 | 5.0 | 100 | Work. Ex. 20 |
| 271 | 1232 | C18 H17 N5 O2 S | 367.9 | 2.6 | 95 | Work. Ex. 20 |
| 272 | 1234 | C19 H16 F3 N5 S | 404.0 | 8.1 | 93 | Work. Ex. 20 |
| 273 | 1235 | C19 H18 F N5 O S | 384.0 | 5.6 | 96 | Work. Ex. 20 |
| 274 | 1236 | C20 H21 N5 O2 S | 396.0 | 6.0 | 95 | Work. Ex. 20 |
| 275 | 1237 | C18 H17 N5 O S | 352.0 | 3.9 | 100 | Work. Ex. 20 |
| 276 | 1238 | C19 H19 N5 O2 S | 382.0 | 4.1 | 86 | Work. Ex. 20 |
| 277 | 1239 | C18 H17 N5 O2 S | 369.0 | 2.6 | 71 | Work. Ex. 20 |
| 278 | 1240 | C19 H16 F3 N5 S | 404.0 | 7.0 | 95 | Work. Ex. 20 |
| 279 | 1241 | C19 H16 N6 S | 361.0 | 5.1 | 94 | Work. Ex. 20 |
| 280 | 1242 | C20 H20 N6 O S | 393.1 | 3.4 | 91 | Work. Ex. 20 |
| 281 | 1243 | C16 H15 N5 S2 | 342.0 | 4.6 | 97 | Work. Ex. 20 |

**Table 67**

| Work. Ex. No. | Compound No. | Composition | ESI MS m/e | HPLC (min) | Purity (%) | Synthetic meth. |
|---|---|---|---|---|---|---|
| 282 | 1244 | C17 H17 N5 S2 | 356.1 | 5.4 | 97 | Work. Ex. 20 |
| 283 | 1245 | C16 H14 Br N5 S2 | 422.0 | 6.8 | 96 | Work. Ex. 20 |
| 284 | 1247 | C16 H14 N6 O2 S2 | 387.0 | 7.8 | 93 | Work. Ex. 20 |
| 285 | 1249 | C17 H17 N5 S2 | 356.1 | 5.7 | 100 | Work. Ex. 20 |
| 286 | 1250 | C16 H15 N5 S2 | 342.0 | 4.6 | 96 | Work. Ex. 20 |
| 287 | 1251 | C19 H19 N5 O2 S | 382.1 | 3.9 | 95 | Work. Ex. 20 |
| 288 | 1252 | C19 H16 F3 N5 S | 404.1 | 7.1 | 100 | Work. Ex. 20 |
| 289 | 1253 | C16 H21 N5 S | 316.1 | 4.9 | 97 | Work. Ex. 20 |
| 290 | 1255 | C16 H21 N5 S | 316.1 | 5.2 | 100 | Work. Ex. 20 |
| 291 | 1256 | C20 H20 N6 O4 S | 441.1 | 5.9 | 100 | Work. Ex. 20 |
| 292 | 1257 | C19 H18 N6 O3 S | 411.1 | 8.4 | 93 | Work. Ex. 20 |
| 293 | 1258 | C20 H18 N6 O2 S | 407.2 | 6.3 | 100 | Work. Ex. 20 |
| 294 | 1259 | C18 H15 C1 N6 O2 S | 415.1 | 8.2 | 100 | Work. Ex. 20 |
| 295 | 1260 | C18 H16 N6 O3 S | 397.2 | 5.4 | 68 | Work. Ex. 20 |
| 296 | 1261 | C20 H18 N6 O2 S | 407.2 | 6.5 | 100 | Work. Ex. 20 |
| 297 | 1262 | C18 H15 F2 N5 S | 372.2 | 5.9 | 100 | Work. Ex. 20 |
| 298 | 1263 | C18 H15 F2 N5 S | 372.2 | 5.6 | 100 | Work. Ex. 20 |
| 299 | 1264 | C18 H15 F2 N5 S | 372.2 | 5.8 | 100 | Work. Ex. 20 |
| 300 | 1265 | C18 H15 F2 N5 S | 372.2 | 6.0 | 94 | Work. Ex. 20 |
| 301 | 1266 | C16 H15 N5 O S | 326.1 | 3.7 | 100 | Work. Ex. 20 |
| 302 | 1267 | C18 H17 N5 O2 S | 368.2 | 3.3 | 81 | Work. Ex. 20 |
| 303 | 1268 | C18 H15 C1 F N5 S | 388.1 | 6.5 | 100 | Work. Ex. 20 |
| 304 | 1269 | C19 H19 N5 O S | 366.2 | 4.7 | 96 | Work. Ex. 20 |
| 305 | 1270 | C21 H21 N5 O S | 392.2 | 6.6 | 100 | Work. Ex. 20 |
| 306 | 1271 | C20 H17 N5 O S | 376.2 | 6.5 | 100 | Work. Ex. 20 |
| 307 | 1272 | C21 H21 N5 O S | 392.3 | 6.5 | 94 | Work. Ex. 20 |
| 308 | 1273 | C19 H16 C1 N5 O2 S | 414.4 | 6.2 | 95 | Work. Ex. 20 |
| 309 | 1274 | C19 H19 N5 O S | 366.2 | 5.7 | 100 | Work. Ex. 20 |
| 310 | 1275 | C18 H15 F N6 O2 S | 399.2 | 6.5 | 100 | Work. Ex. 20 |
| 311 | 1276 | C18 H15 C1 F N5 S | 388.1 | 6.6 | 94 | Work. Ex. 20 |
| 312 | 1277 | C22 H18 N6 O3 S | 447.2 | 7.2 | 91 | Work. Ex. 20 |
| 313 | 1278 | C18 H16 Cl N5 O S | 386.1 | 5.0 | 96 | Work. Ex. 20 |
| 314 | 1279 | C19 H15 F4 N5 S | 422.2 | 7.5 | 100 | Work. Ex. 20 |
| 315 | 1280 | C19 H15 F4 N5 S | 422.2 | 7.7 | 100 | Work. Ex. 20 |
| 316 | 1281 | C19 H15 F4 N5 S | 422.2 | 7.5 | 100 | Work. Ex. 20 |
| 317 | 1282 | C19 H15 F4 N5 S | 422.2 | 8.9 | 94 | Work. Ex. 20 |
| 318 | 1283 | C19 H15 F4 N5 S | 422.2 | 7.4 | 94 | Work. Ex. 20 |

**Table 68**

| Work. Ex. No. | Compound No. | Composition | ESI MS m/e | HPLC (min) | Purity (%) | Synthetic meth. |
|---|---|---|---|---|---|---|
| 319 | 1284 | C19 H19 N5 O S | 366.2 | 6.1 | 92 | Work. Ex. 20 |
| 320 | 1285 | C19 H18 F N5 O S | 384.2 | 5.7 | 100 | Work. Ex. 20 |
| 321 | 1286 | C22 H20 C1 N7 S | 450.4 | 6.7 | 95 | Work. Ex. 20 |
| 322 | 1287 | C22 H18 N6 O3 S | 447.2 | 7.3 | 100 | Work. Ex. 20 |
| 323 | 1288 | C19 H15 C1 F3 N5 S | 438.3 | 8.9 | 100 | Work. Ex. 20 |
| 324 | 1289 | C15 H14 N6 S2 | 343.2 | 4.8 | 84 | Work. Ex. 20 |
| 325 | 1290 | C19 H18 F N5 O S | 384.2 | 5.8 | 94 | Work. Ex. 20 |
| 326 | 1291 | C25 H30 N6 O2 S | 479.3 | 7.9 | 86 | Work. Ex. 20 |
| 327 | 1291 | C25 H30 N6 O2 S | 479.3 | 7.9 | 87 | Work. Ex. 20 |
| 328 | 1292 | C19 H17 N5 O S | 364.2 | | 84 | Work. Ex. 20 |
| 329 | 1293 | C19 H19 N5 O2 S | 382.2 | 5.3 | 100 | Work. Ex. 20 |
| 330 | 1294 | C16 H14 Br N5 O S | 406.1 | 5.7 | 89 | Work. Ex. 20 |
| 331 | 1295 | C20 H21 N5 O S | 380.2 | 6.2 | 53 | Work. Ex. 20 |
| 332 | 1296 | C22 H30 N6 O3 S | 459.3 | 7.6 | 85 | Work. Ex. 20 |
| 333 | 1297 | C19 H17 N5 O2 S | 380.2 | | 64 | Work. Ex. 20 |
| 334 | 1298 | C22 H18 C1 N5 O S | 436.1 | 7.8 | 100 | Work. Ex. 20 |
| 335 | 1299 | C22 H18 N6 O3 S | 447.1 | 6.8 | 100 | Work. Ex. 20 |
| 336 | 1300 | C19 H15 F4 N5 S | 422.2 | 7.9 | 96 | Work. Ex. 20 |
| 337 | 1301 | C17 H21 N5 O2 S | 360.2 | 4.4 | 71 | Work. Ex. 20 |
| 338 | 1302 | C21 H28 N6 O2 S | 429.3 | 7.1 | 100 | Work. Ex. 20 |
| 339 | 1303 | C19 H19 N5 O S | 366.2 | 5.5 | 100 | Work. Ex. 20 |
| 340 | 1304 | C22 H20 N6 O2 S2 | 465.2 | 7.2 | 92 | Work. Ex. 20 |
| 341 | 1306 | C19 H15 C1 F3 N5 S | 438.1 | 8.5 | 100 | Work. Ex. 20 |
| 342 | 1308 | C23 H21 N7 O2 S | 460.2 | 7.6 | 88 | Work. Ex. 20 |
| 343 | 1309 | C18 H16 F N5 O S | 370.2 | 5.1 | 68 | Work. Ex. 20 |
| 344 | 1310 | C20 H18 N6 S | 375.2 | 6.2 | 95 | Work. Ex. 20 |
| 345 | 1311 | C16 H17 N7 S | 340.2 | 3.9 | 100 | Work. Ex. 20 |
| 346 | 1312 | C18 H15 C1 F N5 S | 388.1 | 6.6 | 100 | Work. Ex. 20 |
| 347 | 1313 | C18 H15 F N6 O2 S | 399.1 | 6.2 | 79 | Work. Ex. 20 |
| 348 | 1315 | C23 H20 N6 S | 413.2 | 3.8 | 64 | Work. Ex. 20 |
| 349 | 1316 | C19 H15 F N6 S | 379.1 | 5.8 | 100 | Work. Ex. 20 |
| 350 | 1317 | C21 H20 N6 S | 389.1 | 7.0 | 100 | Work. Ex. 20 |
| 351 | 1318 | C19 H18 N6 O3 S | 411.1 | 5.7 | 100 | Work. Ex. 20 |
| 352 | 1319 | C20 H19 N5 O S | 378.1 | 4.9 | 98 | Work. Ex. 20 |
| 353 | 1322 | C18 H15 F2 N5 S | 372.2 | 5.4 | 100 | Work. Ex. 20 |
| 354 | 2164 | C13 H14 N4 S | 259.4 | 9.1 | 89 | Work. Ex. 22 |
| 355 | 2190 | C12 H13 N5 O | 244.1 | 1.6 | 97 | Work. Ex. 4 |

**Table 69**

| Work. Ex. No. | Compound No. | Composition | ESI MS m/e | HPLC (min) | Purity (%) | Synthetic meth. |
|---|---|---|---|---|---|---|
| 356 | 2203 | C17 H21 N5 O3 | 344.3 | 7.2 | 100 | Work. Ex. 1 |
| 357 | 2240 | C18 H23 N7 O S2 | 418.2 | 4.9 | 88 | Work. Ex. 16 |
| 358 | 2241 | C24 H27 N7 O2 S2 | 510.3 | 9.6 | 75 | Work. Ex. 16 |
| 359 | 2242 | C18 H16 N6 O2 S | 381.2 | 5.9 | 81 | Work. Ex. 20 |
| 360 | 2243 | C15 H17 N5 O2 | 300.3 | 6.0 | 97 | Work. Ex. 6 |
| 361 | 2244 | C23 H24 N6 O3 | 433.3 | 8.0 | 90 | Work. Ex. 6 |

### [Working Example 362]

¹H-NMR (400 MHz, DMSO-d₆) of the compounds of the present invention was determined. The following Table 70 - Table 71 show the data of chemical shift (δ: ppm) and coupling constant (J: Hz). Working Example Nos. in the Tables indicate those described in the above Working Examples, and Compound Nos. in the Tables indicate those in Table 1 - Table 59 listed as the above preferred examples.

**Table 70**

| Compound No. | Work. Ex. No. | δ (ppm) |
|---|---|---|
| 161 | 7 | 2.23(s, 3H), 3.15(m, 2H), 3.74(m, 2H), 3.81(m, 2H), 4.91(m, 2H), 7.05(d, 8.6Hz, 2H), 7.35(d, 8.5Hz, 2H), 7.97(s, 1H), 8.52(s, 1H), 12.38(br, 1H) |
| 165 | 70 | 3.16(m, 2H), 3.70(s, 3H), 3.74(m, 2H), 3.80(m, 2H), 4.91(m, 2H), 6.83(d, 9.0Hz, 2H), 7.35(d, 8.8Hz, 2H), 7.97(s, 1H), 8.47(s, 1H), 12.38(br, 1H) |
| 236 | 8 | 3.20(m, 2H), 3.25-3.43(m, 4H), 3.81(s, 3H), 4.90(m, 2H), 7.10(d, 9.0Hz, 2H), 7.70(d, 9.0Hz, 2H), 7.93(d, 3.7Hz, 1H), 12.38(br, 1H) |
| 280 | 11 | 3.32(m, 2H), 3.89(m, 4H), 5.70(m, 2H), 8.17(s, 1H), 13.78(s, 1H) |
| 351 | 126 | 3.25(m, 2H), 3.81(m, 4H), 5.61(m, 2H), 5.95(t, 7.4Hz, 1H), 7.24(t, 7.3Hz, 2H), 7.46(d, 7.6Hz, 2H), 8.15(d, 3.7Hz, 1H), 8.66(s, 1H), 13.74(br, 1H) |
| 355 | 129 | 2.25(s, 3H), 3.25(m, 2H), 3.79(m, 4H), 5.59(m, 2H), 6.77(d, 7.6Hz, 1H), 7.12(t, 7.7Hz, 1H), 7.27(m, 2H), 8.15(d, 3.4Hz, 1H), 8.58(s, 1H), 13.74(br, 1H) |
| 356 | 16 | 2.22(s, 3H), 3.23(m, 2H), 3.79(m, 4H), 5.59(m, 2H), 7.05(d, 8.6Hz, 2H),7.33(d, 8.6Hz, 2H), 8.14(d, 3.6Hz, 1H), 8.56(s, 1H), 13.73(br, 1H) |
| 413 | 13 | 2.91(br, 4H), 3.14(m, 2H), 5.48(br, 2H), 7.04(br, 1H), 8.12(s, 1H) |
| 792 | 18 | 3.27(m, 2H), 3.81(br, 4H), 5.62(m, 2H), 7.36(t, 7.8Hz, 1H), 7.53(m, 1H), 7.78(m, 1H), 8.09(s, 1H), 8.15(s, 1H), 8.87(s, 1H), 12.89(br, 1H), 13.74(br, 1H) |
| 796 | 202 | 3.26(m, 2H), 3.82(br, 4H), 5.63(m, 2H), 7.60(m, 2H), 7.83(m, 2H), 8.15(m, 1H), 9.00(s, 1H), 13.77(m, 1H) |
| 811 | 203 | 3.33(m, 2H), 3.84(m, 4H), 5.68(m, 2H), 7.04(t, 7.6Hz, 1H), 7.54(m, 1H), 7.96(d, 8.1Hz, 1H), 8.16(s, 1H), 8.34(d, 8.6Hz, 1H), 11.09(s, 1H), 13.75(br, 1H) |
| 847 | 19 | 3.26(br, 2H), 3.81(br, 4H), 5.62(br, 2H), 7.30(m, 2H), 7.68(m, 1H), 7.88(s, 1H), 8.15(m, 1H), 8.85(s, 1H), 11.14(s, 1H), 13.76(s, 1H) |

**Table 71**

| Compound No. | Work. Ex. No. | δ (ppm) |
|---|---|---|
| 1088 | 218 | 3.35(m, 2H), 4.28(m, 4H), 5.68(m, 2H), 7.43(t, 7.8Hz, 1H), 7.58(m, 1H), 7.71(m, 1H), 7.88(s, 1H), 8.17(d, 3.4Hz, 1H), 9.63(s, 1H), 12.98(br, 1H), 13.77(s, 1H) |
| 1090 | 219 | 3.33(m, 2H), 4.27(m, 4H), 5.67(br, 2H), 7.42(m, 2H), 7.86(m, 2H), 8.16(s, 1H), 9.69(s, 1H), 12.79(br, 1H), 13.77(br, 1H) |
| 1096 | 222 | 3.35(m, 2H), 4.33(br, 4H), 5.72(br, 2H), 7.21(t, 7.6Hz, 1H), 7.55(t, 7.7Hz, 1H), 7.91(d, 7.8Hz, 1H), 8.08(d, 8.3Hz, 1H), 8.17(s, 1H), 10.71(br, 1H), 13.45(br, 1H), 13.78(s, 1H) |
| 1501 | 22 | 1.57(br, 3H), 3.07-4.33(m, 9H), 7.45(m, 5H), 8.19(s, 1H), 13.85(br, 1H) |

### [Working Example 363]

### Determination of inhibition of GSK-3 enzyme activity

To five µl of a test compound in 5% DMSO as a solvent, 25 µl of phospho-glycogen synthase peptide-2 substrate solution [6 µm phospho-glycogen synthase peptide-2, 20 µm ATP, 16 mM MOPS buffer, pH 7.0, 0.2 mM EDTA, 20 mM magnesium acetate, 0.1 µl [γ-³³P]ATP (specific activity: about 110 TBq/mmol)] was added, and 20 µl of a GSK-3β enzyme solution [10 mU recombinant human GSK-3β, 20 mM MOPS buffer, pH 7.0, 1 mM EDTA, 0.1% polyoxyethylenelauryl ether (23 Lauryl Ether; Brij 35), 5% glycerol, 0.1% β-mercaptoethanol] was further added to initiate the reaction. After reacting at room temperature for 20 minutes, an equal amount of 200 mM phosphate solution was added to stop the reaction. 90 µl of the reaction product was allowed to adsorb to the MultiScreen PH plate (Millipore), and washed with 100 mM phosphate solution. After drying said plate, 30 µl of MicroScint-O (Packard Bioscience) was added, and cpm was measured by a scintilation counter to examine the inhibition activity. As used herein, Phospho GS Peptide 2 means Tyr-Arg-Arg-Ala-Ala-Val-Pro-Pro-Ser-Pro-Ser-Leu-Ser-Arg-His-Ser-Ser-Pro-His-Gln-Ser(P)-Glu-Asp-Glu-Glu-Glu (SEQ ID NO: 1).

After determining the GSK-3 enzyme-inhibiting activity (IC₅₀ value) of the compounds of the present invention, the inhibiting activity of IC₅₀ < 50 nM was noted in Compound Nos. 692(R), 731, 732, 735, 736, 792, 796, 2164. The inhibiting activity of 50 nM ≤ IC₅₀ < 100 nM was noted in Compound Nos. 692(±), 696(R), 734, 836, 1088, 1090, 1179, 1203, 1290, and 1295. Also, the inhibiting activity of 100 nM ≤ IC₅₀ < 1 µM was noted in Compound Nos. 137, 280, 282, 283, 297, 330, 331, 332, 333, 335, 336, 337, 340, 341, 342, 344, 351, 353, 354, 355, 356, 359, 360, 361, 365, 366, 368, 374, 375, 391, 405, 413, 414, 415, 420, 421, 422, 423, 424, 425, 426, 427, 428, 668, 672, 676, 678, 680, 682, 686, 688, 696(S), 747, 752, 753, 755, 756, 758, 759, 760, 763, 764, 765, 769, 771, 772, 793(S), 811, 837, 838, 847, 1069, 1072, 1074, 1076, 1078, 1080, 1082, 1084, 1096, 1098, 1100, 1104, 1114, 1141, 1161, 1166, 1172, 1174, 1176, 1180, 1181, 1183, 1184, 1192, 1193, 1194, 1196, 1198, 1200, 1201, 1202, 1204, 1205, 1214, 1222, 1223, 1224, 1225, 1226, 1227, 1228, 1229, 1230, 1231, 1232, 1235, 1236, 1237, 1238, 1239, 1241, 1242, 1243, 1244, 1245, 1247, 1249, 1250, 1251, 1253, 1255, 1256, 1257, 1258, 1259, 1260, 1261, 1262, 1263, 1264, 1265, 1266, 1267, 1268, 1269, 1270, 1271, 1272, 1273, 1274, 1275, 1277, 1278, 1284, 1285, 1286, 1287, 1289, 1291, 1291, 1292, 1293, 1294, 1296, 1297, 1299, 1301, 1302, 1303, 1308, 1309, 1310, 1312, 1313, 1315, 1316, 1318, 1319, 1322, 2240, 2241, 2242, 1501. Also, the inhibiting activity of 1 µM ≤ IC₅₀ < 10 µM was noted in Compound Nos. 47, 50, 114, 119, 135, 136, 141, 143, 144, 147, 150, 155, 158, 160, 161, 164, 165, 167, 172, 173, 176, 177, 179, 180, 182, 183, 188, 189, 191, 193, 196, 198, 199, 200, 334, 347, 363, 410, 432, 433, 521, 674, 684, 739, 740, 741, 749, 750, 770, 773, 791, 1067, 1087, 1092, 1094, 1102, 1106, 1108, 1110, 1112, 1116, 1118, 1163, 1177, 1178, 1182, 1234, 1240, 1252, 1276, 1279, 1280, 1281, 1282, 1283, 1288, 1298, 1300, 1304, 1306, 1311, 1317, 1799. Compound Nos. in the Tables indicate those in Table 1 - Table 59 listed as the above preferred examples.

As described above, the pyrrolopyrimidine derivatives of the present invention exhibit potent GSK-3-inhibiting activity. Thus, it is clear now that they are clinically applicable as GSK-3 activity-inhibiting substance for use in the prevention and/or treatment of various diseases in which GSK-3 is involved.

### [Working Example 364]

### Preparation of tablets

Tablets were prepared with one tablet having the following composition:

| | |
|---|---|
| Compound (Working Example 1) | 50 mg |
| Lactose | 230 mg |
| Potato starch | 80 mg |
| Polyvinyl pyrrolidone | 11 mg |
| Magnesium stearate | 5 mg |

The compound (the compound of Working Example 1) of the present invention, lactose, and potato starch were mixed, which were evenly swelled in a 20% polyvinyl pyrrolidone in ethanol, sieved through a 20 nm mesh, dried at 45°C, and sieved through a 15 nm mesh again. Granules thus obtained were blended with magnesium stearate and compressed to tablets.

### INDUSTRIAL APPLICABILITY

The pyrrolo[3,2-d]pyrimidine derivatives of the present invention and pharmaceutically acceptable salts thereof exhibit excellent activity of inhibiting GSK-3. Thus, it was revealed that they are fully clinically applicable as GSK-3 activity-inhibiting substance for use in the prevention and/or treatment of various diseases in which GSK-3 is involved.

## Claims

1. A pyrrolo[3,2-d]pyrimidine derivative represented by Formula (I) or a pharmaceutically acceptable salt thereof 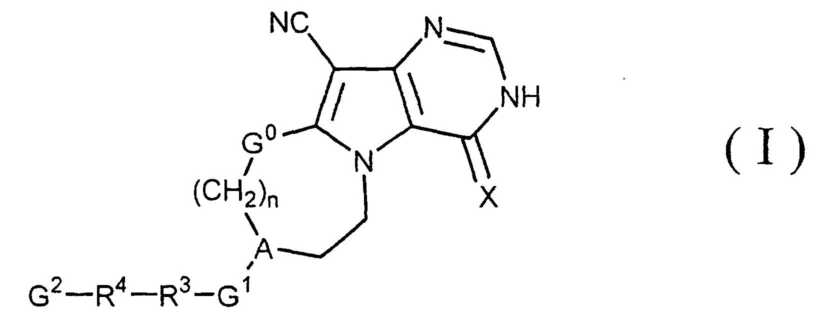 [In Formula (I), X represents an oxygen atom or a sulfur atom.
In Formula (I), n represents 0, 1, or 2.
In Formula (I), A represents a nitrogen atom or CH.
In Formula (I), G⁰ represents a divalent group of substituted or unsubstituted benzene, furan, thiophene, pyrrole, isoxazole, cyclopentane or cyclohexane, or a divalent group represented by -CR¹R²- (R¹ and R², which may be the same or different, represent a hydrogen atom, a substituted or unsubstituted aliphatic hydrocarbon group having one to four carbons, or NR¹⁰R²⁰ (R¹⁰ and R²⁰, which may be the same or different, represent a hydrogen atom, a substituted or unsubstituted aliphatic hydrocarbon group having one to four carbons), or an optionally substituted group in which R¹ and R² bind to each other and form a 3- to 7-membered ring together with a carbon atom (C in -CR¹R²-) to which R¹ and R² are bound, provided that R¹ and R² are not NR¹⁰R²⁰ at the same time).
In Formula (I), G¹ represents a binding hand which is a single bond, or a group that binds A to which G¹ binds and R³ in the form of A-C(=O)-O-R³, A-C(=O)-R³, A-C(=O)-NR³⁰-R³, A-C(=S)-NR³¹-R³, A-C(=O)-NR³²-S(=O)₂-R³, or A-S(=O)₂-R³ (R³⁰ to R³² represent, independently from one another, a hydrogen atom or a substituted or unsubstituted aliphatic hydrocarbon group having one to four carbons).
In Formula (I), R³ represents a group selected from the following 1)-5).
1) a single bond,
2) a substituted or unsubstituted alicyclic hydrocarbon group having three to eight carbons (substituents are one or more substituents selected from the group consisting of a fluorine atom, a chlorine atom, a bromine atom, an iodine atom, a hydroxy group, an optionally substituted alkoxy group having one to seven carbons, an aryloxy group having six to ten carbons, an aralkoxy group having seven to nine carbons, an acyloxy group having two to seven carbons, an oxo group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, and an optionally substituted aliphatic hydrocarbon group having one to six carbons),
3) a substituted or unsubstituted aromatic hydrocarbon group having six to 14 carbons (substituents are one or more substituents selected from the group consisting of a fluorine atom, a chlorine atom, a bromine atom, an iodine atom, a hydroxy group, an optionally substituted alkoxy group having one to seven carbons, an aryloxy group having six to ten carbons, an aralkoxy group having seven to nine carbons, an acyloxy group having two to seven carbons, an oxo group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, and an optionally substituted aliphatic hydrocarbon group having one to six carbons),
4) a substituted or unsubstituted heterocyclic group containing, in the ring, one to four atoms selected from the group consisting of an oxygen atom, a nitrogen atom, and a sulfur atom (substituents are one or more substituents selected from the group consisting of a fluorine atom, a chlorine atom, a bromine atom, an iodine atom, a hydroxy group, an optionally substituted alkoxy group having one to seven carbons, an aryloxy group having six to ten carbons, an aralkoxy group having seven to nine carbons, an acyloxy group having two to seven carbons, an oxo group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, and an optionally substituted aliphatic hydrocarbon group having one to six carbons),
5) a substituted or unsubstituted aliphatic hydrocarbon group having one to ten carbons (substituents are one or more substituents selected from the group consisting of a fluorine atom, a chlorine atom, a bromine atom, an iodine atom, a hydroxy group, an optionally substituted alkoxy group having one to seven carbons, an optionally substituted phenylalkoxy group having seven to ten carbons, an alkoxy group having one to four carbons substituted with an optionally substituted heterocyclic group (containing, in the ring, one to four atoms selected from the group consisting of an oxygen atom, a nitrogen atom, and a sulfur atom), an aryloxy group having six to ten carbons, an acyloxy group having two to seven carbons, an oxo group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, an optionally substituted aromatic hydrocarbon group having six to 14 carbons, and an optionally substituted heterocyclic group (containing, in the ring, one to four atoms selected from the group consisting of an oxygen atom, a nitrogen atom, and a sulfur atom)).
In Formula (I), R⁴ represents a group selected from the following 1)-4).
1) a single bond,
2) a substituted or unsubstituted alicyclic hydrocarbon group having three to eight carbons (substituents are one or more substituents selected from the group consisting of a fluorine atom, a chlorine atom, a bromine atom, an iodine atom, a hydroxy group, an optionally substituted alkoxy group having one to seven carbons, an aryloxy group having six to ten carbons, an aralkoxy group having seven to nine carbons, an acyloxy group having two to seven carbons, an oxo group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, and an optionally substituted aliphatic hydrocarbon group having one to six carbons),
3) a substituted or unsubstituted aromatic hydrocarbon group having six to 14 carbons (substituents are one or more substituents selected from the group consisting of a fluorine atom, a chlorine atom, a bromine atom, an iodine atom, a hydroxy group, an optionally substituted alkoxy group having one to seven carbons, an aryloxy group having six to ten carbons, an aralkoxy group having seven to nine carbons, an acyloxy group having two to seven carbons, an oxo group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, and an optionally substituted aliphatic hydrocarbon group having one to six carbons),
4) a substituted or unsubstituted heterocyclic group containing, in the ring, one to four atoms selected from the group consisting of an oxygen atom, a nitrogen atom, and a sulfur atom (substituents are one or more substituents selected from the group consisting of a fluorine atom, a chlorine atom, a bromine atom, an iodine atom, a hydroxy group, an optionally substituted alkoxy group having one to seven carbons, an aryloxy group having six to ten carbons, an aralkoxy group having seven to nine carbons, an acyloxy group having two to seven carbons, an oxo group, an alkylsulfonyloxy group having one to six carbons, an optionally substituted acyl group having two to seven carbons, a carboxyl group, an alkoxycarbonyl group having two to seven carbons, a carbamoyl group, an optionally substituted alkylcarbamoyl group having two to seven carbons, an amino group, an optionally substituted alkylamino group having one to six carbons, an optionally substituted acylamino group having two to seven carbons, an alkoxycarbonylamino group having two to eight carbons, an alkylsulfonylamino group having one to six carbons, a cyano group, a nitro group, an alkylthio group having one to six carbons, an alkylsulfinyl group having one to six carbons, an alkylsulfonyl group having one to six carbons, a sulfamoyl group, an alkylaminosulfonyl group having one to six carbons, a sulpho group, an optionally substituted alicyclic hydrocarbon group having three to six carbons, and an optionally substituted aliphatic hydrocarbon group having one to six carbons).
In Formula (I), G² represents a hydrogen atom, -C(=O)-OH, -C(=O)-NH-OH, -S(=O)₂-OH, or a 5-tetrazolyl group].

2. A pyrrolo[3,2-d]pyrimidine derivative according to claim 1 or a pharmaceutically acceptable salt thereof, wherein A represents a nitrogen atom.

3. A pyrrolo[3,2-d]pyrimidine derivative according to claim 2 or a pharmaceutically acceptable salt thereof, wherein G⁰ is a divalent group represented by -CR¹R²- (R¹ and R² are as defined above).

4. A pyrrolo[3,2-d]pyrimidine derivative according to claim 2 or a pharmaceutically acceptable salt thereof, wherein G⁰ is a divalent group represented by -CR¹R²- wherein R¹ and R², which may be the same or different, are a hydrogen atom or an optionally substituted aliphatic hydrocarbon group having one to four carbons, or R¹ and R² bind to each other and form a cyclopropane ring together with a carbon atom to which R¹ and R² are bound.

5. A pyrrolo[3,2-d]pyrimidine derivative according to claim 2 or a pharmaceutically acceptable salt thereof, wherein G⁰ is a divalent group represented by -CR¹R²- wherein R¹ and R², which may be the same or different, are a hydrogen atom or a methyl group, or R¹ and R² bind to each other and form a cyclopropane ring together with a carbon atom to which R¹ and R² are bound.

6. A pyrrolo[3,2-d]pyrimidine derivative according to claim 2 or a pharmaceutically acceptable salt thereof, wherein G⁰ is a divalent group represented by -CR¹R²- wherein R¹ is an optionally substituted aliphatic hydrocarbon group having one to four carbons and R² is a hydrogen atom.

7. A pyrrolo[3,2-d]pyrimidine derivative according to claim 2 or a pharmaceutically acceptable salt thereof, wherein G⁰ is a divalent group represented by -CR¹R²- wherein R¹ is a methyl group and R² is a hydrogen atom.

8. A pyrrolo[3,2-d]pyrimidine derivative according to claim 2 or a pharmaceutically acceptable salt thereof, wherein G⁰ is a divalent group represented by -CR¹R²- wherein each of R¹ and R² is a methyl group, or R¹ and R² bind to each other and form a cyclopropane ring together with a carbon atom to which R¹ and R² are bound.

9. A pyrrolo[3,2-d]pyrimidine derivative according to claim 2 or a pharmaceutically acceptable salt thereof, wherein G⁰ is a divalent group of an optionally substituted benzene, furan, thiophene, pyrrole, isoxazole, cyclopentane or cyclohexane, and G⁰, (CH₂)ₙ, A, -(CH₂)₂-, and a nitrogen atom and a carbon atom in the pyrrole ring of the pyrrolopyrimidine ring form a 10- to 12-membered bicyclic structure.

10. A pyrrolo[3,2-d]pyrimidine derivative according to claim 2 or a pharmaceutically acceptable salt thereof, wherein G⁰ is a divalent group of an optionally substituted benzene, and G⁰, (CH₂)ₙ, A, -(CH₂)₂-, and a nitrogen atom and a carbon atom in the pyrrole ring of the pyrrolopyrimidine ring form a 10- to 12-membered bicyclic structure.

11. A pyrrolo[3,2-d]pyrimidine derivative according to claim 2 or a pharmaceutically acceptable salt thereof, wherein G⁰ is a divalent group of benzene, furan, thiophene, pyrrole, isoxazole, cyclopentane or cyclohexane, and G⁰, (CH₂)ₙ, A, -(CH₂)₂-, and a nitrogen atom and a carbon atom in the pyrrole ring of the pyrrolopyrimidine ring form a 10- to 12-membered bicyclic structure, and said bicyclic structure has 3-5 substituents.

12. A pyrrolo[3,2-d]pyrimidine derivative according to claim 2 or a pharmaceutically acceptable salt thereof, wherein G⁰ is a divalent group of an optionally substituted isoxazole, and G⁰, (CH₂)ₙ, A, -(CH₂)₂-, and a nitrogen atom and a carbon atom in the pyrrole ring of the pyrrolopyrimidine ring form a 10- to 12-membered bicyclic structure.

13. A pyrrolo[3,2-d]pyrimidine derivative according to any one of claims 2 to 12 or a pharmaceutically acceptable salt thereof, wherein R³ is a divalent group of an optionally substituted, saturated aliphatic hydrocarbon group having five to ten carbons, an optionally substituted alicyclic hydrocarbon group having five to eight carbons, an optionally substituted aromatic hydrocarbon group having six to ten carbons, or an optionally substituted heterocyclic group (containing one to four atoms selected from the group consisting of an oxygen atom, a nitrogen atom, and a sulfur atom).

14. A pyrrolo[3,2-d]pyrimidine derivative according to any one of claims 2 to 12 or a pharmaceutically acceptable salt thereof, wherein R³ is a divalent group of an optionally substituted heterocyclic group (containing, in the ring, one to four atoms selected from the group consisting of an oxygen atom, a nitrogen atom, and a sulfur atom).

15. A pyrrolo[3,2-d]pyrimidine derivative according to any one of claims 2 to 12 or a pharmaceutically acceptable salt thereof, wherein A-G¹-R³ represents a group that binds in the form of A-C(=O)-NH-R³, A-C(=S)-NH-R³, or A-C(=O)-NH-S(=O)₂-R³, and R³ is a divalent group of an optionally substituted aliphatic hydrocarbon group having one to ten carbons, an optionally substituted alicyclic hydrocarbon group having three to eight carbons, an optionally substituted aromatic hydrocarbon group having six to ten carbons, or an optionally substituted heterocyclic group (containing, in the ring, one to four atoms selected from the group consisting of an oxygen atom, a nitrogen atom, and a sulfur atom).

16. A pyrrolo[3,2-d]pyrimidine derivative according to any one of claims 2 to 12 or a pharmaceutically acceptable salt thereof, wherein A-G¹-R³ represents a group that binds in the form of A-C(=O)-NH-R³ or A-C(=S)-NH-R³, and R³ is a divalent group of an optionally substituted aliphatic hydrocarbon group having one to ten carbons, an optionally substituted alicyclic hydrocarbon group having three to eight carbons, an optionally substituted aromatic hydrocarbon group having six to ten carbons, or an optionally substituted heterocyclic group (containing, in the ring, one to four atoms selected from the group consisting of an oxygen atom, a nitrogen atom, and a sulfur atom).

17. A pyrrolo[3,2-d]pyrimidine derivative according to any one of claims 2 to 12 or a pharmaceutically acceptable salt thereof, wherein A-G¹-R³ represents a group that binds in the form of A-C(=O)-NH-R³, and R³ is a divalent group of an optionally substituted aliphatic hydrocarbon group having one to ten carbons, an optionally substituted alicyclic hydrocarbon group having three to eight carbons, an optionally substituted aromatic hydrocarbon group having six to ten carbons, or an optionally substituted heterocyclic group (containing, in the ring, one to four atoms selected from the group consisting of an oxygen atom, a nitrogen atom, and a sulfur atom).

18. A pyrrolo[3,2-d]pyrimidine derivative according to any one of claims 2 to 12 or a pharmaceutically acceptable salt thereof, wherein A-G¹-R³ represents a group that binds in the form of A-C(=O)-NH-R³, and R³ is a divalent group of an optionally substituted alkane having five to ten carbons, an optionally substituted alicyclic hydrocarbon group having five to eight carbons, an optionally substituted aromatic hydrocarbon group having six to ten carbons, or an optionally substituted heterocyclic group (containing, in the ring, one to four atoms selected from the group consisting of an oxygen atom, a nitrogen atom, and a sulfur atom).

19. A pyrrolo[3,2-d]pyrimidine derivative according to any one of claims 2 to 12 or a pharmaceutically acceptable salt thereof, wherein A-G¹-R³ represents a group that binds in the form of A-C(=O)-NH-R³, and R³ is a divalent group of an optionally substituted heterocyclic group (containing, in the ring, one to four atoms selected from the group consisting of an oxygen atom, a nitrogen atom, and a sulfur atom).

20. A pyrrolo[3,2-d]pyrimidine derivative according to any one of claims 2 to 19 or a pharmaceutically acceptable salt thereof, wherein A-G¹-R³ represents a group that binds in the form of A-C(=O)-R³, A-C(=O)-NH-R³, or A-C(=S)-NH-R³, and G² represents any of -C(=O)-OH, -C(=O)-NH-OH, -S(=O)₂-OH, and 5-tetrazolyl group.

21. A pyrrolo[3,2-d]pyrimidine derivative according to any one of claims 2 to 19 or a pharmaceutically acceptable salt thereof, wherein A-G¹-R³ represents a group that binds in the form of A-C(=O)-R³, A-C(=O)-NH-R³, or A-C(=S)-NH-R³, and G² represents -C(=O)-OH.

22. A pyrrolo[3,2-d]pyrimidine derivative according to any one of claims 2 to 19 or a pharmaceutically acceptable salt thereof, wherein A-G¹-R³ represents a group that binds in the form of A-C(=O)-NH-R³, and G² represents any of -C(=O)-OH, -C(=O)-NH-OH, -S(=O)₂-OH, and 5-tetrazolyl group.

23. A pyrrolo[3,2-d]pyrimidine derivative according to any one of claims 2 to 19 or a pharmaceutically acceptable salt thereof, wherein A-G¹-R³ represents a group that binds in the form of A-C(=O)-NH-R³, and G² represents -C(=O)-OH.

24. A pyrrolo[3,2-d]pyrimidine derivative according to any one of claims 2 to 12 or a pharmaceutically acceptable salt thereof, wherein -G¹- represents a single bond, and R³ is a divalent group of an alkane having two to six carbons substituted with an optionally substituted alkoxy group having one to four carbons, an optionally substituted phenylalkoxy group having seven to ten carbons, or an optionally substituted aryloxy group having six to ten carbons.

25. A pyrrolo[3,2-d]pyrimidine derivative according to any one of claims 2 to 12 or a pharmaceutically acceptable salt thereof, wherein -G¹- represents a single bond, and R³ is a divalent group of an alkane having two to four carbons substituted with an optionally substituted alkoxy group having one to four carbons.

26. A pyrrolo[3,2-d]pyrimidine derivative according to any one of claims 2 to 12 or a pharmaceutically acceptable salt thereof, wherein -G¹- represents a single bond, and R³ is a divalent group of an alkane having two to four carbons substituted with a phenylalkoxy group having seven to ten carbons.

27. A pyrrolo[3,2-d]pyrimidine derivative according to any one of claims 2 to 12 or a pharmaceutically acceptable salt thereof, wherein -G¹- represents a single bond, and R³ is a divalent group of an alkane having two to four carbons substituted with an alkoxy group having one to four carbons substituted with an optionally substituted heterocyclic group (containing, in the ring, one to four atoms selected from the group consisting of an oxygen atom, a nitrogen atom, and a sulfur atom).

28. A pyrrolo[3,2-d]pyrimidine derivative according to any one of claims 2 to 12 or a pharmaceutically acceptable salt thereof, wherein -G¹- represents a single bond, and R³ is a divalent group of an alkane having two to four carbons substituted with an optionally substituted phenoxy group.

29. A pyrrolo[3,2-d]pyrimidine derivative according to any one of claims 2 to 12 or a pharmaceutically acceptable salt thereof, wherein -G¹- represents a single bond, and R³ is a divalent group of an alkane having two to four carbons substituted with an optionally substituted benzyloxy group.

30. A pyrrolo[3,2-d]pyrimidine derivative according to any one of claims 2 to 12 or a pharmaceutically acceptable salt thereof, wherein -G¹- represents a single bond, and R³ represents -CH2-, and R⁴ is a divalent group of an aromatic hydrocarbon group having six to ten carbons said group having G² other than a hydrogen atom or a substituent at a carbon atom of R⁴ at a position adjacent to the carbon atom of R⁴ at which -R³- binds, or a heterocyclic group (containing, in the ring, one to four atoms selected from the group consisting of an oxygen atom, a nitrogen atom, and a sulfur atom) having G² other than a hydrogen atom or a substituent at an atom at a position adjacent to the carbon atom of R⁴ at which -R³- binds.

31. A pyrrolo[3,2-d]pyrimidine derivative according to any one of claims 2 to 30 or a pharmaceutically acceptable salt thereof, wherein X is an oxygen atom.

32. A pyrrolo[3,2-d]pyrimidine derivative according to any one of claims 2 to 30 or a pharmaceutically acceptable salt thereof, wherein X is a sulfur atom.

33. A pyrrolo[3,2-d]pyrimidine derivative according to any one of claims 2 to 30 or a pharmaceutically acceptable salt thereof, wherein G⁰ is a divalent group represented by -CR¹R²-, wherein R¹ and R², which may be the same or different, are a hydrogen atom or a methyl group, n represents 1, and X is a sulfur atom.

34. A pyrrolo[3,2-d]pyrimidine derivative according to claim 1 or a pharmaceutically acceptable salt thereof, wherein A represents CH.

35. A pyrrolo[3,2-d]pyrimidine derivative according to claim 34 or a pharmaceutically acceptable salt thereof, wherein G⁰ is a divalent group represented by -CR¹R²-, wherein R¹ and R², which may be the same or different, are a hydrogen atom or a substituted or unsubstituted aliphatic hydrocarbon group having one to four carbons, or R¹ and R² bind to each other and form a cyclopropane ring together with a carbon atom to which R¹ and R² are bound.

36. A pyrrolo[3,2-d]pyrimidine derivative according to claim 34 or a pharmaceutically acceptable salt thereof, wherein G⁰ is a divalent group represented by -CR¹R²-, wherein R¹ and R², which may be the same or different, are a hydrogen atom or a methyl group, or R¹ and R² bind to each other and form a cyclopropane ring together with a carbon atom to which R¹ and R² are bound.

37. A pyrrolo[3,2-d]pyrimidine derivative according to claim 34 or a pharmaceutically acceptable salt thereof, wherein G⁰ is a divalent group represented by -CR¹R²-, wherein R¹ is a substituted or unsubstituted aliphatic hydrocarbon group having one to four carbons and R² is a hydrogen atom.

38. A pyrrolo[3,2-d]pyrimidine derivative according to claim 34 or a pharmaceutically acceptable salt thereof, wherein G⁰ is a divalent group represented by -CR¹R²-, wherein R¹ is a methyl group and R² is a hydrogen atom.

39. A pyrrolo[3,2-d]pyrimidine derivative according to claim 34 or a pharmaceutically acceptable salt thereof, wherein G⁰ is a divalent group represented by -CR¹R²-, wherein both of R¹ and R² are a methyl group, or R¹ and R² bind to each other and form a cyclopropane ring together with a carbon atom to which R1 and R² are bound.

40. A pyrrolo[3,2-d]pyrimidine derivative according to claim 34 or a pharmaceutically acceptable salt thereof, wherein G⁰ represents a divalent group of an optionally substituted benzene, furan, thiophene, pyrrole, isoxazole, cyclopentane or cyclohexane, and G⁰, (CH₂)ₙ, A, -(CH₂)₂-, and a nitrogen atom and a carbon atom in the pyrrole ring of the pyrrolopyrimidine ring form a 10- to 12-membered bicyclic structure.

41. A pyrrolo[3,2-d]pyrimidine derivative according to claim 34 or a pharmaceutically acceptable salt thereof, wherein G⁰ represents a divalent group of optionally substituted benzene, and G⁰, (CH₂)ₙ, A, -(CH₂)₂-, and a nitrogen atom and a carbon atom in the pyrrole ring of the pyrrolopyrimidine ring form a 10- to 12-membered bicyclic structure.

42. A pyrrolo[3,2-d]pyrimidine derivative according to claim 34 or a pharmaceutically acceptable salt thereof, wherein G⁰ represents a divalent group of a substituted benzene, furan, thiophene, pyrrole, isoxazole, cyclopentane or cyclohexane, and G⁰, (CH₂)ₙ, A, - (CH₂)₂-, and a nitrogen atom and a carbon atom in the pyrrole ring of the pyrrolopyrimidine ring form a 10- to 12-membered bicyclic structure and said bicyclic structure has 3-5 substituents.

43. A pyrrolo[3,2-d]pyrimidine derivative according to claim 34 or a pharmaceutically acceptable salt thereof, wherein G⁰ represents a divalent group of an optionally substituted isoxazole, and G⁰, (CH₂)ₙ, A, -(CH₂)₂-, and a nitrogen atom and a carbon atom in the pyrrole ring of the pyrrolopyrimidine ring form a 10- to 12-membered bicyclic structure.

44. A GSK-3 inhibitor comprising a pyrrolo[3,2-d]pyrimidine derivative according to any one of claims 1 to 43 or a pharmaceutically acceptable salt thereof.

45. A pharmaceutical composition comprising a pyrrolo[3,2-d]pyrimidine derivative according to any one of claims 1 to 43 or a pharmaceutically acceptable salt thereof and a pharmaceutically acceptable carrier.

46. A therapeutic or preventive agent for a disease in which GSK-3 is involved, said agent comprising as an active ingredient a pyrrolo[3,2-d]pyrimidine derivative according to any one of claims 1 to 43 or a pharmaceutically acceptable salt thereof.

47. A therapeutic or preventive agent according to claim 46 wherein a disease in which GSK-3 is involved is one selected from the group consisting of diabetes, diabetic complications, Alzheimer's disease, neurodegenerative diseases, manic-depressive psychosis, traumatic encephalopathy, alopecia, inflammatory diseases, cancer, and immune deficiency.

48. A pyrrolo[3,2-d]pyrimidine derivative represented by Formula (II) 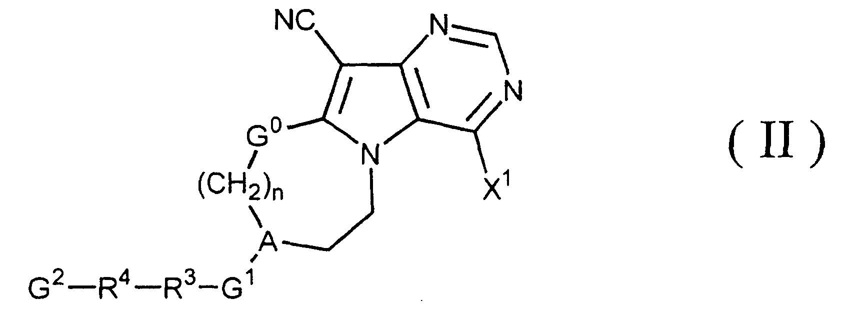 [In Formula (II), n, A, R³, R⁴, G⁰, G¹, and G² are as defined for Formula (I). X¹ represents a chlorine atom, a bromine atom, an iodine atom, or an alkyl or arylsulfonyl group having one to eight carbons that may be substituted with a fluorine atom, a chlorine atom, or a bromine atom.]

49. A pyrrolo[3,2-d]pyrimidine derivative according to claim 48 wherein X¹ is a chlorine atom or a trifluoromethylsulfonyloxy group.
